(19) Europäisches Patentamt
European Patent Office
Office européen des brevets

(11) EP 4 023 250 A1

(12) **EUROPEAN PATENT APPLICATION**

(43) Date of publication:
**06.07.2022 Bulletin 2022/27**

(21) Application number: **21150122.6**

(22) Date of filing: **04.01.2021**

(51) International Patent Classification (IPC):
***A61K 51/04** (2006.01)* ***A61P 35/00** (2006.01)*
***A61K 103/00** (2006.01)* ***A61K 103/30** (2006.01)*

(52) Cooperative Patent Classification (CPC):
**A61K 51/0402; A61K 51/0497; A61P 35/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Technische Universität München**
**80333 München (DE)**

(72) Inventors:
- **Felber, Veronika Barbara**
**D-84048 Mainburg (DE)**
- **Valentin, Manuel Amando**
**D-90419 Nürnberg (DE)**
- **Wester, Hans-Jürgen**
**D-85301 Schweitenkirchen (DE)**

(74) Representative: **Stratagem IPM Limited**
**Meridian Court**
**Comberton Road**
**Toft, Cambridge CB23 2RY (GB)**

# (54) DUAL MODE RADIOTRACER AND -THERAPEUTICS

(57) The present disclosure relates to compounds of Formula (1a), (1b), (1c) or (1d):

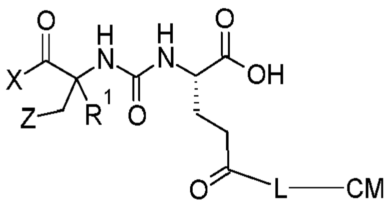
(1a);

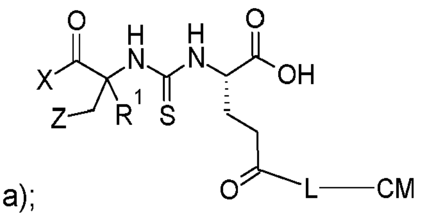
(1b);

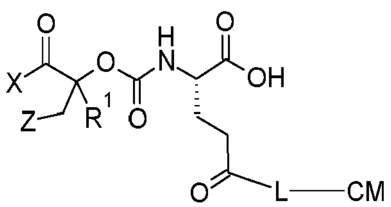
(1c);

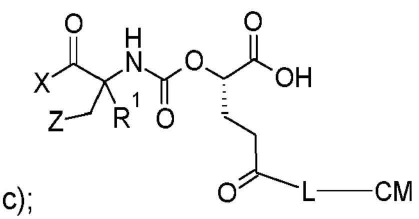
(1d);

and pharmaceutically acceptable salts thereof, wherein X, Z, L, CM and $R^1$ are defined herein, and their use as cancer diagnostic or imaging agents.

EP 4 023 250 A1

## Description

The present invention relates to compounds that bind to prostate-specific membrane antigen (PSMA) comprising a PSMA binding moiety, a linker group comprising a silicon-fluoride acceptor (SIFA) moiety and a chelator moiety, optionally containing a chelated nonradioactive or radioactive cation, wherein the SIFA moiety comprises a covalent bond between a silicon and a fluorine atom which can be $^{18}F$.

## BACKGROUND OF THE INVENTION

### *Prostate cancer*

Prostate Cancer (PCa) remained over the last decades the most common malignant disease in men with high incidence for poor survival rates. Due to its overexpression in prostate cancer (Silver et al., Clinical Cancer Research 3, 81-85 (1997)), prostate-specific membrane antigen (PSMA) or glutamate carboxypeptidase II (GCP II) proved its eligibility as excellent target for the development of highly sensitive radiolabelled agents for endoradiotherapy and imaging of PCa (Afshar-Oromieh et al., European journal of nuclear medicine and molecular imaging 42, 197-209 (2015); Benešová et al., Journal of Nuclear Medicine 56, 914-920 (2015); Robu et al., Journal of Nuclear Medicine, jnumed. 116.178939 (2016); Weineisen et al.; Journal of Nuclear Medicine 55, 1083-1083 (2014); Rowe et al., Prostate cancer and prostatic diseases (2016); Maurer et al., Nature Reviews Urology (2016)). Prostate-specific membrane antigen is an extracellular hydrolase whose catalytic center comprises two zinc(II) ions with a bridging hydroxido ligand. It is highly upregulated in metastatic and hormone-refractory prostate carcinomas, but its physiologic expression has also been reported in kidneys, salivary glands, small intestine, brain and, to a low extent, also in healthy prostate tissue. In the intestine, PSMA facilitates absorption of folate by conversion of pteroylpoly-γ-glutamate to pteroylglutamate (folate). In the brain, it hydrolyses N-acetyl-L-aspartyl-L-glutamate (NAAG) to N-acetyl-L-aspartate and glutamate.

### *Prostate-specific membrane antigen (PSMA)*

Prostate-specific membrane antigen (PSMA) is a type II transmembrane glycoprotein that is highly overexpressed on prostate cancer epithelial cells. Despite its name, PSMA is also expressed, to varying degrees, in the neovasculature of a wide variety of nonprostate cancers. Among the most common nonprostate cancers to demonstrate PSMA expression include breast, lung, colorectal, and renal cell carcinoma.

The general necessary structures of PSMA targeting molecules comprise a binding unit that encompasses a zinc-binding group (such as urea (Zhou et al., Nature Reviews Drug Discovery 4, 1015-1026 (2005)), phosphinate or phosphoramidate) connected to a P1' glutamate moiety, which warrants high affinity and specificity to PSMA and is typically further connected to an effector functionality (Machulkin et al., Journal of drug targeting, 1-15 (2016)). The effector part is more flexible and to some extent tolerant towards structural modifications. The entrance tunnel accommodates two other prominent structural features, which are important for ligand binding. The first one is an arginine patch, a positively charged area at the wall of the entrance funnel and the mechanistic explanation for the preference of negatively charged functionalities at the P1 position of PSMA. This appears to be the reason for the preferable incorporation of negative charged residues within the ligand-scaffold. An in-depth analysis about the effect of positive charges on PSMA ligands has been, to our knowledge, so far not conducted. Upon binding, the concerted repositioning of the arginine side chains can lead to the opening of an S1 hydrophobic accessory pocket, the second important structure that has been shown to accommodate an iodo-benzyl group of several urea based inhibitors, thus contributing to their high affinity for PSMA (Barinka et al., Journal of medicinal chemistry 51, 7737-7743 (2008)).

Zhang et al. discovered a remote binding site of PSMA, which can be employed for bidentate binding mode (Zhang et al., Journal of the American Chemical Society 132, 12711-12716 (2010)). The so called arene-binding site is a simple structural motif shaped by the side chains of Arg463, Arg511 and Trp541, and is part of the GCPII entrance lid. The engagement of the arene binding site by a distal inhibitor moiety can result in a substantial increase in the inhibitor affinity for PSMA due to avidity effects. PSMA I&T was developed with the intention to interact this way with PSMA, albeit no crystal structure analysis of binding mode is available. A necessary feature according to Zhang et al. is a linker unit (Suberic acid in the case of PSMA I&T) which facilitates an open conformation of the entrance lid of GCPII and thereby enabling the accessibility of the arene-binding site. It was further shown that the structural composition of the linker has a significant impact on the tumor-targeting and biologic activity as well as on imaging contrast and pharmacokinetics (Liu et al., Bioorganic & medicinal chemistry letters 21, 7013-7016 (2011)), properties which are crucial for both high imaging quality and efficient targeted endoradiotherapy.

Two categories of PSMA-targeting inhibitors are currently used in clinical settings. On the one side there are tracers with chelating units for radionuclide complexation such as PSMA I&T or related compounds (Kiess et al., The quarterly journal of nuclear medicine and molecular imaging 59, 241 (2015)). On the other side there are small molecules,

comprising a targeting unit and effector molecules.

The most often used agents for selective PSMA imaging are PSMA HBED-CC (Eder et al., Bioconjugate chemistry 23, 688-697 (2012)), PSMA-617 (Benešová et al., Journal of Nuclear Medicine 56, 914-920 (2015)) and PSMA I&T (Weineisen et al.; Journal of Nuclear Medicine 55, 1083-1083 (2014)), which are predominantly labelled with $^{68}$Ga (88.9% $\beta^+$, $E_{\beta+, max}$ = 1.89 MeV, $t_{1/2}$ = 68 min). Among these $^{68}$Ga-PSMA-HBED-CC (also known as $^{68}$Ga-PSMA-11), is so far considered as the golden standard for PET imaging of PCa.

*$^{18}$F labelling*

Recently, several groups have focused on the development of novel $^{18}$F-labelled urea-based inhibitors for PCa diagnosis. In contrast to the radiometal $^{68}$Ga, which can be obtained from commercially distributed $^{68}$Ge/$^{68}$Ga radionuclide generators ($^{68}$Ge; $t_{1/2}$ = 270.8 d), the radioisotope $^{18}$F-fluorine (96.7% $\beta^+$, $E_{\beta+, max}$ = 634 keV) requires an on-site cyclotron for its production. Despite this limitation, $^{18}$F offers due to its longer half-live ($t_{1/2}$= 109.8 min) and its lower positron energy, significant advantages in terms of routine-handling and image quality. Additionally, there is the possibility for largescale production in a cyclotron, which would be beneficial for a higher patient throughput and reduction of production costs. The $^{18}$F-labelled urea-based PSMA inhibitor $^{18}$F-DCFPyl demonstrated promising results in the detection of primary and metastatic PCa (Rowe et al., Molecular Imaging and Biology, 1-9 (2016)) and superiority to $^{68}$Ga-PSMA-HBED-CC in a comparative study (Dietlein et al., Molecular Imaging and Biology 17, 575-584 (2015)). Based on the structure of PSMA-617, the $^{18}$F-labelled analogue PSMA-1007 was recently developed, which showed comparable tumor-to-organ ratios (Cardinale et al., Journal of nuclear medicine: official publication, Society of Nuclear Medicine 58, 425-431 (2017); Giesel et al., European journal of nuclear medicine and molecular imaging 43, 1929-1930 (2016)). A comparative study with $^{68}$Ga-PSMA-HBED-CC revealed similar diagnostic accuracy of both tracers and a reduced urinary clearance of $^{18}$F-PSMA-1007, enabling a better assessment of the prostate (Giesel et al., European journal of nuclear medicine and molecular imaging 44, 678-688 (2017)).

An attractive approach for introducing $^{18}$F labels is the use of silicon fluoride acceptors (SIFA). Silicon fluoride acceptors are described, for example, in Lindner et al., Bioconjugate Chemistry 25, 738-749 (2014). In order to preserve the silicon-fluoride bond, the use of silicon fluoride acceptors introduces the necessity of sterically demanding groups around the silicone atom. This in turn renders silicon fluoride acceptors highly hydrophobic. In terms of binding to the target molecule, in particular to the target molecule which is PSMA, the hydrophobic moiety provided by the silicone fluoride acceptor may be exploited for the purpose of establishing interactions of the radio-diagnostic or -therapeutic compound with the hydrophobic pocket described in Zhang et al., Journal of the American Chemical Society 132, 12711-12716 (2010). Yet, prior to binding, the higher degree of lipophilicity introduced into the molecule poses a severe problem with respect to the development of radiopharmaceuticals with suitable in vivo biodistribution, i.e. low unspecific binding in non-target tissue.

*Failure to solve the hydrophobicity problem*

Despite many attempts, the hydrophobicity problem caused by silicon fluoride acceptors has not been satisfactorily solved in the prior art.

To explain further, Schirrmacher E. et al. (Bioconjugate Chem. 2007, 18, 2085-2089) synthesized different $^{18}$F-labelled peptides using the highly effective labelling synthon p-(di-tert-butylfluorosilyl) benzaldehyde ([$^{18}$F]SIFA-A), which is one example of a silicon fluoride acceptor. The SIFA technique resulted in an unexpectedly efficient isotopic $^{19}$F-$^{18}$F exchange and yielded the $^{18}$F-synthon in almost quantitative yields in high specific activities between 225 and 680 GBq/$\mu$mol (6081-18 378 Ci/mmol) without applying HPLC purification. [$^{18}$F]SIFA-benzaldehyde was finally used to label the N-terminal amino-oxy (N-AO) derivatized peptides AO-Tyr3 -octreotate (AO-TATE), cyclo(fK(AO-N)RGD) and N-AO-PEG$_2$-[D-Tyr-Gln-Trp-Ala-Val-Ala-His-Thi-Nle-NH$_2$] (AO-BZH3, a bombesin derivative) in high radiochemical yields. Nevertheless, the labelled peptides are highly lipophilic (as can be taken from the HPLC retention times using the conditions described in this paper) and thus are unsuitable for further evaluation in animal models or humans.

In Wängler C. et al. (Bioconjugate Chem., 2009, 20 (2), pp 317-321), the first SIFA-based Kit-like radio-fluorination of a protein (rat serum albumin, RSA) has been described. As a labelling agent, 4-(di-tert-butyl[$^{18}$F]fluorosilyl)benzenethiol (Si[$^{18}$F]FA-SH) was produced by simple isotopic exchange in 40-60% radiochemical yield (RCY) and coupled the product directly to maleimide derivatized serum albumin in an overall RCY of 12% within 20-30 min. The technically simple labelling procedure does not require any elaborated purification procedures and is a straightforward example of a successful application of Si-$^{18}$F chemistry for in vivo imaging with PET. The time-activity cureves and $\mu$PET images of mice showed that most of the activity was localized in the liver, thus demonstrating that the labelling agent is too lipophilic and directs the in vivo probe to hepatobiliary excretion and extensive hepatic metabolism.

Wängler C. et al. (see Bioconjug Chem. 2010 Dec 15;21(12):2289-96) subsequently tried to overcome the major drawback of the SIFA technology, the high lipophilicity of the resulting radiopharmaceuticals, by synthesizing and

evaluating new SIFA-octreotate analogues (SIFA-Tyr3-octreotate, SIFA-Asn(AcNH-β-Glc)-Tyr3-octreotate and SIFA-Asn(AcNH-β-Glc)-PEG-Tyr3-octreotate). In these compounds, hydrophilic linkers and pharmacokinetic modifiers were introduced between the peptide and the SIFA-moiety, i.e. a carbohydrate and a PEG linker plus a carbohydrate. As a measure of lipophilicity of the conjugates, the log P(ow) was determined and found to be 0.96 for SIFA-Asn(AcNH-β-Glc)-PEG-$Tyr^3$-octreotate and 1.23 for SIFA-Asn(AcNH-β-Glc)-$Tyr^3$-octreotate. These results show that the high lipophilicity of the SIFA moiety can only be marginally compensated by applying hydrophilic moieties. A first imaging study demonstrated excessive hepatic clearance /liver uptake and thus has never been transferred into a first human study.

Bernard-Gauthier et al. (Biomed Res Int. 2014;2014:454503) reviews a great plethora of different SIFA species that have been reported in the literature ranging from small prosthetic groups and other compounds of low molecular weight to labelled peptides and most recently affibody molecules. Based on these data the problem of lipophilicity of SIFA-based prosthetric groups has not been solved sofar; i.e. a methodology that reduces the overall lipophilicity of a SIFA conjugated peptide to a log D lower than approx. -2,0 has not been described.

In Lindner S. et al. (Bioconjug Chem. 2014 Apr 16;25(4):738-49) it is described that PEGylated bombesin (PESIN) derivatives as specific GRP receptor ligands and RGD (one-letter codes for arginine-glycine-aspartic acid) peptides as specific αvβ3 binders were synthesized and tagged with a silicon-fluoride-acceptor (SIFA) moiety. To compensate the high lipophilicity of the SIFA moiety various hydrophilic structure modifications were introduced leading to reduced logD values. SIFA-Asn(AcNH-β-Glc)-PESIN, SIFA-Ser(β-Lac)-PESIN, SIFA-Cya-PESIN, SIFA-LysMe3-PESIN, SIFA-γ-carboxy-d-Glu-PESIN, SIFA-Cya2-PESIN, SIFA-LysMe3-γ-carboxy-d-Glu-PESIN, SIFA-(γ-carboxy-d-Glu)2-PESIN, SIFA-RGD, SIFA-γ-carboxy-d-Glu-RGD, SIFA-(γ-carboxy-d-Glu)2-RGD, SIFA-LysMe3-γ-carboxy-d-Glu-RGD. All of these peptides - already improved and derivatized with the aim to reduce the lipophilicity - showed a logD value in the range between +2 and -1.22.

In Niedermoser S. et al. (J Nucl Med. 2015 Jul;56(7):1100-5), newly developed $^{18}$F-SIFA- and $^{18}$F-SIFAlin-(SIFA = silicon-fluoride-acceptor) modified TATE derivatives were compared with the current clinical gold standard $^{68}$Ga-DOTATATE for high-quality imaging of somatostatin receptor-bearing tumors. For this purpose, $^{18}$F-SIFA-TATE and two quite complex analogues, $^{18}$F-SIFA-Glc-PEG1-TATE, $^{18}$F-SIFAlin-Glc-Asp2-PEG1-TATE were developed. None of the agents showed a logD <-1.5.

In view of the above, the technical problem underlying the present invention can be seen in providing radio-diagnostics and radio-therapeutics which contain a silicone fluoride acceptor and which are, at the same time, characterized by favourable in-vivo properties.

WO2019/020831 and WO2020/157184 disclose ligand-SIFA-chelator conjugates.

In the present invention a proof-of-principle has been established using specific conjugates which bind with high affinity to prostate-specific membrane antigen (PSMA) as target. Accordingly, a further technical problem underlying the present invention can be seen in providing improved radio-therapeutics and -diagnostics for the medical indication which is cancer, preferably prostate cancer.

## THE INVENTION

The present disclosure relates to compounds of Formula (1a), (1b), (1c) or (1d):

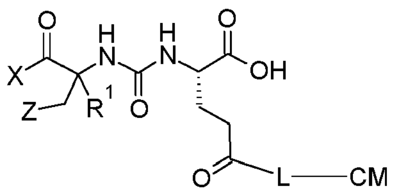 (1a);

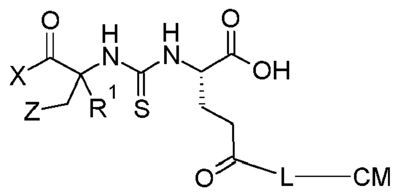 (1b);

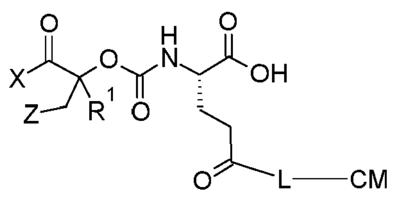 (1c);

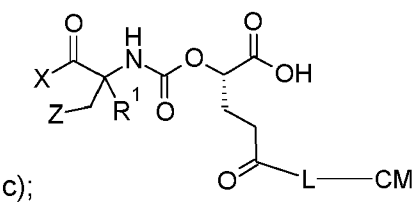 (1d);

or a pharmaceutically acceptable salt thereof, wherein;

L represents a linker group comprising a silicon-fluoride acceptor (SIFA) moiety which comprises a covalent bond between a silicon and a fluorine atom;

CM represents a chelator moiety, optionally containing a chelated nonradioactive or radioactive cation;
$R^1$ is H or a $C_{1-3}$ alkyl group optionally substituted with 1 to 3 fluorine atoms;
X is selected from OH and an amino acid group;
Z is selected from $-V-CO_2H$, $-V-NH_2$, $-V-PO_3H_2$, -V-COY, -V-W and a $C_{1-6}$ saturated or unsaturated hydrocarbon group optionally substituted with 1 to 3 fluorine atoms, where Y is an amino acid, W is a 5- or 6-membered heterocyclic ring, and V is a bond or a $C_{1-3}$ alkyl group optionally substituted with 1 to 3 fluorine atoms;
and when the compound is a compound of formula (1a) and X is OH, Z is not $CH_2CO_2H$.

Also provided is a pharmaceutical or diagnostic composition comprising or consisting of one or more compounds of Formula (1a), (1b), (1c) or (1d). The compounds of the invention may be for use as a cancer diagnostic or imaging agent. Accordingly also provided is a method of imaging and/or diagnosing cancer comprising administering a compound of Formula (1a), (1b), (1c) or (1d) or a composition comprising a compound of Formula (1a), (1b), (1c) or (1d). The compounds or compositions of the invention may be for use in the treatment of cancer. The compounds or compositions of the invention may be for use in the diagnosis, imaging or prevention of neoangiogenesis/angiogenesis. The compounds or compositions of the invention may be for use as a cancer diagnostic or imaging agent or for use in the treatment of cancer. The compounds or compositions of the invention may be for use as a cancer diagnostic or imaging agent or for use in the treatment of cancer wherein the cancer is prostate, breast, lung, colorectal or renal cell carcinoma.

## DETAILED DESCRIPTION OF THE INVENTION

The present disclosure relates to compounds of Formula (1a), (1b), (1c) or (1d):

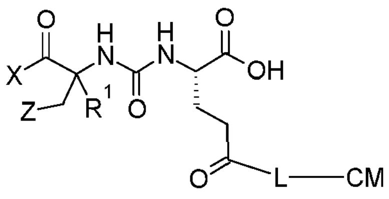 (1a); 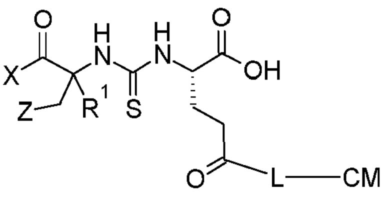 (1b);

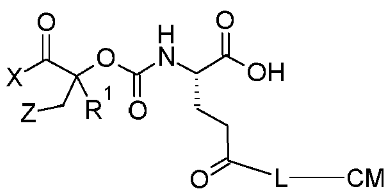 (1c); 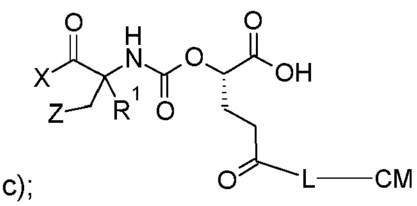 (1d);

or a pharmaceutically acceptable salt thereof, wherein;
L represents a linker group comprising a silicon-fluoride acceptor (SIFA) moiety which comprises a covalent bond between a silicon and a fluorine atom;
CM represents a chelator moiety, optionally containing a chelated nonradioactive or radioactive cation;
$R^1$ is H or a $C_{1-3}$ alkyl group optionally substituted with 1 to 3 fluorine atoms;
X is selected from OH and an amino acid group;
Z is selected from $-V-CO_2H$, $-V-NH_2$, $-V-PO_3H_2$, -V-COY, -V-W and a $C_{1-6}$ saturated or unsaturated hydrocarbon group optionally substituted with 1 to 3 fluorine atoms, where Y is an amino acid, W is 5- or 6-membered heterocyclic ring, and V is a bond or a $C_{1-3}$ alkyl group optionally substituted with 1 to 3 fluorine atoms;
and when the compound is a compound of formula (1a) and X is OH, Z is not $CH_2CO_2H$.

The invention relates to compounds of Formula (1a):

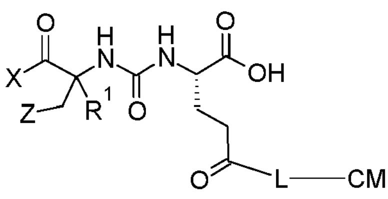 (1a);

or a pharmaceutically acceptable salt thereof, wherein;

L represents a linker group comprising a silicon-fluoride acceptor (SIFA) moiety which comprises a covalent bond between a silicon and a fluorine atom;
CM represents a chelator moiety, optionally containing a chelated nonradioactive or radioactive cation;
$R^1$ is H or a $C_{1-3}$ alkyl group optionally substituted with 1 to 3 fluorine atoms;
X is selected from OH and an amino acid group;
Z is selected from $-V-CO_2H$, $-V-NH_2$, $-V-PO_3H_2$, -V-COY, -V-W and a $C_{1-6}$ saturated or unsaturated hydrocarbon group optionally substituted with 1 to 3 fluorine atoms, where Y is an amino acid, W is 5- or 6-membered heterocyclic ring, and V is a bond or a $C_{1-3}$ alkyl group optionally substituted with 1 to 3 fluorine atoms;
and when X is OH, Z is not $CH_2CO_2H$.

The compounds of the invention comprise three separate moieties. The three separate moieties are a PSMA binding moiety, a linker group (L) comprising a silicon-fluoride acceptor (SIFA) moiety and a chelator moiety (CM), optionally containing a chelated nonradioactive or radioactive cation, wherein the SIFA moiety comprises a covalent bond between a silicon and a fluorine atom which can be $^{18}F$.

For diagnostic imaging, the fluorine atom on the SIFA moiety may be $^{18}F$. The $^{18}F$ can be introduced by isotopic exchange with $^{19}F$.

The compounds of the invention require a hydrophilic chelator moiety (CM) in addition to the PSMA binding moiety. The hydrophilic chelator moiety (CM) is required to reduce the hydrophobic nature of the compounds caused by the presence of the SIFA moiety. A key aspect of the invention is the combination, within a single molecule, of a silicon fluoride acceptor and a chelator moiety or a chelate. These two structural elements, SIFA and the chelator, exhibit a spatial proximity. Preferably, the shortest distance between two atoms of the two elements is less or equal 25 Å, more preferably less than 20 Å and even more preferably less than 15 Å. Alternatively or in addition, it is preferred that not more than 25 covalent bonds separate an atom of the SIFA moiety and an atom the chelator, preferably not more than 20 chemical bonds and even more preferably not more than 15 chemical bonds.

The cation which may be optionally chelated to the chelator moiety may be a radioactive or non-radioactive cation. It is preferably a non-radioactive metal cation. Examples of suitable cations are provided below.

The compounds of the invention may be radioactively labelled at the SIFA moiety. Also included are molecules which are not radiolabelled at all. The chelator moiety may be either a complex of a cold (non-radioactive) ion or may be devoid of any ion.

The present inventors surprisingly discovered that placement of the silicone fluoride acceptor in the neighbourhood of a hydrophilic chelator such as, but not limited to, DOTAGA or DOTA, shields or compensates efficiently the lipophilicity of the SIFA moiety to an extent which shifts the overall hydrophobicity of compound in a range which renders the compound suitable for in-vivo administration.

A further advantage of the compounds of the present invention is their surprisingly low accumulation in the kidneys of mice when compared to other PSMA targeted radiopharmaceuticals, such as PSMA I&T. Without wishing to be bound by a particular theory, it seems to be the combination of the structural element SIFA with a chelator which provides for the unexpected reduction of accumulation in the kidneys.

In terms of lipophilicity/hydrophilicity, the logP value (sometimes also referred to as logD value) is an art-established measure.

The term "lipophilicity" relates to the strength of being dissolved in, or be absorbed in lipid solutions, or being adsorbed at a lipid-like surface or matrix. It denotes a preference for lipids (literal meaning) or for organic or apolar liquids or for liquids, solutions or surfaces with a small dipole moment as compared to water. The term "hydrophobicity" is used with equivalent meaning herein. The adjectives lipophilic and hydrophobic are used with corresponding meaning to the substantives described above.

The mass flux of a molecule at the interface of two immiscible or substantially immiscible solvents is governed by its lipophilicity. The more lipophilic a molecule is, the more soluble it is in the lipophilic organic phase. The partition coefficient of a molecule that is observed between water and n-octanol has been adopted as the standard measure of lipophilicity. The partition coefficient P of a species A is defined as the ratio $P = [A]_{n\text{-octanol}} / [A]_{water}$. A figure commonly reported is the logP value, which is the logarithm of the partition coefficient. In case a molecule is ionizable, a plurality of distinct microspecies (ionized and not ionized forms of the molecule) will in principle be present in both phases. The quantity describing the overall lipophilicity of an ionizable species is the distribution coefficient D, defined as the ratio $D = [\text{sum of the concentrations of all microspecies}]_{n\text{-octanol}} / [\text{sum of the concentrations of all microspecies}]_{water}$. Analogous to logP, frequently the logarithm of the distribution coefficient, logD, is reported. Often, a buffer system, such as phosphate buffered saline is used as alternative to water in the above described determination of logP.

If the lipophilic character of a substituent on a first molecule is to be assessed and/or to be determined quantitatively, one may assess a second molecule corresponding to that substituent, wherein said second molecule is obtained, for example, by breaking the bond connecting said substituent to the remainder of the first molecule and connecting (the) free valence(s) obtained thereby to hydrogen(s).

Alternatively, the contribution of the substituent to the logP of a molecule may be determined. The contribution

$\pi_{Xx}$ of a substituent X to the logP of a molecule R-X is defined as $\pi_{Xx} = logP_{R-X} - logP_{R-H}$, wherein R-H is the unsubstituted parent compound.

Values of P and D greater than one as well as logP, logD and $\pi_{Xx}$ values greater than zero indicate lipophilic/hydrophobic character, whereas values of P and D smaller than one as well as logP, logD and $\pi_{Xx}$ values smaller than zero indicate hydrophilic character of the respective molecules or substituents.

The above described parameters characterizing the lipophilicity of the lipophilic group or the entire molecule according to the invention can be determined by experimental means and/or predicted by computational methods known in the art (see for example Sangster, Octanol-water Partition Coefficients: fundamentals and physical chemistry, John Wiley & Sons, Chichester. (1997)).

The logP value of compounds of the invention may be between -5 and -1.5. It is particularly preferred that the logP value is between -3.5 and -2.0.

The compounds are preferably high affinity PSMA ligands with preferable affinity, expressed as $IC_{50}$, being below 50 nM, below 20 nM or below 5 nM.

The compounds of the invention may be compounds of Formula (2a), (2b), (2c) or (2d):

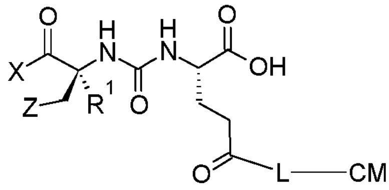 (2a); 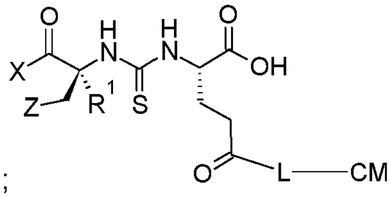 (2b);

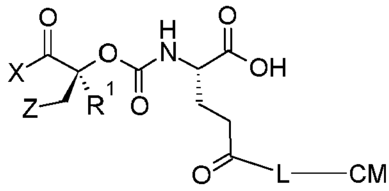 (2c); 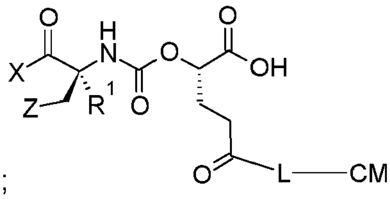 (2d);

or a pharmaceutically acceptable salt thereof, wherein X, Z, L, CM and $R^1$ are as defined herein.

In the compounds herein, $R^1$ can be H or a $C_{1-3}$ alkyl group optionally substituted with 1 to 3 fluorine atoms. $R^1$ can be H or a $C_{1-3}$ alkyl group. $R^1$ can be H or methyl. $R^1$ can be H. $R^1$ can be methyl.

The compounds of the invention may be compounds of Formula (3a), (3b), (3c) or (3d):

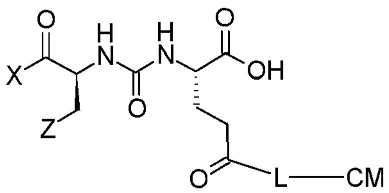 (3a); 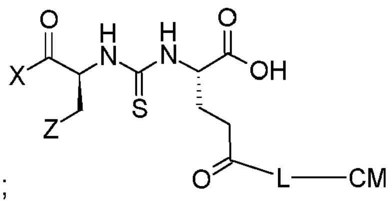 (3b);

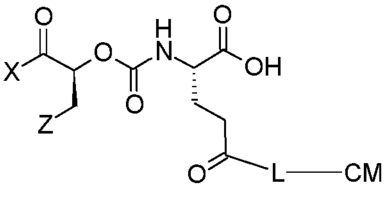 (3c); 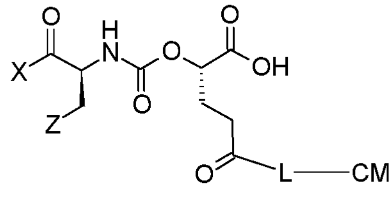 (3d);

or a pharmaceutically acceptable salt thereof, wherein X, Z, L and CM are as defined herein.

In the compounds herein, X can be OH or an amino acid group. X can be OH. X can be OH or $-NH-CH(C_6H_{13})CO_2H$. X can be $-NHCH(C_6H_{13})CO_2H$.

In the compounds herein, Z can be selected from $-V-CO_2H$, $-V-NH_2$, $-V-PO_3H_2$, -V-COY, -V-W and a $C_{1-6}$ saturated or unsaturated hydrocarbon group optionally substituted with 1 to 3 fluorine atoms, where Y is an amino acid, W is 5- or 6-membered heterocyclic ring, and V is a bond or a $C_{1-3}$ alkyl group optionally substituted with 1 to 3 fluorine

atoms.

In the compounds herein, V can be a bond. V can be a $C_{1-3}$ alkyl group optionally substituted with 1 to 3 fluorine atoms. V can be a $C_{1-3}$ alkyl group. V can be $-CH_2-$. V can be -CHF-. V can be $-CH_2CH_2-$. V can be $-CH_2CH_2CH_2-$.

In the compounds herein, W can be a 5-membered heterocyclic ring. W can be a 6-membered heterocyclic ring. W can be a 5- or 6-membered heterocyclic ring containing 1 to 4 heteroatom ring members selected from N, O and S. W can be a tetrazole ring. W can be a furan ring.

In the compounds herein, Y can be an amino acid. Y can be $-NHCH(C_6H_{13})CO_2H$. Y can be - $NH-CH(CH_2CH_3SCH_3)CO_2H$.

In the compounds herein of Formula (1a), when X is OH, Z is not $CH_2CO_2H$.

In the compounds herein, X can be $-NHCH(C_6H_{13}CO_2H$. X can be $-NHCH(C_6H_{13})CO_2H$ and Z can be $CH_2CO_2H$.

Z can be $-CHFCO_2H$, $-CH_2CONH_2$, $-CH_2PO_3H_2$, n-butyl, acetylene, furan, $-CH_2$-tetrazole, - $NH-CH(C_6H_{13})CO_2H$, $CH_2CO_2H$ or $-NHCH(CH_2CH_2SCH_3)CO_2H$.

The term amino acid or amino acid group as used in relation to groups X and Y, includes any amino acid, i.e. any group of formula $-NHCHR^{SC}CO_2H$, where $R^{SC}$ is any amino acid sidechain. The amino acid group may be an essential or non-essential amino acid. The amino acid group may be arginine, histidine, isoleucine, leucine, lysine, methionine, phenylalanine, threonine, tryptophan, valine, alanine, asparagine, aspartic acid, cysteine, glutamic acid, glutamine, glycine, proline, serine, tyrosine, or any derivative thereof.

The compounds of the invention may be compounds of Formula (4a), (4b), (4c), (4d), (4e), (4f), (4g), (4h), (4i), (4j), (4k) or (4l):

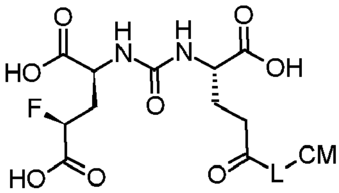 (4a); 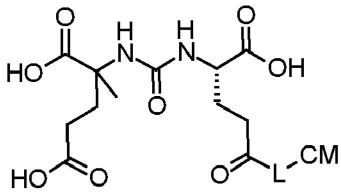 (4b); 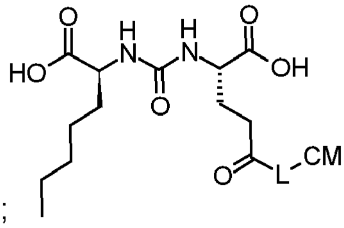 (4c);

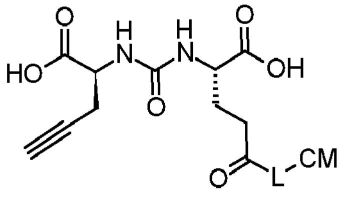 (4d); 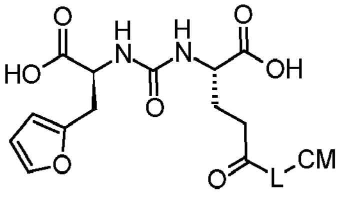 (4e); 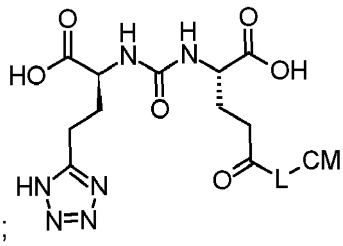 (4f);

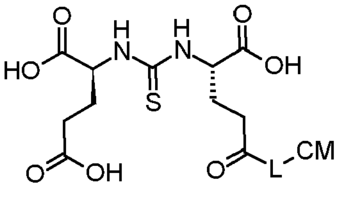 (4g); 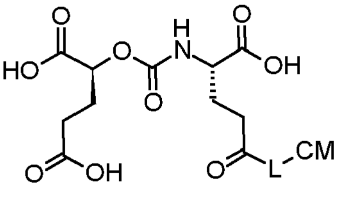 (4h); 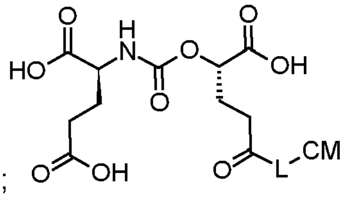 (4i);

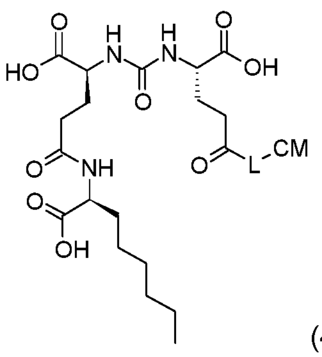 (4j); 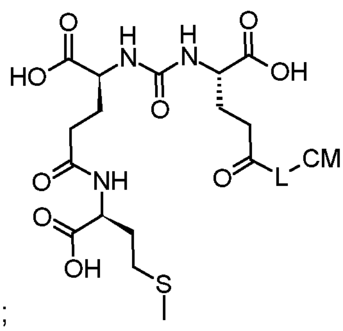 (4k); 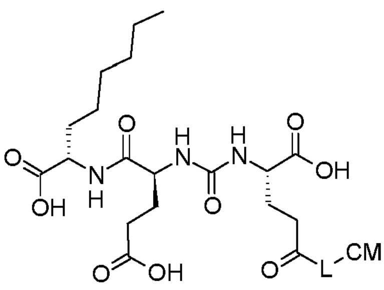 (4l);

or a pharmaceutically acceptable salt thereof, wherein L and CM are as defined herein.

In the compounds herein, the silicon-fluoride acceptor (SIFA) moiety may have the structure represented by formula (5):

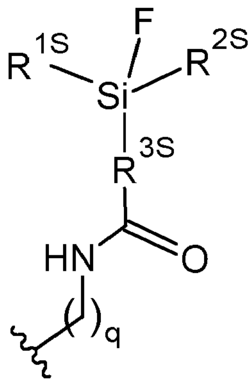

(5),

wherein $R^{1S}$ and $R^{2S}$ are independently a linear or branched $C_{3-10}$ alkyl group;

$R^{3S}$ is a $C_{1-20}$ hydrocarbon group comprising one or more aromatic and/or aliphatic units; q is 0 to 3;

and wherein the SIFA moiety is attached to L via the bond marked by .

$R^{1S}$ and $R^{2S}$ are independently a linear or branched $C_{3-10}$ alkyl group. Preferably $R^{1S}$ and $R^{2S}$ are selected from isopropyl and tert-butyl, and are more preferably $R^{1S}$ and $R^{2S}$ are tert-butyl; $R^{3S}$ is a $C_{1-20}$ hydrocarbon group comprising one or more aromatic and/or aliphatic units, preferably $R^{3S}$ is a $C_{6-10}$ hydrocarbon group which comprises an aromatic ring; more preferably $R^{3S}$ comprises a phenyl ring, and most preferably, $R^{3S}$ is a phenyl ring wherein the Si-containing substituent and the amide group are in a para-position. q may be 0, 1, 2 or 3. Preferably q is 1. The SIFA moiety is attached to the remainder of the conjugate via the bond marked by in formula (5).

The silicon-fluoride acceptor (SIFA) moiety may have the structure represented by formula (5a):

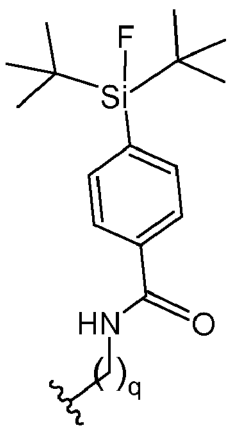

(5a),

wherein q is 0 to 3.

The silicon-fluoride acceptor (SIFA) moiety may have the structure represented by formula (5b):

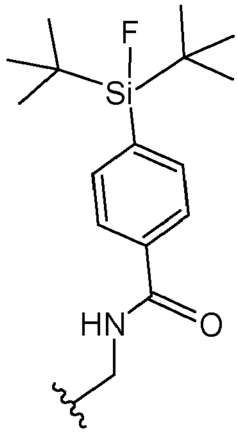

(5b).

In the compounds and moieties represented structurally herein F is to be understood to encompass both $^{19}F$ and $^{18}F$. The fluorine atom of the silicon-fluoride acceptor (SIFA) moiety may be $^{18}F$.

In the compounds herein, a preferred chelating group comprises at least one of the following (i), (ii) or (iii):

(i) A macrocyclic ring structure with 8 to 20 ring atoms of which 2 or more, more preferably 3 or more, are selected from oxygen atoms or nitrogen atoms. Preferably, 6 or less ring atoms are selected from oxygen atoms or nitrogen atoms. Especially preferred is that 3 or 4 ring atoms are nitrogen atoms or oxygen atoms. Among the oxygen and nitrogen atoms, preference is given to the nitrogen atoms. In combination with the macrocyclic ring structure, the preferred chelating group may comprise 2 or more, such as 2 to 6, preferably 2 to 4, carboxyl groups and/or hydroxyl groups. Among the carboxyl groups and the hydroxyl groups, preference is given to the carboxyl groups.

(ii) An acyclic, open chain chelating structure with 8 to 20 main chain (back bone) atoms of which 2 or more, more preferably 3 or more are heteroatoms selected from oxygen atoms or nitrogen atoms. Preferably, 6 or less back bone atoms are selected from oxygen atoms or nitrogen atoms. Among the oxygen and nitrogen atoms, preference is given to the nitrogen atoms. More preferably, the open chain chelating structure is a structure which comprises a combination of 2 or more, more preferably 3 or more heteroatoms selected from oxygen atoms or nitrogen atoms, and 2 or more, such as 2 to 6, preferably 2 to 4, carboxyl groups and/or hydroxyl groups. Among the carboxyl groups and the hydroxyl groups, preference is given to the carboxyl groups.

(iii) A branched chelating structure containing a quaternary carbon atom. Preferably the quaternary carbon atom is substituted with 3 identical chelating groups in addition to the SIFA/ligand moiety. The substituted chelating groups can comprise an amide. The substituted chelating groups can comprise an aromatic group. The substituted chelating groups can comprise a hydroxypyridinone.

The chelator moiety may comprise at least one of:

(i) a macrocyclic ring structure with 8 to 20 ring atoms of which 2 or more are heteroatoms selected from oxygen atoms and nitrogen atoms;

(ii) an acyclic, open chain chelating structure with 8 to 20 main chain atoms of which 2 or more are heteroatoms selected from oxygen atoms and nitrogen atoms; or

(iii) a branched chelating structure containing a quaternary carbon atom.

In preferred specific examples, the chelating group is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetracyclododecan-N,N',N",N'"-tetraacetic acid (DOTA), α-(2-carboxyethyl)-1,4,7,10-tetraazacyclododecane-1,4,7,10-tetraacetic acid (DOTAGA), 1,4,7,10 tetraazacyclododecane N, N', N", N'" 1,4,7,10-tetra(methylene) phosphonic acid (DOTMP), N,N'-dipyridoxylethylendiamine-N,N'-diacetate-5,5'-bis(phosphat) (DPDP), diethylene triamine N,N',N" penta(methylene) phosphonic acid (DTMP), diethylenetriaminepentaacetic acid (DTPA), ethylenediamine-N,N'-tetraacetic acid (EDTA), ethyleneglycol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), tetra 3-hydroxy-N-methyl-2-pyridinone chelators (4-((4-(3-(bis(2-(3-hydroxy-1-methyl-2-oxo-1,2-dihydropyridine-4-carboxamido)ethyl)amino)-2-((bis(2-(3-hydroxy-1-methyl-2-oxo-1,2-dihydropyridine-4-carboxamido)ethyl)amino)methyl)propyl)phenyl)amino)-4-oxobutanoic acid), abbreviated as Me-3,2-HOPO, 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), tris(hydroxypyridinone) (THP), terpyridin-bis(methyleneamintetraacetic acid (TMT), 1,4,7-triazacyclononane-1,4,7-tris[methylene(2-carboxyethyl)phosphinic acid] (TRAP), 1,4,7,10-tetraazacyclotridecan-N,N',N",N"'-tetraacetic acid (TRITA), 3-[[4,7-bis[[2-carboxyethyl(hydroxy)phosphoryl]methyl]-1,4,7-triazonan-1-yl]methyl-hydroxy-phosphoryl]propanoic acid, and triethylenetetraaminehexaacetic acid (TTHA), which residue is provided by covalently binding a carboxyl group contained in the chelating agent to the remainder of the conjugate via an ester or an amide bond.

The chelator moiety may be 1,4,7,10-tetracyclododecan-N,N',N",N"'-tetraacetic acid (DOTA) or α-(2-carboxyethyl)-1,4,7,10-tetraazacyclododecane-1,4,7,10-tetraacetic acid (DOTAGA).

Particular chelators are shown below:

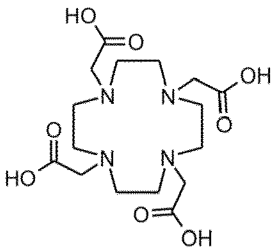

DOTA

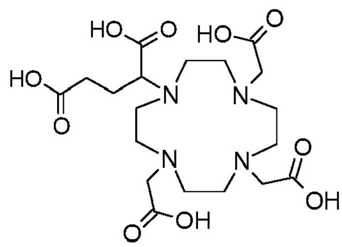

DOTAGA

, ,

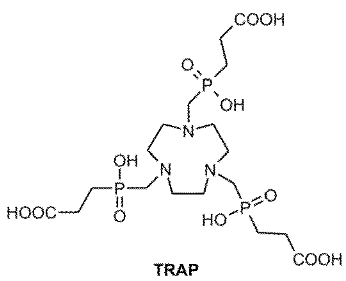

TRAP

,

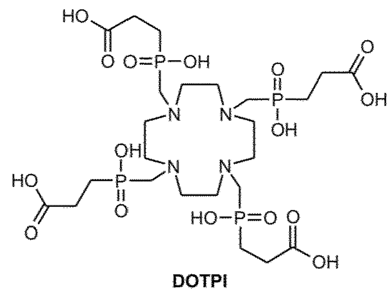

DOTPI

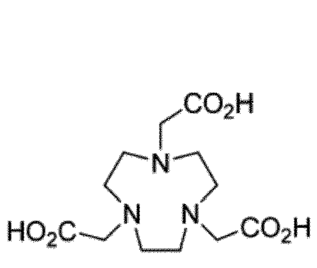

NOTA

,

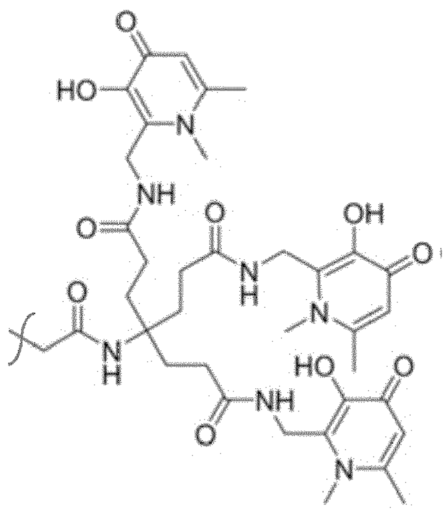

,

THP

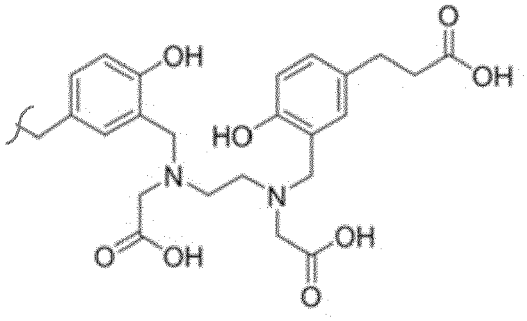

,

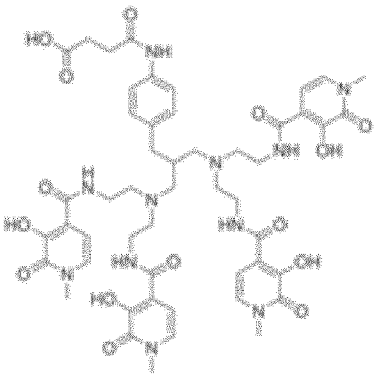

.

**HBED** **Me-3,2-HOPO**

Among the above exemplary chelating agents, particular preference is given to a chelating agent selected from TRAP, DOTA and DOTAGA.

Metal- or cation-chelating macrocyclic and acyclic compounds are well-known in the art and available from a number of manufacturers. While the chelating moiety in accordance with the present invention is not particularly limited, it is understood that numerous moieties can be used in an off-the-shelf manner by a skilled person without further ado.

The chelating group may comprise a chelated cation which may be radioactive or non-radioactive, preferably a chelated metal cation which may be radioactive or non-radioactive. The chelating group may comprise a chelated cation which is radioactive. The chelating group may comprise a chelated cation which is non-radioactive.

Especially preferred is that CM represents a chelating agent selected from DOTA and DOTAGA bound with one of its carboxylic groups via an amide bond to the remainder of the conjugate.

In order to be used in PET imaging, the compounds require a positron emitting atom. The compounds include $^{18}$F for medical use. Most preferred compounds of the invention are wherein F includes $^{18}$F and CM comprises a nonradioactive metal cation.

Preferred examples of cations that may be chelated by the chelating group are the non-radioactive cations of Sc, Cr, Mn, Co, Fe, Ni, Cu, Ga, Zr, Y, Tc, Ru, Rh, Pd, Ag, In, Sn, te, Pr, Pm, Tb, Sm, Gd, Tb, Ho, Dy, Er, Yb, Tm, Lu, Re, Pt, Hg, Au, Pb At, Bi, Ra, Ac, Th; more preferably the cations of Sc, Cu, Ga, Y, In, Tb, Ho, Lu, Re, Pb, Bi, Ac, Th and Er. The cation may be Ga. The cation may be Lu.

The chelator moiety may contain a chelated cation or cationic species selected from the cations of $^{43}$Sc, $^{44}$Sc, $^{47}$Sc, $^{51}$Cr, $^{52m}$Mn, $^{58}$Co, $^{52}$Fe, $^{56}$Ni, $^{57}$Ni, $^{62}$Cu, $^{64}$Cu, $^{67}$Cu, $^{66}$Ga, $^{67}$Ga $^{68}$Ga, $^{89}$Zr, $^{90}$Y, $^{89}$Y, <Tc, $^{99m}$Tc, $^{97}$Ru, $^{105}$Rh, $^{109}$Pd, $^{111}$Ag, $^{110m}$In, $^{111}$In, $^{113m}$In, $^{114m}$In, $^{117m}$Sn, $^{121}$Sn, $^{127}$Te, $^{142}$Pr, $^{143}$Pr, $^{149}$Pm, $^{151}$Pm, $^{149}$Tb, $^{152}$Tb, $^{155}$Tb, $^{161}$Tb, $^{153}$Sm, $^{157}$Gd, $^{161}$Tb, $^{166}$Ho, $^{165}$Dy, $^{169}$Er, $^{169}$Yb, $^{175}$Yb, $^{172}$Tm, $^{177}$Lu, $^{186}$Re, $^{188}$Re, $^{191}$Pt, $^{197}$Hg, $^{198}$Au, $^{199}$Au, $^{212}$Pb, $^{203}$Pb, $^{211}$At, $^{212}$Bi, $^{213}$Bi, $^{223}$Ra, $^{225}$Ac, $^{227}$Th, a cationic molecule comprising $^{18}$F or a cation such as $^{18}$F-[AlF]$^{2+}$; more preferably the cations of $^{44}$Sc, $^{47}$Sc, $^{64}$Cu, $^{67}$Cu, $^{68}$Ga, $^{90}$Y, $^{111}$In, $^{161}$Tb, $^{166}$Ho, $^{177}$Lu, $^{188}$Re, $^{212}$Pb, $^{212}$Bi, $^{213}$Bi, $^{225}$Ac, and $^{227}$Th or a cationic molecule comprising $^{18}$F.

The chelator moiety may contain a chelated cation selected from the cations of $^{43}$Sc, $^{44}$Sc, $^{47}$Sc, $^{64}$Cu, $^{67}$Cu, $^{67}$Ga, $^{68}$Ga, $^{90}$Y, $^{111}$In, $^{149}$Tb, $^{152}$Tb, $^{155}$Tb, $^{161}$Tb, $^{166}$Ho, $^{177}$Lu, $^{186}$Re, $^{188}$Re, $^{212}$Pb, $^{212}$Bi, $^{213}$Bi, $^{225}$Ac, and $^{227}$Th or a cationic molecule comprising $^{18}$F. The chelator moiety may contain a chelated cation selected from the cations of $^{68}$Ga or $^{177}$Lu. The chelator moiety may contain a chelated $^{68}$Ga cation. The chelator moiety may contain a chelated $^{177}$Lu cation.

In the compounds herein, the group -L-CM may be:

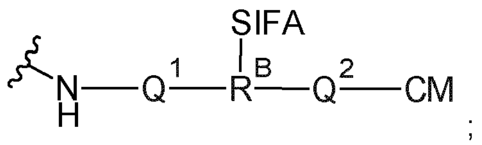

;

wherein $Q^1$ is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea), wherein $Q^1$ is optionally substituted with one or more substituents independently selected from -OH, $-OCH_3$, $-CH_2OH$, $-CO_2H$, $-CO_2CH_3$, $-NH_2$, $-CH_2NH_2$ and -NHC(NH)$NH_2$;

$Q^2$ is selected from an amide bond, an ether bond, a thioether bond, an ester bond, a thioester bond, a urea bridge, an amine bond and linking groups of the formula:

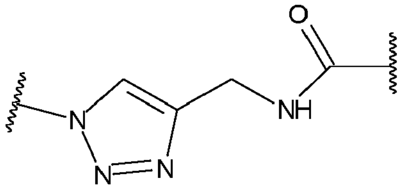
;
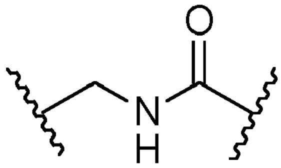
;

$R^B$ is a trivalent coupling group;
and SIFA is the silicon-fluoride acceptor moiety.

In the compounds herein, $-Q^1-$ can be selected from:

$-R^8-NH-C(O)-R^9-C(O)-NH-R^{10}-NH-C(O)-$
$-R^8-NH-C(O)-R^9-NH-C(O)-R^{10}-NH-C(O)-R^{11}-NH-C(O)-$
wherein $R^6$, $R^7$, $R^8$, $R^9$, $R^{10}$ and $R^{11}$ are independently $C_{1-10}$ alkyl, which alkyl groups may each be substituted by one or more substitutents independently selected from - OH, $-OCH_3$, $-CH_2OH$, $-CO_2H$, $-CO_2CH_3$, $-NH_2$, $-CH_2NH_2$ and $-NHC(NH)NH_2$.
$-Q^1-$ can be selected from:

$-CH(COOH)-R^{13}-NH-C(O)-R^{14}-C(O)-NH-R^{15}-CH(COOH)-NH-C(O)-$
$-CH(COOH)-R^{13}-NH-C(O)-R^{14}-NH-C(O)-R^{15}-NH-C(O)-CH(CH_2OH)-NH-C(O)-$

wherein $R^{13}$, $R^{14}$ and $R^{15}$ are independently $C_{1-6}$ alkyl.

In the compounds herein, $-Q^2-$ can be -NH-, -NH-C(O)-, $-NH-C(O)-CH_2-$, $-NH-C(O)-CH_2CH_2-$ or $-NH-C(O)-CH_2CH_2-CH(COOH)-$. $-Q^2-$ can be -NH-.

In the compounds herein $R^B$ may be:

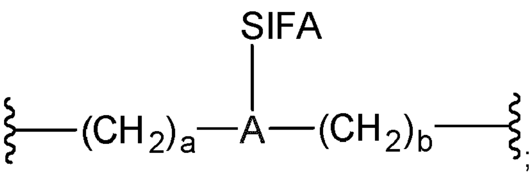

wherein:

A is selected from N, $CR^{16}$, wherein $R^{16}$ is H or $C_{1-6}$ alkyl, and a 5 to 7 membered carbocyclic or heterocyclic group; preferably A is selected from N and CH, and more preferably A is CH;
the bond marked by at $(CH_2)_a$ is formed with $Q^1$, and a is an integer of 0 to 4, preferably 0 or 1, and most preferably 0; and
the bond marked by at $(CH_2)_b$ is formed with $Q^2$, and b is an integer of 0 to 4, preferably of 0 to 2, and most preferably 0.
$R^B$ can be:

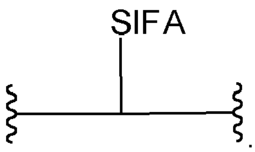

In the compounds herein, the group L-CM may be selected from:

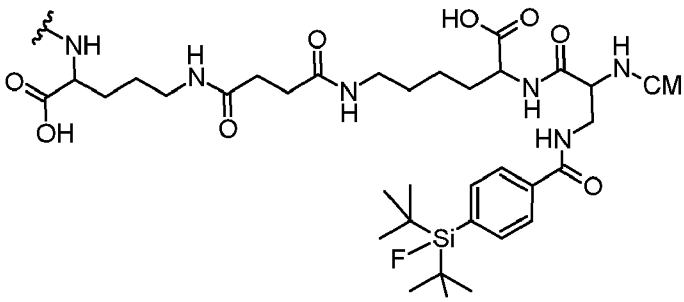
;

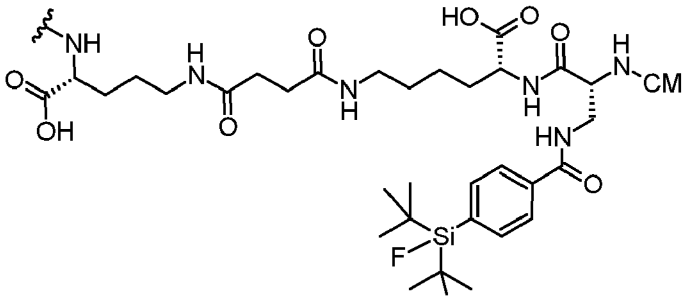
;

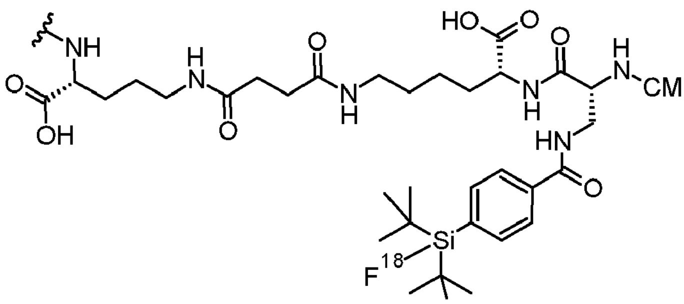
;

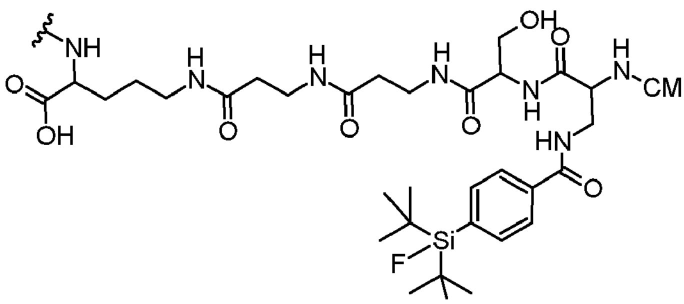
;

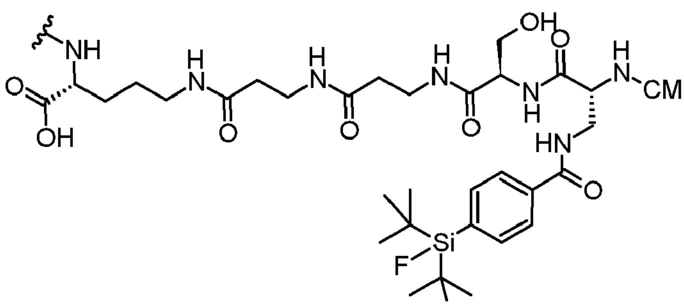
;

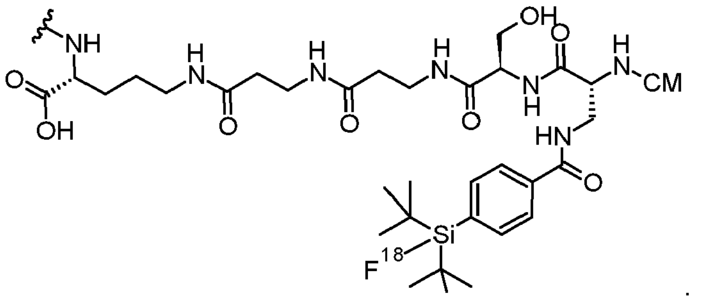

.

In the compounds herein, CM may be selected from:

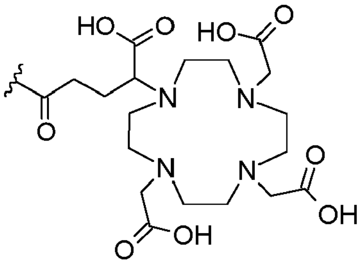

;

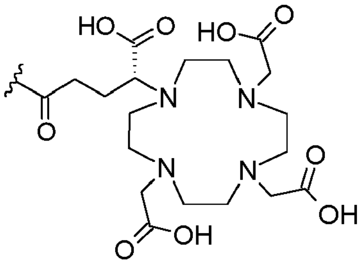

;

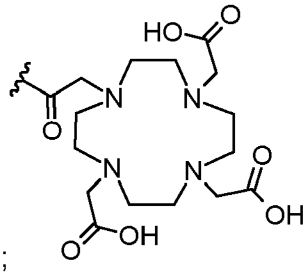

;

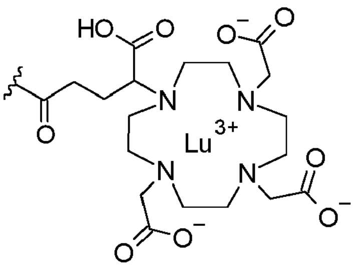

;

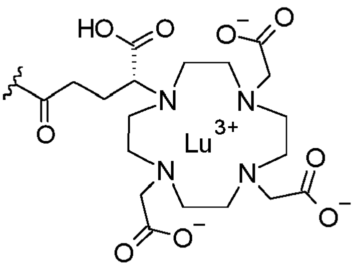

;

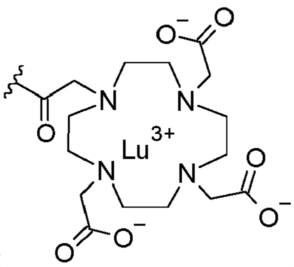

.

In the compounds herein, the group L-CM may be selected from:

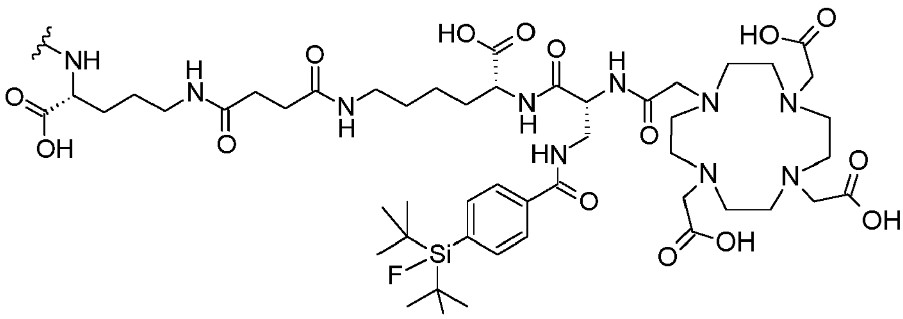

;

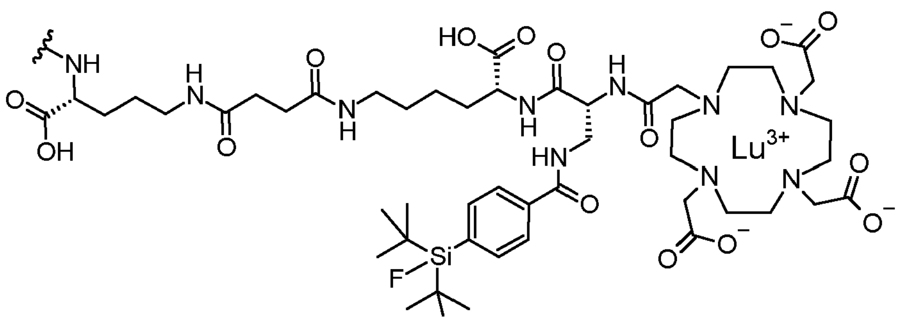

;

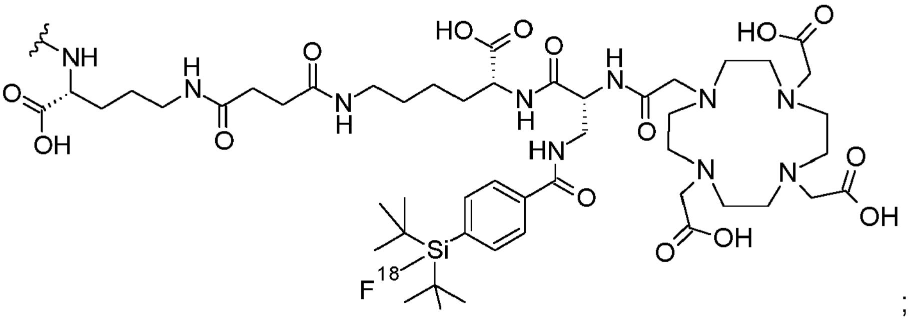

;

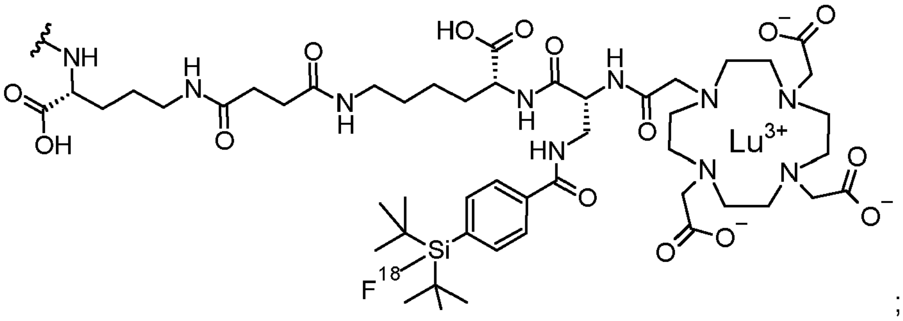

;

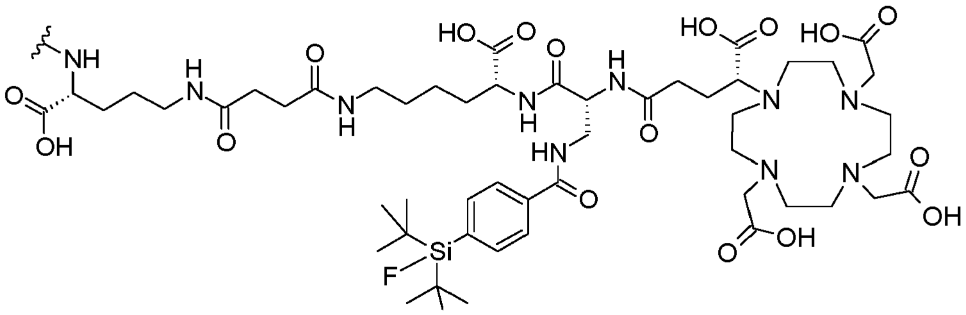

;

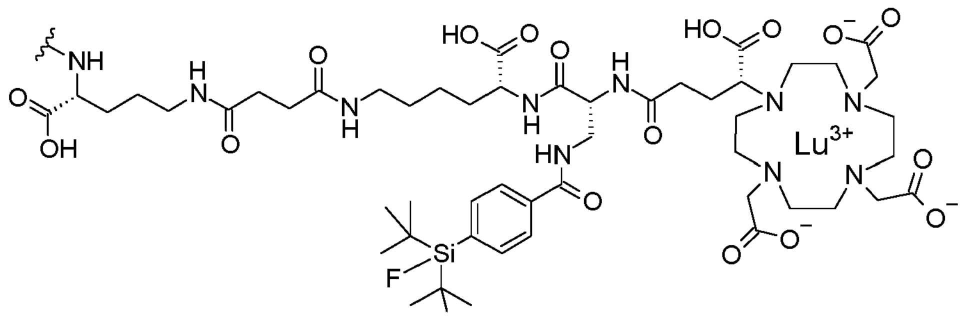

;

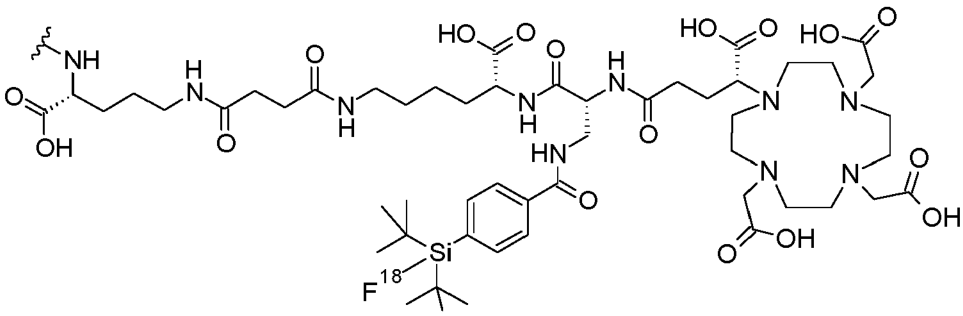

;

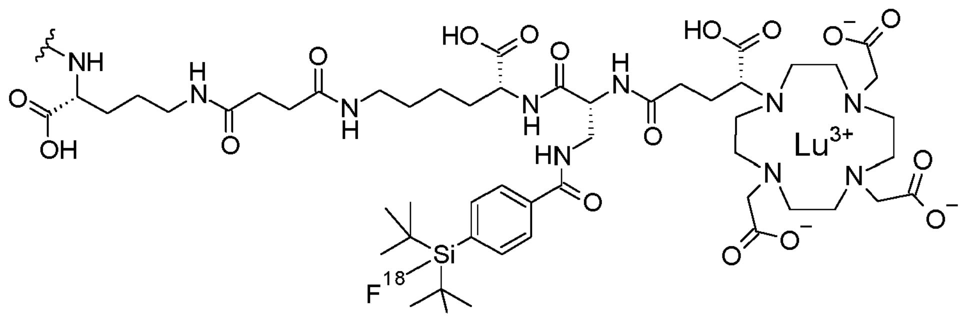

;

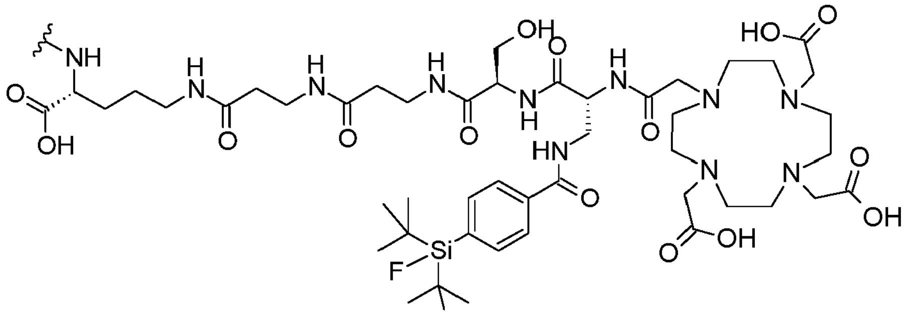

;

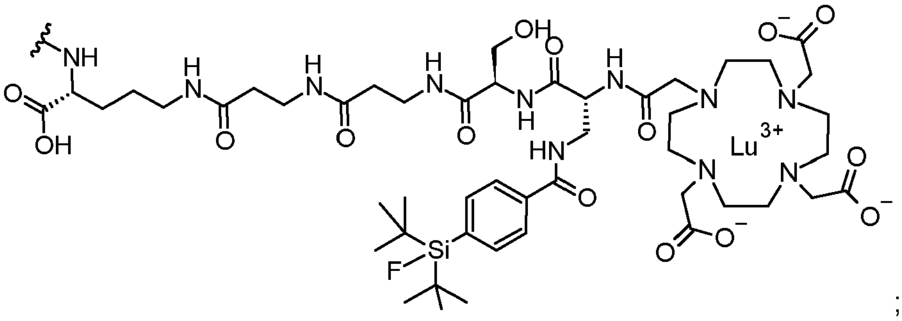

;

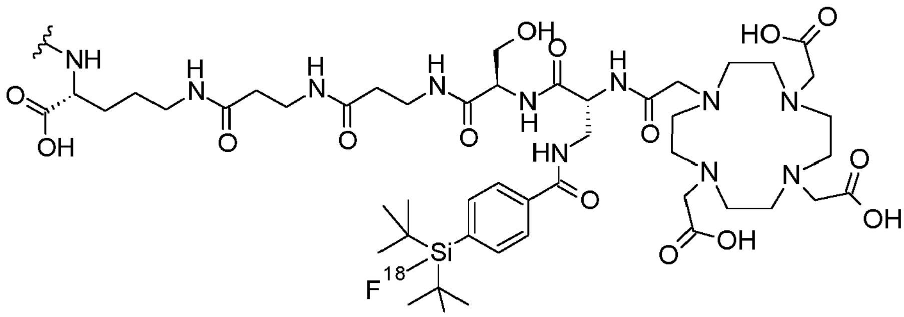

;

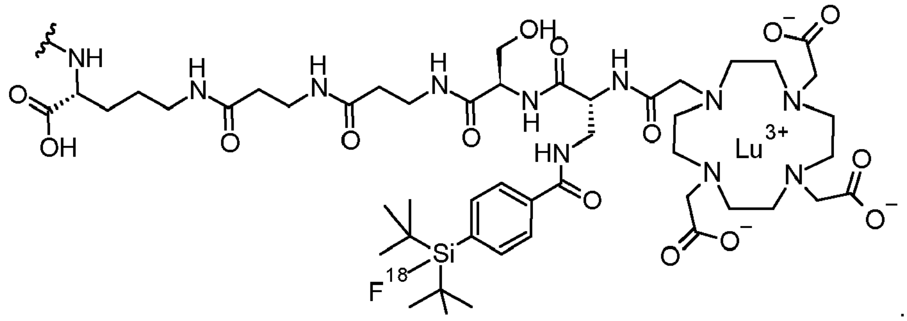

.

The term "oligo" as used in oligoamide, oligoether, oligothioether, oligoester, oligothioester, oligourea, oligo(ether-amide), oligo(thioether-amide), oligo(ester-amide), oligo(thioester-amide), oligo(urea-amide), oligo(ether-thioether), oligo(ether-ester), oligo(ether-thioester), oligo (ether-urea), oligo(thioether-ester), oligo(thioether-thioester), oligo(thioether-urea), oligo(ester-thioester), oligo(ester-urea), and oligo(thioester-urea) is preferably to be understood as referring to a group wherein 2 to 20, more preferably wherein 2 to 10 subunits are linked by the type of bonds specified in the same terms. As will be understood by the skilled reader, where two different types of bonds are indicated in brackets, both types of bonds are contained in the concerned group (e.g. in "oligo (ester-amide)", ester bonds and amide bonds are contained).

It is preferred that $L^1$ comprises a total of 1 to 5, more preferably a total of 1 to 3, and most preferably a total of 1 or 2 amide and/or ester bonds, preferably amide bonds, within its backbone.

The term oligoamide therefore describes a moiety having a chain of $CH_2$ or CHR groups interspersed with groups selected from NHCO or CONH. Each occurrence of the R moiety is an optional substituent selected from -OH, $-OCH_3$, $-CH_2OH$, $-CO_2H$, $-CO_2CH_3$, $-NH_2$, - $CH_2NH_2$ and $-NHC(NH)NH_2$.

In the compounds herein, the chelated nonradioactive or radioactive cation of the chelator moiety may be chelated to one or more $COO^-$ groups. The chelated nonradioactive or radioactive cation of the chelator moiety may be chelated to one or more N atoms. The chelated nonradioactive or radioactive cation of the chelator moiety may be chelated to one or more N atoms or one or more $COO^-$ groups. The chelated nonradioactive or radioactive cation of the chelator moiety may be chelated to one or more N atoms and one or more $COO^-$ groups. Where the chelated nonradioactive or radioactive cation is shown, the acid groups to which it is chelated are merely representatively shown as $COO^-$.

The compound may be selected from:

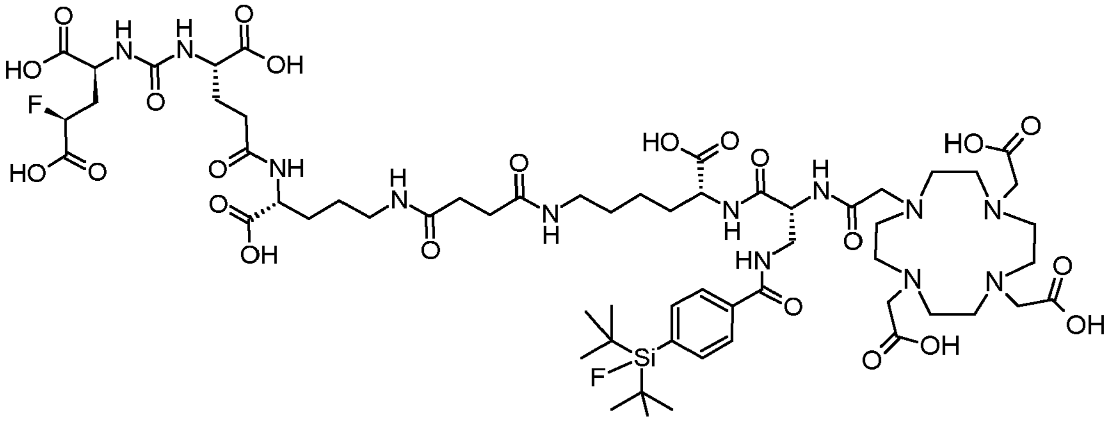

;

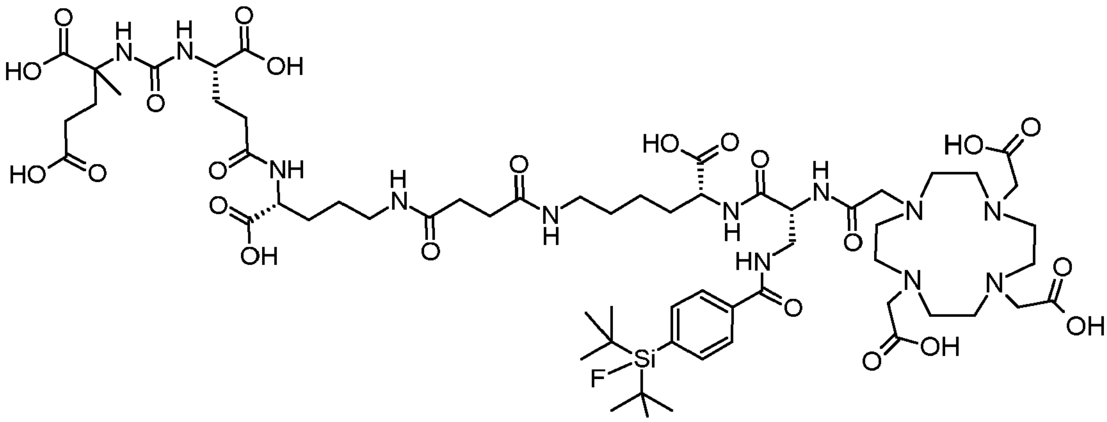

;

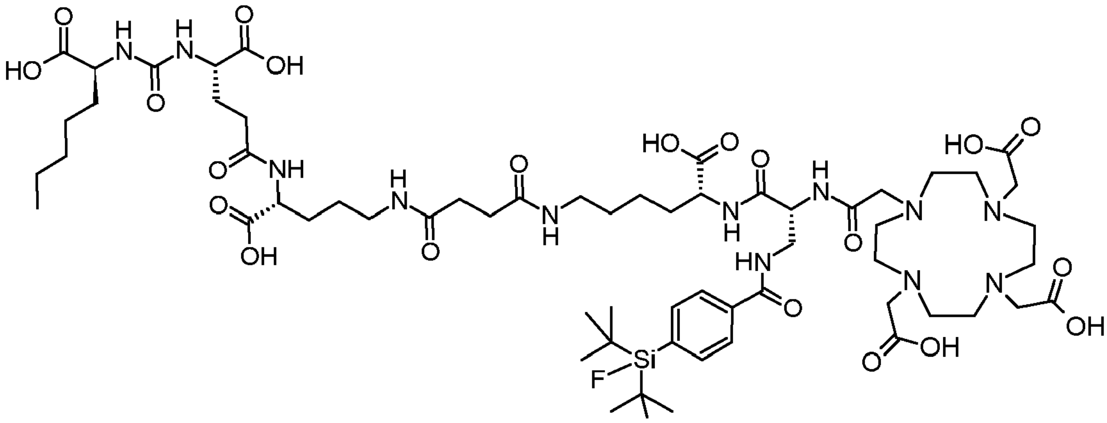

;

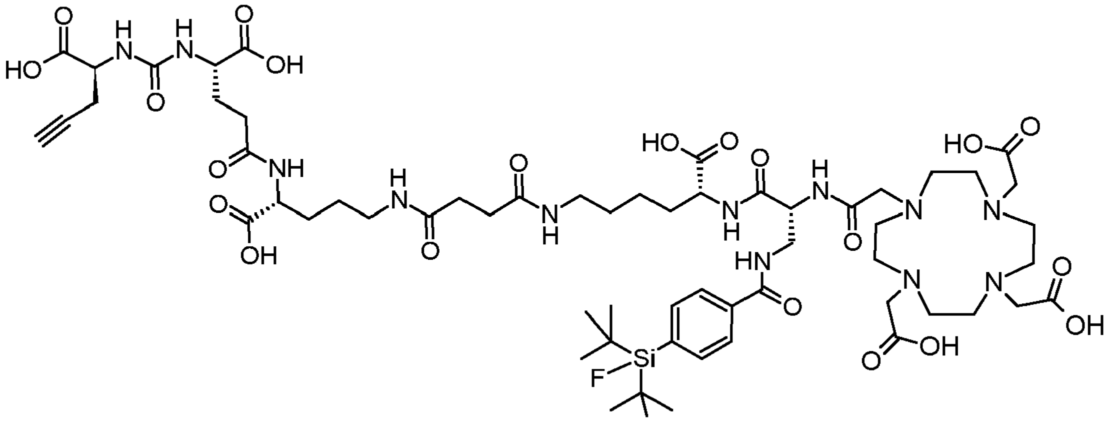

;

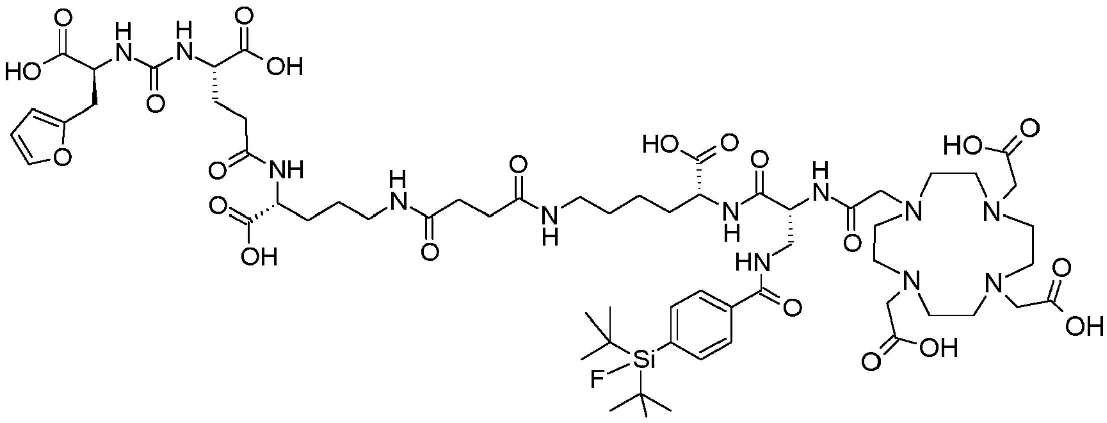

;

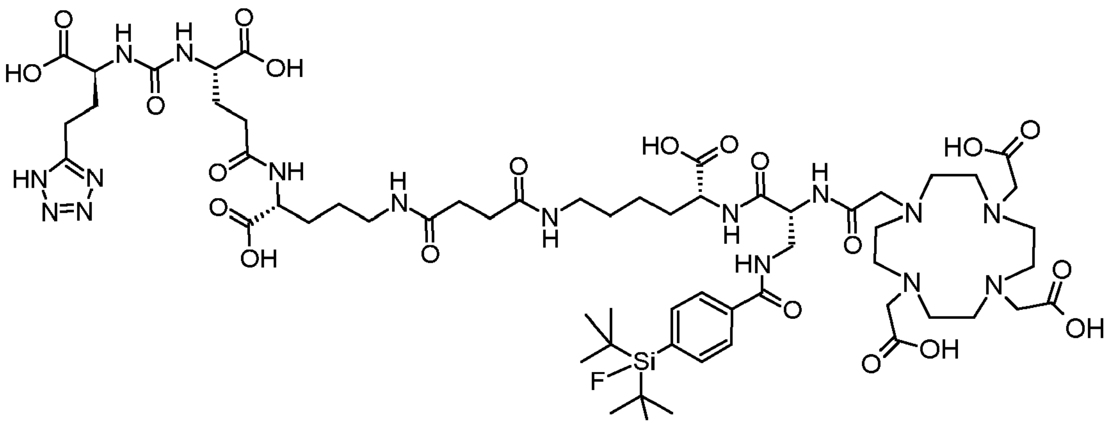

;

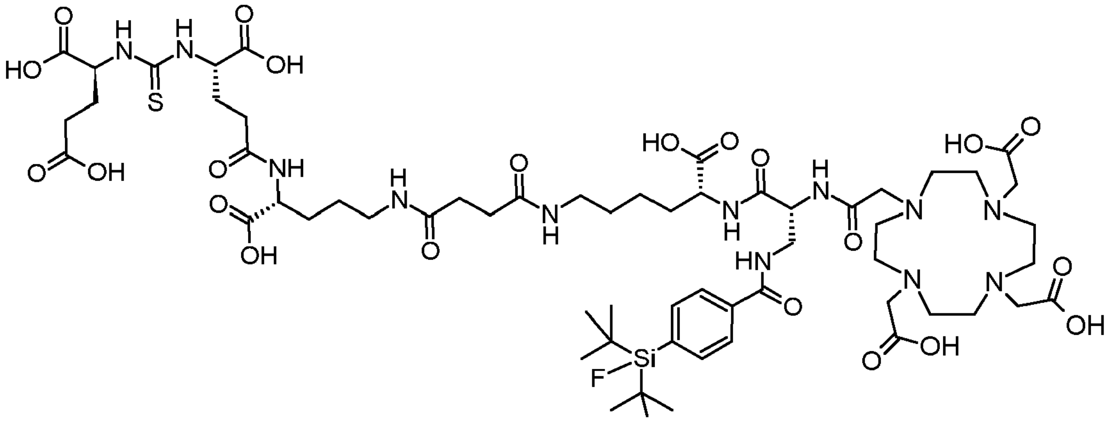

;

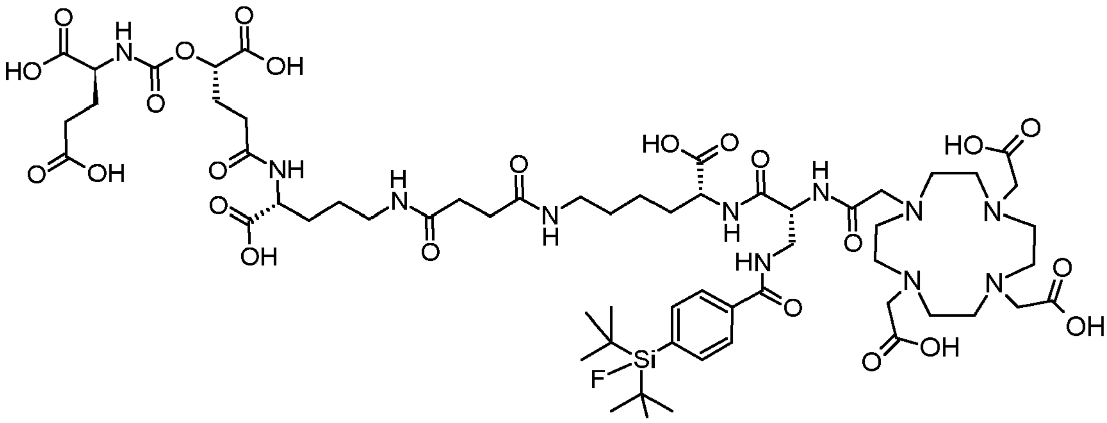

;

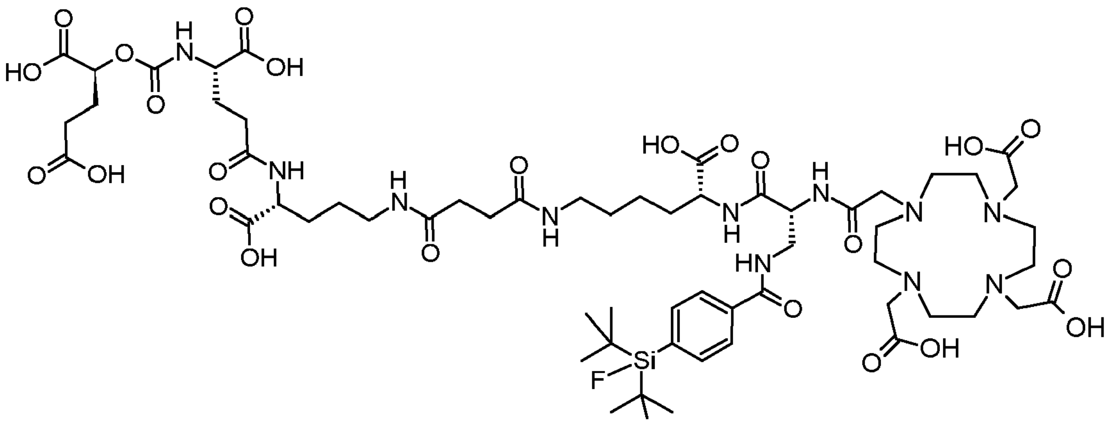

;

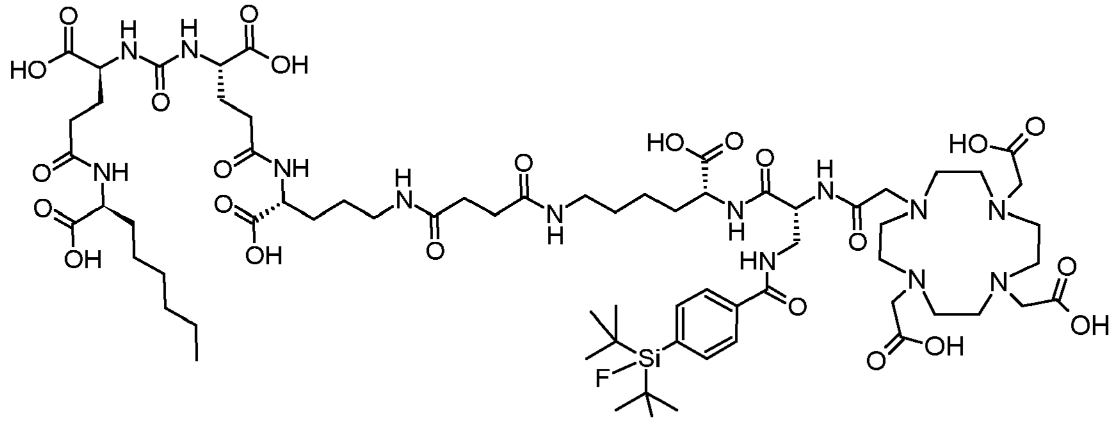

;

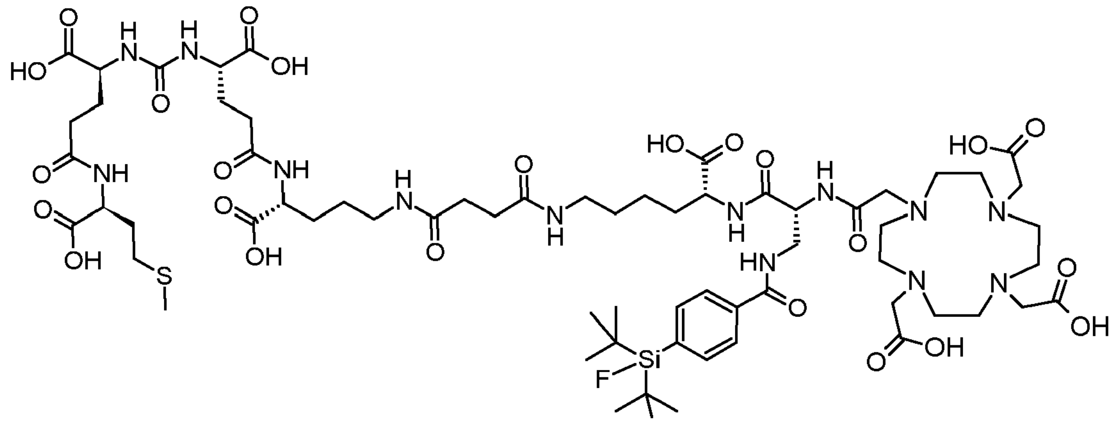

;

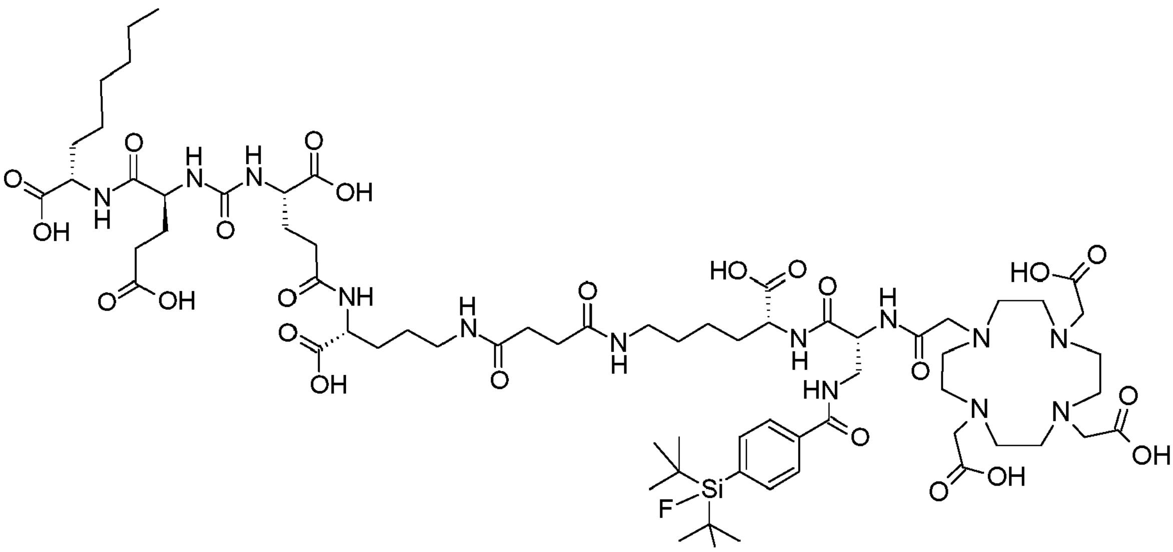

;

or a pharmaceutically acceptable salt thereof, wherein the fluorine atom is optionally $^{18}F$ and wherein the chelator moiety is optionally coordinated to $Lu^{3+}$.

The compound may be selected from:

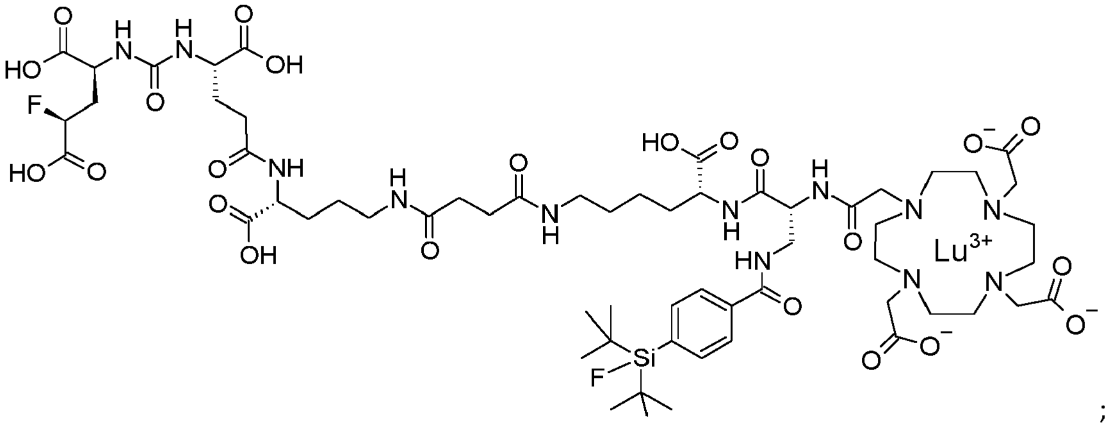

;

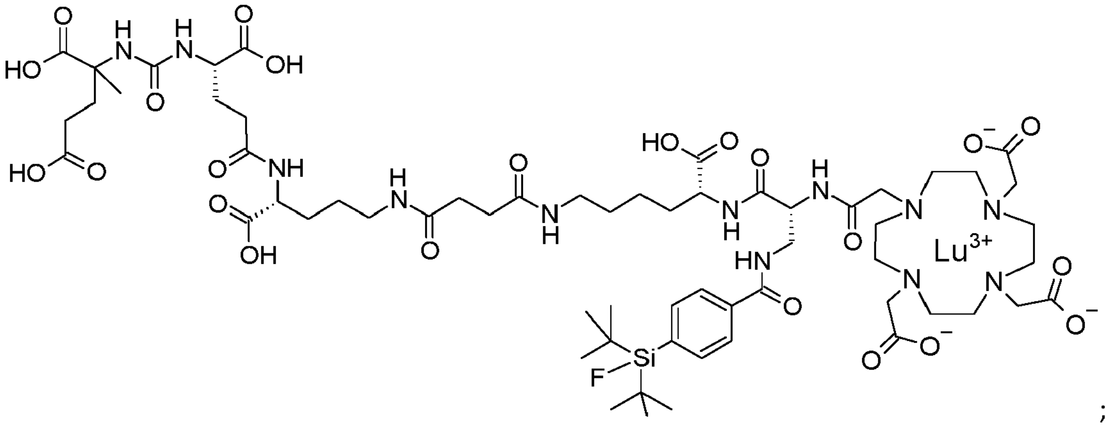

;

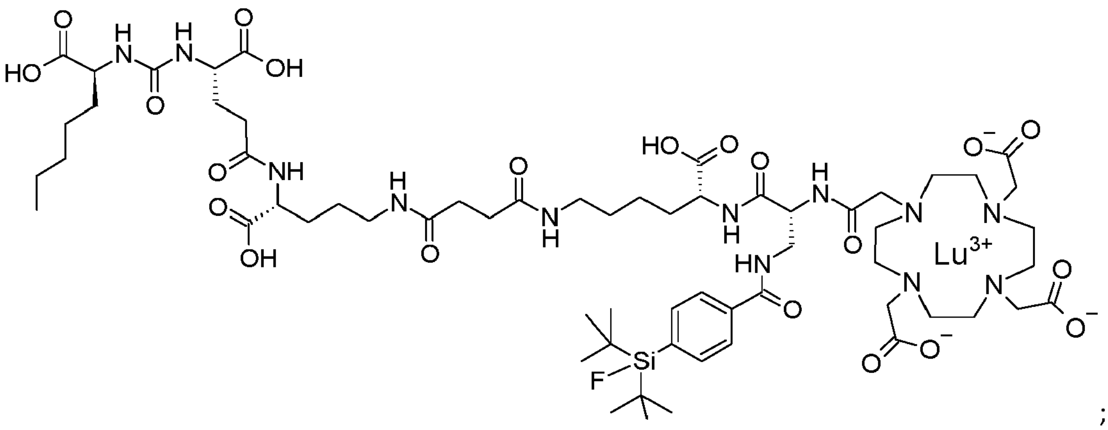

;

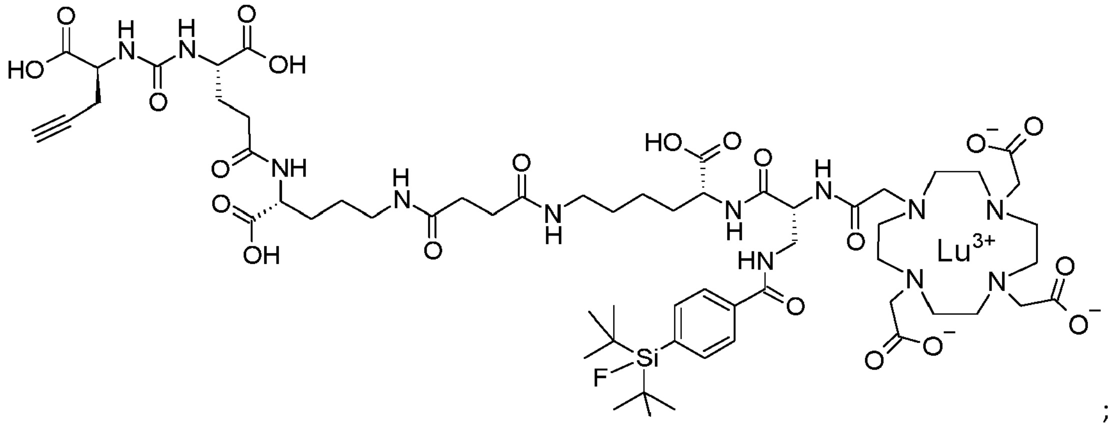

;

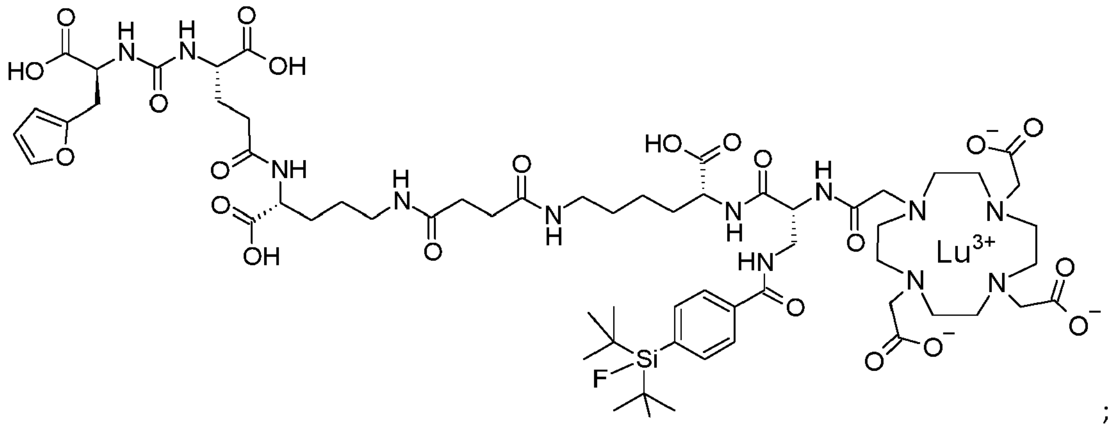

;

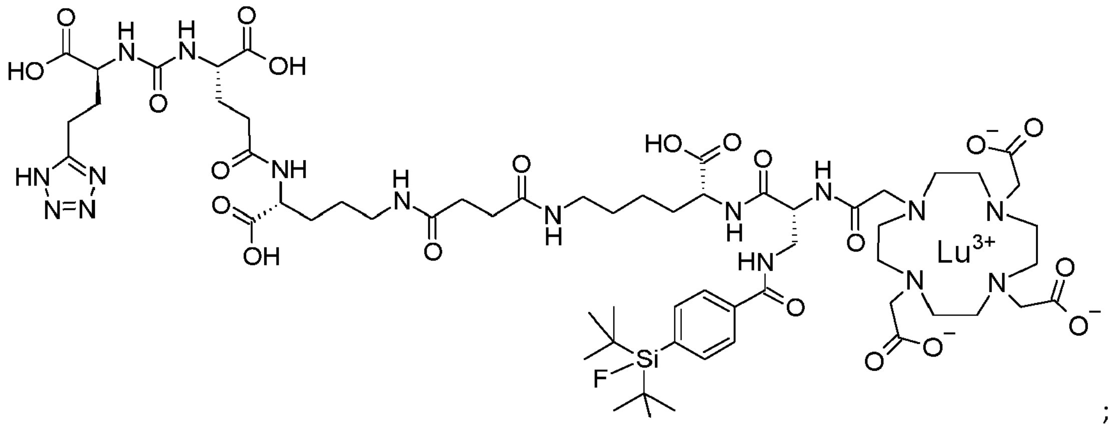

;

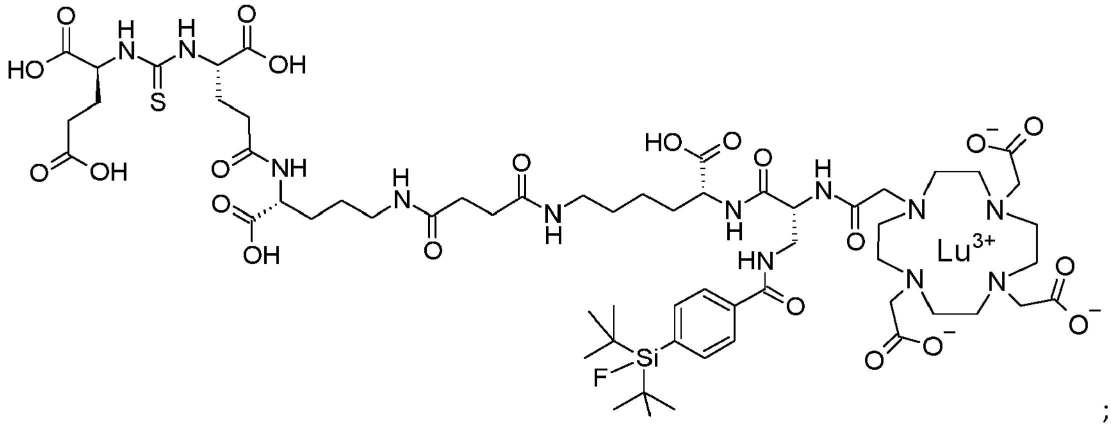

;

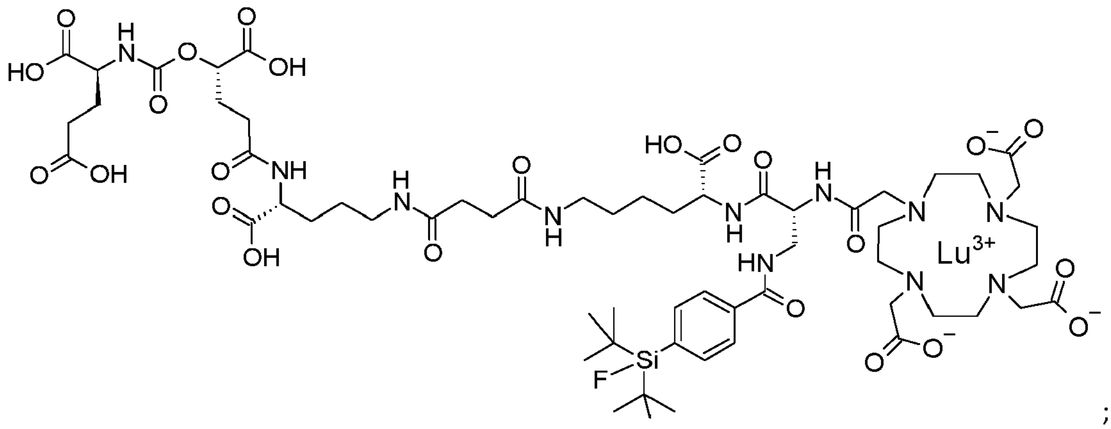

;

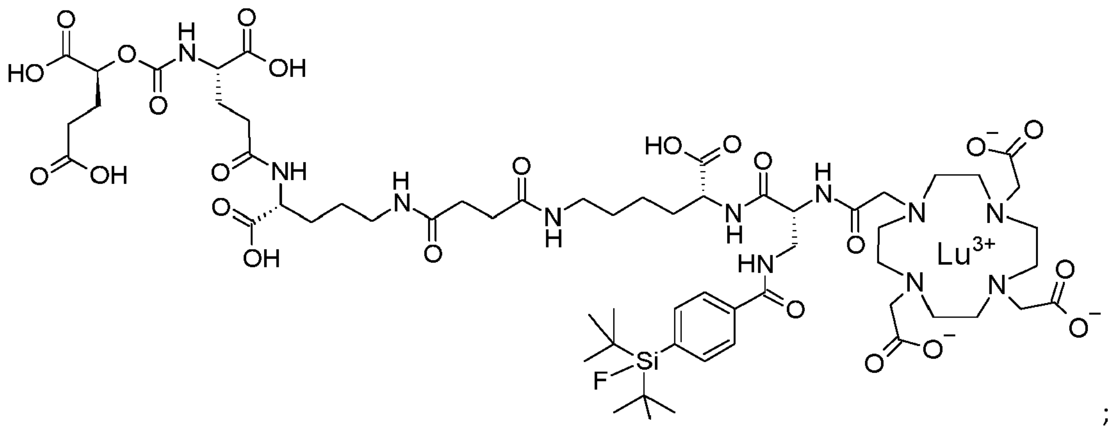

;

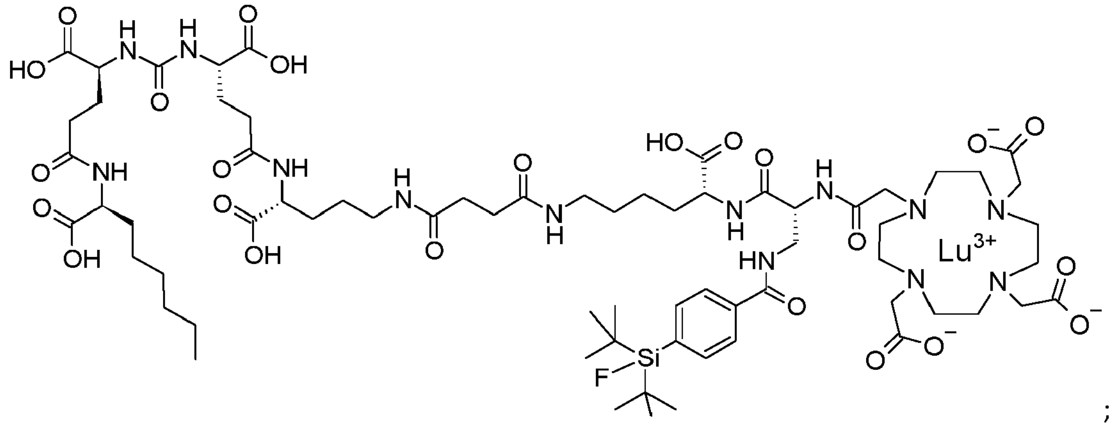

;

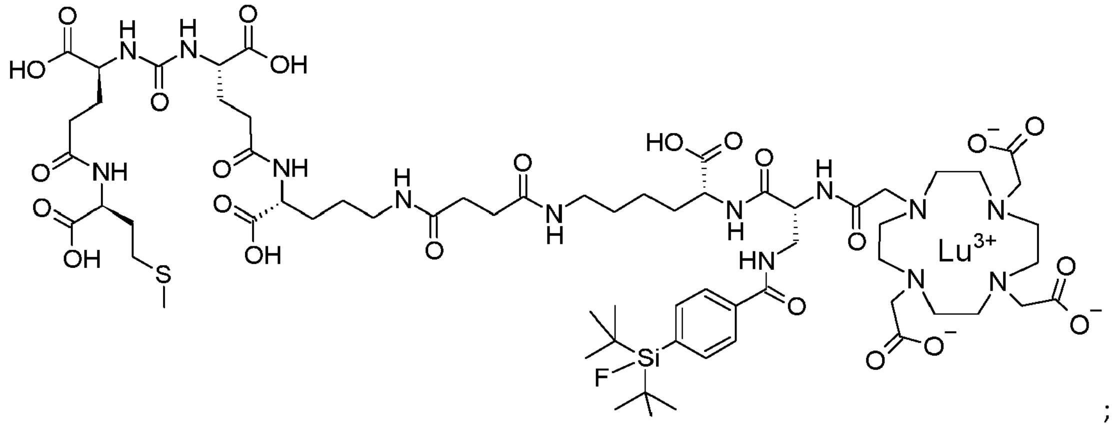

;

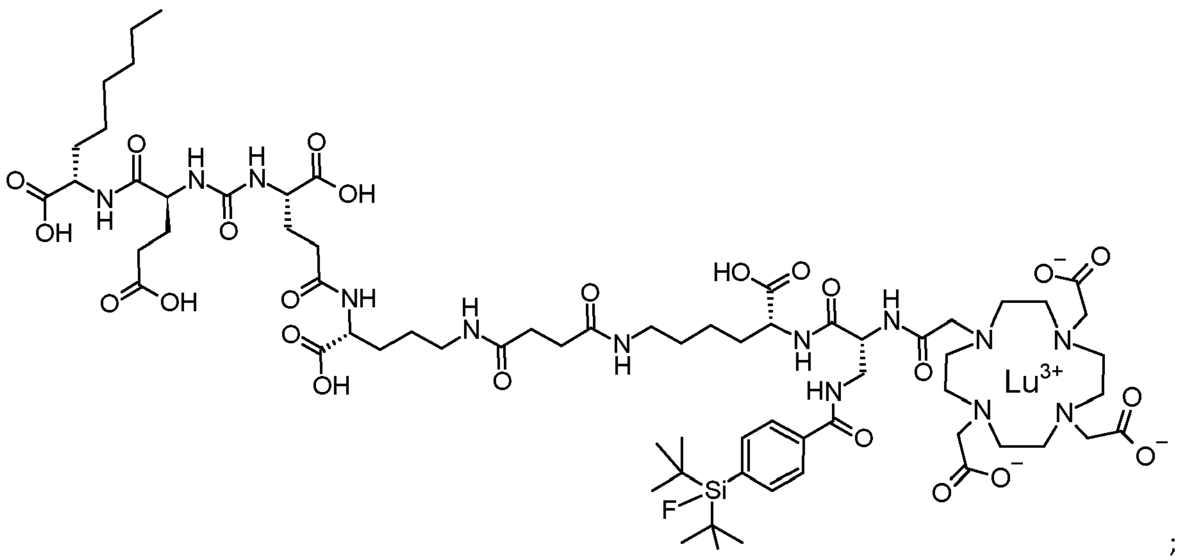

;

or a pharmaceutically acceptable salt thereof, wherein the fluorine atom is optionally $^{18}F$.

Preferred labelling schemes for these most preferred compounds are as defined herein above.

Also provided is a compound [$^{177}$Lu]Lu-PSMA-10 ([$^{177}$Lu]Lu-1 below),

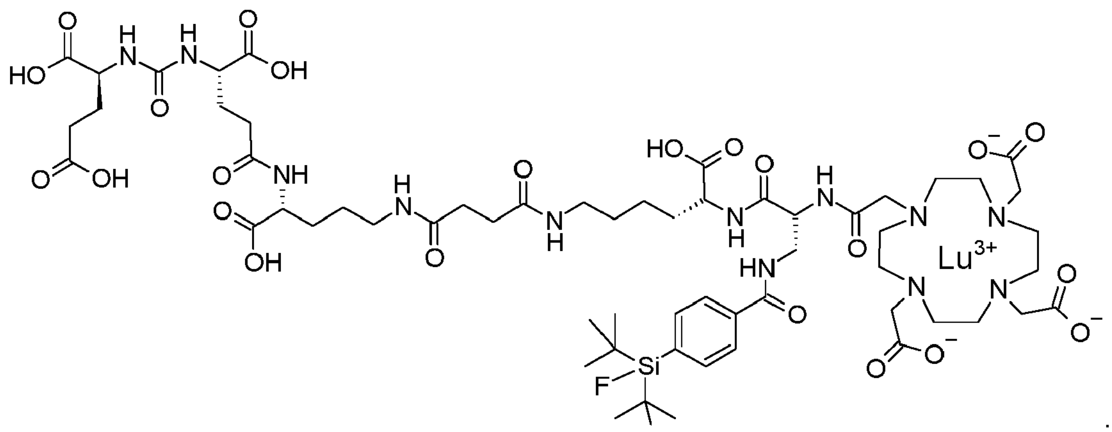

Compound [$^{177}$Lu]Lu-PSMA-10 is the Lutetium 177 chelate of PMSA 10 shown below,

(D)

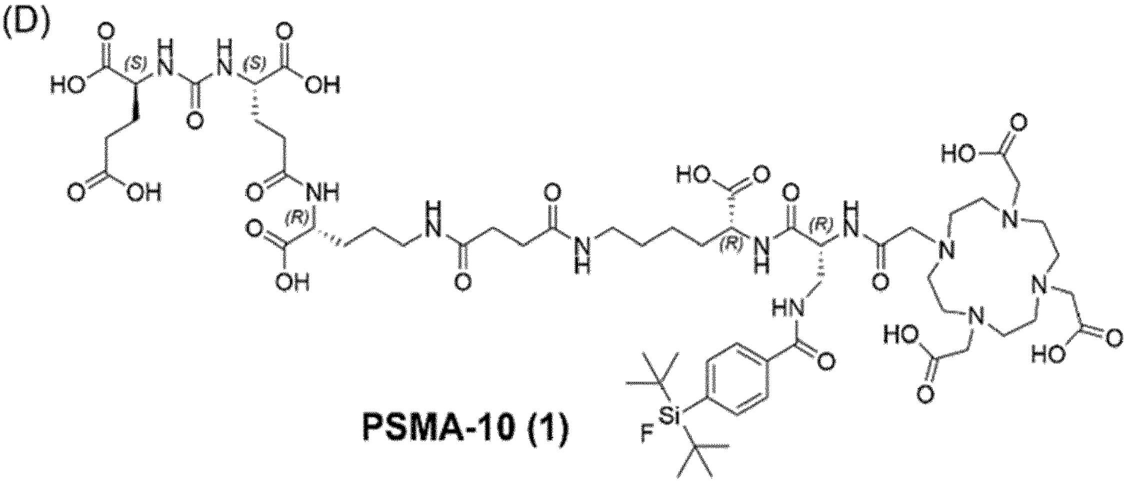

PSMA-10 (1)

Also provided is a pharmaceutical imaging composition comprising or consisting of one or more compounds of the invention as disclosed herein above.

Also provided is a diagnostic composition comprising or consisting of one or more compounds of the invention as disclosed herein above.

The pharmaceutical composition may further comprise pharmaceutically acceptable carriers, excipients and/or diluents. Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected in different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present in an effective therapeutic amount, which may be between 0.1 ng and 10 mg/kg body weight per dose; however, doses below or above this exemplary range are envisioned, especially considering the aforementioned factors.

Also provided is one or more compounds of the invention as disclosed herein above for use in diagnostic medicine.

Preferred uses in medicine are in nuclear medicine such as nuclear diagnostic imaging, also named nuclear molecular imaging, and/or targeted radiotherapy of diseases associated with an overexpression, preferably of PSMA on the diseased tissue.

Also provided is a compound of the invention as defined herein above for use in a method of diagnosing and/or staging cancer, preferably prostate cancer. Prostate cancer is not the only cancer to express PSMA. Nonprostate cancers to demonstrate PSMA expression include breast, lung, colorectal, and renal cell carcinoma. Thus, any compound described herein having a PSMA binding moiety can be used in the diagnosis, imaging or treatment of a cancer having PSMA expression.

Preferred indications are the detection or staging of cancer, such as, but not limited high grade gliomas, lung cancer and especially prostate cancer and metastasized prostate cancer, the detection of metastatic disease in patients with primary prostate cancer of intermediate-risk to high-risk, and the detection of metastatic sites, even at low serum PSA values in patients with biochemically recurrent prostate cancer. Another preferred indication is the imaging and visualization of neoangiogensis.

Also provided is a compound of the invention as defined herein above for use in a method of diagnosing and/or staging cancer, preferably prostate cancer.

Also provided is a pharmaceutical or diagnostic composition comprising or consisting of one or more compounds of Formula (1a), (1b), (1c) or (1d). The compounds of the invention may be for use as a cancer diagnostic or imaging agent. Accordingly also provided is a method of imaging and/or diagnosing cancer comprising administering a compound of Formula (1a), (1b), (1c) or (1d) or a composition comprising a compound of Formula (1a), (1b), (1c) or (1d). The compounds or compositions of the invention may be for use in the treatment of cancer. The compounds or compositions of the invention may be for use in the diagnosis, imaging or prevention of neoangiogenesis/angiogenesis. The compounds or compositions of the invention may be for use as a cancer diagnostic or imaging agent or for use in the treatment of cancer. The compounds or compositions of the invention may be for use as a cancer diagnostic or imaging agent or for use in the treatment of cancer wherein the cancer is prostate, breast, lung, colorectal or renal cell carcinoma.

The term "treatment", in relation to the uses of any of the compounds described herein, including those of Formula (1a), (1b), (1c) and (1d) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect.

Terms such as "alkyl", "hydrocarbon" and "heterocyclic" are all used in their conventional sense (e.g. as defined in the IUPAC Gold Book), unless indicated otherwise. "optionally substituted" as applied to any group means that the said group may if desired be substituted with one or more substituents, which may be the same or different.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts as well as salt forms arising due to the presence of the chelated nonradioactive or radioactive cation. Such salts may be formed by conventional means, for example by reaction of a free acid or a free base form of a compound with one or more equivalents of an appropriate acid or base, optionally in a solvent, or in a medium in which the salt is insoluble, followed by removal of said solvent, or said medium, using standard techniques (e.g. in vacuo, by freeze-drying or by filtration). Salts may also be prepared by exchanging a counter-ion of a compound in the form of a salt with another counter-ion, for example using a suitable ion exchange resin.

Beyond the suitable chelated nonradioactive or radioactive cations described herein above, further examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), $\alpha$-oxoglutaric, glycolic, hippuric, hydrobromic, hydrochloric, hydriodic, isethionic, lactic (e.g. (+)-L-lactic and ($\pm$)-DL-lactic), lactobionic, maleic, malic (e.g. (-)-L-malic), malonic, ($\pm$)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed

by the incorporation into the solid state structure (e.g. crystal structure) of the compounds of the invention of molecules of a non-toxic pharmaceutically acceptable solvent (referred to below as the solvating solvent). Examples of such solvents may include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The compounds of the invention may contain one or more isotopic substitutions, and a reference to a particular element includes within its scope all isotopes of the element. For example, a reference to hydrogen includes within its scope 1H, 2H (D), and 3H (T). Similarly, references to carbon and oxygen include within their scope respectively 12C, 13C and 14C and 160 and 180. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

## PREPARATION OF THE COMPOUNDS OF THE INVENTION

Some compounds of Formula (1a), (1b), (1c) and (1d) and derivatives or synthetic intermediates thereof can be prepared in accordance with synthetic methods known to the skilled person. In some embodiments, the invention provides a process for the preparation of a compound as defined in Formula (1a), (1b), (1c) and (1d). Certain compounds of the invention may be prepared according to the methods described below.

PSMA ligands containing modified binding motifs (Figure 1) were synthesized according to known or modified organic chemical synthesis procedures. On-resin synthesis of binding motifs was established and adjusted in individual cases. Peptide chain elongation was performed according to standard solid phase peptide synthesis protocols for PSMA derivatives and optimizations concerning (radio)metal complexation reactions were performed if necessary. The following sections cover the syntheses of compounds 2 to 11, highlighting special synthetic aspects, improvements to already described procedures as well as methods for preservation of the mandatory L-configuration of the PSMA-binding motif during inhibitor modification.

### General Information

All reagents were purchased from Merck KGaA (Darmstadt, Germany), Sigma-Aldrich Chemie GmbH (Steinheim, Germany), VWR International GmbH (Darmstadt, Germany), TCI (Eschborn, Germany), Iris Biotech (Marktredwitz, Germany) and Carbolution (St. Ingbert, Germany) in the quality grade "for synthesis". Racemic 2-PMPA was purchased from Bio-Techne GmbH (Wiesbaden-Nordenstadt, Germany). Chematech (Dijon, France) delivered the chelator DOTA and derivatives thereof. Cell culture media and buffer solutions were purchased from Merck KGaA (Darmstadt, Germany) and Sigma Aldrich Chemie GmbH (Steinheim, Germany). ([$^{125}$I]NaI was purchased from Hartmann Analytic (Braunschweig, Germany), n.c.a. [$^{177}$Lu]$LuCl_3$ was delivered by ITG (Garching, Germany). Solvents were purchased from VWR International GmbH (Darmstadt, Germany) in the quality grade "HPLC grade" and used for column chromatography or liquid-liquid extraction. Dry solvents were purchased from Sigma-Aldrich Chemie GmbH (Steinheim, Germany), Alfa Aesar (Karlsruhe, Germany) and VWR International GmbH (Darmstadt, Germany). Silica gel (high purity grade, 60 Å, 0.040 - 0.063 particle size) used for column chromatography was purchased from Sigma-Aldrich Chemie GmbH (Steinheim, Germany). Solid phase synthesis of the peptides was carried out by manual operation using an Intelli-Mixer syringe shaker (Neolab, Heidelberg, Germany).

### Materials

*Analytical* and *preparative* RP-HPLC were performed using Shimadzu gradient systems (Shimadzu Deutschland GmbH, Neufahrn, Germany), each equipped with a SPD-20A UV/Vis detector (220 nm, 254 nm). All systems were operated by the LabSolutions software. Prior to quality control acquisitions, a control run was performed, in which only water/acetonitrile (1/1) was injected. Thereby, the system was checked for impurities in the injection port or on the column.

As different eluents and flow rates have been used for several compounds, the used methods are cited in the

text and described as follows:

Method A: solvent A = water + 0.1% TFA, solvent B = acetonitrile + 2% water + 0.1% TFA
Method B: solvent A = water + 0.1% TFA, solvent B = acetonitrile + 5% water + 0.1% TFA
Method C: solvent A = water, solvent B: acetonitrile + 5% water
Method D: solvent A = water, solvent B: acetonitrile

*Analytical RP-HPLC* was performed on a Nucleosil 100-C18 (5 μm, 125 mm x 4.6 mm) column (CS GmbH, Langerwehe, Germany) applying different linear solvent gradients (Method A) and a constant flow rate of 1 mL/min. Both, specific gradients and the corresponding retention times $t_R$ as well as capacity factor *k* are cited in the text. The capacity factor was calculated from the experimentally determined dead time ($t_0$ = 1.6 min) of the HPLC system and the respective retention time $t_R$:

$$k = \frac{t_R - t_0}{t_0}$$

*Preparative RP-HPLC* was performed on a Multospher 100 RP C18 (5 μm, 250 x 20 mm) column (CS GmbH, Langerwehe, Germany) applying different linear solvent gradients (Method B or C) and different constant flow rates of 5, 8, 9 or 10 mL/min.

*Flash chromatography* was performed with a Biotage gradient HPLC system (Biotage Europe, Uppsala, Sweden), using HP-Sphere C18-25 catridges (micron spherical silica, Biotage SNAP Ultra C18, 12 g). The compounds were eluted applying different solvent gradients (Method D).

*Radio-RP-HPLC* was performed on a Nucleosil 100-C18 (5 μm, 125 mm x 4.6 mm) column (CS GmbH, Langerwehe, Germany) using a Shimadzu gradient system (Shimadzu Deutschland GmbH, Neufahrn, Germany) with a linear solvent gradient (Method A) and a constant flow rate of 1 mL/min. For radioactivity detection, the outlet of the UV detector was connected to a HERM LB 500 NaI detector (Berthold Technologies, Bad Wildbad, Germany). For metabolite analysis a FlowStar$^2$ LB 514 detector (Berthold Technologies, Bad Wildbad, Germany) was additionally connected to the HERM detector.

*Mass spectra* were acquired with an Advion expression L compact mass spectrometer (Advion Ltd., Harlow, UK) with electrospray ionization (positive ion mode) and an orthogonal ion sampling from the heated capillary. The system was operated by the Mass Express software and spectra were processed using the Data Express software.

All *$^1$H- and $^{13}$C-NMR spectra* were measured at room temperature in either DMSO-$d_6$ or $CDCl_3$ on Bruker (Rheinstetten, Germany) instruments (AHV HD-300, AHV HD-400). Chemical shifts ($\delta$) are reported in parts per million (ppm) and calibrated on the residual solvent signal (DMSO-$d_6$: 2.50 ppm for $^1$H and 39.5 ppm for $^{13}$C, $CDCl_3$: 7.26 ppm for $^1$H and 77.0 ppm for $^{13}$C). Multiplicities are described as follows: s = singlet, d = doublet, t = triplet, q = quartet, br = broad singlet m = multiplet.

*Analytical thin layer chromatography* (TLC) was carried out on precoated silica gel plates form Merck KGaA (Darmstadt, Germany) (TLC plates silica gel 60 F254). Substance spots were visualized either via UV illumination at 254 nm or with a 0.75% (m/v) potassium permanganate stain solution.

*Radio-thin layer chromatography* (TLC) was carried out with a Scan-RAM detector (LabLogic Systems, Sheffield, United Kingdom). Cellulose strips were used for citrate-buffer (0.1 M disodium citrate sesquihydrate in $H_2O$). Normal-phase TLC plates (Silica gel 60 RP-18 $F_{254}$s) were used for analyses in $NH_4OAc$ buffer (1.0 M $NH_4OAc$ in $H_2O$)/DMF (1/1).

*Activity measurements* of the respective probes obtained from competitive binding assays, internalization assays, log D determinations, biodistribution or metabolism studies were measured by a 2480 Wizard$^2$ automatic γ-counter (PerkinElmer, Waltham, USA).

**Methods**

General remarks on peptide synthesis

The used equivalents of the reactants for the solid phase synthesis refer to the calculated load after attaching the first amino acid onto the resin. The specific loads are cited in the text. Prior to any reaction, dry resin was swelled in NMP for at least 30 min and then filtered. Unless otherwise indicated, the resin was washed with DMF (6x) after each reaction step. For storage, the resin was washed with DMF (3x) and DCM (3x) and dried in a desiccator.

General procedure for loading the first amino acid onto 2-CTC resin (GP1)

The first amino acid (1.50 eq.) and DIPEA (1.33 eq.) were dissolved in DCM (5.00 mL) and stirred for 5 min at r.t. prior to addition of the resin (1.00 eq.). After 15 min further DIPEA (2.67 eq.) was added and the reaction mixture was stirred for 75 min. Afterwards, MeOH (2 mL) was added and stirred for 15 min. The resin was washed successively with MeOH (4x), DMF (4x) and DCM (4x) and dried at least two hours or overnight in a desiccator. The load was calculated using the following formula:

$$\text{load} = \frac{(m_2 - m_1) \cdot 1000}{(M_W - M_{HCl}) \cdot m_2} \left[\frac{\text{mmol}}{\text{g}}\right]$$

$M_W$ = molecular weight of the amino acid [g/mol]
$M_{HCl}$ = molecular weight of HCl [g/mol]
$m_1$ = mass of dry 2-CTC resin before coupling [g]
$m_2$ = mass of dried resin after coupling [g]

General procedure for on-resin peptide bond formation (GP2)

To a solution of TBTU (2.00 eq.), HOAt (2.00 eq.) and the amino acid (2.00 eq.) in DMF (~ 10 mL/g resin), DIPEA (6.00 - 9.00 eq.) was added to adjust the pH value to 9 - 10 and the mixture was allowed to preactivate for five minutes. In the case of Fmoc-D-Dap(Dde)-OH *sym*-collidine (6.00 - 8.00 eq.) was added instead of DIPEA. Unless otherwise noted, the solution was added to the resin-coupled peptide and shaken for 2 h at room temperature. Afterwards the resin was washed with DMF (6x).

General procedure for the on-resin Fmoc-removal (GP3)

The resin was shaken 5 × 5 min in 20% piperidine in DMF (v/v) to remove the Fmoc-protective group and afterwards washed with DMF (7x). If Ornithin was the first amino acid bound to the resin, Fmoc-removal was performed 12 x 5min in 20% piperidine.

General procedures for the on-resin Dde-removal (GP4 & GP5)

GP4: If no Fmoc-group was present in the resin bound peptide, the resin was treated with 2% hydrazine in DMF (10 mL) for 20 min and afterwards washed with DMF (7x).
GP5: If an Fmoc-group was present in the resin bound peptide, the resin was treated with a solution of imidazole (0.46 g/g resin) and hydroxylamine hydrochloride (0.63 g/g resin) in NMP (5.0 mL/g resin) and DMF (1.0 mL/g resin) for 2 x 3 h. Afterwards, the resin was washed with DMF (7x).

General procedures for monitoring the reaction progress (GP6 & GP7)

For a test cleavage with TFA (GP6) a small aliquot of the resin was taken and treated with 100 μL of TFA for 15 min at r.t. in an Eppendorf tube.
To avoid cleavage of *tert*-butyl groups or formation of other unidentifiable by-products, test cleavage with HFIP/DCM (GP7) was used. The resin aliquot was treated with 100 μL of HFIP/DCM (1/4, v/v) for 30 min at r.t.
For both procedures the respective solution (without beads!) was transferred into another Eppendorf tube and the solvent was evaporated under a stream of nitrogen. The residue was dissolved in a mixture of $H_2O$ and MeCN (1/1, v/v), now ready for RP-HPLC analysis.

Cleaving the peptide off the resin with simultaneous removal of all acid-labile protective groups (GP8)

The resin was treated with TFA/TIPS/DCM (95/2.5/2.5, 10.0 mL) twice for 30 min at r.t. and washed with DCM afterwards (3x). The solvent was evaporated under $N_2$ flow and after lyophilisation the crude product was obtained.

General procedure for reactions under air- and moisture free conditions (GP9)

The used Schlenk flask, further glassware as well as the agitator were heated properly three times under vacuum ($2.0 \times 10^{-3}$ - $8.0 \times 10^{-3}$ mbar) prior to the reaction. The apparatus was flushed with argon after each heating cycle. Only dry solvents and dry reagents were used for the reactions. The addition of reagents or reactants to the reaction mixture was only performed under argon counterflow. Probes for HPLC control were also only taken under argon counterflow. If the reaction mixture was heated or evolution of gas was expected, the stop cock at the top of the apparatus was replaced by a balloon.

**Abbreviations**

| | |
|---|---|
| 2-Aha | 2-aminoheptanoic acid |
| 2-Aoc | 2-aminooctanoic acid |
| 2-CT | 2-chlorotrityl |
| 2-PMPA | 2-(phosphonomethyl)pentane-1,5-dioic acid |
| $A_m$ | molar activity |
| BOP | (benzotriazol-1-yloxy)tris(dimethylamino)phosphonium hexafluorophosphate |
| BSA | bovine serum albumin |
| Bzl | benzyl |
| CAN | cerium(IV) ammonium nitrate |
| Cbz | carbobenzoxy |
| CDI | 1,1'-carbonyldiimidazole |
| CT | computed tomography |
| Dap | 2,3-diaminopropionic acid |
| DCE | 1,2-dichloroethane |
| DCM | dichloromethane |
| Dde | *N*-1-(4,4-dimethyl-2,6-dioxocyclohex-1-ylidene)ethylamine |
| DIAD | diisopropyl azodicarboxylate |
| DIPEA | *N,N*-diisopropylethylamine |
| DMAP | 4-dimethylaminopyridine |
| DMEM | Dulbecco's Modified Eagle's Medium |
| DMF | dimethylformamide |
| DMSO | dimethyl sulfoxide |
| DOTA | 1,4,7,10-tetraazacyclododecane-1,4,7,10-tetraacetic acid |
| EDC | *N*-(3-dimethylaminopropyl)-*N'*-ethylcarbodiimide |
| FWHM | full width at half maximum |
| HBSS | Hank's buffered salt solution |
| HFIP | 1,1,1,3,3,3-hexafluoro-2-propanol |
| HOAt | 1-hydroxy-7-azabenzotriazole |
| $IC_{50}$ | half maximal inhibitory concentration |
| ID/g | injected dose per gram |
| MeCN | acetonitrile |
| MeOH | methanol |
| NHS | *N*-hydroxysuccinimide |
| NMP | *N*-methyl-2-pyrrolidone |
| OI | optical imaging |
| PBS | phosphate-buffered saline |
| PET | positron emission tomography |
| $PPh_3$ | triphenylphosphane |
| PSMA | prostate-specific membrane antigen |
| RCP | radiochemical purity |
| RCY | radiochemical yield |
| RIPA | radioimmunoprecipitation assay |
| RP-HPLC | reversed-phase high-performance liquid chromatography |
| r.t. | room temperature |
| SiFA-BA | silicon-based fluoride acceptor-benzoic acid |

| | |
|---|---|
| SPECT | single-photon emission computed tomography |
| TBTU | 2-(1*H*-benzotriazole-1-yl)-1,1,3,3-tetramethylaminium tetrafluoroborate |
| *t*Bu | *tert*-butyl |
| *t*BuOH | *tert*-butanol |
| TEA | triethylamine |
| TFA | trifluoroacetic acid |
| TIPS | triisopropylsilane |
| TLC | thin layer chromatography |
| $TMSN_3$ | trimethylsilyl azide |

**Synthesis, cold complexation and radiolabeling of ligands with different binding motifs**

Reference compound PSMA-10 (1) in its free chelator form was synthesized according to a previously published protocol (Wurzer A. et al. Journal of Nuclear Medicine 2020, 61(5), 735-42). Preparation of $^{nat}$Lu-**1** and [$^{177}$Lu]Lu-**1** followed similar procedures to those conducted in the literature (WO2019/020831). Hence, their analytical data can be found elsewhere and are not again listed. $IC_{50}$ data for $^{nat}$Lu-**1** as well as internalization, log D, biodistribution and μSPECT/CT data of [$^{177}$Lu]Lu-**1** were not adopted from previously published studies. Instead, they were again determined to ensure a valid comparability of the obtained results and to investigate salivary gland uptake of the reference [$^{177}$Lu]Lu-**1** at 24 h p.i.

**Synthesis of PSMA derivatives containing modifications within the central $Zn^{2+}$-binding unit**

**Thioureate 2**

Di-tert-butyl (1H-imidazole-1-carbonothioyl)-L-glutamate (**12**)

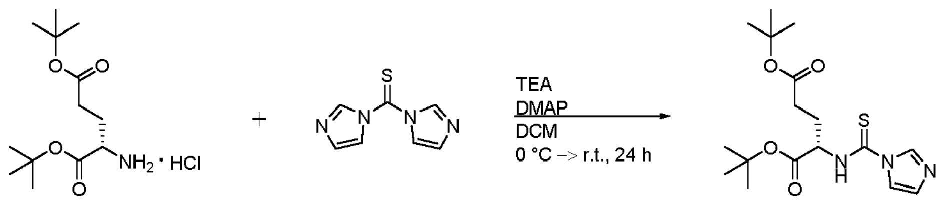


S-1
Exact Mass: 295,16
Molecular Weight: 295,81

S-2
Exact Mass: 178,03
Molecular Weight: 178,21

12
Exact Mass: 369,17
Molecular Weight: 369,48

Compound **S-1** was dissolved in dry DCM (14.9 mL) and cooled to 0 °C. Triethylamine (1.79 mL, 12.8 mmol, 2.53 eq.) and DMAP (24.8 mg, 0.20 mmol, 0.04 eq.) were added and stirred for five minutes. Afterwards, 1,1'-thiocarbonyldiimidazole (**S-2**) (1.36 g, 7.61 mmol, 1.50 eq.) was added, the solution was allowed to warm to room temperature and stirred overnight. The mixture was dissolved with ~ 15 mL DCM and washed with $NaHCO_3$ (1x), $H_2O$ (1x) and brine (1x). The organic layer was dried over $Na_2SO_4$, filtered and the solvent removed under reduced pressure. This afforded 1.67 g (89.2%) of crude product **12** as a yellow oil, which was used without further purification in the next step.

Chemical formula: $C_{17}H_{27}N_3O_4S$
Molecular weight: 369.48 g/mol
Exact mass: 369.17 g/mol
$t_R$-value: 13.0 min (10 - 90% B in 15 min, Method A)
Capacity factor k: 6.2
ESI-MS: calculated monoisotopic mass ($C_{17}H_{27}N_3O_4S$): 369.17;
found: ESI (positive ion mode): m/z = 370.2 [M(**12**)+H]$^+$, 411.2 [M(**12**)+MeCN+H]$^+$; $^1$H-NMR (300 MHz, $CDCl_3$) δ(ppm): 8.45 (virt. d, 1H, H(1)), 7.65 (virt. 1H, H(2)), 7.23 (s, 1H, H(3)), 7.18 - 7.01 (m, 1H, H(4)), 4.83 (t, $^3J$ = 5.7 Hz, 1H, H(5)), 2.56 - 2.44 (m, 2H, H(6)), 2.30 - 2.20 (m, 2H, H(7)), 1.48 (s, 9H, H(8, 8', 8")), 1.45 (s, 9H, H(9, 9', 9")).

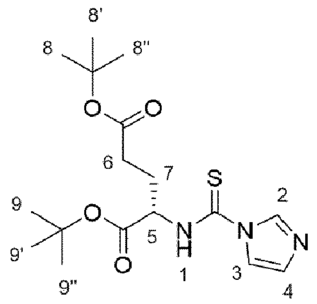

**12**

L-Glu-[D-Orn(Dde)-2-CT]-OtBu (**13**)

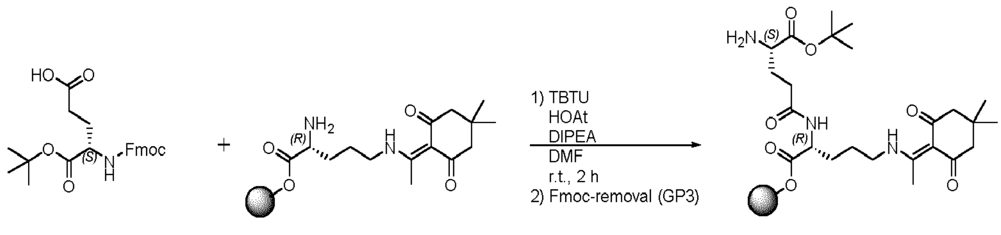


**S-3** Exact Mass: 425,18 Molecular Weight: 425,48

**18** Exact Mass: 296,17 Molecular Weight: 296,37

**13** Exact Mass: 481,28 Molecular Weight: 481,59

Fmoc-D-Orn(Dde)-OH was coupled to 2-CTC resin according to GP1 (load: 0.71 mmol/g, 0.34 mmol, 1.00 eq.). After removal of the Fmoc protective group (GP3), Fmoc-L-Glu-OtBu (**S-3**) (294 mg, 0.69 mmol, 2.00 eq.) was coupled to **18** according to GP2 (2.00 eq. TBTU, 2.00 eq. HOAt, 9.00 eq. DIPEA). After two hours, the Fmoc group of the resin-bound dipeptide was cleaved off, again according to GP3. Some resin beads were taken according to GP6 and analyzed via RP-HPLC, which indicated nearly complete conversion to **13.**

Chemical formula: $C_{24}H_{39}N_3O_7$; -tBu: $C_{20}H_{31}N_3O_7$
Molecular weight: 481.59 g/mol; -tBu: 425.48 g/mol
Exact mass: 481.28 g/mol; -tBu: 425.22 g/mol
$t_R$-value (-tBu): 8.47 min (10 - 90% B in 15 min, Method A)
Capacity factor k (-tBu): 4.6
ESI-MS: calculated monoisotopic mass ($C_{24}H_{39}N_3O_7$): 481.28, -tBu ($C_{20}H_{31}N_3O_7$): 425.22; found: ESI (positive ion mode): m/z = 426.0 [M(**13**)-tBu+H]$^+$.

L-Glu(OtBu)$_2$-(thiocarbonyl)-L-Glu-[D-Orn(Dde)-2-CT]-OtBu (**S-4**)

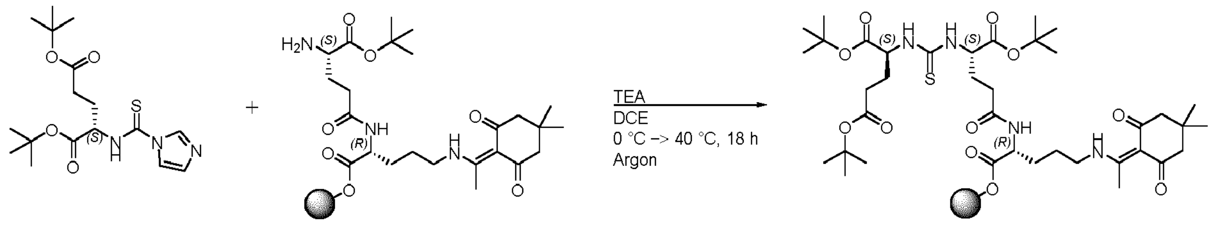


12 Exact Mass: 369,17 Molecular Weight: 369,48

13 Exact Mass: 481,28 Molecular Weight: 481,59

S-4 Exact Mass: 782,41 Molecular Weight: 782,99

Reactant **12** (160 mg of crude product, contain ~ 60% of **12** (RP-HPLC), 0.26 mmol, 1.50 eq.) was dissolved

in 2 mL DCM and cooled to 0 °C. Triethylamine (59.6 μL, 0.43 mmol, 2.50 eq.) and the resin-bound dipeptide **13** (0.17 mmol, 1.00 eq) were added and stirred for five minutes at 0 °C. The reaction mixture was heated to 40 °C and stirred overnight under argon atmosphere. The resin was transferred to a syringe for peptide synthesis (equipped with a frit, pore size 25 μm) and washed with DCM (4x). Some resin beads were taken according to GP7 and analyzed via RP-HPLC, which indicated nearly complete conversion to **S-4.**

Chemical formula: $C_{38}H_{62}N_4O_{11}S$;
Molecular weight: 782.99 g/mol;
Exact mass: 782.41 g/mol;
$t_R$-value: 12.7 min (40 - 100% B in 15 min, Method A)
Capacity factor k: 7.5
ESI-MS: calculated monoisotopic mass ($C_{38}H_{62}N_4O_{11}S$): 782.41;
found: ESI (positive ion mode): m/z = 783.2 [M(**S-4**)+H]$^+$, 805.4 [M(**S-4**)+Na]$^+$, 822.1 [M(**S-4**)+K]$^+$.

Thioureate **2**

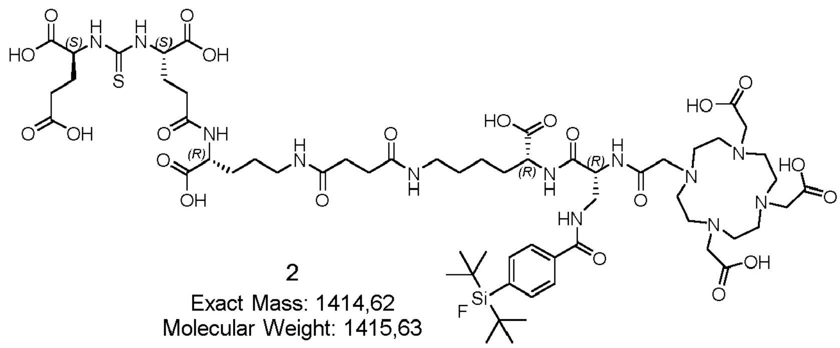

2
Exact Mass: 1414,62
Molecular Weight: 1415,63

Further reactions on resin-bound compound **S-4** were performed according to standard Fmoc-SPPS on 2-CT resin, applying the above-mentioned methods (GP2 - GP8). In brief, the Dde protective group was removed (GP4) and succinic anhydride (119 mg, 1.19 mmol, 7.00 eq.) was coupled (GP2) over a period of at least 2.5 h, only using DIPEA (202 μL, 1.19 mmol, 7.00 eq.) and no further coupling reagents. The peptide was elongated with Fmoc-D-Lys-OtBu*HCl (157 mg, 0.34 mmol, 2.00 eq.). Therefore, the resin-bound acid was preactivated for five minutes using TBTU (109 mg, 0.34 mmol, 2.00 eq.), HOAt (46.3 mg, 0.34 mmol, 2.00 eq.) and DIPEA (173 μL, 1.02 mmol, 6.00 eq.). The amino acid was dissolved in DMF, added to the preactivated resin and shaken for at least 2.5 h. Fmoc-removal was conducted (GP3) and Fmoc-D-Dap(Dde)-OH was coupled for at least 2.5 h using TBTU (109 mg, 0.34 mmol, 2.00 eq.), HOAt (46.3 mg, 0.34 mmol, 2.00 eq.) and sym-collidine (158 μL, 1.19 mmol, 7.00 eq.) as coupling reagents. Afterwards, Dde-removal was performed according to GP5. The SiFA-BA moiety (73.4 mg, 0.26 mmol, 1.50 eq.) was attached using TBTU (109 mg, 0.34 mmol, 2.00 eq.), HOAt (46.3 mg, 0.34 mmol, 2.00 eq.) and sym-collidine (158 μL, 1.19 mmol, 7.00 eq.) as coupling reagents. After an incubation time of at least 2 h, the Fmoc protective group was removed (GP3) and the DOTA chelator moiety was added to half of the resin. Therefore, DOTA*6 $H_2O$ (43.6 mg, 85.0 μmol, 1.00 eq.), TBTU (22.8 mg, 71.0 μmol, 0.84 eq.), HOAt (9.66 mg, 71.0 μmol, 0.84 eq.) and sym-collidine (66.3 μL, 0.50 mmol, 5.88 eq.) were dissolved in a mixture of DMF/DMSO (5/1, v/v) and incubated with the resin-bound amine for 23 h. As RP-HPLC/MS analysis (GP6) revealed successful coupling, the peptide was cleaved off the resin with TFA/TIPS/$H_2O$ (95/2.5/2.5, slightly modified to GP8) (2 x 30 min) and purified afterwards by preparative RP-HPLC (30 - 50% B in 20 min, Method B). Subsequent lyophilization afforded 1.99 mg (1.65% yield) of pure product **2** as a colorless powder.

Chemical formula: $C_{60}H_{95}FN_{12}O_{22}SSi$;
Molecular weight: 1415.63 g/mol;
Exact mass: 1414.62 g/mol;
$t_R$-value: 7.82 min (40 - 100% B in 15 min, Method A)
Capacity factor k: 4.2
ESI-MS: calculated monoisotopic mass ($C_{60}O_{95}FN_{12}O_{22}SSi$): 1414.62;
found: ESI (positive ion mode): m/z = 708.6 [M(**2**)+2H]$^{2+}$, 1415.8 [M(**2**)+H]$^+$.

natGa-thioureate **2** (natGa-**2**)

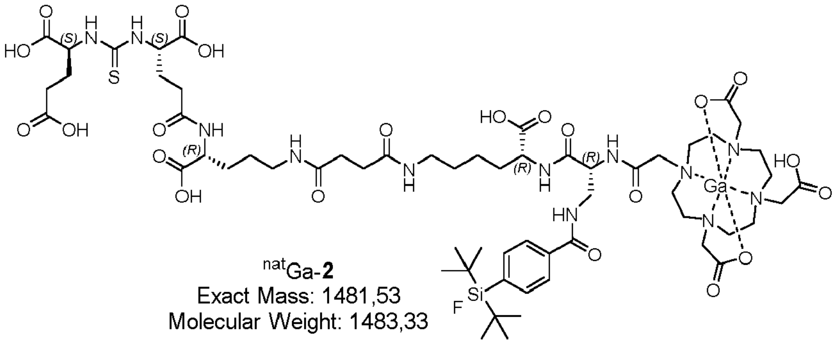

natGa-**2**
Exact Mass: 1481,53
Molecular Weight: 1483,33

Thioureate **2** (1.99 mg, 1.41 μmol, 1.00 eq.) was dissolved in 300 μL of tBuOH, and $Ga(NO_3)_3$*6 $H_2O$ (1.79 mg, 4.92 μmol, 3.50 eq.) dissolved in 100 μL $H_2O$ was added. The mixture was stirred for 30 min at 75 °C and afterwards filtered through single use syringe filters (Sartorius Minisart®) to remove $Ga(OH)_3$ precipitate. The natGa-complex of thioureate **2** (= natGa-**2**) was purified by RP-HPLC (30 - 50% B in 20 min, Method B) and afforded 70.0 μg (3.35%) of pure product natGa-**2** as a colorless powder after lyophilization.

Chemical formula: $C_{60}H_{93}FGaN_{12}O_{22}SSi$;
Molecular weight: 1483.33 g/mol;
Exact mass: 1481.53 g/mol;
$t_R$-value: 8.65 min (10 - 90% B in 15 min, Method A)
Capacity factor k: 4.7
ESI-MS: calculated monoisotopic mass ($C_{60}H_{93}FGaN_{12}O_{22}SSi$): 1481.53;
found: ESI (positive ion mode): m/z = 742.5 [M(natGa-**2**)+2H]$^{2+}$, 1483.0 [M(natGa-**2**)+H]$^+$, 1503.6 [M(natGa-**2**)+Na]$^+$.

**Carbamate I (3)**

Tert-butyl (S)-5-oxotetrahydrofuran-2-carboxylate (**S-6**)

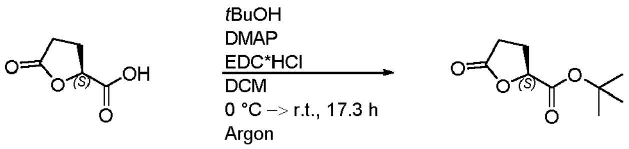


S-5
Exact Mass: 130,03
Molecular Weight: 130,10

S-6
Exact Mass: 186,09
Molecular Weight: 186,21

According to a slightly modified procedure by Zhang et al. (Tetrahedron. 2009, 65(48), 9997-10001), (S)-5-oxotetrahydrofuran-2-carboxylic acid (**S-5**) (1.50 g, 11.5 mmol, 1.00 eq.) was weighed in a 100 mL round bottom flask and dissolved in 36 mL dry DCM. DMAP on polystyrene (1.60 mmol/g, 718 mg, 1.15 mmol, 0.10 eq.) and dry tBuOH (1.40 mL, 15.0 mmol, 1.30 eq.) were added and the reaction mixture was cooled to 0 °C. EDC*HCl (2.87 g, 15.0 mmol, 1.30 eq.) in 12 mL dry DCM was added slowly, the ice bath was removed, the solution was allowed to warm to room temperature and stirred under argon atmosphere for 17.3 h. DMAP on polystyrene was filtered off, the organic layer was washed once with $H_2O$ (spiked with some drops of brine), dried over $Na_2SO_4$ and the solvent was removed under reduced pressure. The crude product was purified by column chromatography (toluene/EtOAc = 3/2) to afford 928 mg (43%) of compound **S-6** as a colorless, crystalline solid.

Chemical formula: $C_9H_{14}O_4$;

Molecular weight: 186.21 g/mol;
Exact mass: 186.09 g/mol;
$t_R$-value: not detectable at 220/254 nm
$R_f$-value: 0.58 (toluene/EtOAc = 3/2)
$^1$H-NMR (300 MHz, $CDCl_3$) δ(ppm): 4.83 - 4.76 (m, 1H, H(1)), 2.68 - 2.39 (m, 3H, H(2, 3, 4)), 2.32 - 2.14 (m, 1H, H(5)), 1.48 (s, 9H, H(6, 6', 6")).
$^{13}$C-NMR (75 MHz, $CDCl_3$) δ(ppm): 176.32 (s, 1C, C(1)), 169.12 ((s, 1C, C(2)), 83.23 (s, 1C, C(3)), 76.40 (s, 1C, C(4)), 28.03 (s, 3C, C(5, 5' 5")), 26.90 (s, 1C, C(6)), 26.00 (s, 1C, C(7)).

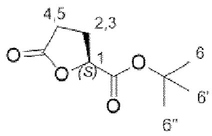

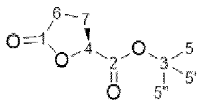

**S-6**

(S)-5-(tert-butoxy)-4-hydroxy-5-oxopentanoic acid (**S-7**)

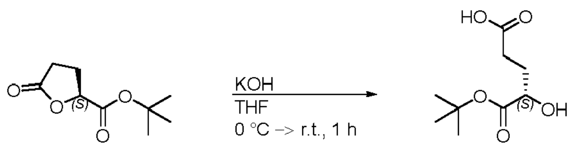


S-6
Exact Mass: 186,09
Molecular Weight: 186,21

S-7
Exact Mass: 204,10
Molecular Weight: 204,22

According to a slightly modified procedure by Zhang et al. (Tetrahedron. 2009, 65(48), 9997-10001), tert-butyl (S)-5-oxotetrahydrofuran-2-carboxylate (**S-6**) (425 mg, 2.28 mmol, 1.00 eq.) was dissolved in 2.40 mL THF. At 0 °C, a 1 M aqueous KOH solution (2.64 mL, 2.64 mmol, 1.16 eq.) was added dropwise over five minutes. The solution was allowed to warm to room temperature and stirred for one hour. As reaction control via TLC revealed almost complete consumption of educt **S-6,** THF was removed under reduced pressure and the pH value of the remaining aqueous layer was adjusted to 3 by adding 2 M HCl. The aqueous residue was extracted with EtOAc (3x), the combined organic phases dried over $Na_2SO_4$, filtered and the solvent removed under reduced pressure. This afforded 381 mg of crude product **S-7** as a colorless solid, which was used without further purification in the next step.

Chemical formula: $C_9H_{16}O_5$;
Molecular weight: 204.22 g/mol;
Exact mass: 204.10 g/mol;
$t_R$-value: not detectable at 220/254 nm
$R_f$-value: 0.0 (toluene/EtOAc = 3/2)

Di-tert-butyl (S)-2-hydroxypentanedioate (**14**)

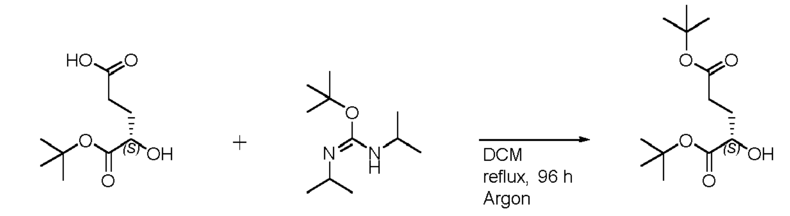


S-7
Exact Mass: 204,10
Molecular Weight: 204,22

S-8
Exact Mass: 200,19
Molecular Weight: 200,33

14
Exact Mass: 260,16
Molecular Weight: 260,33

Compound **14** was synthesized in analogy to a previously published procedure by Bergmeier et al. (J Org Chem. 1993, 58(9), 2369-76) with some minor modifications. Glassware and reagents were handled under air- and moisture-free conditions (GP9). Lyophilized educt **S-7** (crude product, -100 mg, 0.49 mmol, 1.00 eq.) was dissolved in 5 mL dry DCM and the first portion of O-tert-butyl-N,N'-diisopropylisourea (**S-8**) (162 μL, 0.73 mmol, 1.50 eq.) was added. The reaction mixture was stirred under reflux (~42 °C) and argon atmosphere for 24 h. A second portion of **S-8** (162 μL, 0.73 mmol, 1.50 eq.) was added and also DCM, in order to keep the solvent amount constantly between 3 and 5 mL. After stirring for further 72 h at reflux temperature and under argon atmosphere, the reaction was terminated by diluting the suspension with DCM. Solid by-products were removed by filtration and the solvent removed under reduced pressure. Purification by column chromatography (PE/EtOAc = 9/1) provided 24.6 mg (19%) of compound **14** as a colorless liquid.

Chemical formula: $C_{13}H_{24}O_5$;
Molecular weight: 260.33 g/mol;
Exact mass: 260.16 g/mol;
$t_R$-value: not detectable at 220/254 nm
$R_f$-value: 0.65 (PE/EtOAc = 9/1) $^1$H-NMR (300 MHz, $CDCl_3$) δ(ppm): 4.07 (dd, $^3J$ = 8.0, 4.1 Hz, 1H, H(1)), 2.88 (s, 1H, H(2)), 2.53 - 2.24 (m, 2H, H(3)), 2.08 (virt. dddd, 1H, H(4)), 1.93 - 1.73 (m, 1H, H(5)), 1.49 (s, 9H, H(6, 6', 6")), 1.44 (s, 9H, H(7, 7' 7")).
$^{13}$C-NMR (75 MHz, $CDCl_3$) δ(ppm): 174.22 (s, 1C, C(1)), 172.72 (s, 1C, C(2)), 82.80 (s, 1C, C(3)), 80.59 (s, 1C, C(4)), 69.89 (s, 1C, C(5)), 31.13 (s, 1C, C(6)), 29.82 (s, 1C, C(7)), 28.25 (s, 3C, C(8, 8', 8")), 28.17 (s, 3C, C(9, 9', 9")).

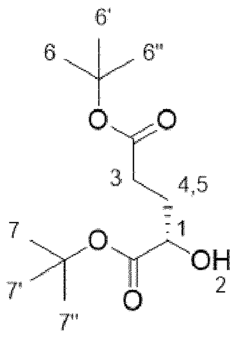

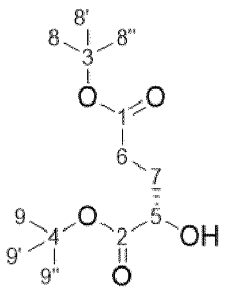

14

Di-tert-butyl (S)-2-((1H-imidazole-1-carbonyl)oxy)pentanedioate (**S-10**)

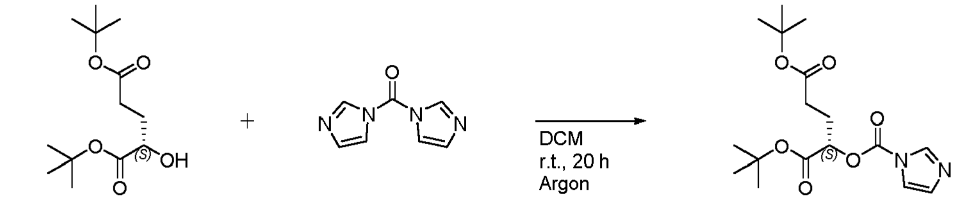


14
Exact Mass: 260,16
Molecular Weight: 260,33

S-9
Exact Mass: 162,05
Molecular Weight: 162,15

S-10
Exact Mass: 354,18
Molecular Weight: 354,40

Compound **S-10** was synthesized in analogy to a previously published procedure by Yang et al. (Journal of Medicinal Chemistry. 2016, 59(1), 206-18), with some minor modifications. Di-tert-butyl (S)-2-hydroxypentanedioate (**14**) (398 mg, 1.53 mmol, 1.00 eq.) was dissolved in 25 mL dry DCM and stirred at room temperature. 1,1'-Carbonyldiimidazole (**S-9**) (478 mg, 2.95 mmol, 1.93 eq.) was added to the solution, which was then stirred under argon atmosphere for 20 h at room temperature. The reaction mixture was diluted with DCM and washed once with $H_2O$. The aqueous layer was extracted two times with DCM, the combined organic phases were dried over $Na_2SO_4$, filtered and the solvent evaporated in vacuo. This afforded 615 mg (>99%) of crude product as slightly yellow solid. RP-HPLC/MS control revealed nearly complete conversion to product **S-10,** which was used in the next step without further purification.

Chemical formula: $C_{17}H_{26}N_2O_6$;
Molecular weight: 354.40 g/mol;
Exact mass: 354.18 g/mol;
$t_R$-value: 10.2 min (40 - 100% B in 15 min, Method A)
Capacity factor k: 5.8
ESI-MS: calculated monoisotopic mass ($C_{17}H_{26}N_2O_6$): 354.18;
found: ESI (positive ion mode): m/z = 355.2 [M(**S-10**)+H]$^+$, 396.2 [M(**S-10**)+MeCN+H]$^+$.

5-Benzyl 1-(tert-butyl) ((((S)-1,5-di-tert-butoxy-1,5-dioxopentan-2-yl)oxy)carbonyl)-L-glutamate (**S-12**)

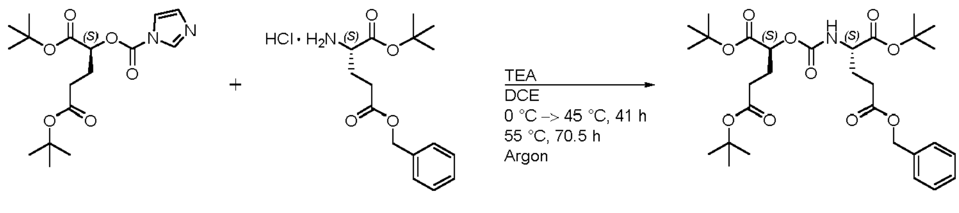


**S-10**
Exact Mass: 354,18
Molecular Weight: 354,40

**S-11**
Exact Mass: 329,14
Molecular Weight: 329,82

**S-12**
Exact Mass: 579,30
Molecular Weight: 579,69

Compound **S-12** was synthesized in analogy to a previously published procedure by Weineisen et al. (EJNMMI Research. 2014, 4(1), 63). with some minor modifications. Di-tert-butyl (S)-2-((1H-imidazole-1-carbonyl)oxy)pentanedioate (**S-10**) (542 mg, 1.53 mmol, 1.00 eq.) was dissolved in 7.29 mL DCE and cooled to 0 °C. H-L-Glu(OBzl)-OtBu*HCl (**S-11**) (1.01 g, 3.06 mmol, 2.00 eq.) and triethylamine (531 μL, 3.83 mmol, 2.50 eq.) were added and stirred for further five minutes at 0 °C. The reaction mixture was warmed to 45 °C and stirred for 41 h under argon atmosphere. As RP-HPLC/MS analysis revealed very low conversion (4.4% **S-12,** 44% **S-10),** again, H-L-Glu(OBzl)-OtBu*HCl (**S-11**) (1.01 g, 3.06 mmol, 2.00 eq.) and triethylamine (531 μL, 3.83 mmol, 2.50 eq.) were added and the temperature was increased to 55 °C. Complete consumption of educt **S-10** was observed after 70.5 h at 55 °C under argon atmosphere. DCM was added for dilution of the reaction mixture and washed once with $H_2O$ (+ 1 mL brine). The aqueous phase was extracted two times with DCM, the combined organic layers were dried over $Na_2SO_4$, filtered and the solvent was removed under reduced pressure. The obtained crude product was purified by preparative RP-HPLC (70 - 80% B in 20 min, Method B, 5 mL/min), which gave 277 mg (31.3%) of compound **S-12** as a colorless oil.

Chemical formula: $C_{30}H_{45}NO_{10}$;
Molecular weight: 579.69 g/mol;

Exact mass: 579.30 g/mol;
$t_R$-value: 12.6 min (40 - 95% B in 15 min, Method A)
Capacity factor k: 7.4
ESI-MS: calculated monoisotopic mass ($C_{30}H_{45}NO_{10}$): 579.30;
found: ESI (positive ion mode): m/z = 412.3 [M(**S-12**)-3tBu+H]$^+$, 468.3 [M(**S-12**)-2tBu+H]$^+$, 524.4 [M(**S-12**)-tBu+H]$^+$, 580.5 [M(**S-12**)+H]$^+$, 597.5 [M(**S-12**)+$H_2O$+H]$^+$,
602.5 [M(**S-12**)+Na]$^+$, 618.5 [M(**S-12**)+K]$^+$;
$^1$H-NMR (300 MHz, $CDCl_3$) δ(ppm): 7.44 - 7.30 (m, 5H, H(1, 1', 1", 1'", 1"")), 5.44 (d, $^3$J = 8.3 Hz, 1H, H(2)), 5.12 (s, 2H, H(3)), 4.84 (dd, $^3$J = 8.1, 4.6 Hz, 1H, H(4)), 4.27 (td, $^3$J = 8.2, 4.9 Hz, 1H, H(5)), 2.60 - 2.41 (m, 2H, H(6)), 2.41 - 2.28 (m, 2H, H(7)), 2.28 - 1.82 (m, 4H, H(8, 9)), 1.46 (s, 9H, H(10, 10', 10")), 1.44 (s, 9H, H(11, 11', 11")), 1.44 (s, 9H, H(12, 12', 12")).
$^{13}$C-NMR (75 MHz, $CDCl_3$) δ(ppm): 172.69 (s, 2C, C(1, 2)), 171.94 (s, 1C, C(3)), 170.95 (s, 1C, C(4)), 155.22 (s, 1C, C(5)), 136.02 (s, 1C, C(6)), 128.67 (s, 2C, C(7, 7')), 128.34 (s, 3C, C(8, 9, 9')), 82.71 (s, 1C, C(10)), 82.31 (s, 2C, C(11, 12)), 80.75 (s, 1C, C(13)), 66.57 (s, 1C, C(14)), 53.82 (s, 1C, C(15)), 31.24 (s, 1C, C(16)), 30.24 (s, 1C, C(17)), 28.23 (s, 3C, C(18, 18', 18")), 28.12 (s, 3C, C(19, 19', 19")), 28.09 (s, 3C, C(20, 20', 20")), 26.80 (s, 2C, C(21, 22)).

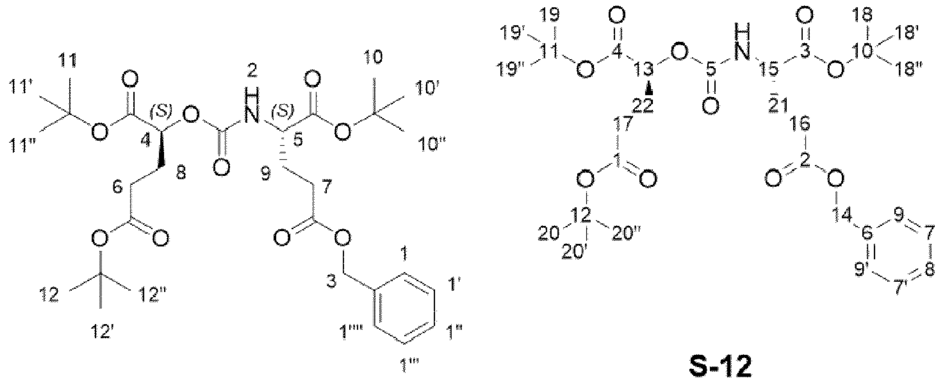

**S-12**

(S)-5-(tert-butoxy)-4-(((((S)-1,5-di-tert-butoxy-1,5-dioxopentan-2-yl)oxy)carbonyl)amino)-5-oxopentanoic acid (**15**)

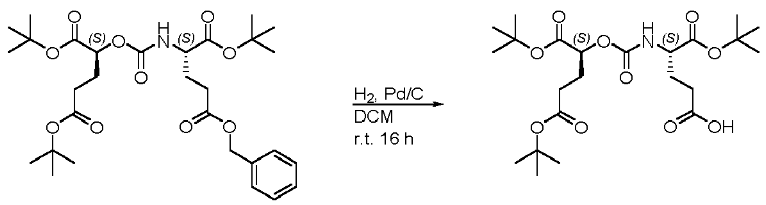


**S-12**
Exact Mass: 579,30
Molecular Weight: 579,69

**15**
Exact Mass: 489,26
Molecular Weight: 489,56

Compound **S-12** (139 mg, 0.24 mmol, 1.00eq.) was dissolved in 12 mL DCM and the solution was flushed 5 minutes with argon to remove dissolved oxygen. 25.5 mg of palladium on carbon (10% wt) (corresponds to 2.55 mg palladium, 24.0 μmol, 0.10 eq.) were added and the flask was sealed with a rubber septum. Remaining air was displaced with argon and subsequently replaced by hydrogen gas. The mixture was stirred under hydrogen atmosphere at room temperature for 16 h. Palladium on carbon was filtered off, and the solvent was evaporated in vacuo. This afforded 59.2 mg (50.4%) of compound **15** as a colorless clear oil, which was used in the next step without further purification.

Chemical formula: $C_{23}H_{39}NO_{10}$;
Molecular weight: 489.56 g/mol;
Exact mass: 489.26 g/mol;
$t_R$-value: 8.80 min (40 - 95% B in 15 min, Method A)
Capacity factor k: 4.9

ESI-MS: calculated monoisotopic mass ($C_{23}H_{39}NO_{10}$): 489.26;
found: ESI (positive ion mode): m/z = 304.2 [M(**15**)-3tBu-OH]$^+$, 322.3 [M(**15**)-3tBu+H]$^+$, 378.3 [M(**15**)-2tBu +H]$^+$, 434.3 [M(**15**)-tBu +H]$^+$, 490.5 [M(**15**)+H]$^+$,
507.5 [M(**15**)+$H_2$O+H]$^+$, 512.5 [M(**15**)+Na]$^+$, 528.4 [M(**15**)+K]$^+$.

[S-2-oxopentanedioic acid(OtBu)$_2$]-(carbonyl)-L-Glu-[D-Orn(Dde)-2-CT]-OtBu (**S-13**)

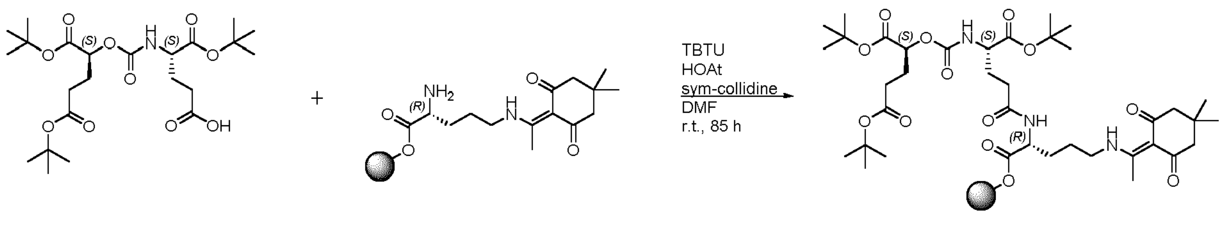


**15** Exact Mass: 489,26 Molecular Weight: 489,56

**18** Exact Mass: 296,17 Molecular Weight: 296,37

**S-13** Exact Mass: 767,42 Molecular Weight: 767,91

According to GP2, fragment **15** (59.2 mg, 0.12 mmol, 1.10 eq.) was coupled to resin-bound H-D-Orn(Dde) (**18**) (0.11 mmol 1.00 eq.), with TBTU (70.6 mg, 0.22 mmol, 2.00 eq.) and HOAt (29.9 mg, 0.22 mmol, 2.00 eq.) as coupling reagents and sym-collidine (131 μL, 0.99 mmol, 9.00 eq.) as base. After shaking for 85 h at room temperature, formation of product **S-13** could be confirmed (GP7). Additionally, some resin beads were taken, treated for 20 minutes with 2% hydrazin/DMF (v/v) and analyzed via RP-HPLC/MS after a test cleavage according to GP7. Thus, stability of the carbamate moiety towards Dde-removal conditions was confirmed, as only one major peak (besides DMF) with the expected m/z-ratio of 604.6 occurred.

Chemical formula: $C_{38}H_{61}N_3O_{13}$;
Molecular weight: 767.91 g/mol;
Exact mass: 767.42 g/mol;
$t_R$-value: 9.21 min (40 - 95% B in 15 min, Method A)
Capacity factor k: 5.1
ESI-MS: calculated monoisotopic mass ($C_{38}H_{61}N_3O_{13}$): 767.42;
found: ESI (positive ion mode): m/z = 768.7 [M(**S-13**)+H]$^+$, 790.6 [M(**S-13**)+Na]$^+$, 806.6 [M(**S-13**)+K]$^+$;

Carbamate I (**3**)

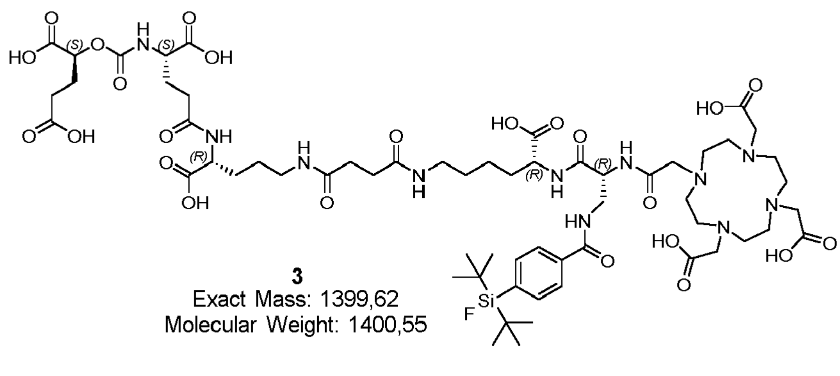

**3**
Exact Mass: 1399,62
Molecular Weight: 1400,55

Further reactions on resin-bound compound **S-13** were performed according to standard Fmoc-SPPS on 2-CT resin, applying the above-mentioned methods (GP2 - GP8). In brief, the Dde protective group was removed (GP4) and succinic anhydride (77.1 mg, 0.77 mmol, 7.00 eq.) was coupled (GP2) over a period of at least 2.5 h, only using DIPEA (131 μL, 0.77 mmol, 7.00 eq.) and no further coupling reagents. The peptide was elongated with Fmoc-D-Lys-OtBu*HCl (101 mg, 0.22 mmol, 2.00 eq.). Therefore, the resin-bound acid was preactivated for five minutes using TBTU (70.6 mg,

0.22 mmol, 2.00 eq.), HOAt (29.9 mg, 0.22 mmol, 2.00 eq.) and DIPEA (112 μL, 0.66 mmol, 6.00 eq.). The amino acid was dissolved in DMF, added to the preactivated resin and shaken for at least 2.5 h. Fmoc-removal was conducted (GP3) and Fmoc-D-Dap(Dde)-OH was coupled for at least 2.5 h using TBTU (70.6 mg, 0.22 mmol, 2.00 eq.), HOAt (29.9 mg, 0.22 mmol, 2.00 eq.) and sym-collidine (102 μL, 0.77 mmol, 7.00 eq.) as coupling reagents. Afterwards, Dde-removal was performed according to GP5. The SiFA-BA moiety (46.6 mg, 0.17 mmol, 1.50 eq.) was attached using TBTU (70.6 mg, 0.22 mmol, 2.00 eq.), HOAt (29.9 mg, 0.22 mmol, 2.00 eq.) and sym-collidine (102 μL, 0.77 mmol, 7.00 eq.) as coupling reagents. After an incubation time of at least 2 h, the Fmoc protective group was removed (GP3) and the DOTA chelator moiety was added to the resin. Therefore, DOTA*6 $H_2O$ (56.4 mg, 0.11 mmol, 1.00 eq.), TBTU (29.3 mg, 91.3 μmol, 0.83 eq.), HOAt (12.4 mg, 91.3 μmol, 0.83 eq.) and sym-collidine (102 μL, 0.77 mmol, 7.00 eq.) were dissolved in a mixture of DMF/DMSO (5/1, v/v) and incubated with the resin-bound amine for 19.5 h. As RP-HPLC/MS analysis (GP6) revealed no sufficient coupling, the resin was divided into two equivalent portions. One portion was again incubated with a freshly prepared DOTA-coupling mixture for 20 h. An only low rise in turnover led to a further DOTA-coupling step, now with DIPEA (101 μL, 0.59 mmol, 10.7 eq.) instead of sym-collidine, in order to monitor and adjust the pH value to 9 - 10. After incubation for 68 h an adequate conversion was achieved and the peptide was cleaved off the resin with TFA/TIPS/$H_2O$ (95/2.5/2.5, slightly modified to GP8) (2 x 30 min) and purified afterwards by preparative RP-HPLC (20 - 70% B in 20 min, Method B, 5 mL/min). Subsequent lyophilization afforded 6.30 mg (8.18%) of pure product **3** as a colorless powder. The second portion was cleaved off the resin with HFIP/DCM (1/4, v/v) at room temperature for 1 h in total (2 x 30 min). Thereby, all acid-labile protective groups were retained. The major portion of solvent was removed under a stream of nitrogen and the residual crude product additionally dried by lyophilization. Crude product (48.0 mg, 39.0 μmol, 1.00 eq.) was dissolved in 3.00 mL DMF and DOTA-NHS (32.5 mg, 43.0 μmol, 1.10 eq.) as well as DIPEA (39.7 μL, 0.23 mmol, 6.00 eq.) was added. The mixture was stirred at room temperature for 18 h. Preparative RP-HPLC (35 - 95% B in 15min, Method B, 1 mL/min) and subsequent lyophilization afforded 23.7 mg of the tert-butyl-functionalized peptide. Incubation with TFA/TIPS/DCM (95/2.5/2.5, slightly modified to GP8) at 0 °C for 1h in total revealed 11.8mg (21.5%) of pure product 3 after RP-HPLC purification (30-50 % B in 15 min, Method B, 1 ml/min) and lyophilization. This resulted in an overall yield of 18.1 mg (13.8 % yield, chemical purity >98% of carbamate I (3).

Chemical formula: $C_{60}H_{94}FN_{11}O_{24}Si$;
Molecular weight: 1400.55 g/mol;
Exact mass: 1399.62 g/mol;
$t_R$-value: 8.58 min (10 - 90% B in 15 min, Method A)
Capacity factor k: 4.72
ESI-MS: calculated monoisotopic mass ($C_{60}H_{94}FN_{11}O_{24}Si$): 1399.62;
found: ESI (positive ion mode): m/z = 701.0 $[M(\mathbf{3})+2H]^{2+}$, 1400.9 $[M(\mathbf{3})+H]^{+}$, 1868.1 $[M_4(\mathbf{3})+3H]^{3+}$.

$^{nat}$Ga-carbamate I ($^{nat}$Ga-**3**)

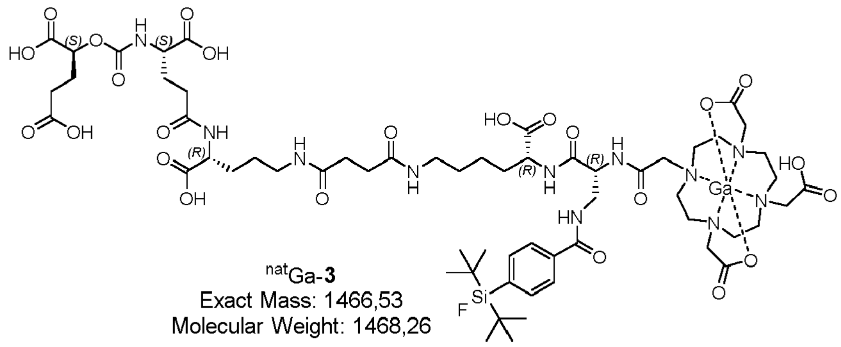

$^{nat}$Ga-**3**
Exact Mass: 1466,53
Molecular Weight: 1468,26

Carbamate I (3) (5.90 mg, 4.22 μmol, 1.00 eq.) was dissolved in 400 μL of HEPES buffer (pH = 3) and $Ga(NO_3)_3$*6 $H_2O$ (5.37 mg, 14.8 μmol, 3.50 eq.) dissolved in 444 μL HEPES buffer was added. The mixture was stirred for 30 min at 75 °C and afterwards filtered through single use syringe filters (Sartorius Minisart®) to remove $Ga(OH)_3$ precipitate. The $^{nat}$Ga-complex of carbamate I (**3**) (= $^{nat}$Ga-**3**) was purified by RP-HPLC (25 - 45% B in 15 min, Method A, 1 mL/min) which afforded 1.64 mg (26.5%) of pure product $^{nat}$Ga-**3** as a colorless powder after lyophilization.

Chemical formula: $C_{60}H_{92}FGaN_{11}O_{24}Si$;

Molecular weight: 1468.26 g/mol;
Exact mass: 1466.53 g/mol;
$t_R$-value: 12.0 min (25 - 45% B in 15 min, Method A)
Capacity factor k: 7.0
ESI-MS: calculated monoisotopic mass ($C_{60}H_{92}FGaN_{11}O_{24}Si$): 1466.53;
found: ESI (positive ion mode): m/z = 734.9 [M($^{nat}$Ga-**3**)+2H]$^{2+}$, 1468.0 [M($^{nat}$Ga-**3**)+H]$^{+}$, 1835.8 [$M_5$($^{nat}$Ga-**3**)+4H]$^{4+}$, 1957.3 [$M_4$($^{nat}$Ga-**3**)+3H]$^{3+}$.

$^{nat}$Lu-carbamate I ($^{nat}$Lu-**3**)

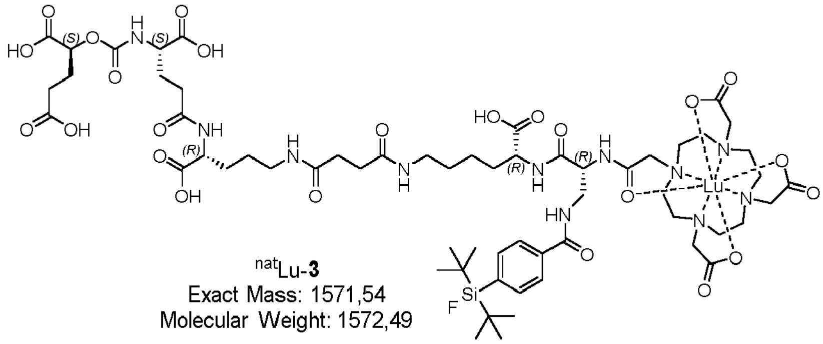

$^{nat}$Lu-**3**
Exact Mass: 1571,54
Molecular Weight: 1572,49

100 μL of the precursor (**3**) (2.00 mM in DMSO, 0.20 μmol, 1.00 eq.) were added to 60.0 μL of $LuCl_3$ (20 mM in Tracepur®-$H_2O$, 1.20 μmol, 6.00 eq.) and 40.0 μL Tracepur®-$H_2O$. The reaction mixture was heated for 30 min at 70 °C and afforded $^{nat}$Lu-**3** in 92.3% chemical purity (>99% yield), determined by RP-HPLC (220 nm). This $^{nat}$Lu-**3** solution (now 1.00 mM) was directly used as stock solution for affinity determination.

Chemical formula: $C_{60}H_{91}FLuN_{11}O_{24}Si$;
Molecular weight: 1572.49 g/mol;
Exact mass: 1571.54 g/mol;
$t_R$-value: 8.48 min (10 - 90% B in 15 min, Method A)
Capacity factor k: 4.7
ESI-MS: calculated monoisotopic mass ($C_{60}H_{91}FLuN_{11}O_{24}Si$): 1571.54;
found: ESI (positive ion mode): m/z = 787.1 [M($^{nat}$Lu-**3**)+2H]$^{2+}$, 1573.2 [M($^{nat}$Lu-**3**)+H]$^{+}$, 1598.0 [M($^{nat}$Lu-**3**)+Na]$^{+}$.

[$^{177}$Lu]Lu-carbamate I ([$^{177}$Lu]Lu-**3**)

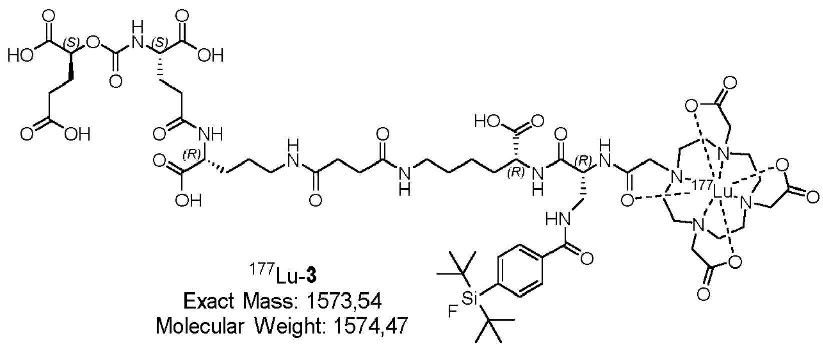

$^{177}$Lu-**3**
Exact Mass: 1573,54
Molecular Weight: 1574,47

5.00 μL of the precursor (**3**) (0.20 mM in DMSO, 1.00 nmol, 1.00 eq.) were added to 10.0 μL of 1 M NaOAc buffer (aq.) (pH = 5.5). Subsequently, 22.3 to 62.2 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0

μL of 0.1 M sodium ascorbate (aq.) (in Tracepur®-$H_2O$) were added and the reaction mixture was heated for 25 min at 70 °C. Due to this lower temperature, removal of free [$^{177}$Lu]$Lu^{3+}$ via HLB cartridge (30 mg) was required. This afforded [$^{177}$Lu]Lu-**3** in 69.7 ± 18.2% (n = 6) isolated radiochemical yield (RCY) (decay corrected (d.c.) to the start of synthesis). The apparent molar activities ($A_m$) were 12.5 to 21.9 GBq/μmol at the end of synthesis. Radiochemical purity (RCP) as determined by radio-RP-HPLC and radio-TLC was 96.9 ± 2.1%.

Chemical formula: $C_{60}H_{91}F^{177}LuN_{11}O_{24}Si$;
Molecular weight: 1574.47 g/mol;
Exact mass: 1573.54 g/mol;
$t_R$-value: 9.59 min (10 - 90% B in 15 min, Method A)
$t_R$-value of co-injected cold standard: 9.49 min (10 - 90% B in 15 min, Method A)
Capacity factor k: 5.4.

**Carbamate II (4)**

5-benzyl 1-(tert-butyl) (S)-2-hydroxypentanedioate (**16**)

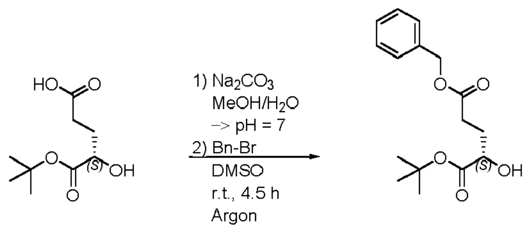


S-7
Exact Mass: 204,10
Molecular Weight: 204,22

16
Exact Mass: 294,15
Molecular Weight: 294,35

According to a slightly modified procedure by Shin et al. (Journal of Organic Chemistry. 2000, 65(22), 7667-75), (S)-5-(tert-butoxy)-4-hydroxy-5-oxopentanoic acid (**S-7**) (crude product, 381 mg, 2.28 mmol, 1.00 eq.) was dissolved in 20.7 mL MeOH/$H_2O$ (10/1, v/v). The solution was stirred during continuous addition of aqueous 10% $Na_2CO_3$, which was terminated as soon as a pH value of 7 was reached (1.10 mL of 10% $Na_2CO_3$ in total). All solvents were removed in vacuo, and the remaining residue was further dried by lyophilization, which afforded a slightly yellow solid. For the subsequent step, the used glassware was pretreated as described in GP9 (air- and moisture-free conditions) and also handling of reactants proceeded by the described methods (GP9). Lyophilized reactant **S-7** was dissolved in 11.4 mL dry DMSO and 5.70 mL thereof (1.14 mmol, 1.00 eq.) were transferred into a Schlenk flask. Benzyl bromide (203 μL, 1.71 mmol, 1.50 eq.) was added and the reaction mixture was stirred at room temperature for 4.5 h under argon atmosphere. Afterwards, the reaction was quenched with $H_2O$, the mixture was extracted with $Et_2O$ (3x). The organic phase was washed once with $H_2O$, dried over $Na_2SO_4$, filtered and the solvent removed under reduced pressure. The obtained crude product was purified by preparative RP-HPLC (20 - 80% B in 20 min, Method C, 5 mL/min). Subsequent lyophilization afforded 178 mg (53.2%) of pure product **16** as a colorless solid.

Chemical formula: $C_{16}H_{22}O_5$;
Molecular weight: 294.35 g/mol;
Exact mass: 294.15 g/mol;
$t_R$-value: 15.7 min (10 - 90% B in 15 min, Method A)
Capacity factor k: 9.5
ESI-MS: calculated monoisotopic mass ($C_{16}H_{22}O_5$): 294.15;
found: ESI (positive ion mode): m/z = 239.1 [M(**16**)-tBu+H]$^+$, 262.2 [M(**16**)-tBu+Na]$^+$, 280.2 [M(**16**)-tBu+MeCN+H]$^+$, 295.2 [M(**16**)+H]+, 317.2 [M(**16**)+Na]$^+$, 333.2 [M(**16**)+K]$^+$, 358.3 [M(**16**)+MeCN+Na]$^+$;
$^1$H-NMR (300 MHz, $CDCl_3$) δ(ppm): 7.38 - 7.32 (m, 5H, H(1, 1', 1", 1'", 1"")), 5.13 (s, 2H, H(2)), 4.09 (dd, $^3J$ = 7.9, 4.1 Hz, 1H, H(3)), 2.87 (s, 1H, H(4)), 2.63 - 2.39 (m, 2H, H(5)), 2.16 (virt. dddd, 1H, H(6)), 1.98 - 1.81 (m, 1H, H(7)), 1.48 (s, 9H, H(8, 8', 8")).
$^{13}$C-NMR (75 MHz, $CDCl_3$) δ(ppm): 174.04 (s, 1C, C(1)), 173.16 (s, 1C, C(2)), 136.06 (s, 1C, C(3)), 128.69 (s, 2C, C(4, 4')), 128.36 (s, 1C, C(5)), 128.32 (s, 2C, C(6, 6')), 82.96 (s, 1C, C(7)), 69.73 (s, 1C, C(8)), 66.49 (s, 1C, C(9)),

29.91 (s, 1C, C(10)), 29.63 (s, 1C, C(11)), 28.15 (s, 3C, C(12, 12', 12")).

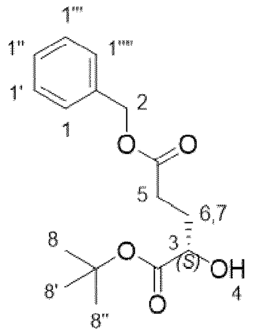

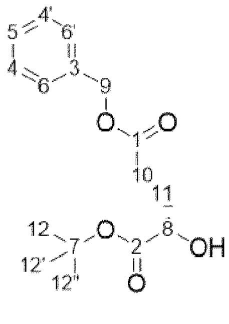

16

5-benzyl 1-(tert-butyl) (S)-2-((1H-imidazole-1-carbonyl)oxy)pentanedioate (**S-14**)

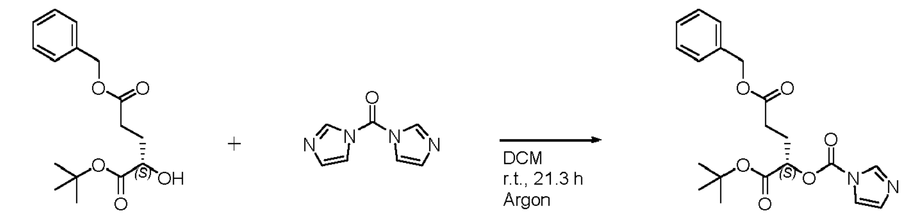


16
Exact Mass: 294,15
Molecular Weight: 294,35

S-9
Exact Mass: 162,05
Molecular Weight: 162,15

S-14
Exact Mass: 388,16
Molecular Weight: 388,42

Compound **S-14** was synthesized in analogy to a previously published procedure by Yang et al. (Journal of Medicinal Chemistry. 2016, 59(1), 206-18) with some minor modifications. 5-benzyl 1-(tert-butyl)(S)-2-hydroxypentanedioate (**16**) (71.6 mg, 0.24 mmol, 1.00 eq.) was dissolved in 4 mL dry DCM and stirred at room temperature. 1,1'-Carbonyldiimidazole (**S-9**) (74.5 mg, 0.46 mmol, 1.92 eq.) was dissolved in 1 mL dry DCM and added to the solution, which was stirred under argon atmosphere for 21.3 h at room temperature. The reaction mixture was diluted with DCM and washed once with $H_2O$ (+ 1 mL brine). The aqueous phase was extracted two times with DCM, the combined organic phases were dried over $Na_2SO_4$, filtered and the solvent evaporated in vacuo. This afforded 112 mg (>99%) of crude product as slightly yellowish oil. RP-HPLC/MS control revealed nearly complete conversion to product **S-14**, which was used in the next step without further purification.

Chemical formula: $C_{20}H_{24}N_2O_6$;
Molecular weight: 388.42 g/mol;
Exact mass: 388.16 g/mol;
$t_R$-value: 10.1 min (40 - 100% B in 15 min, Method A)
Capacity factor k: 5.7
ESI-MS: calculated monoisotopic mass ($C_{20}H_{24}N_2O_6$): 388.16;
found: ESI (positive ion mode): m/z = 389.2 [M(**S-14**)+H]$^+$, 839.5 [$M_2$(**S-14**)+MeCN+Na]$^+$.

5-benzyl 1-(tert-butyl) (S)-2-((((S)-1,5-di-tert-butoxy-1,5-dioxopentan-2-yl)carbamoyl)oxy)pentanedioate (**S-15**)

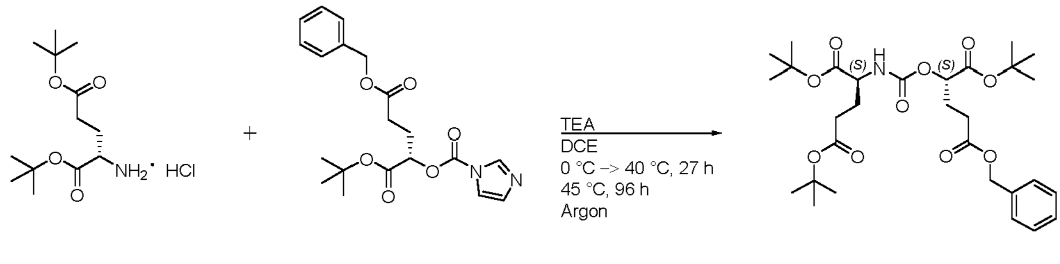


S-1
Exact Mass: 295,16
Molecular Weight: 295,81

S-14
Exact Mass: 388,16
Molecular Weight: 388,42

S-15
Exact Mass: 579,30
Molecular Weight: 579,69

Compound **S-15** was synthesized in analogy to a previously published procedure by Weineisen et al. (EJNMMI Research. 2014, 4(1), 63) with some minor modifications. 5-benzyl 1-(tert-butyl)(S)-2-((1H-imidazole-1-carbonyl)oxy)pentanedioate (**S-14**) (93.2 mg, 0.24 mmol, 1.00 eq.) was dissolved in 2.50 mL DCE and cooled to 0 °C. H-L-Glu(OtBu)-OtBu*HCl (**S-1**) (142 mg, 0.48 mmol, 2.00 eq.) and triethylamine (83.2 μL, 0.60 mmol, 2.50 eq.) were added and stirred for further five minutes at 0 °C. The reaction mixture was warmed to 40 °C and stirred for 27 h under argon atmosphere. As RP-HPLC/MS analysis revealed very low conversion (7% **S-15**, 90.7% **S-14**), again, H-L-Glu(OtBu)-OtBu*HCl (**S-1**) (284 mg, 0.96 mmol, 4.00 eq.) and triethylamine (166 μL, 1.20 mmol, 5.00 eq.) were added and the temperature was increased to 45 °C. Complete consumption of educt **S-14** was observed after 96 h at 45 °C under argon atmosphere. DCM was added for dilution of the reaction mixture and once washed with $H_2O$ (+ 1 mL brine). The aqueous phase was extracted two times with DCM, the combined organic layers were dried over $Na_2SO_4$, filtered and the solvent was removed under reduced pressure. The obtained crude product was purified by flash chromatography (65 - 98% B in 10 min, Method D, 12 mL/min), which gave 84.1 mg (60.5%) of compound **S-15** as a colorless viscous oil.

Chemical formula: $C_{30}H_{45}NO_{10}$;
Molecular weight: 579.69 g/mol;
Exact mass: 579.30 g/mol;
$t_R$-value: 16.9 min (40 - 100% B in 15 min, Method A)
Capacity factor k: 10.3
ESI-MS: calculated monoisotopic mass ($C_{30}H_{45}NO_{10}$): 579.30;
found: ESI (positive ion mode): m/z = 602.1 [M(**S-15**)+Na]$^+$, 618.2 [$M_2$(**S-15**)+K]$^+$.

(S)-5-(tert-butoxy)-4-((((S)-1,5-di-tert-butoxy-1,5-dioxopentan-2-yl)carbamoyl)oxy)-5-oxopentanoic acid (**17**)

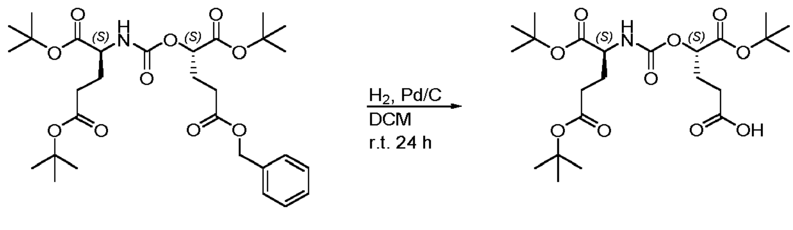


S-15
Exact Mass: 579,30
Molecular Weight: 579,69

17
Exact Mass: 489,26
Molecular Weight: 489,56

Compound **S-15** (84.1mg, 0.15 mmol, 1.00eq.) was dissolved in 6.20 mL DCM and the solution was flushed 5 minutes with argon to remove dissolved oxygen. 16.0 mg of palladium on carbon (10% wt) (corresponds to 1.60 mg palladium, 15.0 μmol, 0.10 eq.) were added and the flask was sealed with a rubber septum. Remaining air was displaced with argon and subsequently replaced by hydrogen gas. The mixture was stirred under hydrogen atmosphere at room temperature for 24 h. Palladium on carbon was filtered off, and the solvent was evaporated in vacuo. This afforded 85.5 mg (>99%) of compound **17** as a colorless clear oil, which was used in the next step without further purification.

Chemical formula: $C_{23}H_{39}NO_{10}$;

Molecular weight: 489.56 g/mol;
Exact mass: 489.26 g/mol;
$t_R$-value: 11.6 min (40 - 100% B in 15 min, Method A)
Capacity factor k: 6.7
ESI-MS: calculated monoisotopic mass ($C_{23}H_{39}NO_{10}$): 489.26;
found: ESI (positive ion mode): m/z = 304.1 [M(**17**)-3tBu-OPH]$^+$ 322.1 [M(**17**)-3tBu+H]$^+$, 378.2 [M(**17**)-2tBu+H]$^+$, 434.2 [M(**17**)-tBu+H]$^+$, 490.4 [M(**17**)+H]$^+$, 512.3 [M(**17**)+Na]$^+$, 528.4 [M(**17**)+K]$^+$.

L-Glu(OtBu)$_2$-(carbonyl)-[(S)-2-oxopentanedioic acid-(D-Orn(Dde)-2-CT)-OtBu] (**S-16**)

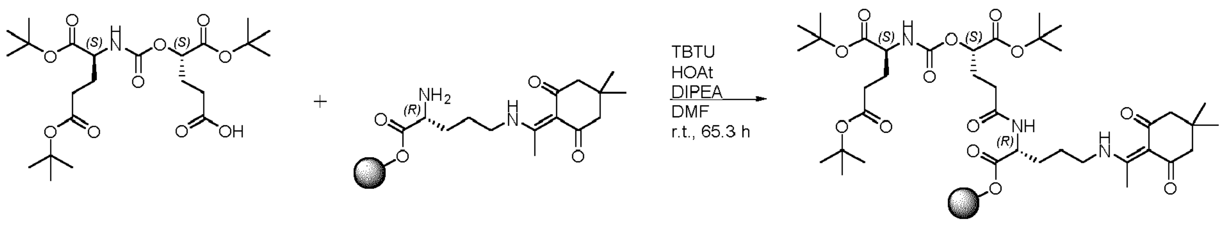


17
Exact Mass: 489,26
Molecular Weight: 489,56

18
Exact Mass: 296,17
Molecular Weight: 296,37

S-16
Exact Mass: 767,42
Molecular Weight: 767,91

According to GP2, fragment **17** (73.4 mg, 0.15 mmol, 1.00 eq.) was coupled to resin bound H-D-Orn(Dde) (**18**) with TBTU (110 mg, 0.34 mmol, 2.29 eq.) and HOAt (46.8 mg, 0.34 mmol, 2.29 eq.) as coupling reagents and DIPEA (264 µL, 1.55 mmol, 10.3 eq.) as base. After shaking for 65.3 h at room temperature, formation of product **S-16** could be confirmed (GP6). Additionally, some resin beads were taken, treated for 20 minutes with 2% hydrazin/DMF (v/v) and analyzed via RP-HPLC/MS after test cleavage according to GP7. Thus, stability of the carbamate moiety towards Dde-removal conditions was confirmed, as only one major peak (besides DMF) with the expected m/z-ratio of 604 occurred.

Chemical formula: $C_{38}H_{61}N_3O_{13}$, -3tBu: $C_{26}H_{37}N_3O_{13}$;
Molecular weight: 767.91 g/mol, -3tBu: 599.59 g/mol;
Exact mass: 767.42 g/mol, -3tBu: 599.23 g/mol;
$t_R$-value (-3tBu): 10.1 min (10 - 90% B in 15 min, Method A)
Capacity factor k (-3tBu): 5.7
ESI-MS: calculated monoisotopic mass ($C_{38}H_{61}N_3O_{13}$): 767.42, -3tBu ($C_{26}H_{37}N_3O_{13}$): 599.23; found: ESI (positive ion mode): m/z = 600.5 [M(**S-16**)-3tBu+H]$^+$, 662.3 [M(**S-16**)-3tBu+MeCN+Na]$^+$.

Carbamate II (**4**)

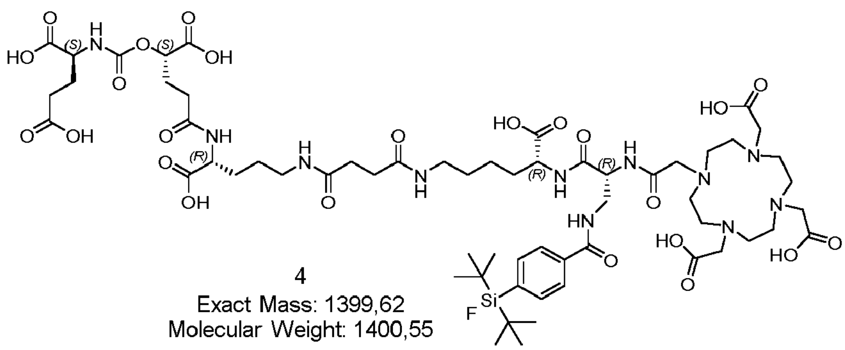

4
Exact Mass: 1399,62
Molecular Weight: 1400,55

Further reactions on resin-bound compound **S-16** were performed according to standard Fmoc-SPPS on 2-CT resin, applying the above-mentioned methods (GP2 - GP8). In brief, the Dde protective group was removed (GP4) and

succinic anhydride (121 mg, 1.20 mmol, 7.00 eq.) was coupled (GP2) over a period of at least 2.5 h, only using DIPEA (156 μL, 0.92 mmol, 5.33 eq.) and no further coupling reagents. The peptide was elongated with Fmoc-D-LysOtBu*HCl (159 mg, 0.34 mmol, 2.00 eq.). Therefore, the resin-bound acid was preactivated for five minutes using TBTU (110 mg, 0.34 mmol, 2.00 eq.), HOAt (46.8 mg, 0.34 mmol, 2.00 eq.) and DIPEA (176 μL, 1.02 mmol, 6.00 eq.). The amino acid was dissolved in DMF, added to the preactivated resin and shaken for at least 2.5 h. Fmoc-removal was conducted (GP3) and Fmoc-D-Dap(Dde)-OH was coupled for at least 2.5 h using TBTU (110 mg, 0.34 mmol, 2.00 eq.), HOAt (46.8 mg, 0.34 mmol, 2.00 eq.) and sym-collidine (160 μL, 1.20 mmol, 7.00 eq.) as coupling reagents. Afterwards, Dde-removal was performed according to GP5. The SiFA-BA moiety (73.4 mg, 0.26 mmol, 1.50 eq.) was attached using TBTU (110 mg, 0.34 mmol, 2.00 eq.), HOAt (46.8 mg, 0.34 mmol, 2.00 eq.) and sym-collidine (160 μL, 1.20 mmol, 7.00 eq.) as coupling reagents. After an incubation time of at least 2 h, the Fmoc protective group was removed (GP3) and the DOTA chelator moiety was added to the resin. Therefore, DOTA*6 $H_2O$ (87.1 mg, 0.17 mmol, 1.00 eq.), TBTU (45.0 mg, 0.14 mmol, 0.83 eq.), HOAt (19.1 mg, 0.14 mmol, 0.83 eq.) and sym-collidine (160 μL, 1.20 mmol, 7.00 eq.) were dissolved in a mixture of DMF/DMSO (5/1, v/v) and incubated with the resin-bound amine for 19.5 h. As RP-HPLC/MS analysis (GP6) revealed successful coupling, the peptide was cleaved off the resin with TFA/TIPS/DCM (95/2.5/2.5, GP8) and purified afterwards by preparative RP-HPLC (35 - 45% B in 20 min, Method B, 5 mL/min). Subsequent lyophilization afforded 8.35 mg (3.51% yield) of pure product **4** as a colorless powder.

Chemical formula: $C_{60}H_{94}FN_{11}O_{24}Si$;
Molecular weight: 1400.55 g/mol;
Exact mass: 1399.62 g/mol;
$t_R$-value: 13.7 min (10 - 90% B in 15 min, Method A)
Capacity factor k: 8.1
ESI-MS: calculated monoisotopic mass ($C_{60}H_{94}FN_{11}O_{24}Si$): 1399.62;
found: ESI (positive ion mode): m/z = 701.1 [M(**4**)+2H]$^{2+}$, 1400.6 [M(**4**)+H]$^{+}$.

$^{nat}$Ga-carbamate II ($^{nat}$Ga-**4**)

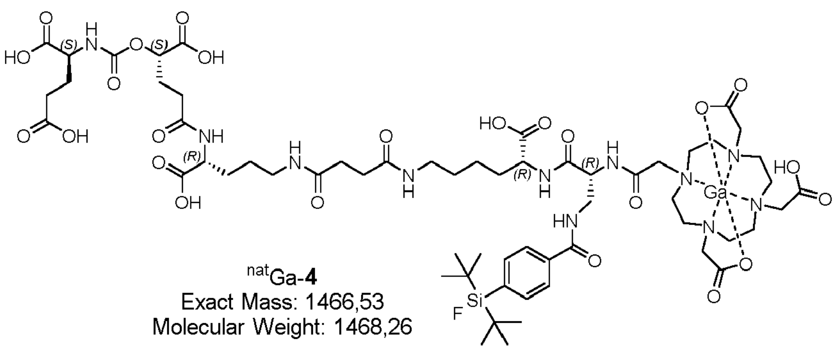

$^{nat}$Ga-**4**
Exact Mass: 1466,53
Molecular Weight: 1468,26

Carbamate II (**4**) (3.22 mg, 2.30 μmol, 1.00 eq.) was dissolved in 200 μL of HEPES buffer (pH = 3) and 260 μL of $Ga(NO_3)_3$*6 $H_2O$ (2.93 mg, 8.05 μmol, 3.50 eq.) dissolved in HEPES buffer was added. The mixture was stirred for 30 min at 75 °C and afterwards filtered through single use syringe filters (Sartorius Minisart®) to remove $Ga(OH)_3$ precipitate. The $^{nat}$Ga-complex of carbamate II (**4**) (= $^{nat}$Ga-**4**) was purified by RP-HPLC (20 - 60% B in 15 min, Method A, 1 mL/min) and afforded 0.53 mg (15.7%) of pure product $^{nat}$Ga-**4** as a colorless powder after lyophilization.

Chemical formula: $C_{60}H_{92}FGaN_{11}O_{24}Si$;
Molecular weight: 1468.26 g/mol;
Exact mass: 1466.53 g/mol;
$t_R$-value: 8.46 min (10 - 90% B in 15 min, Method A)
Capacity factor k: 4.6
ESI-MS: calculated monoisotopic mass ($C_{60}H_{92}FGaN_{11}O_{24}Si$): 1466.53;
found: ESI (positive ion mode): m/z = 734.3 [M($^{nat}$Ga-**4**)+2H]$^{2+}$, 1468.2 [M($^{nat}$Ga-**4**)+H]$^{+}$, 1835.5 [$M_5$($^{nat}$Ga-**4**)+4H]$^{4+}$, 1957.4 [$M_4$($^{nat}$Ga-**4**)+3H]$^{3+}$.

**Synthesis of proinhibitors I, II & III Proinhibitor I (5)**

Tert-butyl N$^5$-((S)-1-(tert-butoxy)-4-(methylthio)-1-oxobutan-2-yl)-L-glutaminate (**19**)

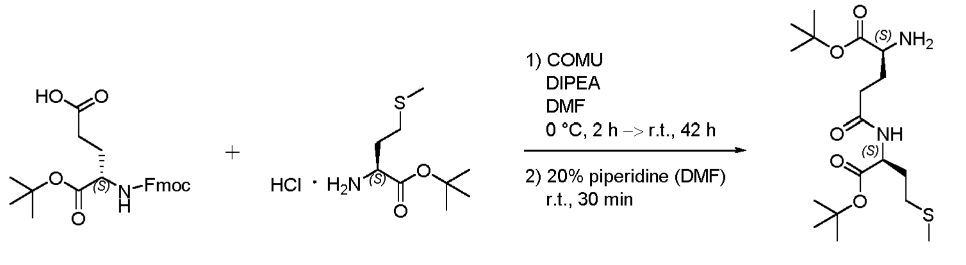


**S-3** Exact Mass: 329,14 Molecular Weight: 329,82

**S-17** Exact Mass: 205,11 Molecular Weight: 205,32

**19** Exact Mass: 390,22 Molecular Weight: 390,54

Fmoc-L-Glu-OtBu (**S-3**) (3.00 g, 7.05 mmol, 1.10 eq.) was dissolved in 40.1 mL DMF and cooled to 0 °C. COMU (3.02 g, 7.05 mmol, 1.10 eq) and DIPEA (5.27 mL, 31.0 mmol, 4.84 eq.) were added to reach a basic pH of 10. H-L-Met-OtBu*HCl (**S-17**) (1.55 g, 6.41 mmol, 1.00 eq.) was added and the reaction mixture was stirred for further 2 h at 0 °C. The ice bath was removed and the solution was stirred at room temperature for 42 h. The reaction was terminated by addition of $H_2O$ and diluted with $Et_2O$. The aqueous phase was extracted three times with $Et_2O$ and the combined organic phases were then washed once with saturated $NaHCO_3$ and brine. The organic layer was dried over $Na_2SO_4$, filtered and the solvent was removed under reduced pressure. As RP-HPLC/MS analysis revealed dipeptide Fmoc-**19** to be the main portion of the crude product, 5 mL of 20% piperidine (DMF) were added and stirred for 30 min at room temperature. As the dibenzofulvene-piperidine byproduct could not be removed sufficiently by preparative RP-HPLC (35 - 60% B in 20 min, Method B), it was removed by column chromatography (n-Hex/EtOAc = 3/2). Afterwards, the solvent was changed (DCM/MeOH/acetone = 5/1/1) by which a distinct separation of product **19** from all remaining by-products was possible. This afforded 287 mg (11.5%) of pure product **19** as a clear, yellow oil.

Chemical formula: $C_{18}H_{34}N_2O_5S$;
Molecular weight: 390.54 g/mol;
Exact mass: 390.22 g/mol;
$t_R$-value: 9.37 min (10 - 90% B in 15 min, Method A)
Capacity factor k: 5.2
$R_f$-value: 0.64 (DCM/MeOH/acetone = 5/1/1)
ESI-MS: calculated monoisotopic mass ($C_{18}H_{34}N_2O_5S$): 390.22;
found: ESI (positive ion mode): m/z = 391.5 [M(**19**)+H]$^+$, 782.0 [M$_2$(**19**)+H]$^+$.

1-Carbonylimidazole-Glu[D-Orn(Dde)-2-CT]-OtBu (20)

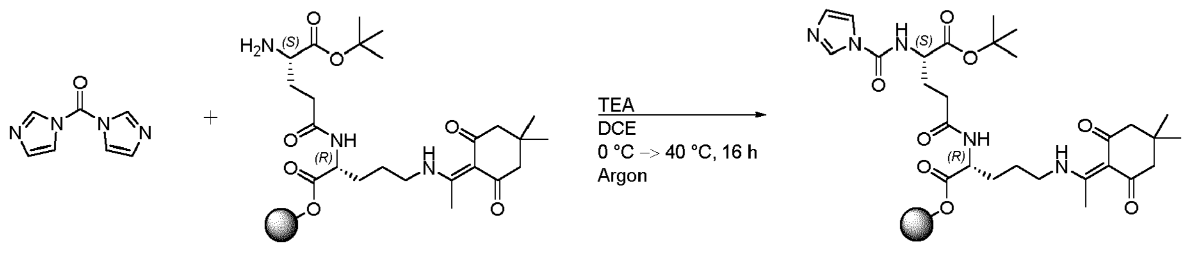


S-9 Exact Mass: 162,05 Molecular Weight: 162,15

S-18 Exact Mass: 481,28 Molecular Weight: 481,59

20 Exact Mass: 575,30 Molecular Weight: 575,66

Compound **S-18** was transferred into a round bottom flask, where it was dissolved in 3.81 mL DCE. At 0 °C triethylamine (146 µL, 1.05 mmol, 2.50 eq.) and 1,1'-carbonyldiimidazole (**S-9**) (68.1 mg, 0.42 mmol, 1.00 eq.) were added and the mixture was stirred for further 5 min at 0 °C. Afterwards, it was warmed to 40 °C and stirred for 16 h under argon atmosphere. The resin was washed with DCM (4x) and again dried in a desiccator for 30 min. Some resin beads were taken and treated with HFIP/DCM (1/4) according to GP7. RP-HPLC/MS analysis revealed nearly complete conversion to product **20.**

Chemical formula: $C_{28}H_{41}N_5O_8$;
Molecular weight: 575.66 g/mol;
Exact mass: 575.30 g/mol;
$t_R$-value: 8.24 min (10 - 90% B in 15 min, Method A)
Capacity factor k: 4.5
ESI-MS: calculated monoisotopic mass ($C_{28}H_{41}N_5O_8$): 575.30;
found: ESI (positive ion mode): m/z = 452.4 [M(**20**)-imidazole-tBu+H]$^+$, 508.5 [M(**20**)-imidazole]$^{\cdot+}$, 576.6 [M(**20**)+H]$^+$.

L-Glu(L-Met-OtBu)-OtBu-(carbonyl)-L-Glu[D-Orn(Dde)-2-CT]-OtBu (S-19)

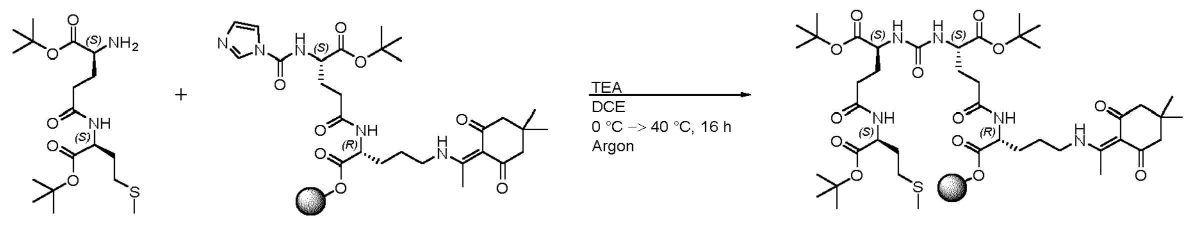


**19**
Exact Mass: 390,22
Molecular Weight: 390,54

**20**
Exact Mass: 575,30
Molecular Weight: 575,66

**S-19**
Exact Mass: 897,48
Molecular Weight: 898,12

Compound **S-19** was synthesized in analogy to a previously published procedure by Weineisen et al. (EJNMMI Research. 2014, 4(1), 63) with some minor modifications. The H-L-Glu(L-Met-OtBu)-OtBu dipeptide **19** (246 mg, 0.63 mmol, 1.50 eq.) was dissolved in 3.81 mL DCE and added to compound **20** (0.42 mmol, 1.00 eq.). At 0 °C triethylamine (146 µL, 1.05 mmol, 2.50 eq.) was added and the mixture was stirred for further five minutes at 0 °C. The solution was warmed to 40 °C and stirred for 16 h under argon atmosphere. The resin was transferred to a syringe for peptide synthesis (equipped with a frit, pore size 25 µm) and washed with DCM (4x). Some resin beads were taken according to GP7 and analyzed via RP-HPLC/MS, which indicated nearly complete conversion to **S-19.**

Chemical formula: $C_{43}H_{71}N_5O_{13}S$;
Molecular weight: 898.12 g/mol;
Exact mass: 897.48 g/mol;
$t_R$-value: 13.2 min (10 - 90% B in 15 min, Method A)
Capacity factor k: 7.8
ESI-MS: calculated monoisotopic mass ($C_{43}H_{71}N_5O_{13}S$): 897.48;
found: ESI (positive ion mode): m/z = 450.2 [M(**S-19**)+2H]$^{2+}$, 470.7 [M(**S-19**)+MeCN+2H]$^{2+}$, 899.2 [M(**S-19**)+H]$^+$.

Proinhibitor I (**5**)

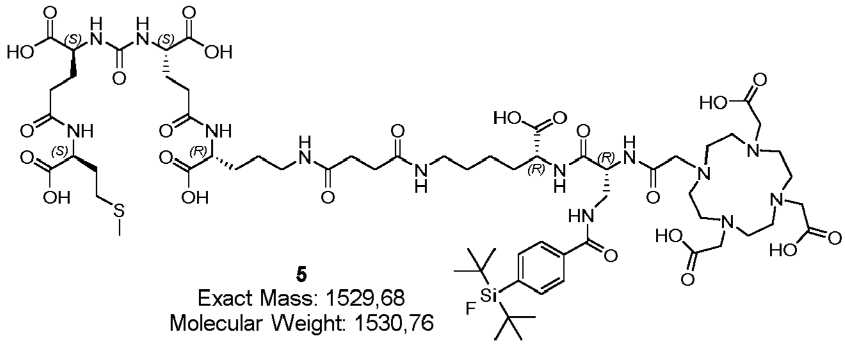

**5**
Exact Mass: 1529,68
Molecular Weight: 1530,76

Further reactions on resin-bound compound **S-19** were performed according to standard Fmoc-SPPS on 2-CT resin, applying the above-mentioned methods (GP2 - GP8). In brief, the Dde protective group was removed (GP4) and succinic anhydride (294 mg, 294 mmol, 7.00 eq.) was coupled (GP2) over a period of at least 2.5 h, only using DIPEA (500 μL, 2.94 mmol, 7.00 eq.) and no further coupling reagents. The peptide was elongated with Fmoc-D-Lys-OtBu*HCl (290 mg, 0.63 mmol, 1.50 eq.). Therefore, the resin-bound acid was preactivated for five minutes using TBTU (270 mg, 0.84 mmol, 2.00 eq.), HOAt (114 mg, 0.82 mmol, 2.00 eq.) and DIPEA (629 μL, 3.70 mmol, 8.81 eq.). The amino acid was dissolved in DMF, added to the preactivated resin and shaken for at least 2.5 h. Fmoc-removal was conducted (GP3) and Fmoc-D-Dap(Dde)-OH (309 mg, 0.63 mmol, 1.50 eq.) was coupled for at least 2.5 h using TBTU (270 mg, 0.84 mmol, 2.00 eq.), HOAt (114 mg, 0.84 mmol, 2.00 eq.) and sym-collidine (490 μL, 3.70 mmol, 8.81 eq.) as coupling reagents. Afterwards, Dde-removal was performed according to GP5. Due to extensive oxidation and other side reactions, some test reactions were performed with a minor part (0.09 mmol) of the resin for optimization of SiFA-BA and DOTA coupling. Hence, further reactions were conducted on the remaining part (0.33 mmol) and all following equivalents refer to this amount of substance. The SiFA-BA moiety was attached via the Pfp-ester, which was generated previously by preactivation of SiFA-BA (93.1 mg, 0.33 mmol, 1.00 eq.) with pentafluorophenol (104 μL, 0.99 mmol, 3.00 eq.), DIC (153 μL, 0.99 mmol, 3.00 eq.) and pyridine (214 μL, 2.64 mmol, 8.00 eq.) in DMF for 1.5 h. The solution was added to the resin-bound peptide and incubated for 22 h prior to Fmoc protective group removal (GP3). For coupling of the DOTA moiety, DOTA-NHS (276 mg, 0.36 mmol, 1.10 eq.) and DIPEA (437 μL, 2.57 mmol, 7.79 eq.) were each dissolved in DMF. First, DIPEA in DMF was added to the resin for preactivation. After five minutes, DOTA-NHS in DMF was added and incubated with the resin-bound amine for 21 h. An adequate conversion (RP-HPLC/MS after GP7) was achieved and the peptide was cleaved off the resin under argon atmosphere with TFA/TIPS/DCM/dithiothreitol (95/2.5/2.5/0.5% wt, slightly modified to GP8) (3 x 30 min) and purified afterwards by preparative RP-HPLC (30 - 60% B in 20 min, Method B and 33 - 40% B in 15 min, Method A). Subsequent lyophilization afforded 0.39 mg (0.08% yield, chemical purity 98.0%) of pure product **5** as a colorless powder.

Chemical formula: $C_{65}H_{104}FN_{13}O_{24}SSi$;
Molecular weight: 1530.76 g/mol;
Exact mass: 1529.68 g/mol;
$t_R$-value: 8.62 min (10 - 90% B in 15 min, Method A)
Capacity factor k: 4.7
ESI-MS: calculated monoisotopic mass ($C_{65}H_{104}FN_{13}O_{24}SSi$): 1529.68;
found: ESI (positive ion mode): m/z = 765.8 [M(**5**)+2H]$^{2+}$, 1531.5 [M(**5**)+H]$^{+}$.

$^{nat}$Lu-proinhibitor I ($^{nat}$Lu-**5**)

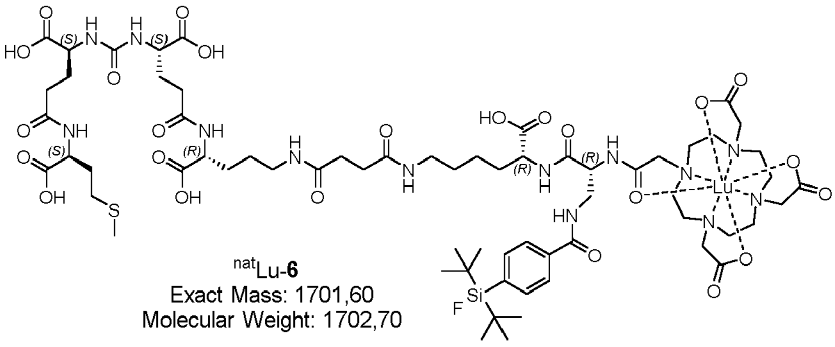

natLu-**6**
Exact Mass: 1701,60
Molecular Weight: 1702,70

50 μL of the precursor (**5**) (2.00 mM in DMSO, 0.10 μmol, 1.00 eq.) were added to 30.0 μL of $LuCl_3$ (20 mM in Tracepur®-$H_2O$, 60.0 μmol, 6.00 eq.) and 20.0 μL Tracepur®-$H_2O$. The reaction mixture was heated for 30 min at 70 °C and afforded $^{nat}$Lu-**5** in >99% chemical purity (>99% yield), determined by RP-HPLC (220 nm). This $^{nat}$Lu-**5** solution (now 0.50 mM) was directly used as stock solution for affinity determination.

Chemical formula: $C_{65}H_{101}FLuN_{13}O_{24}SSi$;
Molecular weight: 1702.70 g/mol;
Exact mass: 1701.60 g/mol;
$t_R$-value: 8.62 min (10 - 90% B in 15 min, Method A)
Capacity factor k: 4.7
ESI-MS: calculated monoisotopic mass ($C_{65}H_{101}FLuN_{13}O_{24}SSi$): 1701.60;
found: ESI (positive ion mode): m/z = 851.3 [M($^{nat}$Lu-**5**)+2H]$^{2+}$, 1701.8 [M($^{nat}$Lu-**5**)+H]$^{+}$.

[$^{177}$Lu]Lu-proinhibitor I ([$^{177}$Lu]Lu-**5**)

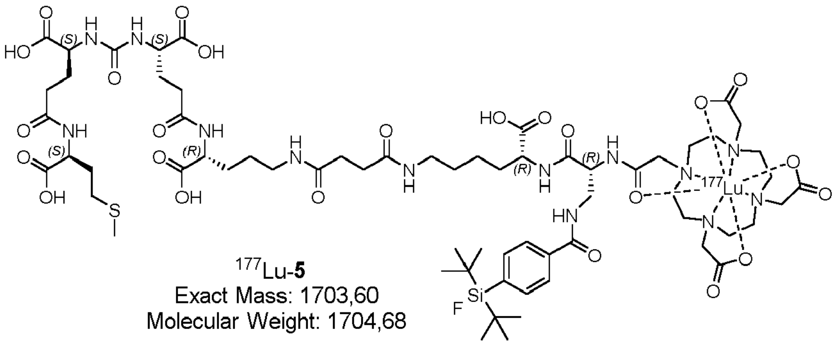

$^{177}$Lu-**5**
Exact Mass: 1703,60
Molecular Weight: 1704,68

5.00 μL of the precursor (**5**) (0.20 mM in DMSO, 1.00 nmol, 1.00 eq.) were added to 10.0 μL of 1 M NaOAc buffer (aq.) (pH = 5.5). Subsequently, 14.0 to 35.9 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0 μL of 0.1 M sodium ascorbate (aq.) (in Tracepur®-$H_2O$) were added and the reaction mixture was heated for 25 min at 80 °C. After removal of free [$^{177}$Lu]$Lu^{3+}$ via HLB cartridge (30 mg), [$^{177}$Lu]Lu-**5** was purified by radio-RP-HPLC, which afforded [$^{177}$Lu]Lu-**5** in 44.0 ± 7.6% (n = 4) isolated RCY (d.c. to the start of synthesis). Exact apparent $A_m$ of products, which were purified by radio-RP-HPLC could not be determined as the amount of cold precursor within the product fraction could only be estimated. In these cases, the amount of substance was roughly determined by the percentage of product, determined by radio-RP-HPLC. This afforded apparent $A_m$ of approximately 4.64 to 14.5 GBq/μmol. RCP as determined by radio-RP-HPLC and radio-TLC was 89.3 ± 1.9%. Higher RCP could not be achieved, as right after purification, quality control revealed up to 13% of oxidized byproduct again.

Chemical formula: $C_{65}H_{101}F^{177}LuN_{13}O_{24}SSi$;
Molecular weight: 1704.68 g/mol;
Exact mass: 1703.60 g/mol;

$t_R$-value: 10.1 min (10 - 90% B in 15 min, Method A)
$t_R$-value of co-injected cold standard: 10.0 min (10 - 90% B in 15 min, Method A) Capacity factor k: 5.7.

Proinhibitor II (6)

(S)-2-((S)-5-(tert-butoxy)-4-(1H-imidazole-1-carboxamido)-5-oxopentanamido) octanoic acid-[2-CT] (**S-21**)

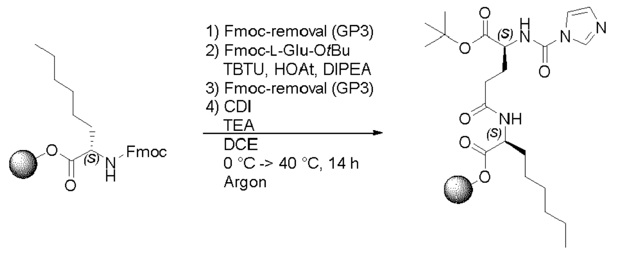


**S-20**
Exact Mass: 381,19
Molecular Weight: 381,47

**S-21**
Exact Mass: 438,25
Molecular Weight: 438,53

Fmoc-L-2-Aminooctanoic acid was coupled to 2-CTC resin according to GP1. Further reactions on compound **S-20** (load: 0.72 mmol/g, 0.86 mmol, 1.00 eq.) were performed according to standard Fmoc-SPPS on 2-CT resin, applying the above-mentioned methods (GP2 & GP3). In brief, the Fmoc protective group was removed (GP3) and Fmoc-L-Glu-OtBu (549 mg, 1.29 mmol, 1.50 eq.) was coupled (GP2) over a period of 16 h using TBTU (552 mg, 1.72 mmol, 2.00 eq.), HOAt (234 mg, 1.72 mmol, 2.00 eq.) and DIPEA (658 µL, 3.87 mmol, 4.50 eq.). After removal of the Fmoc protective group (GP3), the resin was dried in a desiccator for 30 min and transferred into a round bottom flask, where it was dissolved in 7.81 mL DCE. At 0 °C, triethylamine (298 µL, 2.15 mmol, 2.50 eq.) and 1,1'-carbonyldiimidazole (154 mg, 0.95 mmol, 1.10 eq.) were added and the mixture was stirred for further 5 min at 0 °C. Afterwards, it was warmed to 40 °C and stirred for 14 h under argon atmosphere. The resin was washed with DCM (4x) and again dried in a desiccator for 30 min. Some resin beads were taken and treated with TFA according to GP7. RP-HPLC/MS revealed nearly complete conversion to product **S-21.**

Chemical formula: $C_{21}H_{34}N_4O_6$;
Molecular weight: 438.53 g/mol;
Exact mass: 438.25 g/mol;
$t_R$-value: 9.61 min (10 - 90% B in 15 min, Method A)
Capacity factor k: 5.4
ESI-MS: calculated monoisotopic mass ($C_{21}H_{34}N_4O_6$): 438.25;
found: ESI (positive ion mode): m/z = 439.3 [M(**S-21**)+H]$^+$, 480.4 [M(**S-21**)+MeCN+H]$^+$.

(6S,10S,15S)-6,10-bis(tert-butoxycarbonyl)-15-hexyl-3,8,13-trioxo-1-phenyl-2-oxa-7,9,14-triazahexadecan-16-oic acid (**S-22**)

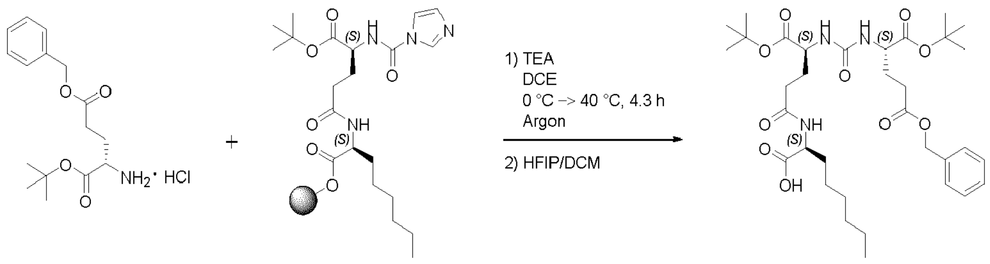


S-11
Exact Mass: 329,14
Molecular Weight: 329,82

S-21
Exact Mass: 438,25
Molecular Weight: 438,53

S-22
Exact Mass: 663,37
Molecular Weight: 663,81

Compound **S-22** was synthesized in analogy to a previously published procedure by Weineisen et al. (EJNMMI Research. 2014, 4(1), 63) with some minor modifications. Resin-bound **S-21** (0.86 mmol, 1.00 eq.) was dissolved in 7.81 mL DCE and cooled to 0 °C. H-L-Glu(OBzl)-OtBu*HCl (**S-11**) (425 mg, 1.29 mmol, 1.50 eq.) and triethylamine (298 μL, 2.15 mmol, 2.50 eq.) were added and stirred for further five minutes at 0 °C. The reaction mixture was warmed to 40 °C and stirred for 4.3 h under argon atmosphere. The resin was transferred to a syringe for peptide synthesis (equipped with a frit, pore size 25 μm) and washed with DCM (4x). Some resin beads were taken according to GP7 and analyzed via RP-HPLC, which indicated nearly complete conversion to **S-22.** The dried resin was treated with a mixture of HFIP/DCM (1/4, v/v) at room temperature for 4 h in total (4 x 30 min, 2 x 1 h). Thereby, all acid-labile protective groups were retained. The major portion of solvent was removed under a stream of nitrogen and the residual crude product additionally dried by lyophilization. The resulting yellow oil was used in the next step without further purification (81.8 mg, 14.3%).

Chemical formula: $C_{34}H_{53}N_3O_{10}$;
Molecular weight: 663.81 g/mol;
Exact mass: 663.37 g/mol;
$t_R$-value: 15.7 min (10 - 90% B in 15 min, Method A)
Capacity factor k: 9.5
ESI-MS: calculated monoisotopic mass ($C_{30}H_{45}NO_{10}$): 663.37;
found: ESI (positive ion mode): m/z = 552.4 [M(**S-22**)-2tBu+H]$^+$, 608.5 [M(**S-22**)-tBu+H]$^+$, 664.6 [M(**S-22**)+H]$^+$.

5-benzyl 1-(tert-butyl) (((S)-1-(tert-butoxy)-5-(((S)-1-(tert-butoxy)-1-oxooctan-2-yl)amino)-1,5-dioxo-pentan-2-yl)carbamoyl)-L-glutamate (**S-23**)

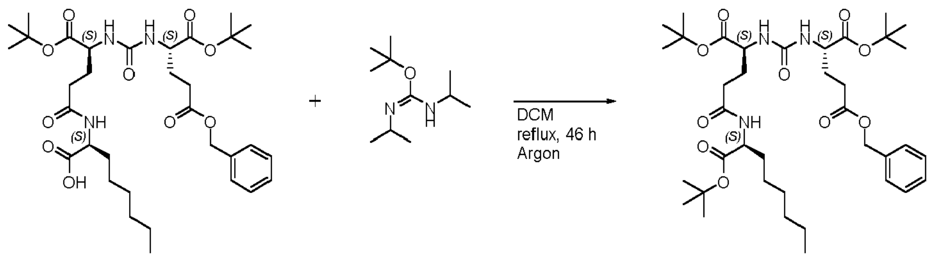


S-22
Exact Mass: 663,37
Molecular Weight: 663,81

S-8
Exact Mass: 200,19
Molecular Weight: 200,33

S-23
Exact Mass: 719,44
Molecular Weight: 719,92

Compound **S-23** was synthesized in analogy to a previously published procedure by Bergmeier et al. (J Org Chem. 1993, 58(9), 2369-76) with some minor modifications. Glassware and reagents were handled under air- and moisture-free conditions (GP9). Lyophilized educt **S-22** (crude product, -81.8 mg, 0.12 mmol, 1.00 eq.) was dissolved in 2 mL dry DCM and the first portion of O-tert-butyl-N,N'-diisopropylisourea (**S-8**) (40.1 μL, 0.18 mmol, 1.50 eq.) was added. The reaction mixture was stirred under reflux (~42 °C) and argon atmosphere for 22 h. A second portion of **S-8** (200 μL, 0.90 mmol, 7.49 eq.) was added and also DCM, in order to keep the solvent amount constantly between 2 and 3 mL. After stirring for further 24 h at reflux temperature and under argon atmosphere, the reaction was terminated by diluting the suspension with DCM. Solid by-products were removed by filtration and the organic layer was washed once

with $H_2O$. The aqueous phase was extracted twice with DCM. The combined organic phases were transferred into a round bottom flask and the solvent removed under reduced pressure. Purification by preparative RP-HPLC (70 - 90% B in 20 min, Method C, 5 mL/min) provided 41.4 mg (48%) of compound **S-23** as a colorless solid.

Chemical formula: $C_{38}H_{61}N_3O_{10}$;
Molecular weight: 719.92 g/mol;
Exact mass: 719.44 g/mol;
$t_R$-value: 19.2 min (10 - 90% B in 15 min, Method A)
Capacity factor k: 11.8
ESI-MS: calculated monoisotopic mass ($C_{38}H_{61}N_3O_{10}$): 719.44;
found: ESI (positive ion mode): m/z = 720.6 [M(**S-23**)+H]$^+$, 742.6 [M(**S-23**)+Na]$^+$, 758.6 [M(**S-23**)+K]$^+$.

(5S,10S,14S)-10,14-bis(tert-butoxycarbonyl)-5-hexyl-2,2-dimethyl-4,7,12-trioxo-3-oxa-6,11,13-triazaheptadecan-17-oic acid (**21**)

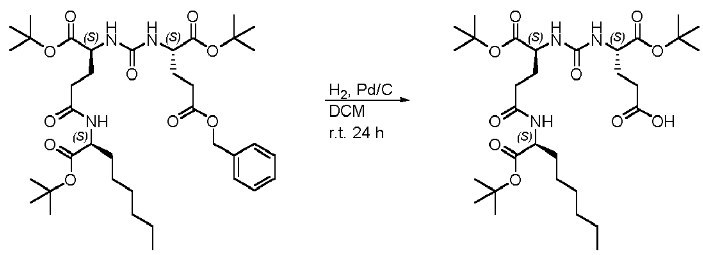


S-23
Exact Mass: 719,44
Molecular Weight: 719,92

21
Exact Mass: 629,39
Molecular Weight: 629,79

Compound **S-23** (41.4 mg, 57.5 μmol, 1.00 eq.) was dissolved in 6 mL DCM and the solution was flushed five minutes with argon to remove dissolved oxygen. 6.12 mg of palladium on carbon (10% wt) (corresponds to 0.61 mg palladium, 5.75 μmol, 0.10 eq.) were added and the flask was sealed with a rubber septum. Remaining air was displaced with argon and subsequently replaced by hydrogen gas. The mixture was stirred under hydrogen atmosphere at room temperature for 24 h. Palladium on carbon was filtered off, and the solvent was evaporated in vacuo. This afforded 35.5 mg (98.1%) of compound **21** as a colorless clear oil, which was used in the next step without further purification.

Chemical formula: $C_{31}H_{55}N_3O_{10}$;
Molecular weight: 629.79 g/mol;
Exact mass: 629.39 g/mol;
$t_R$-value: 15.8 min (10 - 90% B in 15 min, Method A)
Capacity factor k: 9.5
ESI-MS: calculated monoisotopic mass ($C_{31}H_{55}N_3O_{10}$): 629.39;
found: ESI (positive ion mode): m/z = 630.5 [M(**21**)+H]$^+$, 652.5 [M(**21**)+Na]$^+$.

L-Glu(L-2-Aoc-OtBu)-OtBu-(carbonyl)-L-Glu[D-Orn(Dde)-2-CT]-OtBu (**S-24**)

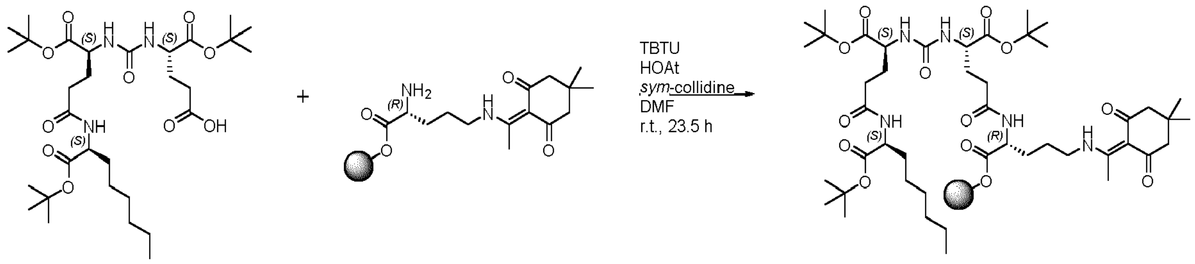


**21**
Exact Mass: 629,39
Molecular Weight: 629,79

**18**
Exact Mass: 296,17
Molecular Weight: 296,37

**S-24**
Exact Mass: 907,55
Molecular Weight: 908,14

According to GP2, fragment **21** (35.5 mg, 56.4 μmol, 1.00 eq.) was coupled to resin-bound H-D-Orn(Dde) (**18**) (81.2 μmol 1.44 eq.), with TBTU (36.2 mg, 0.11 mmol, 2.00 eq.) and HOAt (15.0 mg, 0.11 mmol, 2.00 eq.) as coupling reagents and sym-collidine (67.7 μL, 0.51 mmol, 9.00 eq.) as base. After shaking for 23.5 h at room temperature, formation of product **S-24** could be confirmed (GP7), as only one major peak with the expected m/z-ratio of 908.4 occurred.

Chemical formula: $C_{46}H_{77}N_5O_{13}$;
Molecular weight: 908.14 g/mol;
Exact mass: 907.55 g/mol;
$t_R$-value: 12.7 min (40 - 95% B in 15 min, Method A)
Capacity factor k: 7.5
ESI-MS: calculated monoisotopic mass ($C_{46}H_{77}N_5O_{13}$): 907.55;
found: ESI (positive ion mode): m/z = 454.8 [M(**S-24**)+2H]$^{2+}$, 908.4 [M(**S-24**)+H]$^{+}$.

Proinhibitor II (6)

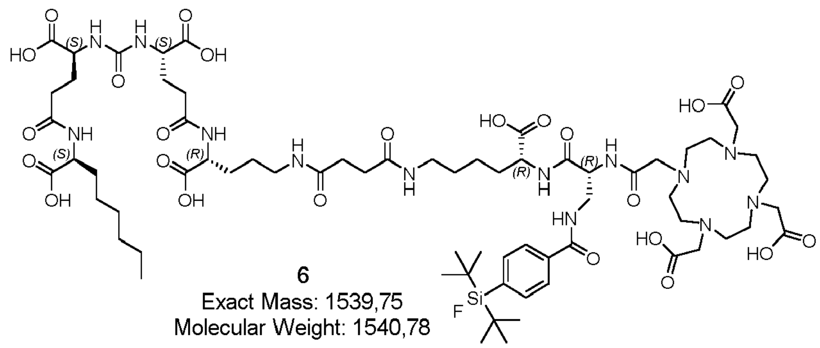

**6**
Exact Mass: 1539,75
Molecular Weight: 1540,78

Further reactions on resin-bound compound **S-24** were performed according to standard Fmoc-SPPS on 2-CT resin, applying the above-mentioned methods (GP2 - GP8). In brief, the Dde protective group was removed (GP4) and succinic anhydride (56.7 mg, 0.57 mmol, 7.00 eq.) was coupled (GP2) over a period of at least 2.5 h, only using DIPEA (96.4 μL, 0.57 mmol, 7.00 eq.) and no further coupling reagents. The peptide was elongated with Fmoc-D-Lys-OtBu*HCl (74.7 mg, 0.16 mmol, 2.00 eq.). Therefore, the resin-bound acid was preactivated for five minutes using TBTU (52.0 mg, 0.16 mmol, 2.00 eq.), HOAt (22.0 mg, 0.16 mmol, 2.00 eq.) and DIPEA (82.7 μL, 0.49 mmol, 6.00 eq.). The amino acid was dissolved in DMF, added to the preactivated resin and shaken for at least 2.5 h. Fmoc-removal was conducted (GP3) and Fmoc-D-Dap(Dde)-OH (79.5 mg, 0.16 mmol, 2.00 eq.) was coupled for at least 2.5 h using TBTU (52.0 mg, 0.16 mmol, 2.00 eq.), HOAt (22.0 mg, 0.16 mmol, 2.00 eq.) and sym-collidine (75.2 μL, 0.57 mmol, 7.00 eq.) as coupling reagents. Afterwards, Dde-removal was performed according to GP5. The SiFA-BA moiety (12.0 mg, 42.5 μmol, 0.53 eq.) was attached using TBTU (52.0 mg, 0.16 mmol, 2.00 eq.), HOAt (22.0 mg, 0.16 mmol, 2.00 eq.) and sym-collidine (75.2 μL, 0.57 mmol, 7.00 eq.) as coupling reagents. After an incubation time of at least 2 h, the Fmoc protective group was removed (GP3) and the DOTA chelator moiety was added to the resin. Therefore, DOTA-NHS (67.8 mg, 89.6 μmol, 1.10eq.) and DIPEA (118 μL, 8.52 mmol, 7.00 eq.) were each dissolved in DMF. First, DIPEA in DMF was added to the resin for preactivation. After five minutes, DOTA-NHS in DMF was added and incubated with the resin-bound amine for

23.2 h. An adequate conversion (RP-HPLC/MS analysis after GP6) was achieved and the peptide was cleaved off the resin with TFA/TIPS/DCM (95/2.5/2.5, GP8) and purified afterwards by preparative RP-HPLC (40 - 70% C(A) in 20 min). Subsequent lyophilization afforded 20.0 mg (16.0% yield, chemical purity >99%) of pure product **6** as a colorless powder.

Chemical formula: $C_{68}H_{110}FN_{13}O_{24}Si$;
Molecular weight: 1540.78 g/mol;
Exact mass: 1539.75 g/mol;
$t_R$-value: 9.44 min (10 - 90% B in 15 min, Method A)
Capacity factor k: 5.5
ESI-MS: calculated monoisotopic mass ($C_{68}H_{110}FN_{13}O_{24}Si$): 1539.75;
found: ESI (positive ion mode): m/z = 770.5 $[M(\mathbf{6})+2H]^{2+}$, 1539.9 $[M(\mathbf{6})+H]^{+}$, 1924.9 $[M_5(\mathbf{6})+4H]^{4+}$.

$^{nat}$Lu-proinhibitor II ($^{nat}$Lu-**6**)

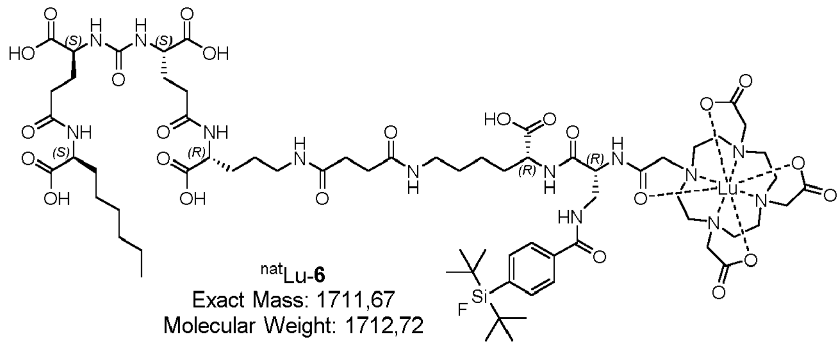

$^{nat}$Lu-**6**
Exact Mass: 1711,67
Molecular Weight: 1712,72

100 µL of the precursor (**6**) (2.00 mM in DMSO, 0.20 µmol, 1.00 eq.) were added to 60.0 µL of $LuCl_3$ (20 mM in Tracepur®-$H_2O$, 1.20 µmol, 6.00 eq.) and 40.0 µL Tracepur®-$H_2O$. The reaction mixture was heated for 30 min at 95 °C and afforded $^{nat}$Lu-**5** in >99% chemical purity (>99% yield), determined by RP-HPLC (220 nm). This $^{nat}$Lu-**6** solution (now 1.00 mM) was directly used as stock solution for affinity determination.

Chemical formula: $C_{68}H_{107}FLuN_{13}O_{24}Si$;
Molecular weight: 1712.72 g/mol;
Exact mass: 1711.67 g/mol;
$t_R$-value: 9.48 min (10 - 90% B in 15 min, Method A)
Capacity factor k: 5.3
ESI-MS: calculated monoisotopic mass ($C_{68}H_{107}FLuN_{13}O_{24}Si$): 1711.67;
found: ESI (positive ion mode): m/z = 856.4 $[M(^{nat}Lu\text{-}\mathbf{6})+2H]^{2+}$, 1711.6 $[M(^{nat}Lu\text{-}\mathbf{6})+H]^{+}$.

[$^{177}$Lu]Lu-proinhibitor II ([$^{177}$Lu]Lu-**6**)

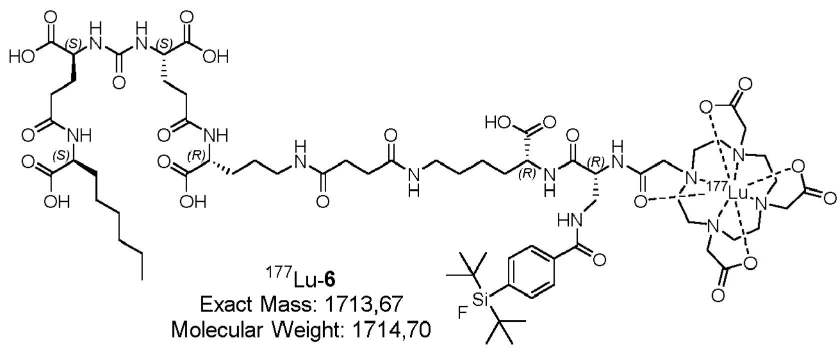

$^{177}$Lu-**6**
Exact Mass: 1713,67
Molecular Weight: 1714,70

5.00 μL of the precursor (**6**) (0.20 mM in DMSO, 1.00 nmol, 1.00 eq.) were added to 10.0 μL of 1 M NaOAc buffer (aq.) (pH = 5.5). Subsequently, 28.0 or 32.5 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0 μL of 0.1 M sodium ascorbate (aq.) (in Tracepur®-$H_2O$) were added and the reaction mixture was heated for 25 min at 95 °C. Removal of free [$^{177}$Lu]$Lu^{3+}$ via HLB cartridge (30 mg) was required, which afforded [$^{177}$Lu]Lu-**6** in 81.4 ± 6.9% (n = 2) isolated RCY (d.c. to the start of synthesis). The apparent $A_m$ were 24.1 and 24.8 GBq/μmol at the end of synthesis. RCP as determined by radio-RP-HPLC and radio-TLC was 98.2 ± 0.1%.

Chemical formula: $C_{68}H_{107}F^{177}LuN_{13}O_{24}Si$;
Molecular weight: 1714.70 g/mol;
Exact mass: 1713.67 g/mol;
$t_R$-value: 11.1 min (10 - 90% B in 15 min, Method A)
$t_R$-value of co-injected cold standard: 11.0 min (10 - 90% B in 15 min, Method A)
Capacity factor k: 6.4.

**Proinhibitor III (7)**

(S)-2-((S)-2-(((benzyloxy)carbonyl)amino)-5-(tert-butoxy)-5-oxopentanamido)octanoic acid **(S-25)**

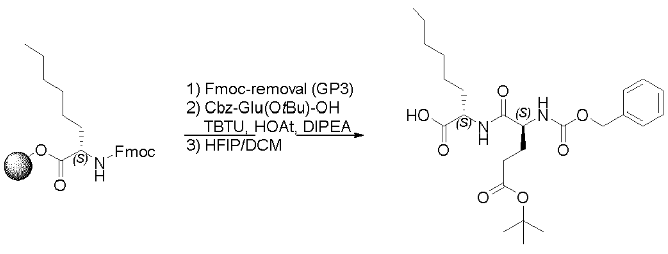


**S-20**
Exact Mass: 381,19
Molecular Weight: 381,47

**S-25**
Exact Mass: 478,27
Molecular Weight: 478,59

Fmoc-L-2-Aminooctanoic acid was coupled to 2-CTC resin according to GP1. Further reactions on compound **S-20** (load: 0.59 mmol/g, 0.63 mmol, 1.00 eq., n = 3) were performed according to standard Fmoc-SPPS on 2-CT resin, applying the above-mentioned methods (GP2 & GP3). In brief, the Fmoc protective group was removed (GP3) and Cbz-L-Glu(OtBu)-OH (321 mg, 0.95 mmol, 1.50 eq.) was coupled (GP2) over a period of 2 h, using TBTU (405 mg, 1.26 mmol, 2.00 eq.), HOAt (171 mg, 1.26 mmol, 2.00 eq.) and DIPEA (582 μL, 3.42 mmol, 5.43 eq.). Some resin beads were taken and treated with TFA according to GP6. RP-HPLC/MS analysis revealed nearly complete conversion to product **S-25**. The protected dipeptide was cleaved off the resin by incubation with HFIP/DCM (1/4) for 4 h in total (4 x 30 min, 2 x 1 h). Flash chromatography purification (40 - 90% B in 10 min, Method D, 12 mL/min) of the crude product provided 649 mg (72.0%) of compound **S-25** as a colorless, viscous oil.

Chemical formula: $C_{25}H_{38}N_2O_7$;
Molecular weight: 478.59 g/mol;
Exact mass: 478.27 g/mol;
$t_R$-value: 14.8 min (10 - 90% B in 15 min, Method A)
Capacity factor k: 8.9
ESI-MS: calculated monoisotopic mass ($C_{25}H_{38}N_2O_7$): 478.27;
found: ESI (positive ion mode): m/z = 422.8 [M(**S-25**)-tBu+H]$^+$, 478.8 [M(**S-25**)+H]$^+$, 500.7 [M(**S-25**)+Na]$^+$, 956.8 [$M_2$(**S-25**)+H]$^+$, 978.8 [$M_2$(**S-25**)+Na]$^+$, 994.7 [$M_2$(**S-25**)+K]$^+$.

Tert-butyl (S)-2-((S)-2-(((benzyloxy)carbonyl)amino)-5-(tert-butoxy)-5-oxopentanamido)octanoate (**S-26**)

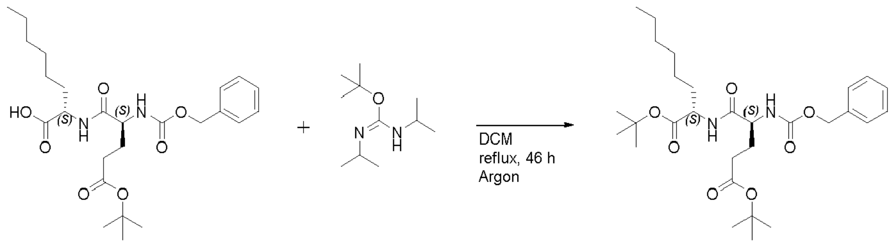


**S-25** Exact Mass: 478,27 Molecular Weight: 478,59

**S-8** Exact Mass: 200,19 Molecular Weight: 200,33

**S-26** Exact Mass: 534,33 Molecular Weight: 534,69

Compound **S-26** was synthesized in analogy to a previously published procedure by Bergmeier et al. (J Org Chem. 1993, 58(9), 2369-76) with some minor modifications. Glassware and reagents were handled under air- and moisture-free conditions (GP9). Lyophilized educt **S-25** (649 mg, 1.36 mmol, 1.00 eq.) was dissolved in 2 mL dry DCM and the first portion of O-tert-butyl-N,N'-diisopropylisourea (**S-8**) (454 μL, 2.04 mmol, 1.50 eq.) was added. The reaction mixture was stirred under reflux (~42 °C) and argon atmosphere for 22 h. A second portion of **S-8** (454 μL, 2.04 mmol, 1.50 eq.) was added and also DCM, in order to keep the solvent amount constantly between 2 and 3 mL. After stirring for further 24 h at reflux temperature and under argon atmosphere, the reaction was terminated by diluting the suspension with DCM. Solid by-products were removed by filtration and the organic layer washed once with $H_2O$. The aqueous phase was extracted twice with DCM. The combined organic phases were transferred into a round bottom flask and the solvent removed under reduced pressure. Purification by flash chromatography (40 - 90% B in 10 min, Method D, 12 mL/min) provided 481 mg (66.2%) of compound **S-26** as a colorless solid.

Chemical formula: $C_{29}H_{46}N_2O_7$;
Molecular weight: 534.69 g/mol;
Exact mass: 534.33 g/mol;
$t_R$-value: 16.7 min (10 - 90% B in 15 min, Method A)
Capacity factor k: 10.1
ESI-MS: calculated monoisotopic mass ($C_{29}H_{46}N_2O_7$): 534.33;
found: ESI (positive ion mode): m/z = 423.0 [M(**S-26**)-2tBu+H]$^+$, 479.1 [M(**S-26**)-tBu+H]$^+$, 535.2 [M(**S-26**)+H]$^+$, 557.0 [M(**S-26**)+Na]$^+$, 579.2 [M(**S-26**)+K]$^+$.

Tert-butyl (S)-2-((S)-2-amino-5-(tert-butoxy)-5-oxopentanamido)octanoate (**S-27**)

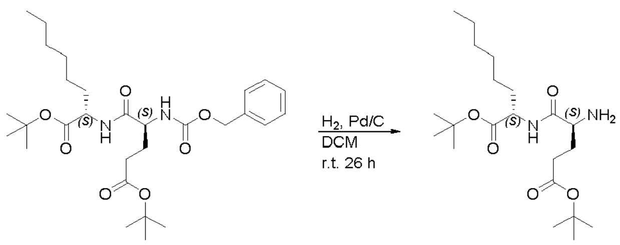


**S-26** Exact Mass: 534,33 Molecular Weight: 534,69

**S-27** Exact Mass: 400,29 Molecular Weight: 400,56

Compound **S-26** (481 mg, 0.89 mmol, 1.00 eq.) was dissolved in 10 mL DCM and the solution was flushed 5 minutes with argon to remove dissolved oxygen. 94.2 mg of palladium on carbon (10% wt) (corresponds to 9.42 mg palladium, 89.0 μmol, 0.10 eq.) were added and the flask was sealed with a rubber septum. Remaining air was displaced with argon and subsequently replaced by hydrogen gas. The mixture was stirred under hydrogen atmosphere at room temperature for 26 h. Palladium on carbon was filtered off, and the solvent was evaporated in vacuo. This afforded 162 mg (45.3%) of compound **S-27** as a slightly brown viscous oil, which was used in the next step without further purification.

Chemical formula: $C_{21}H_{40}N_2O_5$;
Molecular weight: 400.56 g/mol;

Exact mass: 400.29 g/mol;
$t_R$-value: 7.07 min (40 - 95% B in 15 min, Method A)
Capacity factor k: 3.7
ESI-MS: calculated monoisotopic mass ($C_{21}H_{40}N_2O_5$): 400.29;
found: ESI (positive ion mode): m/z = 401.0 $[M(\textbf{S-27})+H]^+$, 422.9 $[M(\textbf{S-27})+Na]^+$, 438.9 $[M(\textbf{S-27})+K]^+$, 801.0 $[M_2(\textbf{S-27})+H]^+$, 822.9 $[M_2(\textbf{S-27})+Na]^+$.

5-Benzyl 1-(tert-butyl) (1H-imidazole-1-carbonyl)-L-glutamate (**S-28**)

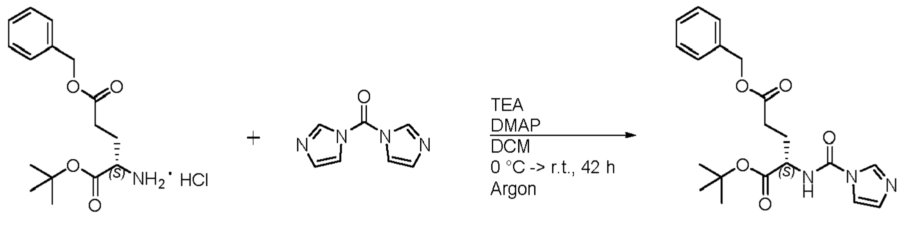


**S-11** Exact Mass: 295,16 Molecular Weight: 295,81

**S-9** Exact Mass: 162,05 Molecular Weight: 162,15

**S-28** Exact Mass: 387,18 Molecular Weight: 387,44

Compound **S-11** (396 mg, 1.20 mmol, 1.00 eq.) was dissolved in 10 mL DCE and cooled to 0 °C. Triethylamine (416 μL, 3.00 mmol, 2.50 eq.), DMAP on polystyrene (3.00 mmol/g, 16.0 mg, 48.0 μmol, 0.04 eq.) and 1,1'-carbonyldiimidazole (**S-9**) (214 mg, 1.32 mmol, 1.10 eq.) were added and the mixture was stirred for further 5 min at 0 °C. Afterwards, it was warmed to room temperature and stirred for 42 h under argon atmosphere. DMAP was filtered off and the solution was washed once with saturated $NaHCO_3$, brine and $H_2O$. The combined organic phases were dried over $Na_2SO_4$, filtered and the solvent was removed under reduced pressure. The obtained crude product (439 mg, 94.5%) was analyzed via RP-HPLC/MS and revealed almost exclusively product **S-28**, which was used in the next step without further purification.

Chemical formula: $C_{20}H_{25}N_3O_5$;
Molecular weight: 387.44 g/mol;
Exact mass: 387.18 g/mol;
$t_R$-value: 10.1 min (10 - 90% B in 15 min, Method A)
Capacity factor k: 5.7
ESI-MS: calculated monoisotopic mass ($C_{20}H_{25}N_3O_5$): 387.18;
found: ESI (positive ion mode): m/z = 388.2 $[M(\textbf{S-28})+H]^+$.

5-Benzyl 1-(tert-butyl) (((S)-5-(tert-butoxy)-1-(((S)-1-(tert-butoxy)-1-oxooctan-2-yl)amino)-1,5-dioxopentan-2-yl)carbamoyl)-L-glutamate (**S-29**)

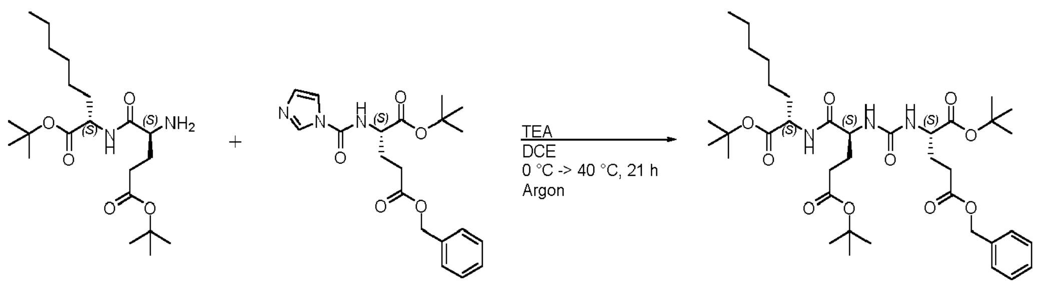


**S-27** Exact Mass: 400,29 Molecular Weight: 400,56

**S-28** Exact Mass: 387,18 Molecular Weight: 387,44

**S-29** Exact Mass: 719,44 Molecular Weight: 719,92

Compound **S-29** was synthesized in analogy to a previously published procedure by Weineisen et al. (EJNMMI Research. 2014, 4(1), 63) with some minor modifications. The H-L-Glu(OtBu)-L-2-Aoc-OtBu dipeptide **S-27** (162 mg, 0.40 mmol, 1.10 eq.) was dissolved in 10 mL DCE and compound **S-28** (141 mg, 0.36 mmol, 1.00 eq.) was added. At 0 °C, triethylamine (139 μL, 1.00 mmol, 2.78 eq.) was added and the mixture was stirred for further five minutes at 0 °C. The solution was warmed to 40 °C and stirred for 21 h under argon atmosphere. The reaction mixture was washed once with $H_2O$ and brine. The combined aqueous phases were extracted once with DCM. The entire organic layer was dried over $Na_2SO_4$, filtered and the solvent removed in vacuo. Purification of the crude product by preparative RP-HPLC (45 - 90% B in 20 min, Method B, 5 mL/min) afforded 128 mg (49%) of compound **S-29** as a colorless clear viscous oil.

Chemical formula: $C_{38}H_{61}N_3O_{10}$;
Molecular weight: 719.92 g/mol;
Exact mass: 719.44 g/mol;
$t_R$-value: 17.5 min (40 - 95% B in 15 min, Method A)
Capacity factor k: 10.7
ESI-MS: calculated monoisotopic mass ($C_{38}H_{61}N_3O_{10}$): 719.44;
found: ESI (positive ion mode): m/z = 720.0 [M(**S-29**)+H]$^+$, 742.0 [M(**S-29**)+MeCN+H]$^+$.

(5S,8S,12S)-8-(3-(tert-butoxy)-3-oxopropyl)-12-(tert-butoxycarbonyl)-5-hexyl-2,2-dimethyl-4,7,10-trioxo-3-oxa-6,9,11-triazapentadecan-15-oic acid (**22**)

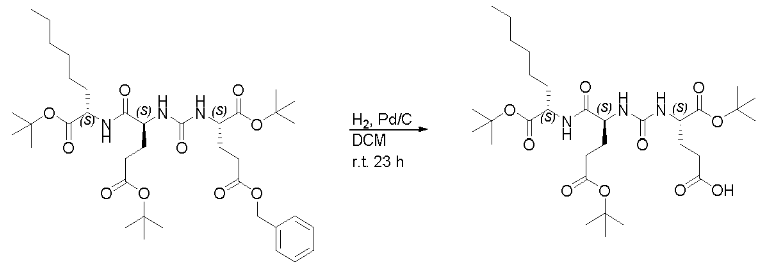


**S-29**
Exact Mass: 719,44
Molecular Weight: 719,92

**22**
Exact Mass: 629,39
Molecular Weight: 629,79

Compound **S-29** (128 mg, 0.18 mmol, 1.00 eq.) was dissolved in 6 mL DCM and the solution was flushed 5 minutes with argon to remove dissolved oxygen. 19.2 mg of palladium on carbon (10% wt) (corresponds to 1.92 mg palladium, 18.0 μmol, 0.10 eq.) were added and the flask was sealed with a rubber septum. Remaining air was displaced with argon and subsequently replaced by hydrogen gas. The mixture was stirred under hydrogen atmosphere at room temperature for 23 h. Palladium on carbon was filtered off, and the solvent was evaporated in vacuo. This afforded 102 mg (89.9%) of compound **22** as a colorless solid, which was used in the next step without further purification.

Chemical formula: $C_{31}H_{55}N_3O_{10}$;
Molecular weight: 629.79 g/mol;
Exact mass: 629.39 g/mol;
$t_R$-value: 13.1 min (40 - 95% B in 15 min, Method A)
Capacity factor k: 7.7
ESI-MS: calculated monoisotopic mass ($C_{31}H_{55}N_3O_{10}$): 629.39;
found: ESI (positive ion mode): m/z = 630.0 [M(**22**)+H]$^+$, 651.9 [M(**22**)+Na]$^+$, 667.9 [M(**22**)+K]$^+$.

L-Glu(L-2-Aoc-OtBu)-OtBu-(carbonyl)-L-Glu[D-Orn(Dde)-2-CT]-OtBu (S-30)

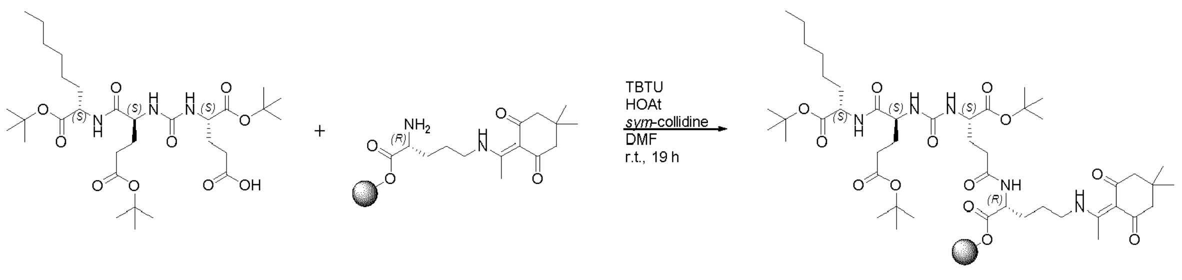


22
Exact Mass: 629,39
Molecular Weight: 629,79

18
Exact Mass: 296,17
Molecular Weight: 296,37

S-30
Exact Mass: 907,55
Molecular Weight: 908,14

According to GP2, fragment **22** (102 mg, 0.16 mmol, 1.00 eq.) was coupled to resin-bound H-D-Orn(Dde) (**18**) (0.23 mmol 1.44 eq.), with TBTU (104 mg, 0.32 mmol, 2.00 eq.) and HOAt (44.0 mg, 0.32 mmol, 2.00 eq.) as coupling reagents and sym-collidine (191 μL, 1.44 mmol, 9.00 eq.) as base. After shaking for 19 h at room temperature, formation of product **S-30** could be confirmed (GP7), as only one major peak with the expected m/z-ratio of 908.0 occurred.

Chemical formula: $C_{46}H_{77}N_5O_{13}$;
Molecular weight: 908.14 g/mol;
Exact mass: 907.55 g/mol;
$t_R$-value: 13.7 min (40 - 95% B in 15 min, Method A)
Capacity factor k: 8.1
ESI-MS: calculated monoisotopic mass ($C_{46}H_{77}N_5O_{13}$): 907.55;
found: ESI (positive ion mode): m/z = 908.0 [M(**S-30**)+H]$^+$, 1814.9 [$M_2$(**S-30**)+H]$^+$.

Proinhibitor III (**7**)

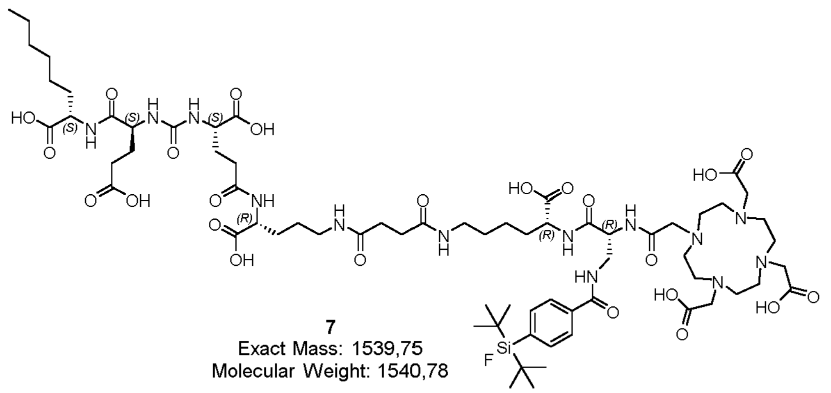

**7**
Exact Mass: 1539,75
Molecular Weight: 1540,78

Further reactions on resin-bound compound **S-30** (0.23 mmol, 1.00 eq.) were performed according to standard Fmoc-SPPS on 2-CT resin, applying the above-mentioned methods (GP2 - GP8). In brief, the Dde protective group was removed (GP4) and succinic anhydride (161 mg, 1.61 mmol, 7.00 eq.) was coupled (GP2) over a period of at least 2.5 h, only using DIPEA (374 μL, 2.20 mmol, 9.56 eq.) and no further coupling reagents. The peptide was elongated with Fmoc-D-LysOtBu*HCl (212 mg, 0.46 mmol, 2.00 eq.). Therefore, the resin-bound acid was preactivated for five minutes by using TBTU (148 mg, 0.46 mmol, 2.00 eq.), HOAt (63.0 mg, 0.46 mmol, 2.00 eq.) and DIPEA (335 μL, 1.97 mmol, 8.56 eq.). The amino acid was dissolved in DMF, added to the preactivated resin and shaken for at least 2.5 h. Fmoc-removal was conducted (GP3) and Fmoc-D-Dap(Dde)-OH (226 mg, 0.46 mmol, 2.00 eq.) was coupled for at least 2.5 h using TBTU (148 mg, 0.46 mmol, 2.00 eq.), HOAt (63.0 mg, 0.46 mmol, 2.00 eq.) and sym-collidine (213 μL, 1.61 mmol, 7.00 eq.) as coupling reagents. Afterwards, Dde-removal was performed according to GP5. The SiFA-BA moiety (64.9 mg, 0.23 mmol, 1.00 eq.) was attached using TBTU (148 mg, 0.46 mmol, 2.00 eq.), HOAt (63.0 mg, 0.46 mmol, 2.00 eq.) and sym-collidine (213 μL, 1.61 mmol, 7.00 eq.) as coupling reagents and incubation for at least 2 h. Fmoc protective group removal (GP3) was performed followed by coupling of the DOTA moiety. Therefore, DOTA-NHS (175 mg, 0.23 mmol, 1.00 eq.) and DIPEA (324 μL, 1.91 mmol, 8.28 eq.) were each dissolved in DMF. First, DIPEA in DMF

was added to the resin for preactivation. After five minutes, DOTA-NHS in DMF was added and incubated with the resin-bound amine for 16 h. An adequate conversion (RP-HPLC/MS analysis after GP6) was reached and the peptide was cleaved off the resin with TFA/TIPS/DCM (95/2.5/2.5/, GP8) and purified afterwards by preparative RP-HPLC (40 - 65% B in 20 min, Method B, 5 mL/min). Subsequent lyophilization afforded 16.6 mg (4.68% yield, chemical purity >99%) of pure product **7** as a colorless powder.

Chemical formula: $C_{68}H_{110}FN_{13}O_{24}Si$;
Molecular weight: 1540.78 g/mol;
Exact mass: 1539.75 g/mol;
$t_R$-value: 9.15 min (10 - 90% B in 15 min, Method A)
Capacity factor k: 5.1
ESI-MS: calculated monoisotopic mass ($C_{68}H_{110}FN_{13}O_{24}Si$): 1539.75; found: ESI (positive ion mode): m/z = 770.5 [M(**7**)+2H]$^{2+}$, 1539.9 [M(**7**)+H]$^+$.

$^{nat}$Lu-proinhibitor III ($^{nat}$Lu-**7**)

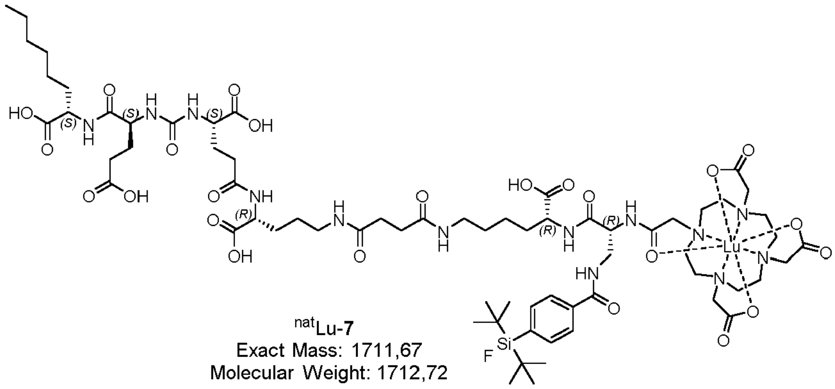

$^{nat}$Lu-**7**
Exact Mass: 1711,67
Molecular Weight: 1712,72

100 μL of the precursor (**7**) (2.00 mM in DMSO, 0.20 μmol, 1.00 eq.) were added to 60.0 μL of $LuCl_3$ (20 mM in Tracepur®-$H_2O$, 1.20 μmol, 6.00 eq.) and 40.0 μL Tracepur®-$H_2O$. The reaction mixture was heated for 30 min at 95 °C and afforded $^{nat}$Lu-**7** in >99% chemical purity (>99% yield), determined by RP-HPLC (220 nm). This $^{nat}$Lu-**7** solution (now 1.00 mM) was directly used as stock solution for affinity determination.

Chemical formula: $C_{68}H_{107}FLuN_{13}O_{24}Si$;
Molecular weight: 1712.72 g/mol;
Exact mass: 1711.67 g/mol;
$t_R$-value: 9.29 min (10 - 90% B in 15 min, Method A)
Capacity factor k: 5.2
ESI-MS: calculated monoisotopic mass ($C_{68}H_{107}FLuN_{13}O_{24}Si$): 1711.67;
found: ESI (positive ion mode): m/z = 856.1 [M($^{nat}$Lu-**7**)+2H]$^{2+}$, 1711.1 [M($^{nat}$Lu-**7**)+H]$^+$.

[$^{177}$Lu]Lu-proinhibitor III ([$^{177}$Lu]Lu-**7**)

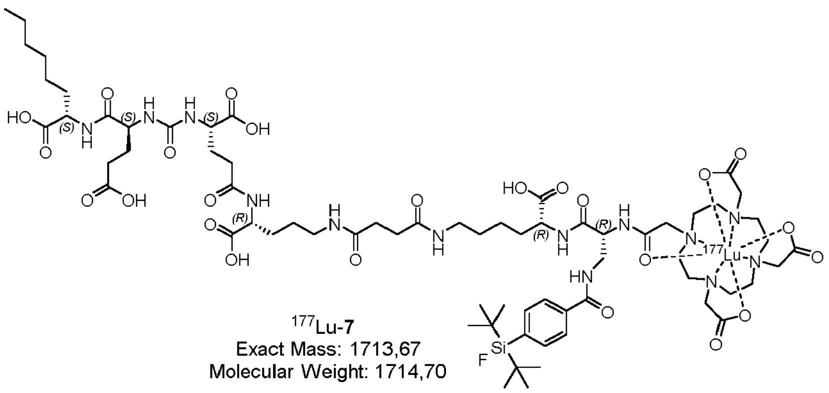

$^{177}$Lu-**7**
Exact Mass: 1713,67
Molecular Weight: 1714,70

5.00 μL of the precursor (**7**) (0.20 mM in DMSO, 1.00 nmol, 1.00 eq.) were added to 10.0 μL of 1 M NaOAc buffer (aq.) (pH = 5.5). Subsequently, 27.4 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0 μL of 0.1 M sodium ascorbate (aq.) (in Tracepur®-$H_2O$) were added and the reaction mixture was heated for 25 min at 95 °C. This afforded [$^{177}$Lu]Lu-**7** in >99% (n = 1) isolated RCY (d.c. to the start of synthesis). The apparent $A_m$ was 27.4 GBq/μmol at the end of synthesis. RCP as determined by radio-RP-HPLC and radio-TLC was 94.7%.

Chemical formula: $C_{68}H_{107}F^{177}LuN_{13}O_{24}Si$;
Molecular weight: 1714.70 g/mol;
Exact mass: 1713.67 g/mol;
$t_R$-value: 11.0 min (10 - 90% B in 15 min, Method A)
$t_R$-value of co-injected cold standard: 10.9 min (10 - 90% B in 15 min, Method A)
Capacity factor k: 6.3.

**Synthesis of PSMA-binding motifs with substituents & bioisosteres of the P1'-γ-carboxylic acid 2-Aminoheptanoic acid derivative 8**

**Tert-butyl (S)-2-aminoheptanoate (23)**

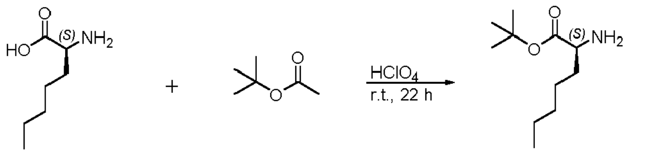


**S-31** Exact Mass: 145,11 Molecular Weight: 145,20

**S-32** Exact Mass: 116,08 Molecular Weight: 116,16

**23** Exact Mass: 201,17 Molecular Weight: 201,31

Compound **23** was synthesized in analogy to a previously published procedure by Hyun et al. (J Am Chem Soc. 2010, 132(48), 17053-5) with some minor modifications. (S)-2-Aminoheptanoic acid (**S-31**) (300 mg, 2.07 mmol, 1.00 eq.) was dissolved in 7.13 mL of tert-butyl acetate (**S-32**) and 70% (v/v) perchloric acid (aq.) (384 μL, 4.74 mmol, 2.29 eq.) was added slowly. The reaction mixture was stirred for 22 h at room temperature. $H_2O$ and some drops of 0.3 M HCl were added for quenching. The pH value of the aqueous phase was adjusted to 9 with 10% (w/v) $Na_2CO_3$ (aq.) and extracted three times with DCM. The combined organic phases were dried over $Na_2SO_4$, filtered and the solvent was removed under reduced pressure. Purification by column chromatography (DCM/MeOH/acetone = 5/1/1) yielded 446 mg (>99%) of pure product **23** as a slightly yellow viscous oil.

Chemical formula: $C_{11}H_{23}NO_2$;
Molecular weight: 201.31 g/mol;
Exact mass: 201.17 g/mol;
$t_R$-value: not detectable at 220/254 nm
$R_f$-value: 0.76 (DCM/MeOH/acetone = 5/1/1)

ESI-MS: calculated monoisotopic mass ($C_{11}H_{23}NO_2$): 201.17;
found: ESI (positive ion mode): m/z = 202.0 [M(**23**)+H]$^+$, 243.0 [M(**23**)+MeCN+H]$^+$;
$^1$H-NMR (300 MHz, $CDCl_3$) $\delta$(ppm): 3.75 (t, $^3J$ = 6.2 Hz, 1H, H(1)), 2.04 (s, 2H, H(2)), 1.91 - 1.70 (m, 2H, H(3)), 1.48 (s, 9H, H(4, 4', 4")), 1.31 (virt. td, 6H, H(5, 6, 7)), 0.93 - 0.78 (m, 3H, H(8)).
$^{13}$C-NMR (75 MHz, $CDCl_3$) $\delta$(ppm): 171.02 (s, 1C, C(1)), 83.45 (s, 1C, C(2)), 54.35 (s, 1C, C(3)), 32.18 (s, 1C, C(4)), 31.41 (s, 1C, C(5)), 28.09 (s, 3C, C(6, 6', 6")), 24.56 (s, 1C, C(7)), 22.43 (s, 1C, C(8)), 14.03 (s, 1C, C(9)).

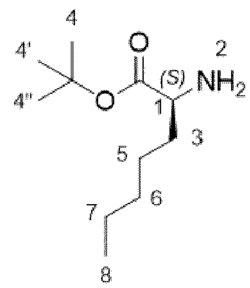

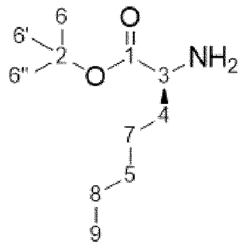

**23**

L-2-Aha-OtBu-(carbonyl)-L-Glu[D-Orn(Dde)-2-CT]-OtBu (S-33)

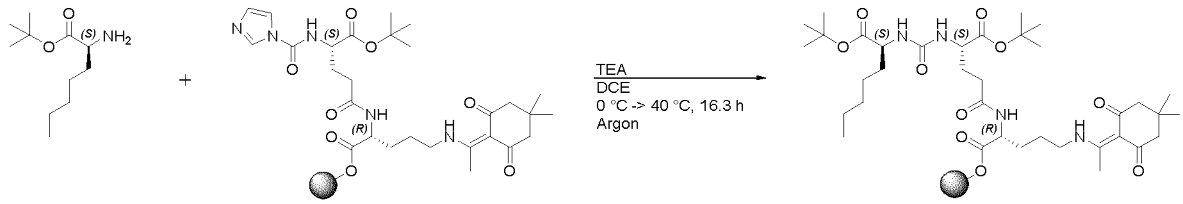


**23**
Exact Mass: 201,17
Molecular Weight: 201,31

**20**
Exact Mass: 575,30
Molecular Weight: 575,66

**S-33**
Exact Mass: 708,43
Molecular Weight: 708,89

Compound **20** (0.24 mmol, 1.00 eq.) was transferred into a round bottom flask and dissolved in 2.50 mL DCE. At 0 °C, triethylamine (83.2 $\mu$L, 0.60 mmol, 2.50 eq.) and tert-butyl (S)-2-aminoheptanoate (23) (60.9 mg, 0.30 mmol, 1.25 eq.) were added and the mixture was stirred for further 5 min at 0 °C. Afterwards, it was warmed to 40 °C and stirred for 16.3 h under argon atmosphere. The resin was washed with DCM (4x), some resin beads were taken and treated with HFIP/DCM (1/4) according to GP7. RP-HPLC/MS analysis revealed nearly complete conversion to product **S-33.**

Chemical formula: $C_{36}H_{60}N_4O_{10}$;
Molecular weight: 708.89 g/mol;
Exact mass: 708.43 g/mol;
$t_R$-value: 14.2 min (10 - 90% B in 15 min)
Capacity factor k: 8.5
ESI-MS: calculated monoisotopic mass ($C_{36}H_{60}N_4O_{10}$): 708.43;
found: ESI (positive ion mode): m/z = 709.1 [M(**S-33**)+H]$^+$, 731.1 [M(**S-33**)+Na]$^+$.

2-Aminoheptanoic acid derivative **8**

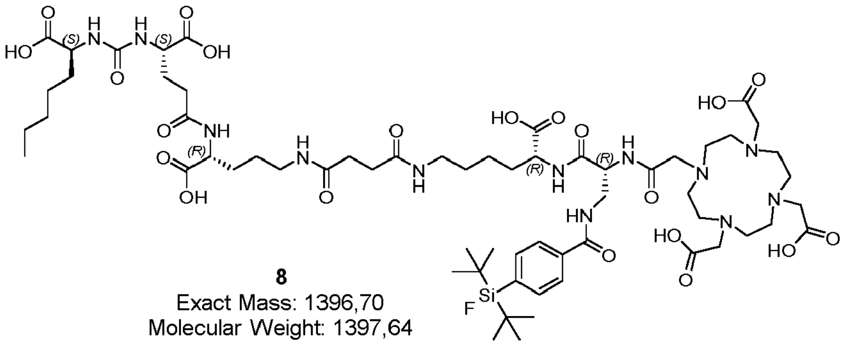

8
Exact Mass: 1396,70
Molecular Weight: 1397,64

Further reactions on resin-bound compound **S-33** (0.24 mmol, 1.00 eq.) were performed according to standard Fmoc-SPPS on 2-CT resin, applying the above-mentioned methods (GP2 - GP8). In brief, the Dde protective group was removed (GP4) and succinic anhydride (170 mg, 1.68 mmol, 7.00 eq.) was coupled (GP2) over a period of at least 2.5 h, only using DIPEA (286 μL, 1.68 mmol, 7.00 eq.) and no further coupling reagents. The peptide was elongated with Fmoc-D-LysOtBu*HCl (221 mg, 0.48 mmol, 2.00 eq.). Therefore, the resin-bound acid was preactivated for five minutes using TBTU (154 mg, 0.48 mmol, 2.00 eq.), HOAt (65.3 mg, 0.48 mmol, 2.00 eq.) and DIPEA (245 μL, 1.44 mmol, 6.00 eq.). The amino acid was dissolved in DMF, added to the preactivated resin and shaken for at least 2.5 h. Fmoc-removal was conducted (GP3) and Fmoc-D-Dap(Dde)-OH (235 mg, 0.48 mmol, 2.00 eq.) was coupled for at least 2.5 h using TBTU (154 mg, 0.48 mmol, 2.00 eq.), HOAt (65.3 mg, 0.48 mmol, 2.00 eq.) and sym-collidine (223 μL, 1.68 mmol, 7.00 eq.) as coupling reagents. Afterwards, Dde-removal was performed according to GP5. The SiFA-BA moiety (67.7 mg, 0.24 mmol, 1.00 eq.) was attached using TBTU (154 mg, 0.48 mmol, 2.00 eq.), HOAt (65.3 mg, 0.48 mmol, 2.00 eq.) and sym-collidine (223 μL, 1.68 mmol, 7.00 eq.) as coupling reagents and incubation for at least 2 h. Removal of the Fmoc protective group (GP3) was followed by coupling of the DOTA moiety. Therefore, DOTA-NHS (91.4 mg, 0.12 mmol, 0.50 eq.) and DIPEA (286 μL, 1.68 mmol, 7.00 eq.) were each dissolved in DMF. First, DIPEA in DMF was added to the resin for preactivation. After five minutes, DOTA-NHS in DMF was added and incubated with the resin-bound amine for 70.5 h. An adequate conversion (RP-HPLC/MS analysis after GP6) was reached and the peptide was cleaved off the resin with TFA/TIPS/DCM (95/2.5/2.5, GP8) and purified afterwards by preparative RP-HPLC (30 - 80% B in 20 min, Method B, 5 mL/min and 25 - 70% B in 15 min, Method B, 1 mL/min). Subsequent lyophilization afforded 3.17 mg (0.95% yield, chemical purity 95.5%) of pure product **8** as a colorless powder.

Chemical formula: $C_{62}H_{101}FN_{12}O_{21}Si$;
Molecular weight: 1397.64 g/mol;
Exact mass: 1396.70 g/mol;
$t_R$-value: 11.8 min (10 - 90% B in 15 min, Method A)
Capacity factor k: 6.9
ESI-MS: calculated monoisotopic mass ($C_{62}H_{101}FN_{12}O_{21}Si$): 1396.70;
found: ESI (positive ion mode): m/z = 699.4 [M(**8**)+2H]$^{2+}$, 1398.9 [M(**8**)+H]$^{+}$.

$^{nat}$Lu-2-Aminoheptanoic acid derivative 8 ($^{nat}$Lu-**8**)

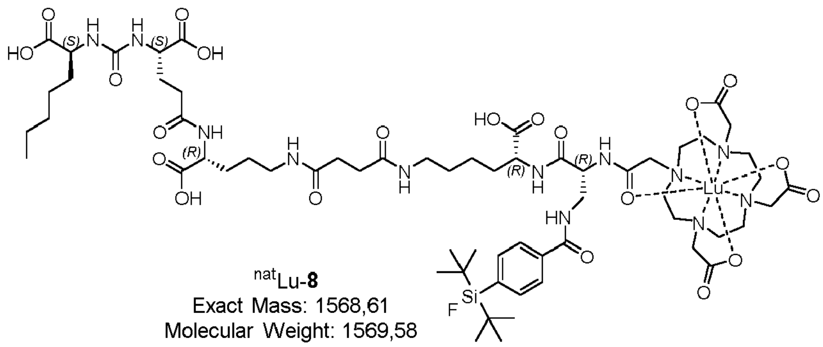

$^{nat}$Lu-**8**
Exact Mass: 1568,61
Molecular Weight: 1569,58

100 μL of the precursor (**8**) (2.00 mM in DMSO, 0.20 μmol, 1.00 eq.) were added to 60.0 μL of $LuCl_3$ (20 mM in Tracepur®-$H_2O$, 1.20 μmol, 6.00 eq.) and 40.0 μL Tracepur®-$H_2O$. The reaction mixture was heated for 30 min at 95 °C and afforded $^{nat}$Lu-**8** in 94.9% chemical purity (>99% yield), determined by RP-HPLC (220 nm). This $^{nat}$Lu-**8** solution (now 1.00 mM) was directly used as stock solution for affinity determination.

Chemical formula: $C_{62}H_{98}FLuN_{12}O_{21}Si$;
Molecular weight: 1569.58 g/mol;
Exact mass: 1568.61 g/mol;
$t_R$-value: 11.7 min (10 - 90% B in 15 min, Method A)
Capacity factor k: 6.8
ESI-MS: calculated monoisotopic mass ($C_{62}H_{98}FLuN_{12}O_{21}Si$): 1568.61;
found: ESI (positive ion mode): m/z = 784.5 [M($^{nat}$Lu-**8**)+2H]$^{2+}$, 1568.0 [M($^{nat}$Lu-**8**)+H]$^{+}$.

**Furyl derivative 9**

Tert-butyl (S)-2-amino-3-(furan-2-yl)propanoate **(24*TFA)**

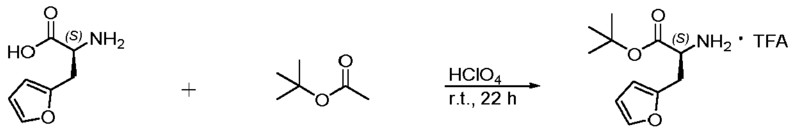


**S-34**
Exact Mass: 155,06
Molecular Weight: 155,15

**S-32**
Exact Mass: 116,08
Molecular Weight: 116,16

**24*TFA**
Exact Mass: 325,14
Molecular Weight: 325,28

Compound **24** was synthesized in analogy to a previously published procedure by Hyun et al. (J Am Chem Soc. 2010, 132(48), 17053-5) with some minor modifications. 3-(2-Furyl)-L-alanine (**S-34**) (300 mg, 2.93 mmol, 1.00 eq.) was dissolved in 6.65 mL of tert-butyl acetate (**S-32**) and 70% (v/v) perchloric acid (aq.) (359 μL, 4.42 mmol, 2.29 eq.) was added slowly. The reaction mixture was stirred for 22 h at room temperature. $H_2O$ and some drops of 0.3 M HCl were added for quenching. The pH value of the aqueous phase was adjusted to 9 with 10% (w/v) $Na_2CO_3$ (aq.) and extracted three times with DCM. The combined organic phases were dried over $Na_2SO_4$, filtered and the solvent was removed under reduced pressure. Purification by column chromatography (DCM/MeOH/acetone = 5/1/1) and preparative RP-HPLC (30 - 75% B in 20 min, Method B, 9 mL/min) yielded 65.5 mg (10.4%) of pure product **24***TFA as a colorless solid.

Chemical formula (w/o TFA): $C_{11}H_{17}NO_3$;
Molecular weight (w/o TFA): 211.26 g/mol;
Exact mass (w/o TFA): 211.12 g/mol;
$t_R$-value: 6.93 min (10 - 90% B in 15 min, Method A)
Capacity factor k: 3.6
$R_f$-value: 0.69 (DCM/MeOH/acetone = 5/1/1)
ESI-MS: calculated monoisotopic mass ($C_{11}H_{17}NO_3$): 211.12;
found: ESI (positive ion mode): m/z = 212.1 [M(**24**)+H]$^{+}$, 253.1 [M(**24**)+MeCN+H]$^{+}$.

3-(2-Furyl)-L-alanine-OtBu-(carbonyl)-L-Glu[D-Orn(Dde)-2-CT]-OtBu (**S-35**)

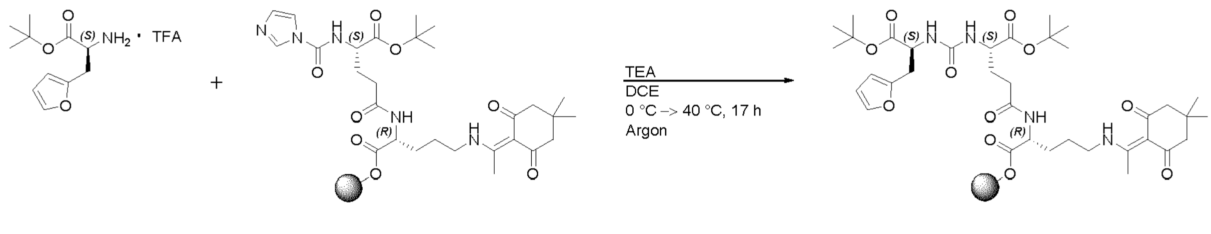


24*TFA
Exact Mass: 325,14
Molecular Weight: 325,28

20
Exact Mass: 575,30
Molecular Weight: 575,66

S-35
Exact Mass: 718,38
Molecular Weight: 718,85

Compound **20** (0.29 mmol, 1.45 eq.) was transferred into a round bottom flask and dissolved in 8.00 mL DCE. At 0 °C, triethylamine (69.8 μL, 0.50 mmol, 2.50 eq.) and **24***TFA (65.5 mg, 0.20 mmol, 1.00 eq.) were added and the mixture was stirred for further 5 min at 0 °C. Afterwards, it was warmed to 40 °C and stirred for 17 h under argon atmosphere. The resin was washed with DCM (4x), some resin beads were taken and treated with HFIP/DCM (1/4) according to GP7. RP-HPLC/MS analysis revealed nearly complete conversion to product **S-35.**

Chemical formula: $C_{36}H_{54}N_4O_{11}$;
Molecular weight: 718.85 g/mol;
Exact mass: 718.38 g/mol;
$t_R$-value: 12.4 min (10 - 90% B in 15 min, Method A)
Capacity factor k: 7.3
ESI-MS: calculated monoisotopic mass ($C_{36}H_{54}N_4O_{11}$): 718.38;
found: ESI (positive ion mode): m/z = 718.8 [M(**S-35**)+H]$^+$, 740.7 [M(**S-35**+Na]$^+$, 756.8 [M(**S-35**)+K]$^+$.

Furyl derivative 9

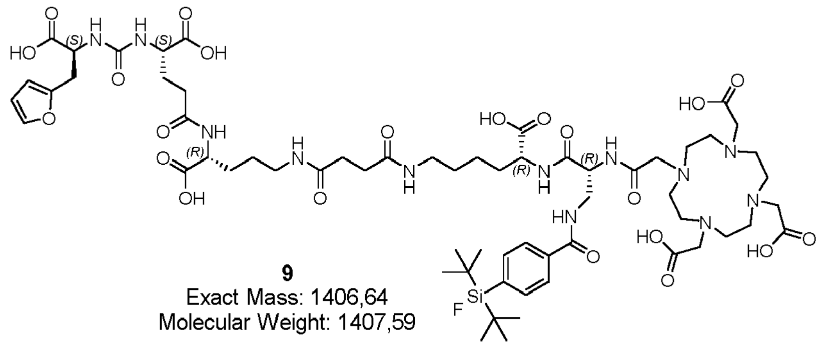

9
Exact Mass: 1406,64
Molecular Weight: 1407,59

Further reactions on resin-bound compound **S-35** (0.29 mmol, 1.00 eq.) were performed according to standard Fmoc-SPPS on 2-CT resin, applying the above-mentioned methods (GP2 - GP8). In brief, the Dde protective group was removed (GP4) and succinic anhydride (203 mg, 2.03 mmol, 7.00 eq.) was coupled (GP2) over a period of at least 2.5 h, only using DIPEA (345 μL, 2.03 mmol, 7.00 eq.) and no further coupling reagents. The peptide was elongated with Fmoc-D-Lys-OtBu*HCl (267 mg, 0.58 mmol, 2.00 eq.). Therefore, the resin-bound acid was preactivated for five minutes using TBTU (186 mg, 0.58 mmol, 2.00 eq.), HOAt (78.9 mg, 0.58 mmol, 2.00 eq.) and DIPEA (296 μL, 1.74 mmol, 6.00 eq.). The amino acid was dissolved in DMF, added to the preactivated resin and shaken for at least 2.5 h. Fmoc-removal was conducted (GP3) and Fmoc-D-Dap(Dde)-OH (285 mg, 0.58 mmol, 2.00 eq.) was coupled for at least 2.5 h using TBTU (186 mg, 0.58 mmol, 2.00 eq.), HOAt (78.9 mg, 0.58 mmol, 2.00 eq.) and sym-collidine (269 μL, 2.03 mmol, 7.00 eq.) as coupling reagents. Afterwards, Dde-removal was performed according to GP5. The SiFA-BA moiety (81.8 mg, 0.29 mmol, 1.00 eq.) was attached using TBTU (186 mg, 0.58 mmol, 2.00 eq.), HOAt (78.9 mg, 0.58 mmol, 2.00 eq.) and sym-collidine (269 μL, 2.03 mmol, 7.00 eq.) as coupling reagents and incubation for at least 2 h. Removal of the Fmoc protective group (GP3) was followed by coupling of the DOTA moiety. Therefore, DOTA-NHS (110 mg, 0.15 mmol, 0.50 eq.) and DIPEA (345 μL, 2.03 mmol, 7.00 eq.) were each dissolved in DMF. First, DIPEA in DMF was added to the resin for preactivation. After five minutes, DOTA-NHS in DMF was added and incubated with the resin-bound amine for 13 h. An adequate conversion (RP-HPLC/MS analysis after GP6) was reached and the peptide was cleaved off the

resin with TFA/TIPS/DCM (95/2.5/2.5, GP8) and purified afterwards by preparative RP-HPLC (35 - 80% B in 20 min, Method B, 5 mL/min). Subsequent lyophilization afforded 8.72 mg (2.14%, yield, chemical purity >99%) of pure product **9** as a colorless powder.

Chemical formula: $C_{62}H_{95}FN_{12}O_{22}Si$;
Molecular weight: 1407.59 g/mol;
Exact mass: 1406.64 g/mol;
$t_R$-value: 11.3 min (10 - 90% B in 15 min, Method A)
Capacity factor k: 6.5
ESI-MS: calculated monoisotopic mass ($C_{62}H_{95}FN_{12}O_{22}Si$): 1406.64;
found: ESI (positive ion mode): m/z = 703.8 $[M(\mathbf{9})+2H]^{2+}$, 1406.6 $[M(\mathbf{9})+H]^{+}$.

$^{nat}$Lu-Furyl derivative 9 ($^{nat}$Lu-**9**)

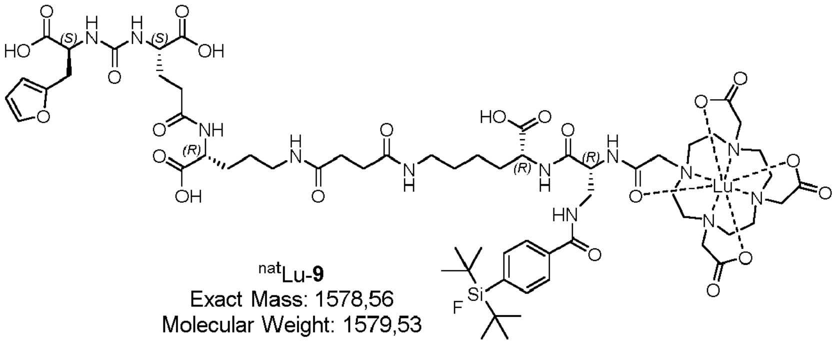

$^{nat}$Lu-**9**
Exact Mass: 1578,56
Molecular Weight: 1579,53

100 µL of the precursor (**9**) (2.00 mM in DMSO, 0.20 µmol, 1.00 eq.) were added to 60.0 µL of $LuCl_3$ (20 mM in Tracepur®-$H_2O$, 1.20 µmol, 6.00 eq.) and 40.0 µL Tracepur®-$H_2O$. The reaction mixture was heated for 30 min at 95 °C and afforded $^{nat}$Lu-**9** in 98.1% chemical purity (>99% yield), determined by RP-HPLC (220 nm). This $^{nat}$Lu-**9** solution (now 1.00 mM) was directly used as stock solution for affinity determination.

Chemical formula: $C_{62}H_{92}FLuN_{12}O_{22}Si$;
Molecular weight: 1579.53 g/mol;
Exact mass: 1578.56 g/mol;
$t_R$-value: 11.2 min (10 - 90% B in 15 min, Method A)
Capacity factor k: 6.5
ESI-MS: calculated monoisotopic mass ($C_{62}H_{92}FLuN_{12}O_{22}Si$): 1578.56;
found: ESI (positive ion mode): m/z = 789.7 $[M(^{nat}Lu\text{-}\mathbf{9})+2H]^{2+}$, 1579.3 $[M(^{nat}Lu\text{-}\mathbf{9})+H]^{+}$.

**Alkyne derivative 10**

(2S)-2-Amino-4-pentynoate-OtBu-(carbonyl)-L-Glu[D-Orn(Dde)-2-CT]-OtBu (**S-36**)

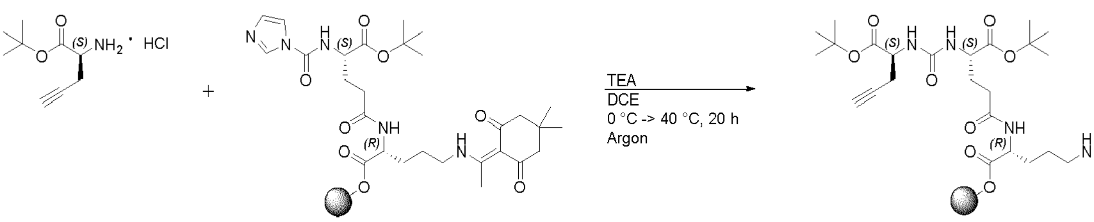


**25*HCl**
Exact Mass: 205,09
Molecular Weight: 205,68

**20**
Exact Mass: 575,30
Molecular Weight: 575,66

**S-36**
Exact Mass: 676,37
Molecular Weight: 676,81

Compound **20** (0.24 mmol, 1.00 eq.) was transferred into a round bottom flask and dissolved in 2.18 mL DCE. At 0 °C, triethylamine (83.2 μL, 0.60 mmol, 2.50 eq.) and tert-butyl (2S)-2-amino-4-pentynoate (**25**)*HCl (59.6 mg, 0.29 mmol, 1.20 eq.) were added and the mixture was stirred for further 5 min at 0 °C. Afterwards, it was warmed to 40 °C and stirred for 20 h under argon atmosphere. The resin was washed with DCM (4x), some resin beads were taken and treated with HFIP/DCM (1/4) according to GP7. RP-HPLC/MS analysis revealed nearly complete conversion to product **S-36.**

Chemical formula: $C_{34}H_{52}N_4O_{10}$;
Molecular weight: 676.81 g/mol;
Exact mass: 676.37 g/mol;
$t_R$-value: 6.05 min (40 - 95% B in 15 min, Method A)
Capacity factor k: 3.0
ESI-MS: calculated monoisotopic mass ($C_{34}H_{52}N_4O_{10}$): 676.37;
found: ESI (positive ion mode): m/z = 677.0 [M(**S-36**)+H]$^+$, 699.0 [M(**S-36**)+Na]$^+$.

Alkyne derivative **10**

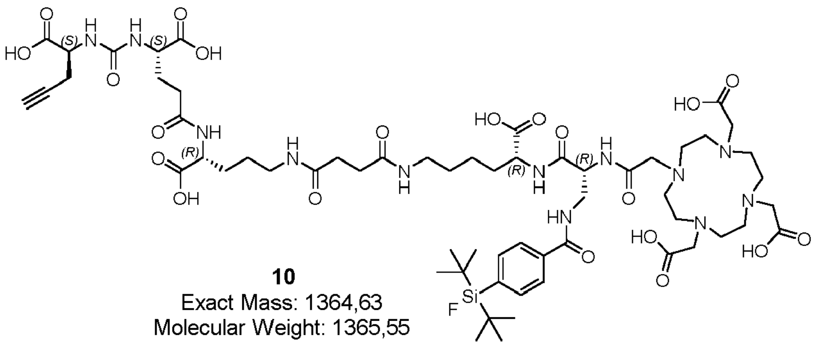

**10**
Exact Mass: 1364,63
Molecular Weight: 1365,55

Further reactions on resin-bound compound **S-36** (0.24 mmol, 1.00 eq.) were performed according to standard Fmoc-SPPS on 2-CT resin, applying the above-mentioned methods (GP2 - GP8). In brief, the Dde protective group was removed (GP4) and succinic anhydride (170 mg, 1.68 mmol, 7.00 eq.) was coupled (GP2) over a period of at least 2.5 h, only using DIPEA (286 μL, 1.68 mmol, 7.00 eq.) and no further coupling reagents. The peptide was elongated with Fmoc-D-Lys-OtBu*HCl (221 mg, 0.48 mmol, 2.00 eq.). Therefore, the resin-bound acid was preactivated for five minutes with TBTU (154 mg, 0.48 mmol, 2.00 eq.), HOAt (65.3 mg, 0.48 mmol, 2.00 eq.) and DIPEA (245 μL, 1.44 mmol, 6.00 eq.). The amino acid was dissolved in DMF, added to the preactivated resin and shaken for at least 2.5 h. Fmoc-removal was conducted (GP3) and Fmoc-D-Dap(Dde)-OH (235 mg, 0.48 mmol, 2.00 eq.) was coupled for at least 2.5 h using TBTU (154 mg, 0.48 mmol, 2.00 eq.), HOAt (65.3 mg, 0.48 mmol, 2.00 eq.) and sym-collidine (223 μL, 1.68 mmol, 7.00 eq.) as coupling reagents. Afterwards, Dde-removal was performed according to GP5. The SiFA-BA moiety (67.7 mg, 0.24 mmol, 1.00 eq.) was attached using TBTU (154 mg, 0.48 mmol, 2.00 eq.), HOAt (65.3 mg, 0.48 mmol, 2.00 eq.) and sym-collidine (223 μL, 1.68 mmol, 7.00 eq.) as coupling reagents and incubation for at least 2 h. Removal of the Fmoc protective group (GP3) was followed by coupling of the DOTA moiety. Therefore, DOTA-NHS (91.4 mg, 0.12 mmol, 0.50 eq.) and DIPEA (286 μL, 1.68 mmol, 7.00 eq.) were each dissolved in DMF. First, DIPEA in DMF was added to the resin for preactivation. After five minutes, DOTA-NHS in DMF was added and incubated with the resin-bound amine for 71 h. An adequate conversion (RP-HPLC/MS analysis after GP6) was reached and the peptide was cleaved off the resin with TFA/TIPS/DCM (95/2.5/2.5, GP8) and purified afterwards by preparative RP-HPLC (30 - 80% B in 20 min, Method B, 5 mL/min). Subsequent lyophilization afforded 10.9 mg (3.33% yield, chemical purity 98.5%) of pure product **10** as a colorless powder.

Chemical formula: $C_{60}H_{93}FN_{12}O_{21}Si$;
Molecular weight: 1365.55 g/mol;
Exact mass: 1364.63 g/mol;
$t_R$-value: 11.1 min (10 - 90% B in 15 min, Method A)
Capacity factor k: 6.4
ESI-MS: calculated monoisotopic mass ($C_{60}H_{93}FN_{12}O_{21}Si$): 1364.63;

found: ESI (positive ion mode): m/z = 682.7 [M(**10**)+2H]$^{2+}$, 1364.4 [M(**10**)+H]$^{+}$.

$^{nat}$Lu-Alkyne derivative **10** ($^{nat}$Lu-**10**)

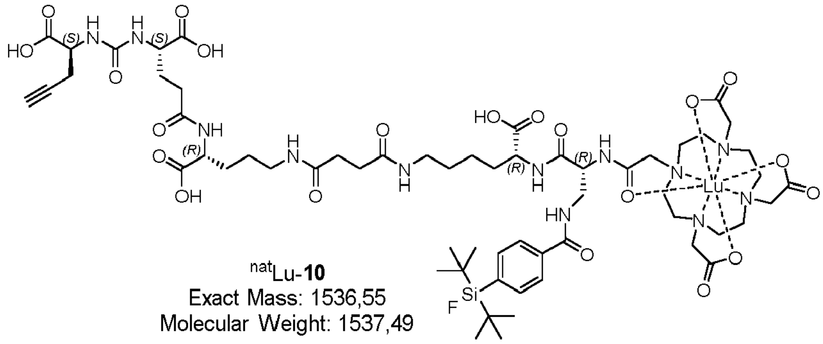

$^{nat}$Lu-**10**
Exact Mass: 1536,55
Molecular Weight: 1537,49

100 μL of the precursor (**10**) (2.00 mM in DMSO, 0.20 μmol, 1.00 eq.) were added to 60.0 μL of $LuCl_3$ (20 mM in Tracepur®-$H_2O$, 1.20 μmol, 6.00 eq.) and 40.0 μL Tracepur®-$H_2O$. The reaction mixture was heated for 30 min at 95 °C and afforded $^{nat}$Lu-**10** in 99.1% chemical purity (>99% yield), determined by RP-HPLC (220 nm). This $^{nat}$Lu-**10** solution (now 1.00 mM) was directly used as stock solution for affinity determination.

Chemical formula: $C_{60}H_{90}FLuN_{12}O_{21}Si$;
Molecular weight: 1537.49 g/mol;
Exact mass: 1536.55 g/mol;
$t_R$-value: 10.9 min (10 - 90% B in 15 min, Method A)
Capacity factor k: 6.3
ESI-MS: calculated monoisotopic mass ($C_{60}H_{90}FLuN_{12}O_{21}Si$): 1536.55;
found: ESI (positive ion mode): m/z = 769.6 [M($^{nat}$Lu-**10**)+2H]$^{2+}$, 1538.4 [M($^{nat}$Lu-**10**)+H]$^{+}$.

[$^{177}$Lu]Lu-Alkyne derivative **10** ([$^{177}$ Lu]Lu-**10**)

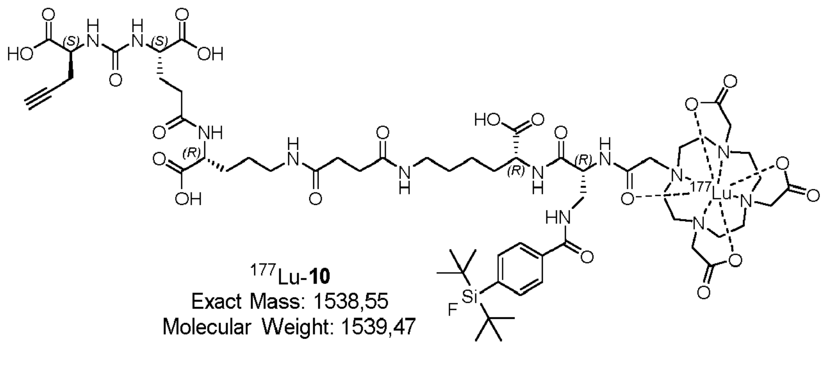

$^{177}$Lu-**10**
Exact Mass: 1538,55
Molecular Weight: 1539,47

5.00 μL of the precursor (**10**) (0.20 mM in DMSO, 1.00 nmol, 1.00 eq.) were added to 10.0 μL of 1 M NaOAc buffer (aq.) (pH = 5.5). Subsequently, 37.5 to 62.0 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0 μL of 0.1 M sodium ascorbate (aq.) (in Tracepur®-$H_2O$) were added and the reaction mixture was heated for 25 min at 95 °C. Occasionally, removal of free [$^{177}$Lu]$Lu^{3+}$ via HLB cartridge (30 mg) was required, which afforded [$^{177}$Lu]Lu-**10** in 70.3 or >99% (n = 3) isolated RCY (d.c. to the start of synthesis). The apparent $A_m$ were 26.3 to 62.0 GBq/μmol at the end of synthesis. RCP as determined by radio-RP-HPLC and radio-TLC was 93.6 ± 2.5%.

Chemical formula: $C_{60}H_{90}F^{177}LuN_{12}O_{21}Si$;

Molecular weight: 1539.47 g/mol;
Exact mass: 1538.55 g/mol;
$t_R$-value: 10.3 min (10 - 90% B in 15 min, Method A)
$t_R$-value of co-injected cold standard: 10.2 min (10 - 90% B in 15 min, Method A)
Capacity factor k: 5.9.

**Tetrazole derivative 11**

Tert-butyl $N^5$-benzyl-$N^2$-((benzyloxy)carbonyl)-L-glutaminate (**S-39**)

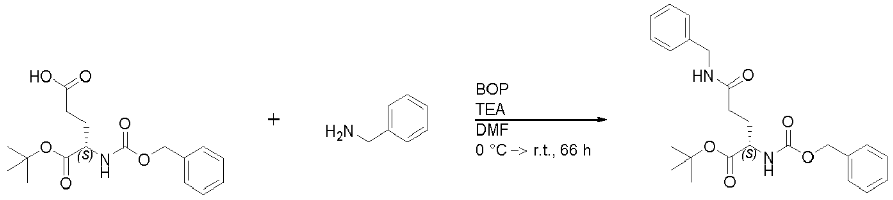


**S-37** Exact Mass: 295,16 Molecular Weight: 295,81

**S-38** Exact Mass: 107,07 Molecular Weight: 107,16

**S-39** Exact Mass: 426,22 Molecular Weight: 426,51

Compound **S-39** was synthesized in analogy to a previously published procedure by Kozikowski et al. (Journal of Medicinal Chemistry. 2004, 47(7), 1729-38) Benzyl amine (**S-38**) (305 μL, 2.80 mmol, 1.40 eq.) and BOP (1.06 g, 2.40 mmol, 1.20 eq.) were added to a solution of N-Cbz-L-Glu-OtBu (**S-37**) (675 mg, 2.00 mmol, 1.00 eq.) in 18.0 mL DMF. The resulting solution was cooled to 0 °C and triethylamine (638 μL, 4.60 mmol, 2.30 eq.) was added. After stirring for 66 h at room temperature the reaction mixture was poured into ice-cold water (100 mL) and extracted four times with EtOAc. The organic layer was washed successively with 1 M HCl, $H_2O$, saturated $NaHCO_3$ and brine. The organic phase was dried over $Na_2SO_4$, filtered and the solvent was removed in vacuo. Purification by column chromatography (EtOAc/n-hexane = 1/1) yielded 876 mg (2.05 mmol, >99%) of N-Cbz-L-Gln(Bn)-OtBu (**S-39**) as a colorless solid.

Chemical formula: $C_{24}H_{30}N_2O_5$;
Molecular weight: 426.51 g/mol;
Exact mass: 426.22 g/mol;
$t_R$-value: n.d.
Capacity factor k: n.d.
$R_f$-value: 0.54 (EtOAc/n-hexane = 1/1)
ESI-MS: n.d.
$^1$H-NMR (400 MHz, $CDCl_3$) δ(ppm): 7.38 - 7.26 (m, 10H, H(1, 1', 2, 2', 3, 4, 4', 5, 6, 6'), 6.27 (s, 1H, H(7)), 5.56 (d, $^3J$ = 8.1 Hz, 1H, H(8)), 5.08 (s, 2H, H(9)), 4.42 (virt. t, 2H, H(10)), 4.23 (td, $^3J$ = 8.8, 3.8 Hz, 1H, H(11)), 2.26 (virt. ddt, 3H, H(12, 13)), 2.02 - 1.86 (m, 1H, H(14)), 1.45 (s, 9H, H(15, 15', 15")).
$^{13}$C-NMR (101 MHz, $CDCl_3$) δ(ppm): 171.92 (s, 1C, C(1)), 171.15 (s, 1C, C(2)), 156.51 (s, 1C, C(3)), 138.37 (s, 1C, C(4)), 136.37 (s, 1C, C(5)), 128.82 (s, 2C, C(6, 6')), 128.65 (s, 2C, C(7, 7')), 128.33 (s, 1C, C(8)), 128.26 (s, 2C, C(9, 9')), 127.95 (s, 2C, C(10, 10')), 127.61 (s, 1C, C(11)), 82.68 (s, 1C, C(12)), 67.16 (s, 1C, C(13)), 54.13 (s, 1C, C(14)), 43.84 (s, 1C, C(15)), 32.72 (s, 1C, C(16)), 29.42 (s, 1C, C(17)), 28.11 (s, 3C, C(18, 18', 18")).

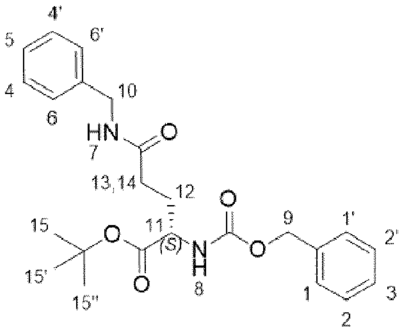

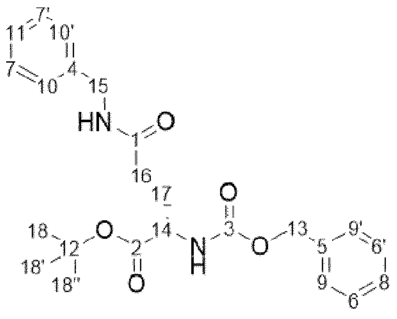

**S-39**

Tert-butyl (S)-4-(1-benzyl-1 H-tetrazol-5-yl)-2-(((benzyloxy)carbonyl)amino)butanoate (**S-40**)

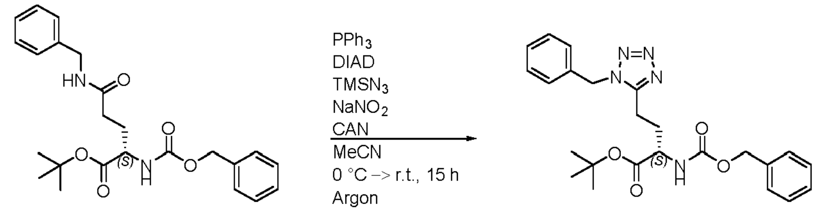


**S-39**
Exact Mass: 426,22
Molecular Weight: 426,51

**S-40**
Exact Mass: 451,22
Molecular Weight: 451,53

Compound **S-40** was synthesized in analogy to a previously published procedure by Kozikowski et al. (Journal of Medicinal Chemistry. 2004, 47(7), 1729-38) Diisopropyl azodicarboxylate (530 μL, 2.70 mmol, 1.35 eq.) was added to a solution of $PPh_3$ (683 mg, 2.60 mmol, 1.30 eq.) and N-Cbz-L-Gln(Bn)-OtBu (**S-39**) (853 mg, 2.00 mmol, 1.00 eq.) in 10 mL ice-cold, anhydrous MeCN over two minutes. Trimethylsilyl azide (372 μL, 2.80 mmol, 1.40 eq.) was added over five minutes, and the solution was allowed to stir for 15 h at room temperature. Afterwards, the solution was cooled to 0 °C and 726 μL of a 3 M $NaNO_2$ solution (aq.) (152 mg, 2.20 mmol, 1.10 eq.) was added. The mixture was allowed to stir for 30 min at room temperature and 3.30 mL of cerium(IV) ammonium nitrate (1.21 g, 2.20 mmol, 1.10 eq.) in $H_2O$ was added. The solution was stirred for further 20 min and subsequently poured into ice-cold $H_2O$. The mixture was extracted three times with DCM and the combined organic phases were washed once with water, then dried over $Na_2SO_4$, filtered and the solvent removed in vacuo. Purification by column chromatography (EtOAc/n-hexane = 1/2) yielded 222 mg (24.6%) of product **S-40** as a colorless solid.

Chemical formula: $C_{24}H_{29}N_5O_4$;
Molecular weight: 451.53 g/mol;
Exact mass: 451.22 g/mol;
$t_R$-value: 14.5 min (10 - 90% B in 15 min, Method A)
Capacity factor k: 8.7
$R_f$-value: 0.28 (EtOAc/n-hexane = 1/2)
ESI-MS: calculated monoisotopic mass ($C_9H_{17}N_5O_2$): 451.22;
found: ESI (positive ion mode): m/z = 395.7 [M(**S-40**)-tBu+H]$^+$, 451.7 [M(**S-40**)+H]$^+$;
$^1$H-NMR (400 MHz, $CDCl_3$) δ(ppm): 7.43 - 7.28 (m, 8H, H(1, 1', 2, 2', 3, 3', 4, 4')), 7.14 (virt. dd, 2H, H(5, 6)), 5.56 - 5.30 (m, 2H, H(7)), 5.24 - 4.99 (m, 2H, H(8)), 4.26 (td, $^3$J=8.2, 4.4 Hz, 1H, H(9)), 2.75 (virt. tq, 2H, H(10)), 2.34 (virt. dq, 1H, H(11)), 2.01 (virt. dddd, 1H, H(12)), 1.42 (s, 9H, H(13, 13', 13")).
$^{13}$C-NMR (101 MHz, $CDCl_3$) δ(ppm): 170.51 (s, 1C, C(1)), 156.19 (s, 1C, C(2)), 154.26 (s, 1C, C(3)), 136.31 (s, 1C, C(4)), 133.33 (s, 1C, C(5)), 129.40 (s, 2C, C(6, 6')), 129.11 (s, 1C, C(7)), 128.73 (s, 2C, C(8, 8')), 128.45 (s, 1C, C(9)), 128.30 (s, 2C, C(10, 10')), 127.67 (s, 2C, C(11, 11')), 83.11 (s, 1C, C(12)), 67.23 (s, 1C, C(13)), 53.93 (s, 1C, C(14)), 50.90 (s, 1C, C(15)), 30.29 (s, 1C, C(16)), 28.06 (s, 3C, C(17, 17', 17")), 19.98 (s, 1C, C(18)).

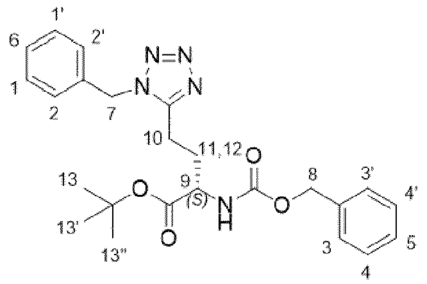

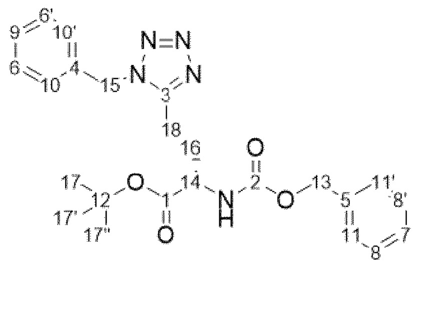

**S-40**

Tert-butyl (S)-2-amino-4-(1H-tetrazol-5-yl)butanoate (**S-41**)

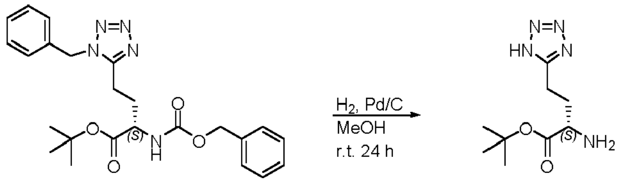


**S-40**
Exact Mass: 451,22
Molecular Weight: 451,53

**S-41**
Exact Mass: 227,14
Molecular Weight: 227,27

Compound **S-40** (120 mg, 0.27 mmol, 1.00 eq.) was dissolved in 15.0 mL MeOH and the solution was flushed 5 minutes with argon to remove dissolved oxygen. 12.0 mg of palladium on carbon (10% wt) (corresponds to 1.20 mg palladium, 11.3 μmol, 0.04 eq.) were added and the flask was sealed with a rubber septum. Remaining air was displaced with argon and subsequently replaced by hydrogen gas. The mixture was stirred under hydrogen atmosphere at room temperature for 23 h. Palladium on carbon was filtered off, and the solvent was evaporated in vacuo. This afforded a mixture of product **S-41** (minor portion) and reactant **S-40** with free α-amine, still bearing the Bn-protective group at the tetrazole moiety (major portion). However, as a certain conversion to product **S-41** could be observed by RP-HPLC/MS analysis and the N-Cbz-protective group was cleaved off efficiently, the crude product was used in the next step without further purification.

Chemical formula: $C_9H_{17}N_5O_2$;
Molecular weight: 227.27 g/mol;
Exact mass: 227.14 g/mol;
$t_R$-value: 4.6 min (10 - 90% B in 15 min, Method A)
Capacity factor k: 2.1
ESI-MS: calculated monoisotopic mass ($C_9H_{17}N_5O_2$): 227.14;
found: ESI (positive ion mode): m/z = 228.0 [M(**S-41**)+H]$^+$, 268.7 [M(**S-41**)+MeCN+H]$^+$.

5-Benzyl 1-(tert-butyl) (((S)-1-(tert-butoxy)-1-oxo-4-(1H-tetrazol-5-yl)butan-2-yl)carbamoyl)-L-glutamate (**S-42**)

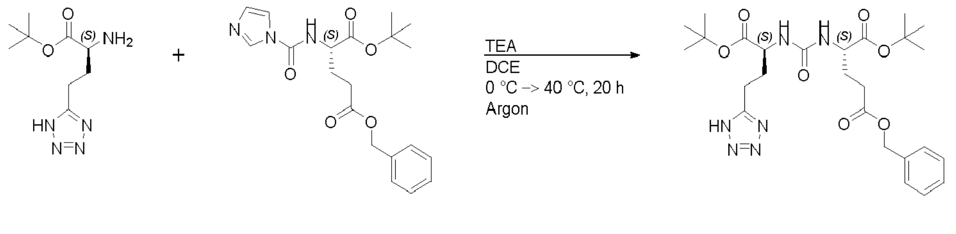


**S-41**
Exact Mass: 227,14
Molecular Weight: 227,27

**S-28**
Exact Mass: 387,18
Molecular Weight: 387,44

**S-42**
Exact Mass: 546,28
Molecular Weight: 546,63

Compound **S-42** was synthesized in analogy to a previously published procedure by Weineisen et al. (EJNMMI Research. 2014, 4(1), 63) with some minor modifications. Tert-butyl (S)-2-amino-4-(1H-tetrazol-5-yl)butanoate (**S-41**) (-100 mg crude product, -266 µmol, 1.20 eq.) was dissolved in 2.00 mL DCE and compound **S-28** (86.0 mg, 222 µmol, 1.00 eq.) was added. At 0 °C, triethylamine (76.9 µL, 555 µmol, 2.50 eq.) was added and the mixture was stirred for further five minutes at 0 °C. The solution was warmed to 40 °C and stirred for 20 h under argon atmosphere. The solvent was removed in vacuo and the crude product was purified via flash chromatography (10 - 90% B in 15 min, Method D, 12 mL/min), which afforded 39.6 mg (32.7%) of compound **S-42** as a colorless solid.

Chemical formula: $C_{26}H_{38}N_6O_7$;
Molecular weight: 546.63 g/mol;
Exact mass: 546.28 g/mol;
$t_R$-value: 11.3 min (30 - 90% B in 15 min, Method A)
Capacity factor k: 6.5
ESI-MS: calculated monoisotopic mass ($C_{26}H_{38}N_6O_7$): 546.28;
found: ESI (positive ion mode): m/z = 490.4 [M(**S-42**)-tBu+H]$^+$, 546.7 [M(**S-42**)+H]$^+$, 568.8 [M(**S-42**)+Na]$^+$.

(S)-5-(tert-butoxy)-4-(3-((S)-1-(tert-butoxy)-1-oxo-4-(1 H-tetrazol-5-yl)butan-2-yl)ureido)-5-oxopentanoic acid (26)

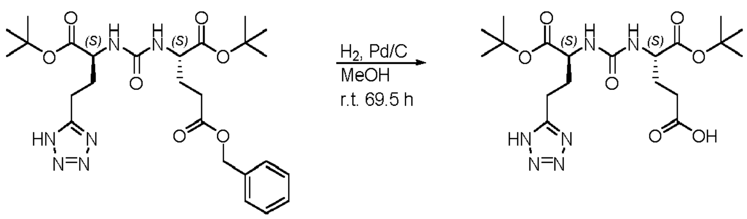


**S-42**
Exact Mass: 546,28
Molecular Weight: 546,63

**26**
Exact Mass: 456,23
Molecular Weight: 456,50

Compound **S-42** (17.0 mg, 31.2 µmol, 1.00 eq.) was dissolved in 6.00 mL MeOH and the solution was flushed 5 minutes with argon to remove dissolved oxygen. 3.30 mg of palladium on carbon (10% wt) (corresponds to 330 µg palladium, 3.12 µmol, 0.10 eq.) were added and the flask was sealed with a rubber septum. Remaining air was displaced with argon and subsequently replaced by hydrogen gas. The mixture was stirred under hydrogen atmosphere at room temperature for 69.5 h. Palladium on carbon was filtered off, and the solvent was evaporated in vacuo. This afforded 44.0 mg of crude product as a colorless oil, with some minor by-products and remaining reactant **S-42.** As the main component could be identified as product **26** by RP-HPLC/MS analysis, the crude product was used in the next step without further purification.

Chemical formula: $C_{19}H_{32}N_6O_7$;
Molecular weight: 456.50 g/mol;
Exact mass: 456.23 g/mol;
$t_R$-value: 8.94 min (10 - 90% B in 15 min, Method A)
Capacity factor k: 5.0
ESI-MS: calculated monoisotopic mass ($C_{19}H_{32}N_6O_7$): 456.23;
found: ESI (positive ion mode): m/z= 345.9 [M(**26**)-2tBu+H]$^+$, 456.7 [M(**26**)+H]$^+$, 478.7 [M(**26**)+Na]$^+$, 912.5

$[M_2(\mathbf{26})+H]^+$, 934.7 $[M_2(\mathbf{26})+Na]^+$, 950.3 $[M_2(\mathbf{26})+K]^+$.

(S)-2-amino-4-(1 H-tetrazol-5-yl)butanoic acid-OtBu-(carbonyl)-L-Glu[D-Orn(Dde)-2-CT]-OtBu (**S-43**)

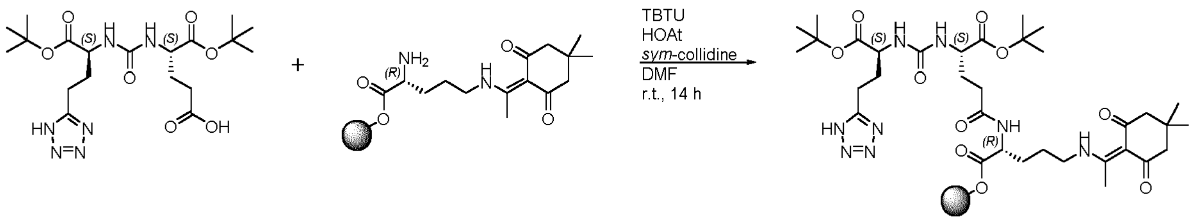


**26**
Exact Mass: 456,23
Molecular Weight: 456,50

**18**
Exact Mass: 296,17
Molecular Weight: 296,37

**S-43**
Exact Mass: 734,40
Molecular Weight: 734,85

According to GP2, fragment **26** (28.5 mg, ~62.4 µmol, 1.00 eq.) was coupled to resin-bound H-D-Orn(Dde) (**18**) (89.0 µmol 1.43 eq.) with TBTU (40.1 mg, 125 µmol, 2.00 eq.) and HOAt (17.0 mg, 125 µmol, 2.00 eq.) as coupling reagents and sym-collidine (74.5 µL, 562 µmol, 9.00 eq.) as base. After shaking for 14 h at room temperature, formation of product **S-43** could be confirmed (GP7), as only one major peak with the expected m/z-ratio of 734.9 occurred.

Chemical formula: $C_{34}H_{54}N_8O_{10}$;
Molecular weight: 734.85 g/mol;
Exact mass: 734.40 g/mol;
$t_R$-value: 9.91 min (10 - 90% B in 15 min, Method A)
Capacity factor k: 5.6
ESI-MS: calculated monoisotopic mass ($C_{34}H_{54}N_8O_{10}$): 734.40;
found: ESI (positive ion mode): m/z = 734.9 $[M(\mathbf{S\text{-}43})+H]^+$.

Tetrazole derivative **11**

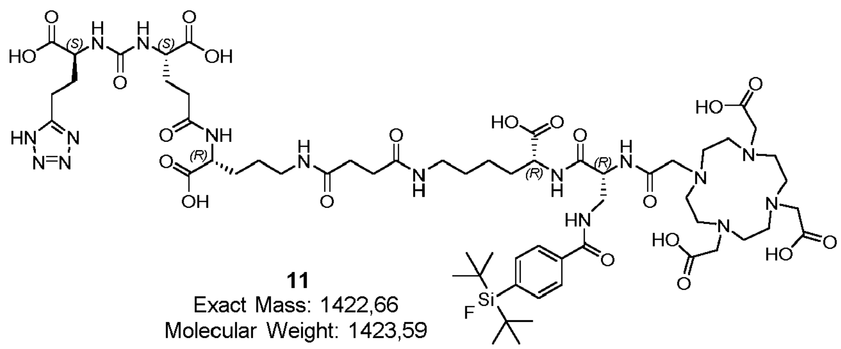

**11**
Exact Mass: 1422,66
Molecular Weight: 1423,59

Further reactions on resin-bound compound **S-43** (89.0 µmol, 1.00 eq.) were performed according to standard Fmoc-SPPS on 2-CT resin, applying the above-mentioned methods (GP2 - GP8). In brief, the Dde protective group was removed (GP4) and succinic anhydride (62.3 mg, 0.62 mmol, 7.00 eq.) was coupled (GP2) over a period of at least 2.5 h, only using DIPEA (106 µL, 0.62 mmol, 7.00 eq.) and no further coupling reagents. The peptide was elongated with Fmoc-D-Lys-OtBu*HCl (82.1 mg, 0.18 mmol, 2.00 eq.). Therefore, the resin-bound acid was preactivated for five minutes with TBTU (57.2 mg, 0.18 mmol, 2.00 eq.), HOAt (24.2 mg, 0.18 mmol, 2.00 eq.) and DIPEA (91.0 µL, 0.53 mmol, 6.00 eq.). The amino acid was dissolved in DMF, added to the preactivated resin and shaken for at least 2.5 h. Fmoc-removal was conducted (GP3) and Fmoc-D-Dap(Dde)-OH (88.3 mg, 0.18 mmol, 2.00 eq.) was coupled for at least 2.5 h using TBTU (57.2 mg, 0.18 mmol, 2.00 eq.), HOAt (24.2 mg, 0.18 mmol, 2.00 eq.) and sym-collidine (83.5 µL, 0.62 mmol, 7.00 eq.) as coupling reagents. Afterwards, Dde-removal was performed according to GP5. The SiFA-BA moiety (25.1 mg, 89.0 µmol, 1.00 eq.) was attached using TBTU (57.2 mg, 0.18 mmol, 2.00 eq.), HOAt (24.2 mg, 0.18 mmol, 2.00

eq.) and sym-collidine (83.5 μL, 0.62 mmol, 7.00 eq.) as coupling reagents and incubation for at least 2 h. Removal of the Fmoc protective group (GP3) was followed by coupling of the DOTA moiety. Therefore, DOTA-NHS (33.9 mg, 44.5 μmol, 0.50 eq.) and DIPEA (106 μL, 0.62 mmol, 7.00 eq.) were each dissolved in DMF. First, DIPEA in DMF was added to the resin for preactivation. After five minutes, DOTA-NHS in DMF was added and incubated with the resin-bound amine for 15 h. As only low conversion (RP-HPLC/MS analysis after GP7) was reached, further DOTA-NHS (33.9 mg, 44.5 μmol, 0.50 eq.) and DIPEA (106 μL, 0.62 mmol, 7.00 eq.) was added and again incubated for 71 h. Again, no increased conversion could be observed and hence, HOAt (24.2 mg, 0.18 mmol, 2.00 eq.) and DIPEA (212 μL, 1.24 mmol, 14.0 eq.) were added to the reaction mixture. RP-HPLC/MS analysis after 20 h revealed no further significant increase of product signal and hence, the peptide was cleaved off the resin with TFA/TIPS/DCM (95/2.5/2.5, GP8) and purified afterwards by preparative RP-HPLC (25 - 80% B in 20 min, Method B, 5 mL/min and 25 - 70% B in 15 min, Method B, 1 mL/min). Subsequent lyophilization afforded 3.55 mg (2.80% yield, chemical purity >99%) of pure product **11** as a colorless powder.

Chemical formula: $C_{60}H_{95}FN_{16}O_{21}Si$;
Molecular weight: 1423.59 g/mol;
Exact mass: 1422.66 g/mol;
$t_R$-value: 10.8 min (10 - 90% B in 15 min, Method A)
Capacity factor k: 6.2
ESI-MS: calculated monoisotopic mass ($C_{60}H_{95}FN_{16}O_{21}Si$): 1422.66;
found: ESI (positive ion mode): m/z = 711.6 $[M(\mathbf{11})+2H]^{2+}$, 1422.5 $[M(\mathbf{11})+H]^{+}$.

$^{nat}$Lu-Tetrazole derivative **11** ($^{nat}$Lu-**11**)

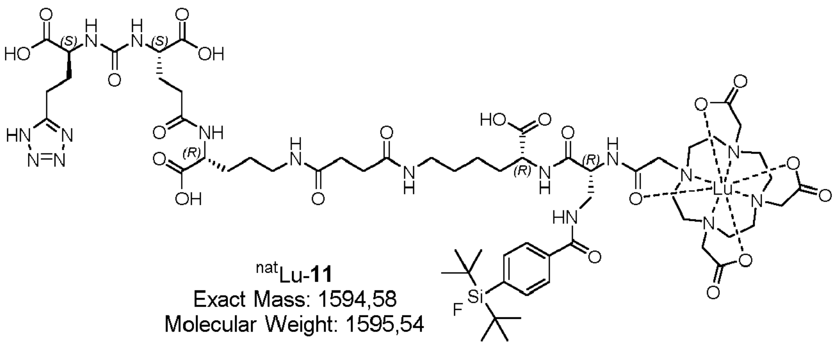

$^{nat}$Lu-**11**
Exact Mass: 1594,58
Molecular Weight: 1595,54

100 μL of the precursor (**11**) (2.00 mM in DMSO, 0.20 μmol, 1.00 eq.) were added to 60.0 μL of $LuCl_3$ (20 mM in Tracepur®-$H_2O$, 1.20 μmol, 6.00 eq.) and 40.0 μL Tracepur®-$H_2O$. The reaction mixture was heated for 30 min at 95 °C and afforded $^{nat}$Lu-**11** in 92.9% chemical purity (>99% yield), determined by RP-HPLC (220 nm). This $^{nat}$Lu-**11** solution (now 1.00 mM) was directly used as stock solution for affinity determination.

Chemical formula: $C_{60}H_{92}FLuN_{16}O_{21}Si$;
Molecular weight: 1595.54 g/mol;
Exact mass: 1594.58 g/mol;
$t_R$-value: 10.6 min (10 - 90% B in 15 min, Method A)
Capacity factor k: 6.1
ESI-MS: calculated monoisotopic mass ($C_{60}H_{92}FLuN_{16}O_{21}Si$): 1594.58;
found: ESI (positive ion mode): m/z = 797.5 $[M(^{nat}Lu\text{-}\mathbf{11})+2H]^{2+}$, 816.6 $[M(^{nat}Lu\text{-}\mathbf{11})+K+H]^{2+}$, 1595.0 $[M(^{nat}Lu\text{-}\mathbf{11})+H]^{+}$.

[$^{177}$Lu]Lu-Tetrazole derivative **11** ([$^{177}$Lu]Lu-**11**)

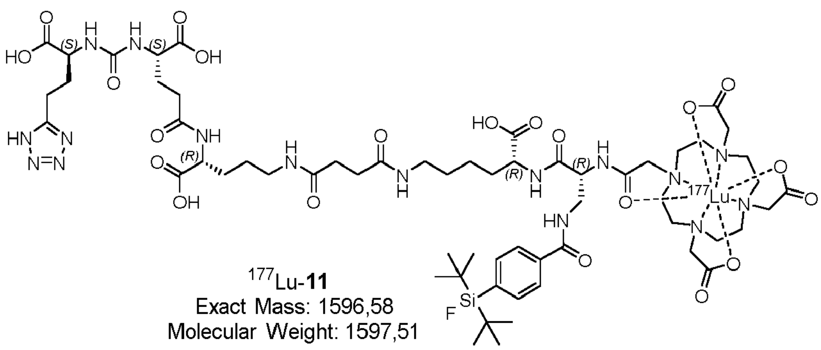

$^{177}$Lu-**11**
Exact Mass: 1596,58
Molecular Weight: 1597,51

5.00 μL of the precursor (**11**) (0.20 mM in DMSO, 1.00 nmol, 1.00 eq.) were added to 10.0 μL of 1 M NaOAc buffer (aq.) (pH = 5.5). Subsequently, 42.2 to 63.5 MBq [$^{177}$Lu]$LuCl_3$ ($A_s$ > 3000 GBq/mg, 740 MBq/mL, 0.04 M HCl, ITG, Garching, Germany) were added and the mixture was filled up to 100 μL with 0.04 M HCl (in Tracepur®-$H_2O$). 10.0 μL of 0.1 M sodium ascorbate (aq.) (in Tracepur®-$H_2O$) were added and the reaction mixture was heated for 25 min at 95 °C. This afforded [$^{177}$Lu]Lu-**11** in >99% (n = 4) isolated RCY (d.c. to the start of synthesis). The apparent $A_m$ ranged from 42.2 to 63.5 GBq/μmol at the end of synthesis. RCP as determined by radio-RP-HPLC and radio-TLC was 97.2 ± 1.2%.

Chemical formula: $C_{60}H_{92}F^{177}LuN_{16}O_{21}Si$;
Molecular weight: 1597.51 g/mol;
Exact mass: 1596.58 g/mol;
$t_R$-value: 10.0 min (10 - 90% B in 15 min, Method A)
$t_R$-value of co-injected cold standard: 9.9 min (10 - 90% B in 15 min, Method A)
Capacity factor k: 5.7.

**Cold complexation and radiolabeling reaction conditions and results**

**Table 1:** Reaction conditions, chemical purity and yield of the investigated $^{nat}$Ga- and $^{nat}$Lu-PSMA ligands.

| PSMA inhibitor | Reaction conditions | Chemical purity | Yield[a] |
|---|---|---|---|
| $^{nat}$Ga-2 | 75 °C, 30 min | 93.0% | 3.35% |
| $^{nat}$Ga-**3** | 75 °C. 30 min | 97.2% | 26.5% |
| $^{nat}$Lu-**3** | 70 °C, 25 min | 92.3% | >99% |
| $^{nat}$Ga-**4** | 75 °C, 30 min | 97.6% | 15.7% |
| $^{nat}$Lu-**5** | 70 °C, 30 min | >99% | >99% |
| $^{nat}$Lu-**6** | 95 °C, 25 min | >99% | >99% |
| $^{nat}$Lu-**7** | 95 °C, 25 min | >99% | >99% |
| $^{nat}$Lu-**8** | 95 °C, 25 min | 94.9% | >99% |
| $^{nat}$Lu-**9** | 95 °C, 25 min | 98.1% | >99% |
| $^{nat}$Lu-**10** | 95 °C, 25 min | 99.1% | >99% |
| $^{nat}$Lu-**11** | 95 °C, 25 min | 92.9% | >99% |

[a]calculated relative to the uncomplexed precursor. Number of experiments is n = 1.

Table 2: Reaction conditions, RCY, apparent $A_m$, used activities and RCP of the investigated [$^{177}$Lu]Lu-PSMA ligands.

| PSMA inhibitor | Reaction conditions | RCY$^a$ | apparent $A_m$ [GBq/μmol] | used activities [MBq] | RCP | No. of experiments |
|---|---|---|---|---|---|---|
| [$^{177}$Lu] Lu-**3** | 70 °C, 25 min | 69.7 ± 18.2% | 12.5 - 21.9 | 22.3 - 62.2 | 96.9 ± 2.1% | 6 |
| [$^{177}$Lu] Lu-**5** | 80 °C, 25 min | 44.0 ± 7.6% | 4.64 - 14.5 | 14.0 - 35.9 | 89.3 ± 1.9% | 4 |
| [$^{177}$Lu] Lu-**6** | 95 °C, 25 min | 81.4 ± 6.9% | 24.1 & 24.8 | 28.0 & 32.5 | 98.2 ± 0.1 % | 2 |
| [$^{177}$Lu] Lu-**7** | 95 °C, 25 min | >99% | 27.4 | 27.4 | 94.7% | 1 |
| [$^{177}$Lu] Lu-**10** | 95 °C, 25 min | 70.3 to >99% | 26.3 - 62.0 | 37.5 - 62.0 | 93.6 ± 2.5% | 3 |
| [$^{177}$Lu] Lu-**11** | 95 °C, 25 min | >99% | 42.2 - 63.5 | 42.2 - 63.5 | 97.2 ± 1.2% | 4 |

$^a$decay corrected to the start of synthesis. Data for isolated RCY and RCP are expressed as mean ± SD.

**In vitro experiments**

**Cell culture**

PSMA-positive LNCaP cells (300265; Cell Lines Service, Eppelheim, Germany) were cultivated in Dulbecco's modified Eagle medium/Nutrition Mixture F-12 with GlutaMAX (1/1, DMEM-F12, Thermo Fisher Scientific, Darmstadt, Germany) supplemented with 10% fetal bovine serum (Merck KGaA, Darmstadt, Germany) and kept at 37 °C in a humidified 5% $CO_2$ atmosphere. One day (24 ± 2 h) prior to all in vitro experiments, the cultivated LNCaP cells were harvested using a mixture of trypsin/ethylenediaminetetraacetic acid (0.05%/0.02%) in phosphate-buffered saline (PBS) (Thermo Fisher Scientific, Darmstadt, Germany) and centrifuged at 1,300 rpm (ca. 190 × g) for 3 min at room temperature (Heraeus Megafuge 16, Thermo Fisher Scientific, Darmstadt, Germany). After centrifugation, the supernatant was disposed and the cell pellet was resuspended in culture medium. Cells were counted with a Neubauer hemocytometer (Paul Marienfeld GmbH & Co. KG, Lauda-Königshofen, Germany) and seeded in 24-well plates (Greiner Bio-One, Kremsmünster, Austria). $IC_{50}$ values were determined by transferring 1.50 x $10^5$ cells/mL per well into 24-well plates, whereas internalization was assessed by transferring 1.25 x $10^5$ cells/mL per well into poly-L-lysine (PLL)-coated 24-well plates (Greiner Bio-One, Kremsmunster, Austria).

**Affinity determinations ($IC_{50}$)**

The culture medium was removed and the cells were washed with 500 μL of Hank's balanced salt solution (HBSS) (Merck KGaA, Darmstadt, Germany), containing 1% bovine serum albumin (BSA) (Merck KGaA, Darmstadt, Germany). Afterwards, 200 μL of HBSS (1% BSA) were added to each well and equilibrated on ice (4 °C) for 15 min. 25 μL/well of either HBSS (1% BSA) (= control) or of solutions, containing the respective unlabeled ligand in increasing concentrations ($10^{-10}$ - $10^{-4}$ M in HBSS) were added, followed by the addition of 25 μL of ([$^{125}$I]I-BA)KuE in HBSS (1%BSA) to each well. Experiments were carried out in triplicates for each concentration. The final concentrations of unlabeled ligand ranged from $10^{-11}$ - $10^{-5}$ M and the final radioligand concentration was 0.2 nM in all binding assays. The cells were incubated for one hour at 4 °C. Incubation was terminated by removal of the incubation medium. The cells were washed with 250 μL of HBSS (1% BSA) and the wash medium was combined with the respective supernatant. This fraction represents the amount of free radioligand. The cells were lysed by addition of 250 μL of 1 M aqueous NaOH. The lysate of each well was transferred to the respective vial as well as 250 μL of 1 M NaOH used for rinsing the well. Quantification of the amount of free and bound activity was performed in a γ-counter. The corresponding $IC_{50}$ values were calculated using the GraphPad PRISM7 software.

Binding to PSMA was determined using LNCaP human prostate cancer cells in a competitive binding assay with $^{nat}$Ga or $^{nat}$Lu complexes of compounds **2** to **11.** For comparison, $IC_{50}$ values of $^{nat}$Lu-PSMA-10 ($^{nat}$Lu-**1**) (7.2 ± 3.7 nM) were also determined. The results show loss of affinity to a varying extent for all modifications differing from

glutamate at P1' position (*Table 3*). Only $^{nat}$Lu-**3** (carbamate I) and $^{nat}$Lu-**11** (tetrazole derivative) still exhibited high affinity (7.1 ± 0.7 nM and 16.4 ± 3.8 nM, respectively) towards PSMA-expressing LNCaP cells.

**Internalization studies**

The culture medium was removed and the cells were washed with 500 μL of DMEM-F12 containing 5% BSA. Afterwards, 200 μL of DMEM-F12 (5% BSA) were added to each well and left to equilibrate at 37 °C for 15 min. 25 μL of DMEM-F12 (5% BSA) were added to each well, followed by the addition of 25 μL of the respective $^{177}$Lu-labeled ligand (10.0 nM in DMEM-F12 (5% BSA)). For blocking PSMA-specific binding and uptake, 25 μL of 2-(phosphonomethyl)pentane-1,5-dioic acid (2-PMPA) (100 μM in DMEM (5% BSA)) instead of DMEM-F12 (5% BSA) were added prior to radiotracer addition. The same procedure was conducted with ([$^{125}$I]I-BA)KuE (2.0 nM in DMEM-F12 (5% BSA)) which served as the reference. Each experiment (control and blockade) was performed in triplicate. The final concentration of the $^{177}$Lu-labeled ligand was 1.0 nM and that of ([$^{125}$I]I-BA)KuE was 0.2 nM in all internalization assays. The cells were incubated for one hour at 37 °C. Incubation was terminated by placing the plate on ice (4 °C, 1 min) and removal of the incubation medium. The cells were washed with 250 μL of ice-cold PBS and the wash medium was combined with the respective supernatant. This fraction represents the amount of free radioligand. 250 μL of ice-cold 2-PMPA (10 μM in PBS) were added and the cells were incubated for 10 min at 4 °C. Afterwards, the cells were rinsed again with 250 μL of ice-cold PBS and the wash medium was combined with the respective supernatant. This fraction represents the amount of cell surface-bound ligand. In the last step, the cells were lysed by addition of 250 μL of 1 M aqueous NaOH. After 20 min, the lysate of each well was transferred to the respective vial as well as 250 μL of 1 M NaOH used for rinsing the well. This fraction represents the amount of internalized radioligand. Quantification of the amount of free, cell surface-bound and internalized activity was performed in a γ-counter. The corresponding internalization values were corrected for non-specific binding and normalized to the specific binding observed for the reference ([$^{125}$I]I-BA)KuE.

LNCaP cells were used to investigate internalization of the $^{177}$Lu-labeled compounds **1**, **3**, **5**, **6**, **7**, **10** and **11.** No internalization studies were performed on thioureate **2,** carbamate II (**4**), L-2-aminoheptanoic acid derivative **8** and furyl derivative **9**, as these candidates were excluded due to poor $IC_{50}$ data. Normalized to the uptake of ([$^{125}$I]I-BA)KuE, the results in *Table 3* show that all modified compounds exhibited significantly lower values in comparison to [$^{177}$Lu]Lu-PSMA-10 ([$^{177}$Lu]Lu-**1**) (177 ± 15%). $^{nat}$Lu-**3** (carbamate I) displayed an internalization of 67.8 ± 0.5% compared to ([$^{125}$I]I-BA)KuE and hence, showed highest internalization of all modified inhibitor derivatives. For [$^{177}$Lu]Lu-**10** (alkyne) and [$^{177}$Lu] Lu-**11** (tetrazole), both lacking a glutamate moiety at the P1' position, only weak internalization was detected (1.2 ± 0.4% and 9.9 ± 3.2% compared to the reference, respectively). For all proinhibitor motifs internalization studies were conducted prior to any affinity determination, in order to investigate possible substrate cleavage kinetics. Internalization at 37 °C was determined at several time points of 0.5 h, 1 h, 2 h and 4 h. All in all, no internalization at any time point could be detected.

**Lipophilicity**

In analogy to internalization studies, log D values were determined for $^{177}$Lu-labeled compounds **1**, **3**, **5**, **6**, **7**, **10** and **11** (*Table 3*). Within the proinhibitor subgroup, methionine-functionalized derivative 5 displayed the most hydrophilic character with a log D value of -2.89 ± 0.18. Moreover, [$^{177}$Lu]Lu-**3** (carbamate I) represents the most hydrophilic compound of all examined and modified PSMA inhibitors (log D = -3.40 ± 0.45), comparable to [$^{177}$ Lu]Lu-**1** (log D = -3.15 ± 0.01).

**Table 3.** PSMA-binding affinities ($IC_{50}$), internalization (%) and lipophilicity (log D) of the investigated compounds.[a]

| PSMA inhibitor | $IC_{50}$ | %Internalization compared to the reference[b] | log D |
|---|---|---|---|
| $^{nat}$Lu/[$^{177}$Lu]Lu-**1** | 2.76 ± 0.51 nM | 1 h: 177 ± 15 | -3.78 ± 0.01 |
| $^{nat}$Ga-**2** | > 3 μM (n = 2) | n.d. | n.d. |
| $^{nat}$Ga-**3** | 21.3 ± 1.7 nM | n.d. | n.d. |
| $^{nat}$Lu/[$^{177}$Lu]Lu-**3** | 7.1 ± 0.7 nM | 1 h: 67.8 ± 0.5 | -3.40 ± 0.45 |
| $^{nat}$Ga-**4** | > 1 μM (n = 2) | n.d. | n.d. |

(continued)

| PSMA inhibitor | $IC_{50}$ | %Internalization compared to the reference[b] | log D |
|---|---|---|---|
| $^{nat}$LU/[$^{177}$ Lu]Lu-**5** | 26 ± 16 μM | 0.5 h: 0.00 ± 0.00<br>1 h: 0.00 ± 0.00<br>2 h: 0.17 ± 0.32<br>4 h: 0.01 ± 0.05 | -2.89 ± 0.18 |
| $^{nat}$Lu/[$^{177}$Lu]Lu-**6** | 2.5 ± 1.2 μM | 0.5 h: 0.00 ± 0.00<br>1 h: 0.00 ± 0.10<br>2 h: 0.03 ± 0.03<br>4 h: 0.01 ± 0.06 | -2.52 ± 0.22 |
| $^{nat}$Lu/[$^{177}$Lu]Lu-**7** | 6.3 ± 3.6 μM | 0.5 h: 0.00 ± 0.00<br>1 h: 0.00 ± 0.00<br>2 h: 0.00 ± 0.00<br>4 h: 0.00 ± 0.00 | -2.37 ± 0.22 |
| $^{nat}$Lu-**8** | > 2 μM (n = 2) | n.d. | n.d. |
| $^{nat}$Lu-**9** | > 440 nM (n = 2) | n.d. | n.d. |
| $^{nat}$Lu/[$^{177}$Lu]Lu-**10** | 138 ± 53 nM | 1 h: 1.2 ± 0.4 | -2.83 ± 0.08 |
| $^{nat}$Lu/[$^{177}$Lu]Lu-**11** | 16.4 ± 3.8 nM | 1 h: 9.9 ± 3.2 | -2.91 ± 0.05 |

[a]Binding assays ($IC_{50}$) were performed using LNCaP cells (150,000 cells/well) and ([$^{125}$I]I-BA)KuE (c = 0.2 nM) as radioligand. Cells were incubated in HBSS (1% BSA) at 4 °C for 1 h. [b]Internalization values were corrected for unspecific binding and normalized to the external reference ([$^{12s}$I]I-BA)KuE (13.0 ± 2.5% internalization at 1 h (n = 21), c = 0.2 nM; 1.0 nM for $^{177}$Lu-compounds; 37 °C, 1 h, 125,000 cells/well, PLL-coated plates). Data for binding ($IC_{50}$) and internalization are expressed as mean ± SD (n = 3) unless otherwise stated. Data are expressed as mean ± SD (n = 6) for log D.

**In vivo experiments**

All animal experiments were conducted in accordance with general animal welfare regulations in Germany (German animal protection act, as amended on 18.05.2018, Art. 141 G v. 29.3.2017 I 626, approval no. 55.2-1-54-2532-71-13) and the institutional guidelines for the care and use of animals. To establish tumor xenografts, LNCaP cells (approx. $10^7$ cells) were suspended in 200 μL of a 1/1 mixture (v/v) of DMEM F-12 and Matrigel (BD Biosciences, Heidelberg, Germany) and inoculated subcutaneously onto the right shoulder of 6 - 8 weeks old CB17-SCID mice (Charles River Laboratories, Sulzfeld, Germany). Mice were used for experiments when tumor size reached 5 - 10 mm in diameter (3 - 6 weeks after inoculation).

**Biodistribution**

Approximately 2 - 10 MBq (0.20 nmol) of the $^{177}$Lu-labeled PSMA inhibitors were injected into the tail vein of LNCaP tumor xenograft-bearing male CB17-SCID mice (n = 3 to 5). They were sacrificed by $CO_2$ asphyxiation and cervical dislocation either 1 h or 24 h post injection (p.i.) (n = 3 for [$^{177}$Lu]Lu-**5**, **-6** & **-7** (proinhibitor compounds I-III), n = 4 for [$^{177}$Lu]Lu-**10** (alkyne) and n = 5 for [$^{177}$Lu]Lu-**3** (carbamate I) and n = 5 for [$^{177}$Lu]Lu-**11** (tetrazole)). Selected organs were removed, weighed and organ activities measured in a γ-counter.

[$^{177}$Lu]Lu-**5**, **-6** and **-7** (proinhibitors I-III) were evaluated (n = 3) in tumor-bearing CB-17 SCID mice prior to any affinity measurement. However, *in vivo* data in general reflected the low internalization values for all proinhibitors, with a maximum tumor accumulation of 0.33 ± 0.11% ID/g for [$^{177}$Lu]Lu-**6** (proinhibitor II) and a minimum tumor accumulation of 0.09 ± 0.02% ID/g for [$^{177}$Lu]Lu-**5** (proinhibitor I) as depicted in *Figure 6.* Furthermore, non-target tissue uptake was on the scale of [$^{177}$Lu]Lu-PSMA-10 ([$^{177}$Lu]Lu-**1**) or even higher.

For further *in vivo* studies, derivatives with medium to high affinity were chosen (i.e. [$^{177}$Lu]Lu-**3**, **-10** and -**11**) (*Figure 7*). For all modified peptides, a distinctly lower tumor accumulation (0.10 to 1.20% ID/g) at 24 h p.i. in comparison to the original [$^{177}$Lu]Lu-PSMA-10 compound (9.82 ± 0.33% ID/g) could be observed with unchanging salivary gland uptake (*Figure 8*). Apparently, tumor-to-submandibular gland and tumor-to-parotid gland ratios declined by a factor of

8 for [$^{177}$Lu]Lu-**11** (tetrazole) up to a factor of 45 for [$^{177}$Lu]Lu-**10** (alkyne). However, salivary gland uptake in general stayed at an equally low level for all EuE- and non-EuE-based inhibitor motifs (between 0.02 ± 0.00% ID/g and 0.09 ± 0.03% ID/g). Residual tumor-to-tissue ratios observed for [$^{177}$Lu]Lu-**3**, **-10** and **-11** were also markedly lower than for [$^{177}$Lu]Lu-PSMA-10 (*Figure 8*). Tumor-to-tissue ratios of proinhibitors were not determined, as uptake in tumor xenografts was very low (≤ 0.33 ±0.11% ID/g) **(Error! Reference source not found**.6), with no significant change in kidney accumulation or other non-target tissues.

Tumor uptake for [$^{177}$Lu]Lu-**3** (carbamate I) and **-11** (tetrazole) was also analyzed 1 h p.i. and compared to [$^{177}$Lu]Lu-**1** (*Table 5*). At this early time point, tumor accumulation of [$^{177}$Lu]Lu-**3** was already only about half of the uptake obtained for [$^{177}$Lu]Lu-**1** (5.31 ± 0.94% ID/g for [$^{177}$Lu]Lu-**3** vs. 12.2 ± 1.8% ID/g for [$^{177}$Lu]Lu-**1**). [$^{177}$Lu]Lu-**11** showed even lower tumor uptake (3.40 ± 0.63% ID/g). Kidney uptake was distinctly lower for both substances (61.8 ± 25.9% ID/g for [$^{177}$Lu]Lu-**3** and 33.2 ± 3.8% ID/g for [$^{177}$Lu]Lu-**11** vs. 173 ± 56% ID/g for [$^{177}$Lu]Lu-**1**) but activity accumulation in all other non-target tissues stayed in the same range. Interestingly, salivary gland uptake of both peptides was found to be distinctly higher at 1 h p.i. (between 0.37 ± 0.08% ID/g and 0.62 ± 0.20% ID/g) than at 24 h p.i. (*Table 5* and 6). This resulted in two-fold lower tumor-to-submandibularis and -parotidea ratios of [$^{177}$Lu]Lu-**3** and **-11** at 1 h p.i. compared to their respective values at 24 h p.i. (*Table 4* and *5*).

For visualization of the biodistribution data, maximum intensity projections (MIPs) of $\mu$SPECT/CT scans in LNCaP xenograft-bearing mice, acquired 24 h p.i. of [$^{177}$Lu]Lu-**3**, [$^{177}$Lu]Lu-**6**, [$^{177}$Lu]Lu-**7**, [$^{177}$Lu]Lu-**10**, [$^{177}$Lu]Lu-**11** and [$^{177}$Lu]Lu-**1** ([$^{177}$Lu]Lu-PSMA-10) are depicted in Figure 9. All images were scaled to the same maximum uptake value (2.5% ID/mL), enabling a clear visual comparison. Arrows indicate apparent tumor uptake in [$^{177}$Lu]Lu-**3**, [$^{177}$Lu]Lu-**11** and [$^{177}$Lu]Lu-**1**.

**In vivo data od PSMA inhibitors**

**Table 4**: Biodistribution of [$^{177}$Lu]Lu-PSMA-10 ([$^{177}$Lu]Lu-**1**) and related derivatives with modified inhibitor motifs at 24 h p.i. in male LNCaP tumor xenograft-bearing CB17-SCID mice. Data are expressed as a percentage of the injected dose per gram (% ID/g), mean ± standard deviation (n = 2 for [$^{177}$Lu]Lu-**5***, n = 3 for [$^{177}$Lu]Lu-**6** and [$^{177}$Lu]Lu-**7**, n = 4 for [$^{177}$Lu]Lu-**10** and n = 5 for [$^{177}$Lu]Lu-**1**, [$^{177}$Lu]Lu-**3** and [$^{177}$Lu]Lu-**11**. *Ingestion of radioactively contaminated animal feed led to putative high activities in stomach and intestine, therefore excluding all values of mouse 3).

| Organ | [$^{177}$Lu]Lu-**1** ([$^{177}$Lu]Lu-PSMA-10) | [$^{177}$Lu]Lu-**3** (carbamate I) | [$^{177}$Lu]Lu-**5** (proinhibitor I) | [$^{177}$Lu]Lu-**6** (proinhibitor II) | [$^{177}$Lu]Lu-**7** (proinhibitor III) | [$^{177}$Lu]Lu-**10** (alkyne) | [$^{177}$Lu]Lu-**11** (tetrazole) |
|---|---|---|---|---|---|---|---|
| **Blood** | 0.00 ± 0.00 | 0.00 ± 0.00 | 0.00 ± 0.00 | 0.00 ± 0.00 | 0.01 ± 0.00 | 0.01 ± 0.01 | 0.00 ± 0.00 |
| **Heart** | 0.02 ± 0.00 | 0.02 ± 0.01 | 0.01 ± 0.00 | 0.03 ± 0.01 | 0.03 ± 0.01 | 0.01 ± 0.00 | 0.02 ± 0.00 |
| **Lung** | 0.03 ± 0.00 | 0.03 ± 0.01 | 0.33 ± 0.30 | 0.10 ± 0.08 | 0.04 ± 0.01 | 0.03 ± 0.03 | 0.05 ± 0.02 |
| **Liver** | 0.18 ± 0.06 | 0.13 ± 0.05 | 0.05 ± 0.00 | 0.14 ± 0.02 | 0.17 ± 0.05 | 0.09 ± 0.04 | 0.18 ± 0.04 |
| **Spleen** | 0.17 ± 0.03 | 0.09 ± 0.01 | 0.05 ± 0.01 | 0.11 ± 0.03 | 0.08 ± 0.02 | 0.04 ± 0.00 | 0.11 ± 0.03 |
| **Pancreas** | 0.01 ± 0.00 | 0.02 ± 0.01 | 0.01 ± 0.00 | 0.03 ± 0.01 | 0.02 ± 0.00 | 0.01 ± 0.01 | 0.02 ± 0.00 |
| **Stomach** | 0.06 ± 0.01 | 0.06 ± 0.04 | 0.14 ± 0.07 | 0.05 ± 0.01 | 0.04 ± 0.01 | 0.12 ± 0.11 | 0.12 ± 0.11 |
| **Intestine** | 0.11 ± 0.05 | 0.12 ± 0.06 | 0.55 ± 0.04 | 0.18 ± 0.06 | 0.10 ± 0.05 | 0.32 ± 0.29 | 0.52 ± 0.68 |
| **Kidneys** | 1.97 ± 0.78 | 0.31 ± 0.05 | 0.48 ± 0.02 | 2.36 ± 0.39 | 2.04 ± 0.75 | 1.29 ± 0.28 | 3.23 ± 0.77 |
| **Adrenals** | 0.06 ± 0.04 | 0.04 ± 0.01 | 0.00 ± 0.00 | 0.04 ± 0.00 | 0.03 ± 0.01 | 0.02 ± 0.02 | 0.17 ± 0.19 |
| **Muscle** | 0.00 ± 0.00 | 0.01 ± 0.01 | 0.00 ± 0.00 | 0.02 ± 0.00 | 0.01 ± 0.00 | 0.00 ± 0.00 | 0.02 ± 0.01 |
| **Bone** | 0.02 ± 0.01 | 0.02 ± 0.01 | 0.00 ± 0.00 | 0.02 ± 0.01 | 0.04 ± 0.01 | 0.03 ± 0.01 | 0.18 ± 0.04 |
| **Tumor** | 9.82 ± 0.30 | 1.20 ± 0.55 | 0.09 ± 0.02 | 0.33 ± 0.11 | 0.14 ± 0.05 | 0.10 ± 0.03 | 0.68 ± 0.16 |
| **Submandibular gland** | 0.04 ± 0.01 | 0.03 ± 0.01 | 0.02 ± 0.01 | 0.07 ± 0.01 | 0.05 ± 0.02 | 0.02 ± 0.00 | 0.04 ± 0.01 |
| **Parotid gland** | 0.04 ± 0.01 | 0.04 ± 0.02 | 0.02 ± 0.00 | 0.09 ± 0.03 | 0.06 ± 0.01 | 0.02 ± 0.00 | 0.06 ± 0.02 |

**Table 5**: Biodistribution of [$^{177}$Lu]Lu-PSMA-10 ([$^{177}$Lu]Lu-**1**) and related derivatives with modified inhibitor motifs at 1 h p.i. in male LNCaP tumor xenograft-bearing CB17-SCID mice. Data are expressed as a percentage of the injected dose per gram (% ID/g), mean ± standard deviation (n = 5 for [$^{177}$Lu]Lu-**1**, [$^{177}$Lu]Lu-**3** and [$^{177}$Lu]Lu-**11**).

| Organ | [$^{177}$Lu]Lu-**1**[a] ([$^{177}$Lu]Lu-PSMA-10) | [$^{177}$Lu]Lu-**3** (carbamate I) | [$^{177}$Lu]Lu-**11** (tetrazole) |
|---|---|---|---|
| **Blood** | 0.48 ± 0.15 | 0.97 ± 0.18 | 1.09 ± 0.30 |
| **Heart** | 0.42 ± 0.15 | 0.36 ± 0.04 | 0.41 ± 0.04 |
| **Lung** | 0.80 ± 0.16 | 2.88 ± 2.96 | 1.23 ± 0.27 |
| **Liver** | 0.40 ± 0.13 | 0.87 ± 0.28 | 0.74 ± 0.17 |
| **Spleen** | 10.7 ± 2.9 | 2.51 ± 0.95 | 1.04 ± 0.15 |
| **Pancreas** | 0.31 ± 0.12 | 0.25 ± 0.06 | 0.33 ± 0.11 |
| **Stomach** | 0.26 ± 0.08 | 0.30 ± 0.09 | 1.55 ± 1.67 |
| **Intestine** | 0.28 ± 0.10 | 0.36 ± 0.17 | 0.67 ± 0.21 |
| **Kidneys** | 173 ± 56 | 61.8 ± 25.9 | 33.2 ± 3.8 |
| **Adrenals** | 1.30 ± 0.37 | 1.46 ± 0.70 | 0.61 ± 0.42 |
| **Muscle** | 0.36 ± 0.10 | 0.13 ± 0.04 | 0.15 ± 0.03 |
| **Bone** | 0.37 ± 0.24 | 0.18 ± 0.02 | 0.47 ± 0.44 |
| **Tumor** | 12.2 ± 1.8 | 5.31 ± 0.94 | 3.40 ± 0.63 |
| **Submandibular gland** | n.d. | 0.37 ± 0.08 | 0.40 ± 0.05 |
| **Parotid gland** | n.d. | 0.62 ± 0.20 | 0.56 ± 0.08 |

[a]Values for [$^{177}$Lu]Lu-PSMA-10 ([$^{177}$Lu]Lu-**1**) at 1 h p.i. taken from WO2019/020831.

**Table 6**: Tumor-to-tissue ratios of [$^{177}$Lu]Lu-PSMA-10 ([$^{177}$Lu]Lu-**1**) and related derivatives with modified inhibitor motifs at 24 h p.i. in male LNCaP tumor xenograft-bearing CB17-SCID mice. Data are expressed as mean ratios ± standard deviation (n = 4 for [$^{177}$Lu]Lu-**10** and n = 5 for [$^{177}$Lu]Lu-**1**, [$^{177}$Lu]Lu-**3** and [$^{177}$Lu]Lu-**11**).

| **Ratio Tumor-to-** | **[$^{177}$Lu]Lu-1 ([$^{177}$Lu]Lu-PSMA-10)** | [$^{177}$Lu]Lu-**3** (carbamate I) | [$^{177}$Lu]Lu-**10** (alkyne) | [$^{177}$Lu]Lu-**11** (tetrazole) |
|---|---|---|---|---|
| **blood** | 11498 ± 1953 | 947 ± 652 | 26.8 ± 16.9 | 227 ± 50 |
| **kidney** | 5.66 ± 1.99 | 3.80 ± 1.36 | 0.08 ± 0.01 | 0.22 ± 0.06 |
| **muscle** | 2441 ± 373 | 127 ± 92 | 27.7 ±10.9 | 55.9 ± 24.3 |
| **liver** | 57.5 ± 13.1 | 8.94 ± 2.94 | 1.46 ± 0.88 | 3.74 ± 0.50 |
| **submandibular gland** | 275 ± 49 | 34.5 ± 9.2 | 6.11 ± 1.78 | 16.9 ± 2.2 |
| **parotid gland** | 250 ± 64 | 31.8 ± 9.3 | 6.38 ± 3.06 | 12.2 ± 1.7 |

**Table 7**: Tumor-to-tissue ratios of [$^{177}$Lu]Lu-**3** (carbamate I) and [$^{177}$Lu]Lu-**11** (tetrazole) at 1 h p.i. in male LNCaP tumor xenograft-bearing CB17-SCID mice. Data are expressed as mean ratios ± standard deviation (n = 5 for [$^{177}$Lu]Lu-**3** and [$^{177}$Lu]Lu-**11**).

| **Ratio Tumor-to-** | [$^{177}$Lu]Lu-**3** (carbamate I) | [$^{177}$Lu]Lu-**11** (tetrazole) |
|---|---|---|
| **blood** | 5.57 ± 1.18 | 3.46 ± 1.62 |
| **kidney** | 0.09 ± 0.03 | 0.10 ± 0.03 |
| **muscle** | 44.1 ± 9.0 | 24.9 ± 10.2 |
| **liver** | 6.61 ± 2.19 | 5.00 ± 2.11 |
| **submandibular gland** | 14.7 ± 3.5 | 8.74 ± 2.52 |
| **parotid gland** | 9.09 ± 3.13 | 6.20 ± 1.74 |

**Metabolite analysis**

[$^{177}$Lu]Lu-**11** (9.64 MBq) was injected into the tail vein of a LNCaP tumor xenograft-bearing CB17-SCID mouse. The animal was sacrificed 1 h p.i. and subjected to the standard procedure for biodistribution studies. In addition, urine was taken from all mice that were investigated in this experiment (8.4 - 9.0 MBq) and pooled (n = 5). Relevant dissected organs (tumor, kidneys, liver) and body fluids (blood and urine) were collected, homogenized if necessary and subjected to mechanochemical as well as solid phase extraction (SPE).

Four 2 mL LoBind tubes were equipped with steel and ceramic beads from Lysis Tubes W (analytikjena, Jena, Germany) for kidneys, tumor and liver (bisected). 1 mL radioimmunoprecipitation assay (RIPA) buffer containing 2.0 μmol of 2-PMPA was added to each tube and the organs were homogenized with a MM-400 ball mill (Retsch GmbH, Haan, Germany) at 30 Hz for 20 min. Afterwards, the homogenates were transferred to LoBind tubes free from steel and ceramic beads and centrifuged (15,200 rpm, 10 min, 21 °C). The first supernatants were stored, and the precipitates were again subjected to mechanochemical extraction with 1 mL RIPA buffer (2.0 μmol PMPA) at 30 Hz for 20 min. After

centrifugation (15,200 rpm, 10 min, 21 °C), also the second supernatants were kept for SPE.

After cardiac puncture, 1 mL Tracepur®-$H_2O$ was directly added to the collected blood sample and centrifuged twice (13,000 rpm, 5 min) to separate the plasma from the blood cells. The precipitate was dissolved in 500 μL Tracepur®-$H_2O$ and together with the first supernatant again centrifuged (13,000 rpm, 5 min). Both supernatants were kept for SPE.

Pooled urine samples were centrifuged (13,000 rpm, 5 min) and the supernatant was directly analyzed via radio-RP-HPLC.

For solid phase extraction, the supernatants were loaded onto Strata-X cartridges (200 mg), which were preconditioned with 5 mL MeOH and 5 mL $H_2O$ (eight cartridges in total, for all organ and blood supernatants). The cartridges were washed with 1 mL Tracepur®-$H_2O$ and dried prior to elution. For each cartridge, 750 μL of MeCN/$H_2O$ (6/4, 0.1% TFA) were used for elution and the extracts were analyzed via radio-RP-HPLC. The extraction efficiencies were calculated before and after SPE and the results are given in *Table* 8.

Besides biodistribution and μSPECT/CT studies, [$^{177}$Lu]Lu-**11** (tetrazole) was analyzed according to its metabolic stability 1 h p.i. Relevant dissected organs (tumor, kidney, liver) and body fluids (blood and urine) were collected, homogenized if necessary and subjected to extraction of the incorporated activity. Tissue extracts were analyzed by radio-RP-HPLC at a predefined gradient (25 - 40% MeCN (0.1% TFA) in 20 min), for which the retention time (18.3 min) of the intact cold standard ($^{nat}$Lu-**11**) was previously determined and hence, served as a reference. Only one metabolite with higher hydrophilicity was detected in tumor (7.1%), blood (8.5%) and kidney (11.7%), with the maximum percentage of metabolite amount found in the urine (18.7%). This metabolite was not detectable in the liver homogenate, instead a more lipophilic metabolite was observed at $t_R$ = 20.5 min (28.0%). The respective radio-RP-HPLC analyses of extracts from homogenized organs and body fluids are depicted in *Figure 10.*

**Table 8**: Extraction efficiencies of [$^{177}$Lu]Lu-**11** from liver, tumor, kidneys and blood using a MM-400 ball mill. The percentage of activity after sample extraction and after SPE purification was quantified, decay corrected and the overall extracted activity was calculated.

| **Organs and body fluids** | **efficiency [% extracted radioactivity]** | | |
|---|---|---|---|
| | **sample extraction** | **SPE purification** | **overall** |
| **liver** | 96.1 | 67.2 | 64.5 |
| **tumor** | 97.3 | 76.1 | 74.0 |
| **kidneys** | 96.0 | 74.8 | 71.8 |
| **blood** | 94.8 | 78.9 | 74.8 |

**μSPECT/CT imaging**

Imaging experiments were conducted using a MILabs VECTor$^4$ small-animal SPECT/PET/OI/CT. The resulting data were analyzed by the associated PMOD (version 4.0) software. Mice were anaesthetized with isoflurane and the $^{177}$Lu-labeled PSMA compounds were injected via the tail vein. Mice were euthanized 1 h or 24 h p.i. and blood samples for later biodistribution or metabolite analysis were taken by cardiac puncture before image acquisition. Static images were acquired with 45 min acquisition time using the HE-GP-RM collimator and a step-wise multi-planar bed movement. All images were reconstructed using the MILabs-Rec software (version 10.02) and a pixel-based Similarity-Regulated Ordered Subsets Expectation Maximization (SROSEM) algorithm with a window-based scatter correction (20% below and 20% above the photopeak, respectively). Voxel size CT: 80 μm, voxel size SPECT: 0.8 mm, 1.6 mm (FWHM) Gaussian blurring post processing filter, with calibration factor in kBq/mL and decay correction, no attenuation correction.

**BRIEF DESCRIPTION OF THE FIGURES**

**Figure 1:** Schematic representation of PSMA inhibitors containing (A) modifications within the central $Zn^{2+}$-binding unit (B) proinhibitor motifs (expected cleavage sites are indicated as red dotted lines) and (C) substituents & bioisosteres of the P1'-γ-carboxylic acid. All compounds were derived from the EuE-based ligand PSMA-10 (**1**) (D) which served as a reference for all obtained in vitro and in vivo data. (E) The reference ligand for $IC_{50}$ determinations was ([$^{125}$I]I-BA)KuE.

**Figure 2:** Detailed structures of the modified PSMA inhibitors, with (A) thioureate **2,** carbamate I & II (**3** & **4**) (B) proinhibitors I, II and III (**5, 6** & **7**) and (C) L-2-aminoheptanoic acid (**8**), furyl (**9**), alkyne (**10**) and tetrazole (**11**) derivatives. All compounds are depicted in their free chelator form and represent PSMA ligands containing modifications within the central $Zn^{2+}$-binding unit (A), proinhibitor motifs (B) and substituents & bioisosteres of the P1'-γ-carboxylic acid (C).

**Figure 3:** General, simplified synthetic routes for the preparation of thioureate **2,** carbamate I (**3**) and carbamate II (**4**). Synthesis of the binding motif of thioureate **2** was conducted by a solid phase procedure, whereas binding motifs **15** (carbamate I) and **17** (carbamate II) were obtained by solution phase synthesis prior to coupling to compound **18.**

**Figure 4:** General, simplified synthetic routes for the preparation of proinhibitors I, II & III (**5**, **6** & **7**). Synthesis of the binding motif of proinhibitor I (**5**) was conducted by a solid phase procedure, whereas binding motif **21** was obtained by a mixed solid/solution phase synthesis prior to coupling to compound **18.** Compound **22** (proinhibitor III) could only be obtained by solution phase synthesis.

**Figure 5:** General, simplified synthetic routes for the preparation of L-2-aha (**8**), furyl (**9**), alkyne (**10**) and tetrazole (**11**) derivatives. Syntheses of the binding motifs of compounds **8** (L-2-aha), **9** (furyl) and **10** (alkyne) were conducted by a solid phase procedure, whereas binding motif **26** (tetrazole) was obtained by solution phase synthesis prior to coupling to compound **18.**

**Figure 6:** Biodistribution data (% ID/g) of [$^{177}$Lu]Lu-PSMA-10 ([$^{177}$Lu]Lu-**1**), [$^{177}$Lu]Lu-**5**, [$^{177}$Lu]Lu-**6** and [$^{177}$Lu]Lu-**7** in tumor xenograft-bearing CB17-SCID mice at 24 h p.i. (n = 2 for [$^{177}$Lu]Lu-**5***, n = 3 for [$^{177}$Lu]Lu-**6** and [$^{177}$Lu]Lu-**7** and n = 5 for [$^{177}$Lu]Lu-PSMA-10 ([$^{177}$Lu]Lu-**1**)). Submandibular and parotid glands were dissected separately and their values are depicted in the columns 'GL. SUBMAND.' and 'GL. PAROTIDEA', respectively. *Ingestion of radioactively contaminated animal feed led to putative high activities in stomach and intestine, therefore excluding all values of mouse 3.

**Figure 7:** Biodistribution data (% ID/g) of [$^{177}$Lu]Lu-PSMA-10 ([$^{177}$Lu]Lu-**1**), [$^{177}$Lu]Lu-**3**, [$^{177}$Lu]Lu-**10** and [$^{177}$Lu]Lu-**11** in tumor xenograft-bearing CB17-SCID mice at 24 h p.i. (n = 4 for [$^{177}$Lu]Lu-**10** and n = 5 for all other experiments). Submandibular and parotid glands were dissected separately and their values are depicted in the columns 'GL. SUBMAND.' and 'GL. PAROTIDEA', respectively.

**Figure 8:** Tumor-to-tissue ratios of [$^{177}$Lu]Lu-PSMA-10 ([$^{177}$Lu]Lu-**1**), [$^{177}$Lu]Lu-**3**, [$^{177}$Lu]Lu-**10** and [$^{177}$Lu]Lu-**11** in selected organs at 24 h p.i. (n = 4 for [$^{177}$Lu]Lu-**10** and n = 5 for all other experiments).

**Figure 9:** Maximum intensity projections (MIPs) of μSPECT/CT scans in LNCaP xenograft-bearing mice, acquired 24 h p.i. of (A) [$^{177}$Lu]Lu-**3** (carbamate I) (3.2 MBq) (B) [$^{177}$Lu]Lu-**6** (proinhibitor II) (5.3 MBq) (C) [$^{177}$Lu]Lu-**7** (proinhibitor III) (4.5 MBq) (D) [$^{177}$Lu]Lu-**10** (alkyne) (9.7 MBq) (E) [$^{177}$Lu]Lu-**11** (tetrazole) (7.0 MBq) (F) [$^{177}$Lu]Lu-PSMA-10 (**1**) (2.8 MBq). For clear comparison, all images were scaled to the same maximum uptake value (2.5% ID/mL). Arrows indicate apparent tumor uptake in (A), (E) and (F). No μSPECT/CT image is displayed for [$^{177}$Lu]Lu-**5** (proinhibitor I), as ingestion of radioactive animal feed led to putative high activities in stomach and intestine. Therefore, all values of this mouse (= mouse 3) from the respective biodistribution study and also its μSPECT/CT image were excluded. Static images were acquired post mortem ($CO_2$ asphyxiation and cervical dislocation) and after cardiac puncture with an acquisition time of 45 min. Further biodistribution studies were performed after the scan and included in the calculation of % ID/g values provided by *Figure 6* and *7* and *Table 4.*

**Figure 10:** Radio-RP-HPLC analyses of extracts from homogenized organs and body fluids of tumor xenograft-bearing CB17-SCID mice, 1 h p.i. of [$^{177}$Lu]Lu-**11** (9.64 MBq, gradient: 25 - 40% MeCN (0.1% TFA) in 20 min, flow rate: 1 mL/min). The retention time (18.3 min) of the intact cold standard ($^{nat}$-Lu-**11**) was previously determined and hence, served as a reference. The radioactivity detector was placed downstream of the UV-detector causing for a slight time delay of the radioactivity signals.

## Claims

1. A compound of Formula (1a):

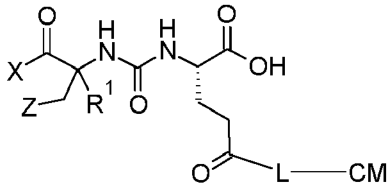

(1a);

or a pharmaceutically acceptable salt thereof, wherein;
L represents a linker group comprising a silicon-fluoride acceptor (SIFA) moiety which comprises a covalent bond between a silicon and a fluorine atom;
CM represents a chelator moiety, optionally containing a chelated nonradioactive or radioactive cation;
$R^1$ is H or a $C_{1-3}$ alkyl group optionally substituted with 1 to 3 fluorine atoms;
X is selected from OH and an amino acid group;
Z is selected from -V-$CO_2H$, -V-$NH_2$, -V-$PO_3H_2$, -V-COY, -V-W and a $C_{1-6}$ saturated or unsaturated hydrocarbon group optionally substituted with 1 to 3 fluorine atoms, where Y is an amino acid, W is 5- or 6-membered heterocyclic ring, and V is a bond or a $C_{1-3}$ alkyl group optionally substituted with 1 to 3 fluorine atoms;
and when X is OH, Z is not $CH_2CO_2H$.

2. The compound according to claim 1 which is a compound of Formula (2a):

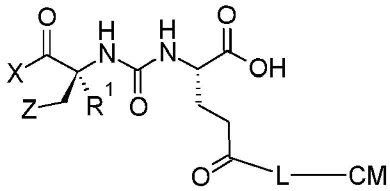

(2a);

or a pharmaceutically acceptable salt thereof.

3. The compound according to claim 1 or claim 2, wherein $R^1$ is H or methyl.

4. The compound according to claim 1 which is a compound of Formula (3a):

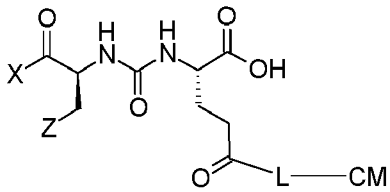

(3a);

or a pharmaceutically acceptable salt thereof.

5. The compound according to any one of claims 1 to 4, wherein X is - $NHCH(C_6H_{13})CO_2H$.

6. The compound according to any one of claims 1 to 4, wherein X is OH and Z is not $CH_2CO_2H$.

7. The compound according to any one of claims 1 to 5, wherein Z is -$CHFCO_2H$, - $CH_2CONH_2$, -$CH_2PO_3H_2$, n-butyl, acetylene, furan, -$CH_2$-tetrazole, -$NHCH(C_6H_{13})CO_2H$, $CH_2CO_2H$ or -$NHCH(CH_2CH_2SCH_3)CO_2H$.

8. The compound according to any one of claims 1 to 7, wherein the silicon-fluoride acceptor (SIFA) moiety has the structure represented by formula (5b):

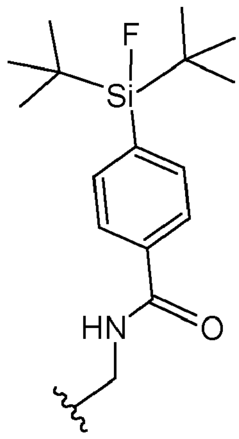

(5b).

9. The compound according to any one of claims 1 to 8, wherein the fluorine atom of the silicon-fluoride acceptor (SIFA) moiety is $^{18}F$.

10. The compound according to any one of claims 1 to 9, wherein the chelator moiety is 1,4,7,10-tetracyclododecan-N,N',N",N"'-tetraacetic acid (DOTA) or α-(2-carboxyethyl)-1,4,7,10-tetraazacyclododecane-1,4,7,10-tetraacetic acid (DOTAGA).

11. The compound according to any one of claims 1 to 10, wherein the chelator moiety contains a chelated cation selected from the cations of $^{68}Ga$ or $^{177}Lu$.

12. The compound according to any one of claims 1 to 11, wherein the group -L-CM is:

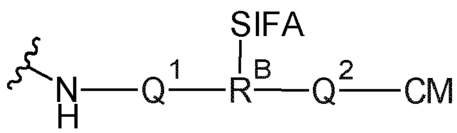

;

wherein $Q^1$ is a divalent linking group with a structure selected from an oligoamide, an oligoether, an oligothioether, an oligoester, an oligothioester, an oligourea, an oligo(ether-amide), an oligo(thioether-amide), an oligo(ester-amide), an oligo(thioester-amide), oligo(urea-amide), an oligo(ether-thioether), an oligo(ether-ester), an oligo(ether-thioester), an oligo ether-urea), an oligo(thioether-ester), an oligo(thioether-thioester), an oligo(thioether-urea), an oligo(ester-thioester), an oligo(ester-urea), and an oligo(thioester-urea), wherein $Q^1$ is optionally substituted with one or more substituents independently selected from -OH, $-OCH_3$, $-CH_2OH$, $-CO_2H$, $-CO_2CH_3$, $-NH_2$, $-CH_2NH_2$ and $-NHC(NH)NH_2$; $Q^2$ is selected from an amide bond, an ether bond, a thioether bond, an ester bond, a thioester bond, a urea bridge, an amine bond and linking groups of the formula:

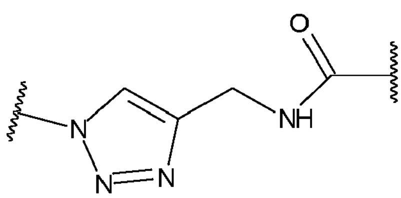

;

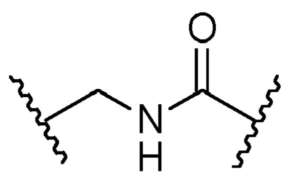

;

$R^B$ is a trivalent coupling group;
and SIFA is the silicon-fluoride acceptor moiety.

13. The compound according to claim 1 which is selected from:

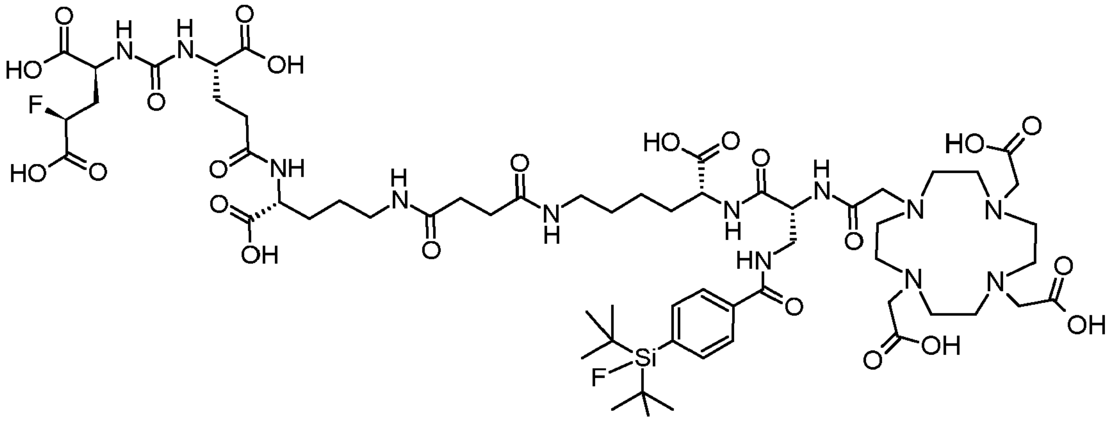

;

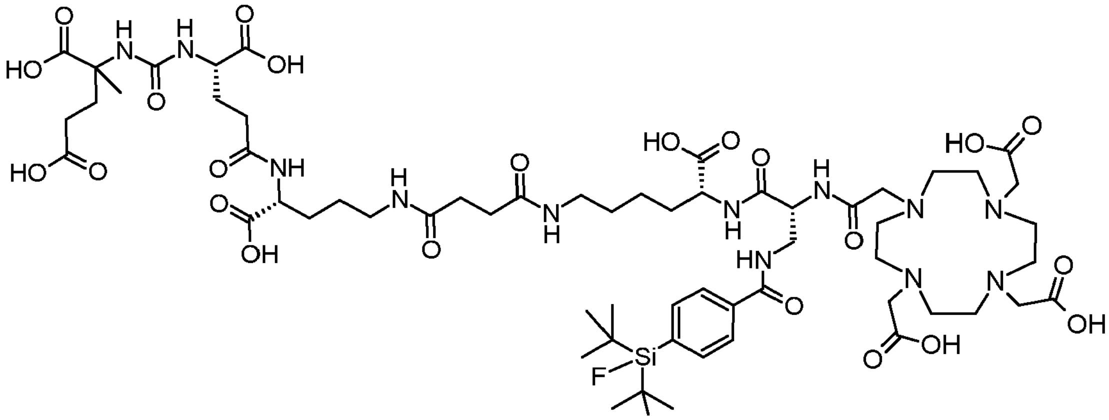

;

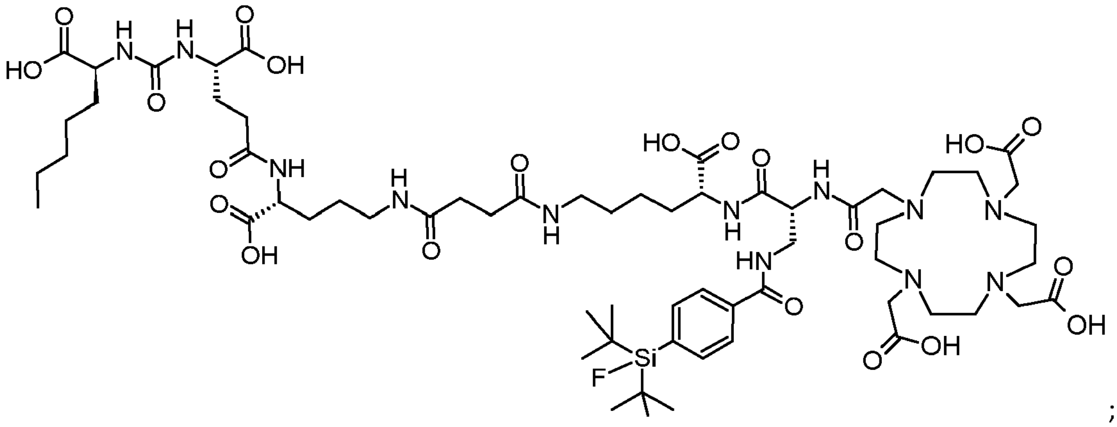

;

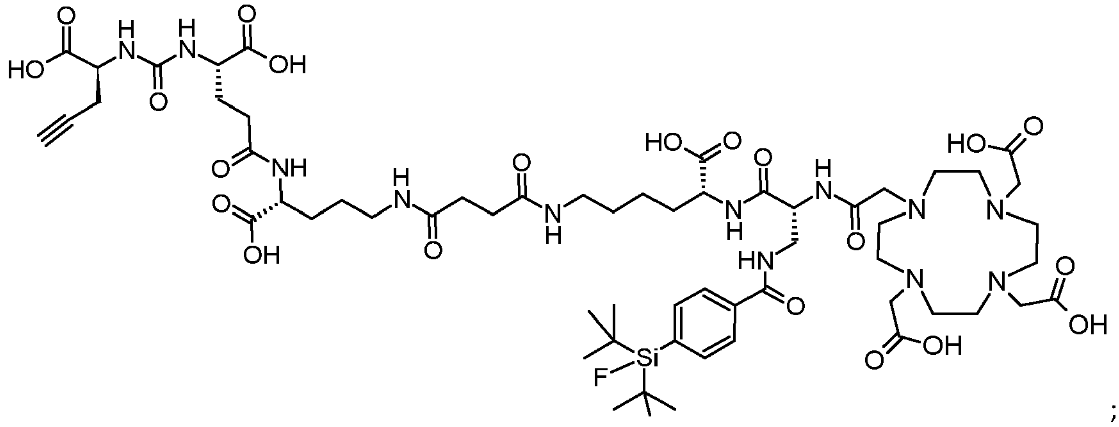

;

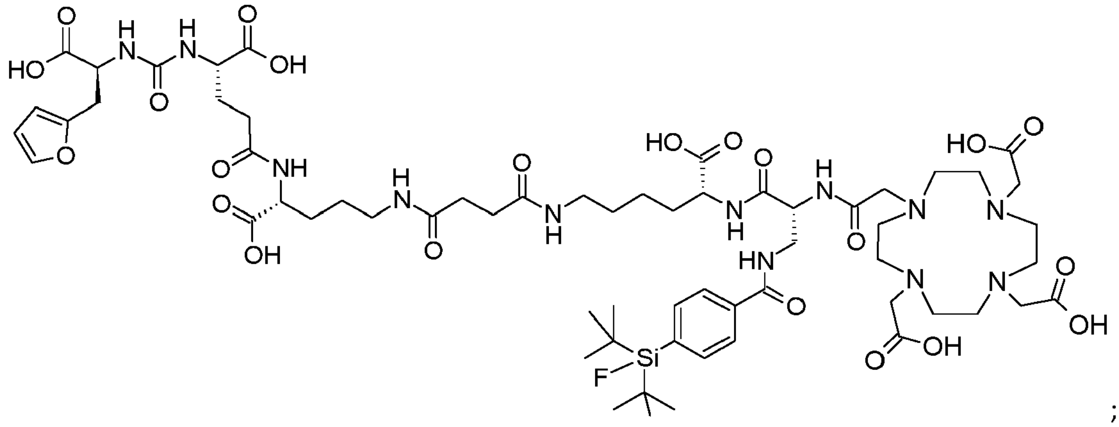

;

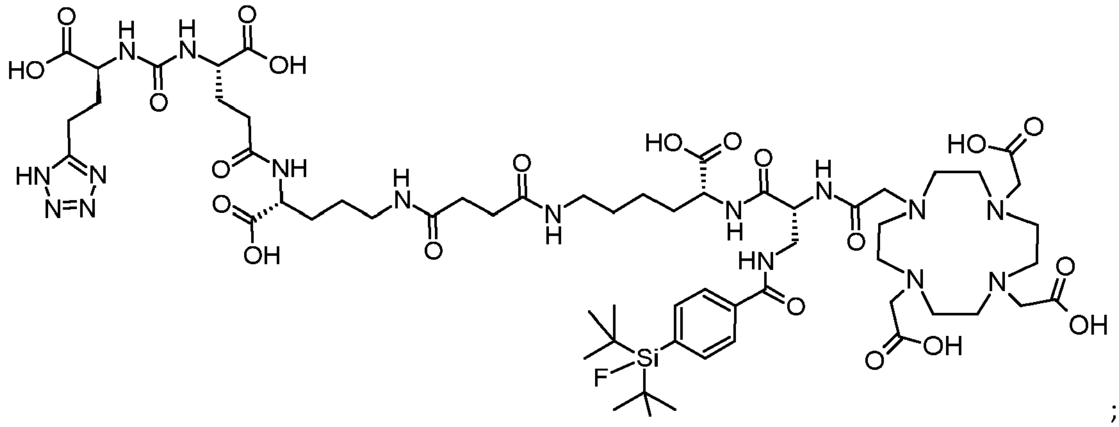

;

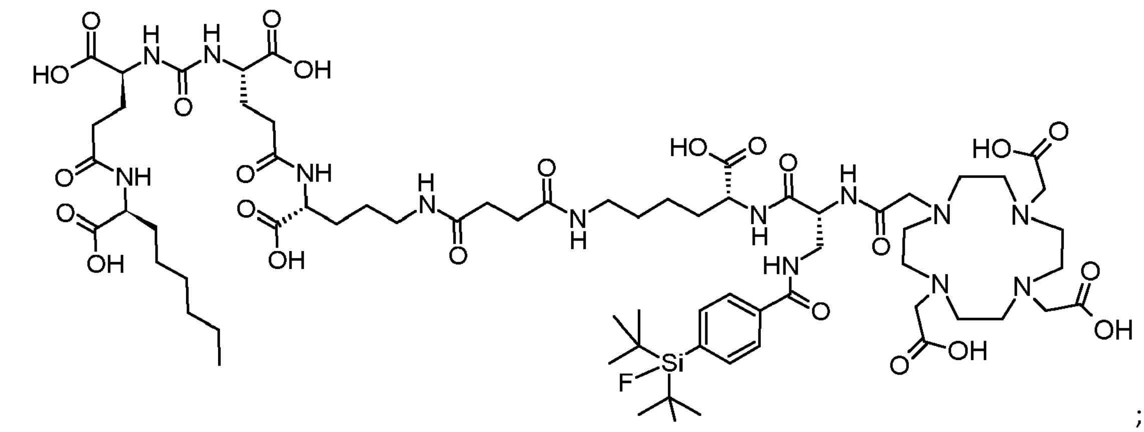

;

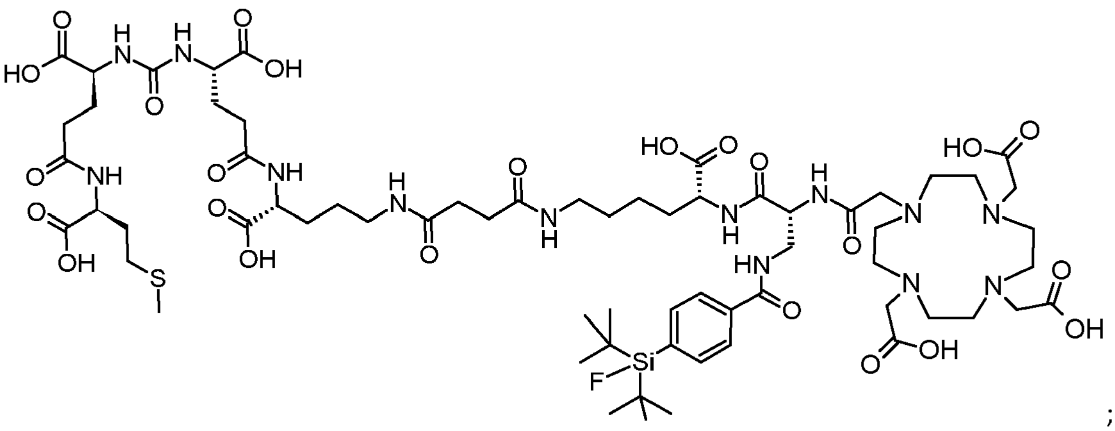

;

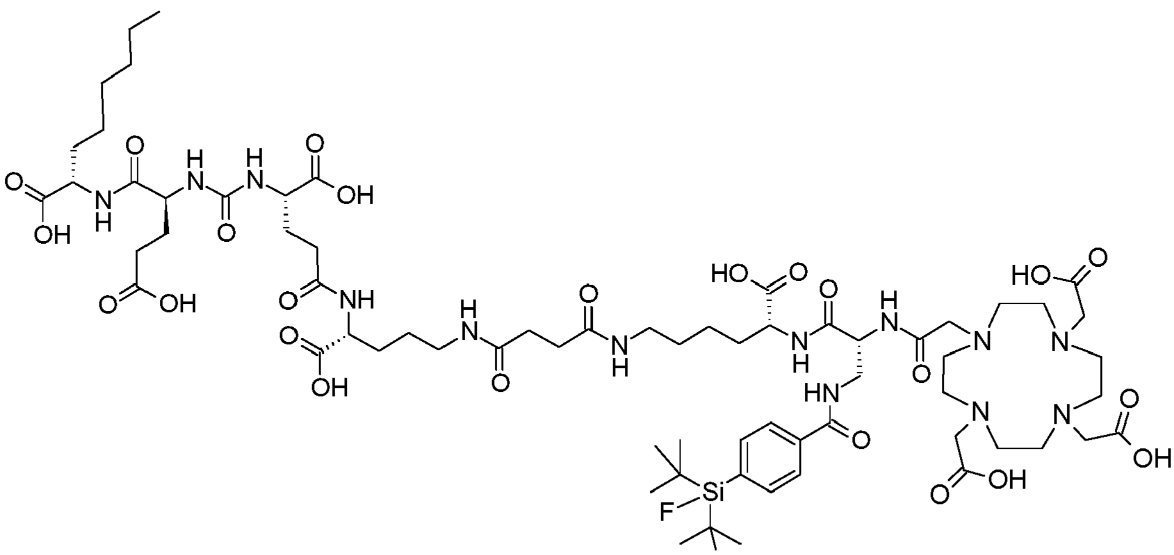

;

or a pharmaceutically acceptable salt thereof, wherein the fluorine atom is optionally $^{18}F$ and wherein the chelator moiety is optionally coordinated to $Lu^{3+}$.

**14.** A pharmaceutical or diagnostic composition comprising or consisting of one or more compounds according to any one of claims 1 to 13.

**15.** A compound or composition according to any one of claims 1 to 14 for use as a cancer diagnostic or imaging agent or for use in the treatment of cancer.

(A) Modifications within the central $Zn^{2+}$-binding unit

(B) Proinhibitor motifs

(C) PSMA binding motifs with substituents & bioisosteres of the P1'-γ-carboxylic acid

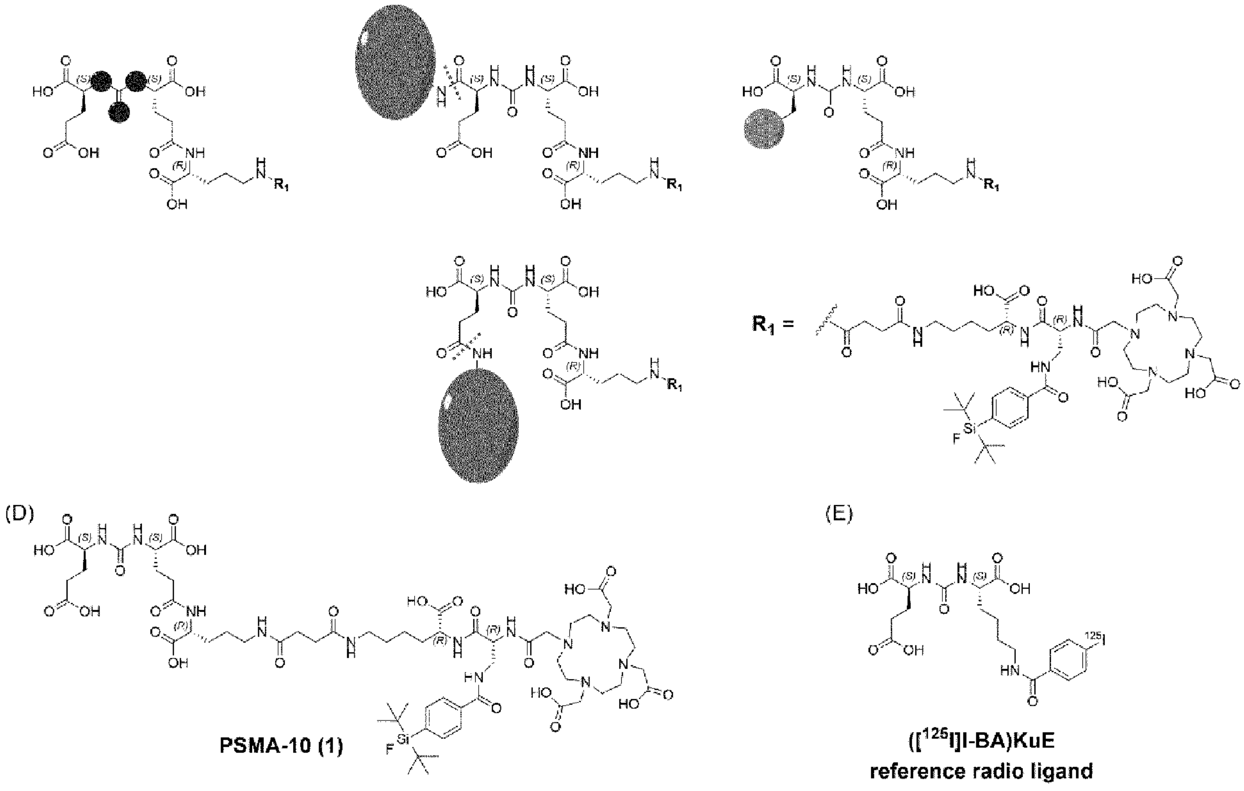

$R_1$ =

(D)

PSMA-10 (1)

(E)

**([125I]I-BA)KuE reference radio ligand**

**Fig. 1**

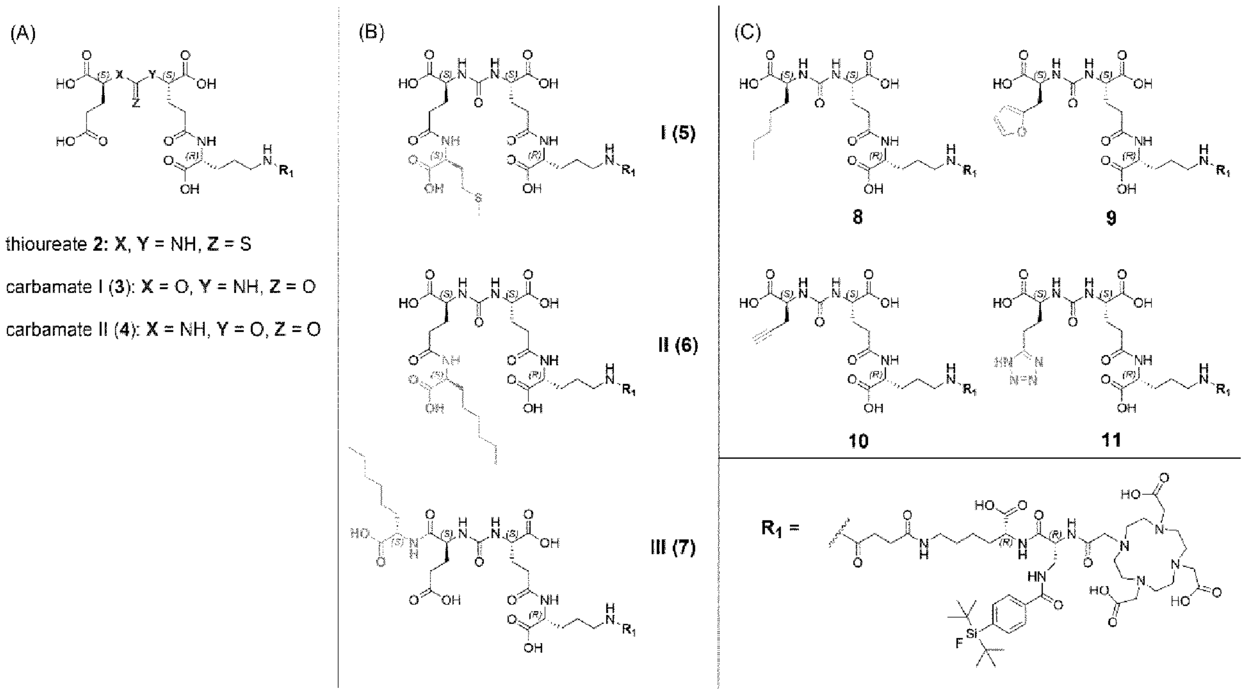

(A)

thioureate **2:** **X**, Y = NH, **Z** = S

carbamate I (**3**): **X** = O, Y = NH, **Z** = O

carbamate II (**4**): **X** = NH, Y = O, Z = O

(B)

**I (5)**

**II (6)**

**III (7)**

(C)

**8**

**9**

**10**

**11**

$R_1$ =

**Fig. 2**

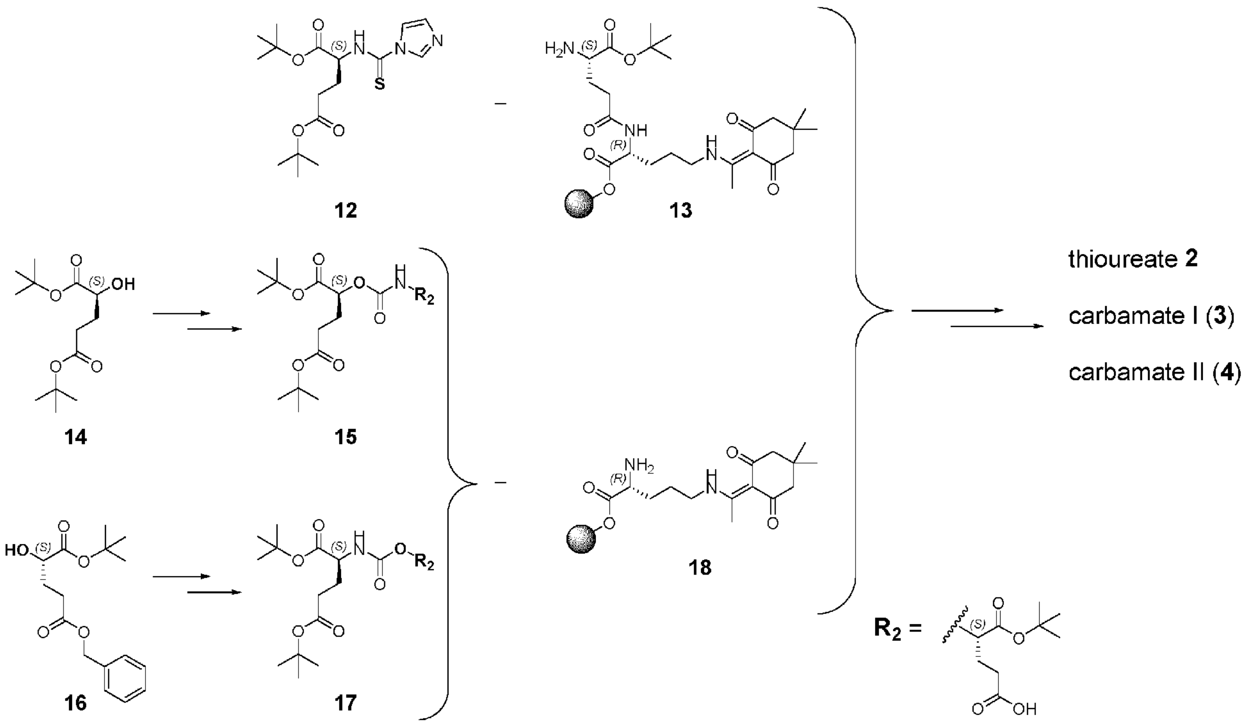

12

13

14

15

16

17

18

thioureate **2**

carbamate I (**3**)

carbamate II (**4**)

$R_2$ =

**Fig. 3**

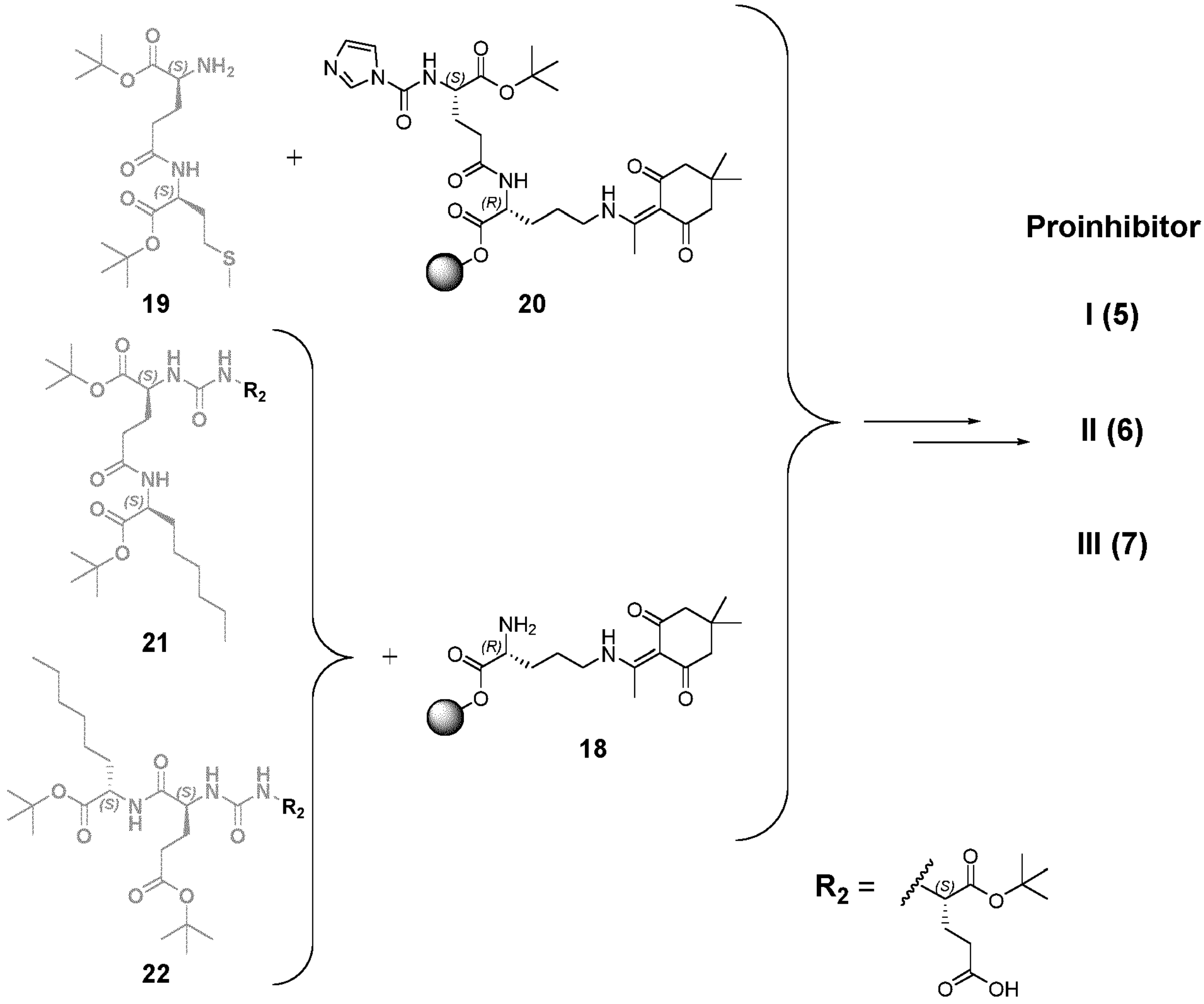

19

20

21

22

18

Proinhibitor

I (5)

II (6)

III (7)

$R_2$ =

Fig. 4

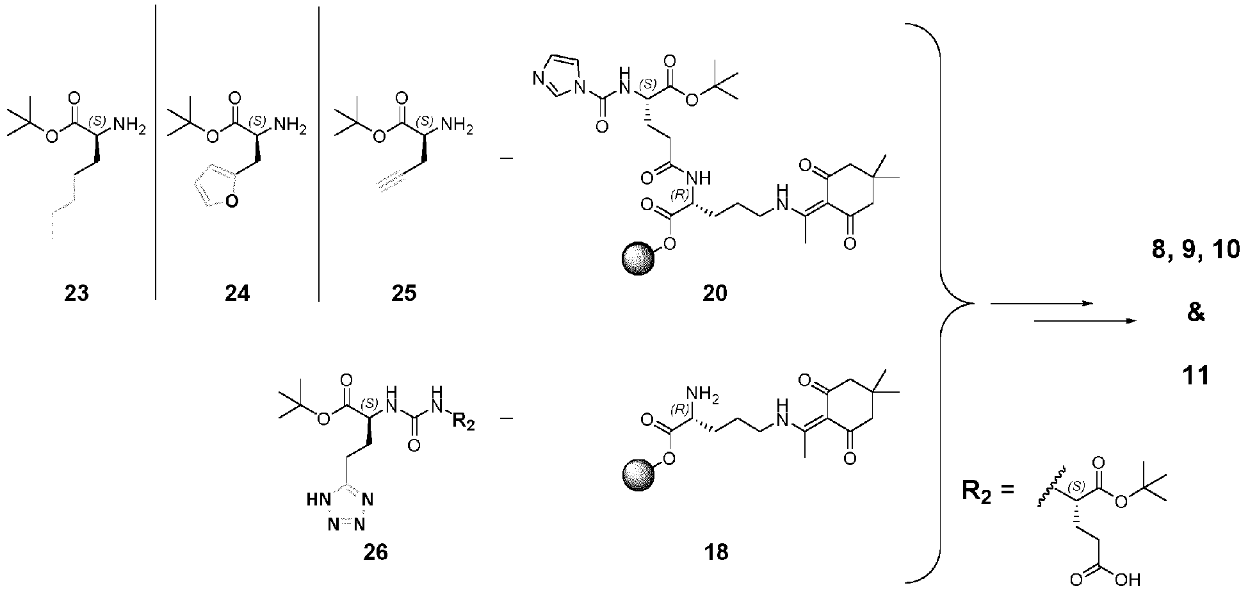

23 24 25 20

26 18

8, 9, 10 & 11

$R_2$ =

Fig. 5

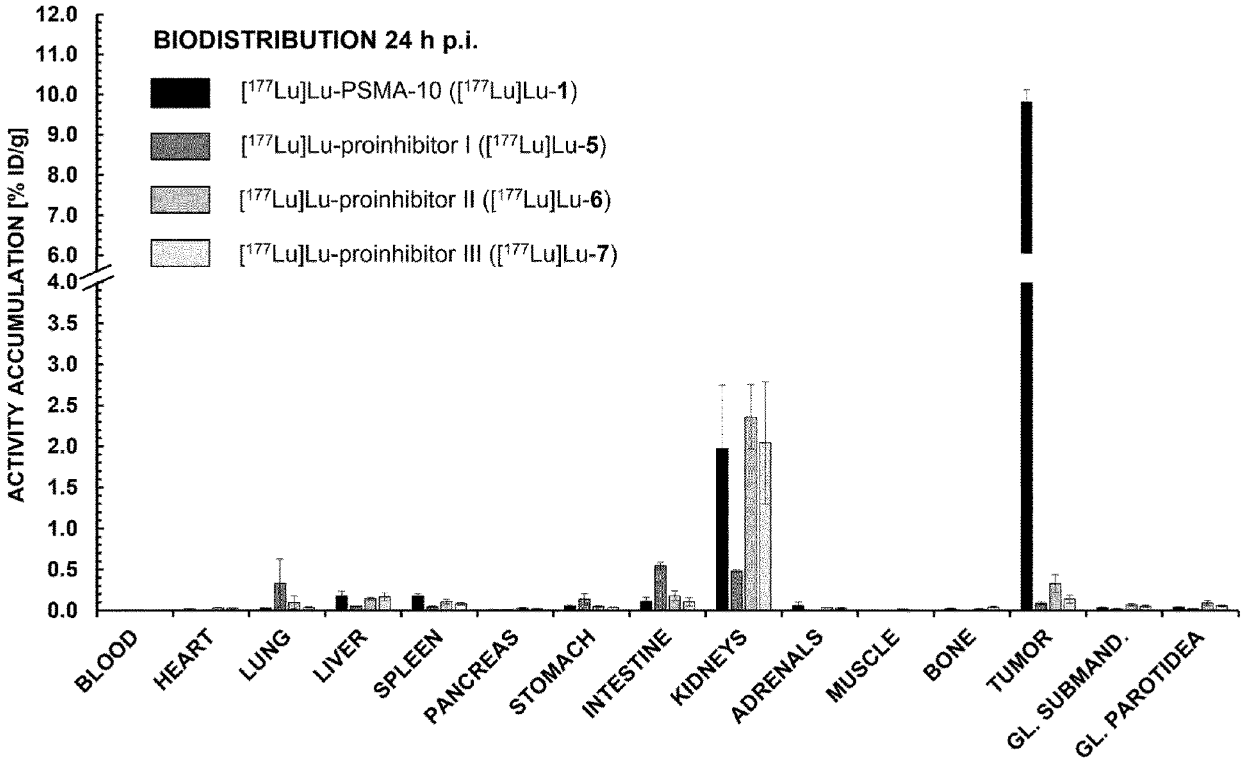
BIODISTRIBUTION 24 h p.i.
[177Lu]Lu-PSMA-10 ([177Lu]Lu-1)
[177Lu]Lu-proinhibitor I ([177Lu]Lu-5)
[177Lu]Lu-proinhibitor II ([177Lu]Lu-6)
[177Lu]Lu-proinhibitor III ([177Lu]Lu-7)
ACTIVITY ACCUMULATION [% ID/g]
12.0
11.0
10.0
9.0
8.0
7.0
6.0
4.0
3.5
3.0
2.5
2.0
1.5
1.0
0.5
0.0
BLOOD
HEART
LUNG
LIVER
SPLEEN
PANCREAS
STOMACH
INTESTINE
KIDNEYS
ADRENALS
MUSCLE
BONE
TUMOR
GL. SUBMAND.
GL. PAROTIDEA

Fig. 6

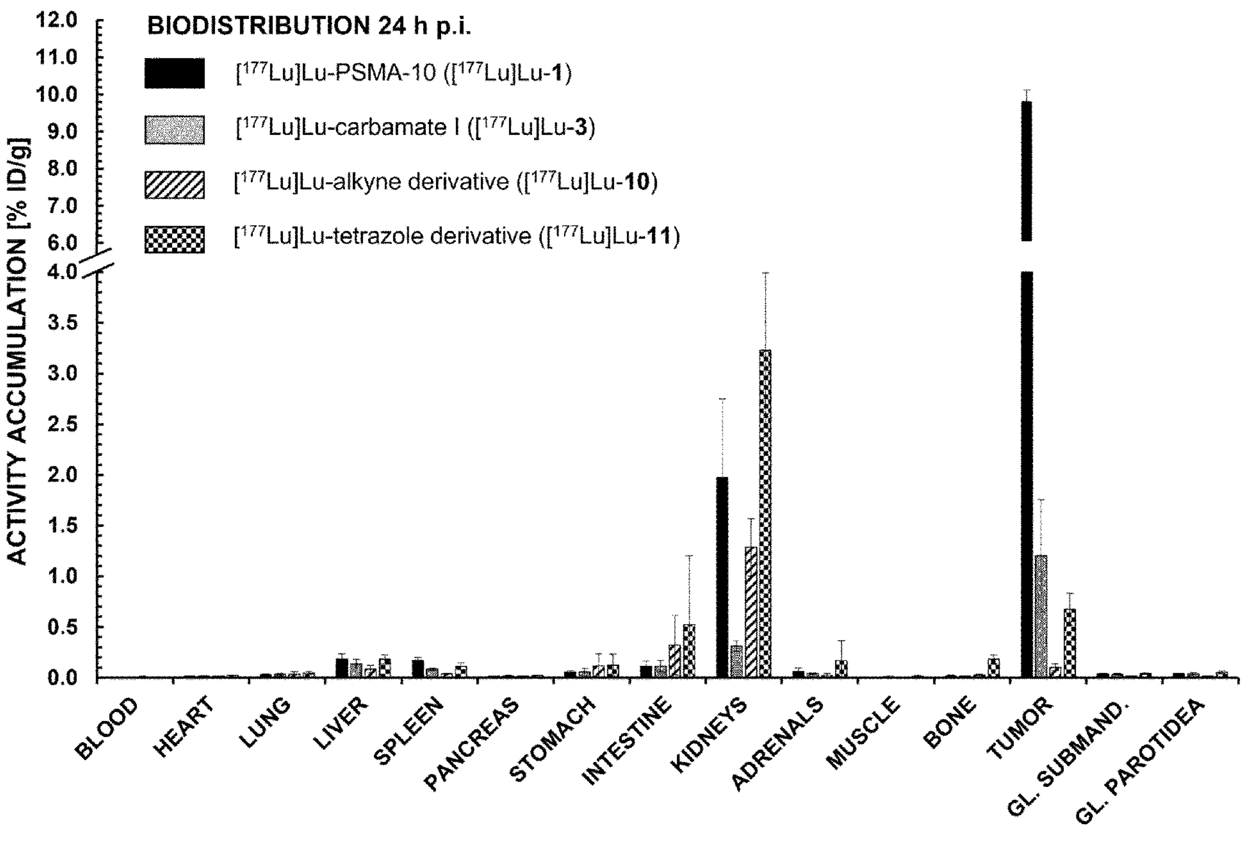
BIODISTRIBUTION 24 h p.i.
[177Lu]Lu-PSMA-10 ([177Lu]Lu-1)
[177Lu]Lu-carbamate I ([177Lu]Lu-3)
[177Lu]Lu-alkyne derivative ([177Lu]Lu-10)
[177Lu]Lu-tetrazole derivative ([177Lu]Lu-11)
ACTIVITY ACCUMULATION [% ID/g]
12.0
11.0
10.0
9.0
8.0
7.0
6.0
4.0
3.5
3.0
2.5
2.0
1.5
1.0
0.5
0.0
BLOOD
HEART
LUNG
LIVER
SPLEEN
PANCREAS
STOMACH
INTESTINE
KIDNEYS
ADRENALS
MUSCLE
BONE
TUMOR
GL. SUBMAND.
GL. PAROTIDEA

Fig. 7

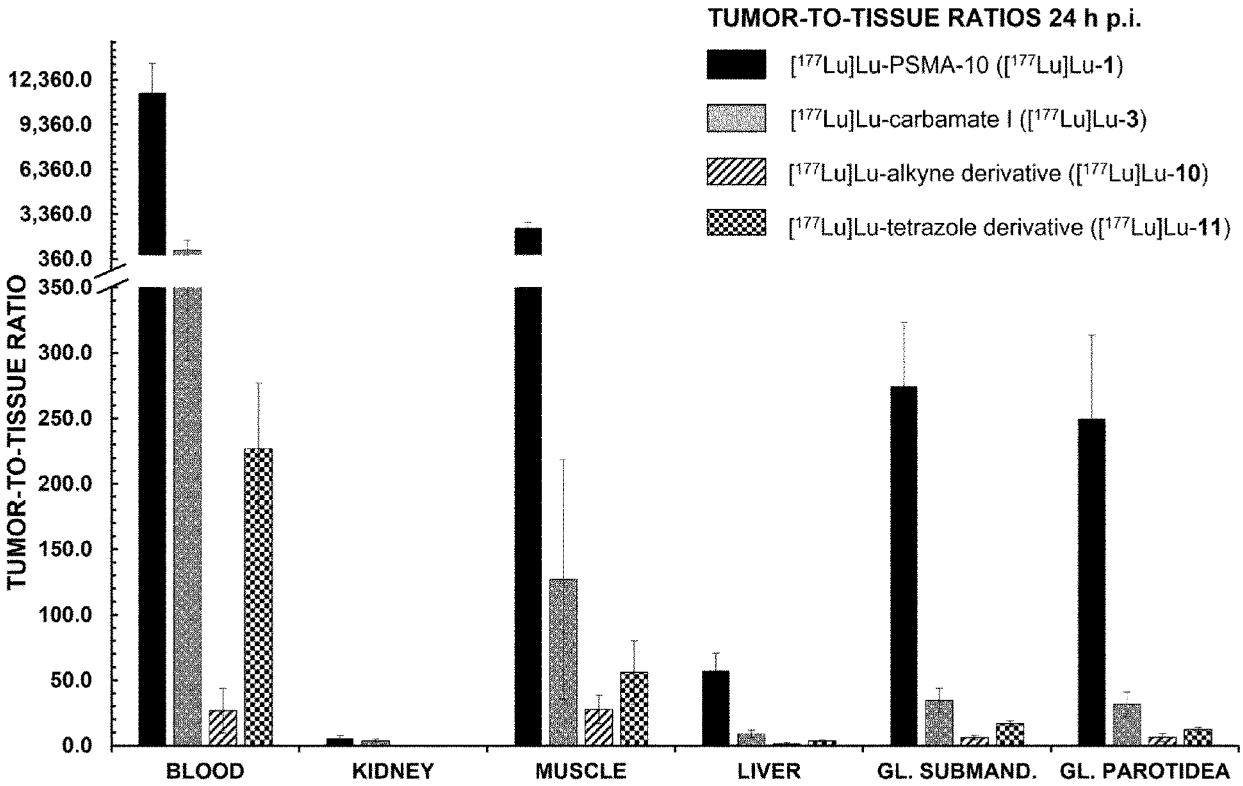
TUMOR-TO-TISSUE RATIOS 24 h p.i.
[177Lu]Lu-PSMA-10 ([177Lu]Lu-1)
[177Lu]Lu-carbamate I ([177Lu]Lu-3)
[177Lu]Lu-alkyne derivative ([177Lu]Lu-10)
[177Lu]Lu-tetrazole derivative ([177Lu]Lu-11)
TUMOR-TO-TISSUE RATIO
12,360.0
9,360.0
6,360.0
3,360.0
360.0
350.0
300.0
250.0
200.0
150.0
100.0
50.0
0.0
BLOOD
KIDNEY
MUSCLE
LIVER
GL. SUBMAND.
GL. PAROTIDEA

Fig. 8

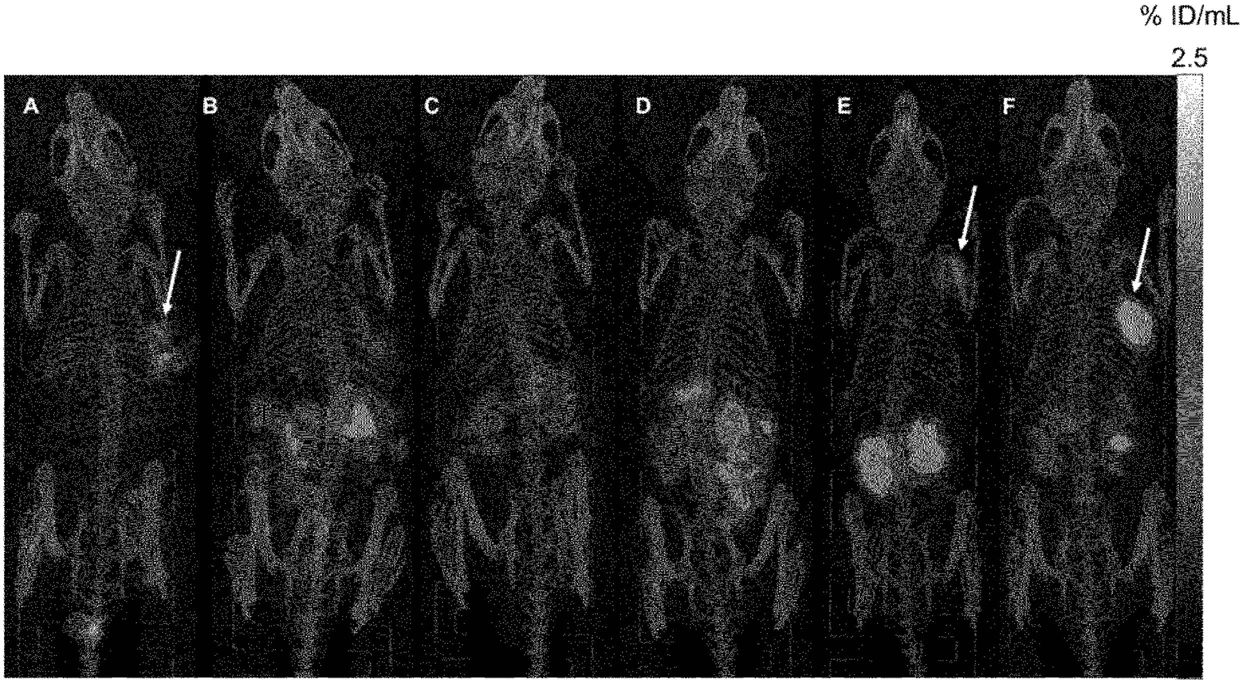


Fig. 9

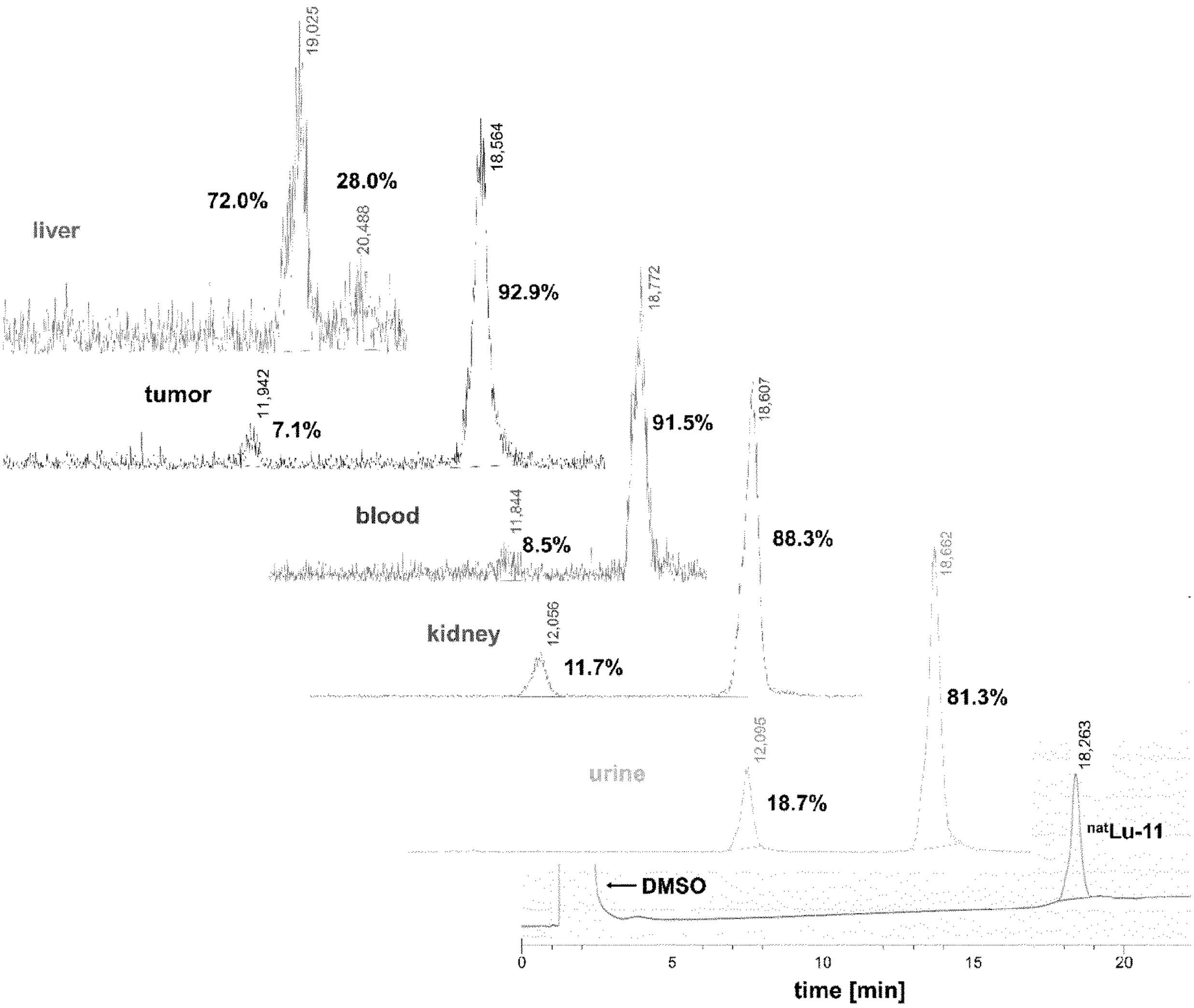
liver
72.0%
28.0%
19,025
20,488
18,564
92.9%
tumor
11,942
7.1%
18,772
91.5%
blood
11,844
8.5%
18,607
88.3%
kidney
12,056
11.7%
18,662
81.3%
urine
12,095
18.7%
18,263
natLu-11
DMSO
0
5
10
15
20
time [min]

Fig. 10

Europäisches Patentamt
European Patent Office
Office européen des brevets

# EUROPEAN SEARCH REPORT

Application Number
EP 21 15 0122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D<br><br>Y | WO 2019/020831 A1 (UNIV MUENCHEN TECH [DE] ET AL.) 31 January 2019 (2019-01-31)<br>* claims 7, 8, 13, 15, 16, 18 *<br>----- | 1-4, 6-12,14, 15<br>5,13 | INV.<br>A61K51/04<br>A61P35/00<br>A61K103/00<br>A61K103/30 |
| X,D<br><br><br>Y | WO 2020/157184 A1 (TECHNISCHE UNIVERSITÄT MÜNCHEN [DE] ET AL.)<br>6 August 2020 (2020-08-06)<br>* claims 6, 13, 16, 17, 19 *<br>----- | 1-4, 6-10,12, 14,15<br>5,13 | |
| Y | WO 2020/157177 A1 (TECHNISCHE UNIVERSITÄT MÜNCHEN [DE] ET AL.)<br>6 August 2020 (2020-08-06)<br>* claim 1 *<br>----- | 5,13 | |
| Y | ALEXANDER WURZER ET AL: "Radiohybrid ligands: a novel tracer concept exemplified by 18 F- or 68 Ga-labeled rhPSMA-inhibitors",<br>THE JOURNAL OF NUCLEAR MEDICINE,<br>20 December 2019 (2019-12-20), XP055686996,<br>US<br>ISSN: 0161-5505, DOI: 10.2967/jnumed.119.234922<br>* rhPSMA-7 in supplemental figure 5 *<br>----- | 5,13 | TECHNICAL FIELDS SEARCHED (IPC)<br>A61K |
| Y | ALEXANDER WURZER ET AL: "Preclinical comparison of four [F, Ga]rhPSMA-7 isomers: influence of the stereoconfiguration on pharmacokinetics",<br>EJNMMI RESEARCH, BIOMED CENTRAL LTD, LONDON, UK,<br>vol. 10, no. 1,<br>7 December 2020 (2020-12-07), pages 1-10, XP021285239,<br>DOI: 10.1186/S13550-020-00740-Z<br>* rhPSMA-7 in figure 1 *<br>-----<br>-/-- | 5,13 | |

The present search report has been drawn up for all claims

1

| Place of search | Date of completion of the search | Examiner |
|---|---|---|
| The Hague | 18 June 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 0122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KOZIKOWSKI A P ET AL: "Synthesis of Urea-Based Inhibitors as Active Site Probes of Glutamate Carboxypeptidase II: Efficacy as Analgesic Agents", JOURNAL OF MEDICINAL CHEMISTRY, AMERICAN CHEMICAL SOCIETY, US, vol. 47, 2 March 2004 (2004-03-02), pages 1729-1738, XP002390387, ISSN: 0022-2623, DOI: 10.1021/JM0306226 * structure 7d, Abstract * ----- | 5,13 | |
| Y | ANNA PLECHANOVOV? ET AL: "Novel Substrate-Based Inhibitors of Human Glutamate Carboxypeptidase II with Enhanced Lipophilicity", JOURNAL OF MEDICINAL CHEMISTRY, vol. 54, no. 21, 10 November 2011 (2011-11-10), pages 7535-7546, XP055239153, US ISSN: 0022-2623, DOI: 10.1021/jm200807m * structure 6i; page 7539 * ----- | 5,13 | TECHNICAL FIELDS SEARCHED (IPC) |
| Y | BARINKA C ET AL: "Substrate specificity, inhibition and enzymological analysis of recombinant human glutamate carboxypeptidase II", JOURNAL OF NEUROCHEMISTRY, WILEY-BLACKWELL PUBLISHING LTD, GB, vol. 80, no. 3, 1 February 2002 (2002-02-01), pages 477-487, XP002338132, ISSN: 0022-3042, DOI: 10.1046/J.0022-3042.2001.00715.X * figure 3 and table 1 (Ac-Glu-Met ) * ----- -/-- | 5,13 | |

The present search report has been drawn up for all claims

1

| Place of search | Date of completion of the search | Examiner |
|---|---|---|
| The Hague | 18 June 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 21 15 0122

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WANG H ET AL: "Bioisosterism of urea-based GCPII inhibitors: Synthesis and structureactivity relationship studies", BIORGANIC & MEDICINAL CHEMISTRY LETTERS, ELSEVIER, AMSTERDAM , NL, vol. 20, no. 1, 1 January 2010 (2010-01-01), pages 392-397, XP026808851, ISSN: 0960-894X [retrieved on 2009-10-24] * scheme 3, structures e and n ; table 1 * ----- | 5,13 | |
| Y | WO 2020/252598 A1 (PROVINCIAL HEALTH SERVICES AUTHORITY [CA]; UNIV BRITISH COLUMBIA [CA]) 24 December 2020 (2020-12-24) * figure 6; structure HTK04050 * ----- | 5,13 | |
| Y | EP 3 636 635 A1 (ACADEMISCH ZIEKENHUIS LEIDEN [NL]; UNIV MUENCHEN TECH [DE] ET AL.) 15 April 2020 (2020-04-15) * page 12; introduction * ----- | 5,13 | TECHNICAL FIELDS SEARCHED (IPC) |
| Y | PAVLICEK JIRI ET AL: "Structural characterization of P1'-diversified urea-based inhibitors of glutamate carboxypeptidas", BIORGANIC & MEDICINAL CHEMISTRY LETTERS, ELSEVIER, AMSTERDAM , NL, vol. 24, no. 10, 28 March 2014 (2014-03-28), pages 2340-2345, XP028646746, ISSN: 0960-894X, DOI: 10.1016/J.BMCL.2014.03.066 * figure 1 * ----- | 5,13 | |

The present search report has been drawn up for all claims

1

| Place of search | Date of completion of the search | Examiner |
|---|---|---|
| The Hague | 18 June 2021 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 21 15 0122

This annex lists the patent family members relating to the patent documents cited in the above-mentioned European search report. The members are as contained in the European Patent Office EDP file on
The European Patent Office is in no way liable for these particulars which are merely given for the purpose of information.

18-06-2021

| Patent document cited in search report | Publication date | Patent family member(s) | Publication date |
|---|---|---|---|
| WO 2019020831 A1 | 31-01-2019 | AU 2018308699 A1 | 23-01-2020 |
| | | BR 112020001785 A2 | 29-09-2020 |
| | | CA 3071315 A1 | 31-01-2019 |
| | | CN 111132700 A | 08-05-2020 |
| | | EA 202090370 A1 | 25-05-2020 |
| | | EP 3658194 A1 | 03-06-2020 |
| | | JP 2020528461 A | 24-09-2020 |
| | | KR 20200064057 A | 05-06-2020 |
| | | SG 11202000725W A | 27-02-2020 |
| | | US 2020197545 A1 | 25-06-2020 |
| | | WO 2019020831 A1 | 31-01-2019 |
| WO 2020157184 A1 | 06-08-2020 | NONE | |
| WO 2020157177 A1 | 06-08-2020 | NONE | |
| WO 2020252598 A1 | 24-12-2020 | NONE | |
| EP 3636635 A1 | 15-04-2020 | AU 2019356168 A1 | 20-05-2021 |
| | | CA 3115725 A1 | 16-04-2020 |
| | | EP 3636635 A1 | 15-04-2020 |
| | | WO 2020074705 A1 | 16-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019020831 A
- WO 2020157184 A


### Non-patent literature cited in the description


- **SILVER et al.** *Clinical Cancer Research,* 1997, vol. 3, 81-85
- **AFSHAR-OROMIEH et al.** *European journal of nuclear medicine and molecular imaging,* 2015, vol. 42, 197-209
- **BENEŠOVÁ et al.** *Journal of Nuclear Medicine,* 2015, vol. 56, 914-920
- **ROBU et al.** *Journal of Nuclear Medicine, jnumed.,* 2016, vol. 116, 178939
- **WEINEISEN et al.** *Journal of Nuclear Medicine,* 2014, vol. 55, 1083-1083
- **ROWE et al.** *Prostate cancer and prostatic diseases,* 2016
- **MAURER et al.** *Nature Reviews Urology,* 2016
- **ZHOU et al.** *Nature Reviews Drug Discovery,* 2005, vol. 4, 1015-1026
- **MACHULKIN et al.** *Journal of drug targeting,* 2016, 1-15
- **BARINKA et al.** *Journal of medicinal chemistry,* 2008, vol. 51, 7737-7743
- **ZHANG et al.** *Journal of the American Chemical Society,* 2010, vol. 132, 12711-12716
- **LIU et al.** *Bioorganic & medicinal chemistry letters,* 2011, vol. 21, 7013-7016
- **KIESS et al.** *The quarterly journal of nuclear medicine and molecular imaging,* 2015, vol. 59, 241
- **EDER et al.** *Bioconjugate chemistry,* 2012, vol. 23, 688-697
- **ROWE et al.** *Molecular Imaging and Biology,* 2016, 1-9
- **DIETLEIN et al.** *Molecular Imaging and Biology,* 2015, vol. 17, 575-584
- **CARDINALE et al.** Journal of nuclear medicine: official publication. Society of Nuclear Medicine, 2017, vol. 58, 425-431
- **GIESEL et al.** *European journal of nuclear medicine and molecular imaging,* 2016, vol. 43, 1929-1930
- **GIESEL et al.** *European journal of nuclear medicine and molecular imaging,* 2017, vol. 44, 678-688
- **LINDNER et al.** *Bioconjugate Chemistry,* 2014, vol. 25, 738-749
- **SCHIRRMACHER E. et al.** *Bioconjugate Chem.,* 2007, vol. 18, 2085-2089
- **WÄNGLER C. et al.** *Bioconjugate Chem.,* 2009, vol. 20 (2), 317-321
- **WÄNGLER C. et al.** *Bioconjug Chem.,* 15 December 2010, vol. 21 (12), 2289-96
- **BERNARD-GAUTHIER et al.** *Biomed Res Int. 2014,* 2014, 454503
- **LINDNER S. et al.** *Bioconjug Chem.,* 16 April 2014, vol. 25 (4), 738-49
- **NIEDERMOSER S. et al.** *J Nucl Med.,* July 2015, vol. 56 (7), 1100-5
- **SANGSTER.** Octanol-water Partition Coefficients: fundamentals and physical chemistry. John Wiley & Sons, 1997
- **BRYN et al.** Solid-State Chemistry of Drugs. SSCI, Inc of West Lafayette, 1999
- **WURZER A. et al.** *Journal of Nuclear Medicine,* 2020, vol. 61 (5), 735-42
- **ZHANG et al.** *Tetrahedron,* 2009, vol. 65 (48), 9997-10001
- **BERGMEIER et al.** *J Org Chem.,* 1993, vol. 58 (9), 2369-76
- **YANG et al.** *Journal of Medicinal Chemistry,* 2016, vol. 59 (1), 206-18
- **WEINEISEN et al.** *EJNMMI Research,* 2014, vol. 4 (1), 63
- **SHIN et al.** *Journal of Organic Chemistry,* 2000, vol. 65 (22), 7667-75
- **WEINEISEN et al.** *EJNMMI Research.,* 2014, vol. 4 (1), 63
- **HYUN et al.** *J Am Chem Soc.,* 2010, vol. 132 (48), 17053-5
- **KOZIKOWSKI et al.** *Journal of Medicinal Chemistry,* 2004, vol. 47 (7), 1729-38